(19) Europäisches Patentamt
European Patent Office
Office européen des brevets

(11) Publication number: **0 332 387 B1**

(12) # EUROPEAN PATENT SPECIFICATION

(45) Date of publication of patent specification: **24.11.93**

(51) Int. Cl.$^5$: **C07D 249/08**, C07D 413/06, C07D 401/06, C07D 403/06, C07D 405/06, A01N 43/653, A01N 43/80, A61K 31/41

(21) Application number: **89302244.2**

(22) Date of filing: **06.03.89**

The file contains technical information submitted after the application was filed and not included in this specification

(54) **Triazole derivatives, their preparation and their use as fungicides.**

(30) Priority: **04.03.88 JP 51312/88**
**23.03.88 JP 68681/88**
**04.10.88 JP 250158/88**
**17.10.88 JP 261211/88**

(43) Date of publication of application:
**13.09.89 Bulletin 89/37**

(45) Publication of the grant of the patent:
**24.11.93 Bulletin 93/47**

(84) Designated Contracting States:
**AT BE CH DE ES FR GB GR IT LI LU NL SE**

(56) References cited:
**EP-A- 0 015 756**
**EP-A- 0 122 693**
**DE-A- 3 406 993**
**US-A- 4 483 863**
**EP-A- 0 097 469**
**DE-A- 3 307 218**
**GB-A- 2 159 148**

(73) Proprietor: **Sankyo Company Limited**
**5-1 Nihonbashi Honcho 3-chome**
**Chuo-ku**
**Tokyo(JP)**

(72) Inventor: **Oida, Sadao**
**c/o**
**Sankyo**
**Co.Ltd.,**
**No.**
**2-58,**
**1-chome,**
**Hiromachi,**
**Shinagawa-ku, Tokyo 140(JP)**
Inventor: **Tajima, Yawara**
**c/o**
**Sankyo**
**Co.Ltd.,**
**No.**
**2-58,**
**1-chome,**
**Hiromachi,**
**Shinagawa-ku, Tokyo 140(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 332 387 B1

Inventor: **Konosu, Toshiyuki c/o Sankyo Co.Ltd., No. 2-58, 1-chome, Hiromachi, Shinagawa-ku, Tokyo 140(JP)**
Inventor: **Iwata, Masayuki c/o Sankyo Co.Ltd., No. 2-58, 1-chome, Hiromachi, Shinagawa-ku, Tokyo 140(JP)**
Inventor: **Takeda, Noriko c/o Sankyo Co.Ltd., No. 2-58, 1-chome, Hiromachi, Shinagawa-ku, Tokyo 140(JP)**
Inventor: **Miyaoka, Takeo c/o Sankyo Co.Ltd., No. 2-58, 1-chome, Hiromachi, Shinagawa-ku, Tokyo 140(JP)**
Inventor: **Takeshiba, Hideo c/o Sankyo Co.Ltd., 1041 Yasu, Yasu-cho Yasu-Gun, Shiga-ken(JP)**
Inventor: **Nakanishi, Toshiro c/o Sankyo Co.Ltd., 1041 Yasu, Yasu-cho Yasu-Gun, Shiga-ken(JP)**

(74) Representative: **Gibson, Christian John Robert et al
MARKS & CLERK
57/60 Lincoln's Inn Fields
London WC2A 3LS (GB)**

## Description

The present invention relates to a series of new triazole derivatives having fungicidal activity, and provides for the agricultural use of the compounds of the present invention as well as for their pharmaceutical use, especially of certain of them. The invention also provides processes for preparing the compounds of the present invention.

The compounds of the present invention are EBI fungicides, i.e. fungicides which act by inhibiting the biosynthesis of ergosterol. Fungicides having this type of activity are known and some are known to be of use for the treatment of various fungal infections of humans and other animals, e.g. by topical application, i.e. for the treatment of dermatophytosis, or by internal application, for the treatment of systemic fungal infections (including opportunistic infections) in various internal organs, such as against fungi of the genus Candida. Such compounds are also, in general, of value as fungicides for plants, to treat or protect the plants from a variety of fungal infections and thus they may be used in agriculture and horticulture.

Certain 1-(1,2,4-triazol-1-yl)-2-aryl-3-(substituted amino)-2-propanol derivatives having this type of activity are disclosed in U.S. Patent Specification No. 4 482 558. These prior art compounds resemble certain of the compounds of the present invention but differ in that the relevant compounds of the present invention are at least butanol derivatives and may have an even longer carbon chain, whereas these prior art compounds are merely propanol derivatives.

Also, certain compounds of this type are disclosed in the Abstract Papers of the 8th Symposium on Medicinal Chemistry, Osaka (1986), page 9, and others were referred to at the Symposium itself, but no use is suggested for those compounds, and, in particular, there was no disclosure that the compounds might be of use as fungicides, whether in the agricultural field or otherwise.

GB 2 159 148 also describes 1,2,4, triazole derivatives having fungicidal activity. Such compounds, although having a structure similar to that of certain of the present compounds, differ in one or more significant respects, for example in the substituent of the 3-carbon of the substituted alkyl side chain.

The compounds to which the present invention relates may be represented by the formula (I):

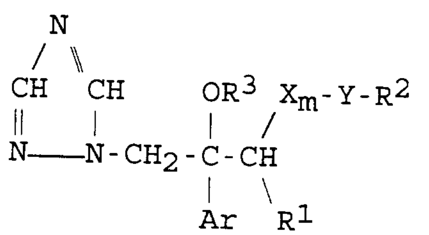

(I)

in which:

Ar represents a phenyl group or a phenyl group having one or two halogen and/or trifluoromethyl substituents;

$R^1$ represents a $C_1$ - $C_6$ alkyl group or, where $R^3$ and $-X_m-Y-R^2$ together represent the group of formula (II) defined below, $R^1$ represents a hydrogen atom or a $C_1$ - $C_6$ alkyl group;

X represents a $C_1$ - $C_6$ alkylene group, a divalent $C_2$ - $C_6$ aliphatic hydrocarbon group having one or two carbon-carbon double bonds, a divalent $C_2$ - $C_6$ aliphatic hydrocarbon group having one or two carbon-carbon triple bonds, a $C_3$ - $C_6$ cycloalkylene group, a $C_3$ - $C_6$ cycloalkylene group having one or two carbon-carbon double bonds, a ($C_1$ - $C_3$ alkylene)-($C_3$ - $C_6$ cycloalkylene) group or a ($C_3$ - $C_6$ cycloalkylene)- ($C_1$ - $C_3$ alkylene) group;

m is 0 or 1;

$-Y-R^2$ represents the azido group ($-N_3$), the phthalimido group, the 1-oxo-2,3-dihydro-2-isoindolyl group, the phthalimido group or the 1-oxo-2,3-dihydro-2-isoindolyl group having at least one of substituents (a), defined below, a protected hydroxy group or a group of formula $-OSO_2R^4$, in which:

$R^4$ represents a $C_1$ - $C_4$ alkyl group, a $C_1$ - $C_4$ haloalkyl group, a phenyl group or a phenyl group having at least one substituent selected from nitro groups, halogen atoms and $C_1$ - $C_4$ alkyl groups;

or

Y represents a group of formula $-N(R^5)CO-$, $-N(R^5)CO-CH=CH-$, -O-CO-, -O-CO-CH=CH-, -S-CO- or -S-CO-CH=CH-, in which:

$R^5$ represents a hydrogen atom or a $C_1$ - $C_4$ alkyl group;

$R^2$ represents a $C_1$ - $C_6$ alkyl group, a $C_1$ - $C_6$ haloalkyl group, a phenyl group, a phenyl group having at

least one of substituents (a), defined below, a naphthyl group or a heterocyclic group having 5 or 6 ring atoms of which from 1 to 3 are nitrogen and/or oxygen and/or sulphur hetero-atoms, said heterocyclic group being unsubstituted or having from 1 to 3 of substituents (b), defined below; and

$R^3$ represents a hydrogen atom;

or

$R^3$ and $-X_m$-Y-$R^2$ together represent a group of formula (II):

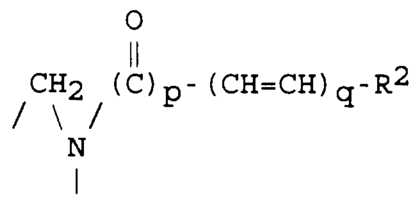

(II)

in which:

$R^2$ is as defined above;

p is 0 or 1; and

q is 0 or 1;

substituents (a):

$C_1$ - $C_4$ alkyl groups, $C_1$ - $C_4$ haloalkyl groups, $C_1$ - $C_4$ alkoxy groups, $C_1$ - $C_4$ haloalkoxy groups, halogen atoms, nitro groups, cyano groups, hydroxy groups, benzyloxy groups, hydroxymethyl groups, groups of formula $-CH_2$-OCO-$R^7$, groups of formula -CO-$R^6$, groups of formula -COO$R^7$, groups of formula $-SO_rR^7$, amino groups, mono- and di- alkylamino groups in which each alkyl group is $C_1$ - $C_4$ and carboxylic acylamino groups;

substituents (b):

$C_1$ - $C_4$ alkyl groups, $C_1$ - $C_4$ haloalkyl groups, halogen atoms, cyano groups and nitro groups;

$R^6$ represents a $C_1$ - $C_6$ alkyl group or a $C_1$ - $C_6$ haloalkyl group;

$R^7$ represents a $C_1$ - $C_6$ alkyl group;

r is 0, 1 or 2; and

acid addition salts thereof; provided that when m is 0 and y is a group of formula -N($R^5$)CO-, $R^5$ must be hydrogen.

Of the above compounds, those in which $R^1$ represents the methyl group, m is 0 and -Y-$R^2$ represents either a protected hydroxy group or a group of formula $-OSO_2R^4$ are known from the Abstract Papers of the 8th Symposium on Medicinal Chemistry, Osaka (1986), page 9, or from the Symposium itself, and these compounds per se are not claimed as part of the present invention.

The invention also provides a process for protecting plants, seeds, and portions of plants or seeds from fungal infection, which comprises applying to said plants, seeds, portions of plants or seeds or a locus including the same a fungicidally effective amount of at least one compound of formula (I) or an acid addition salt thereof.

The invention still further provides an agricultural fungicide comprising a fungicidally effective amount of at least one compound of formula (I) or an acid addition salt thereof in admixture with an agriculturally acceptable carrier or diluent.

The invention still further provides the use for the manufacture of a medicament for the treatment or prophylaxis of fungal infections in an animal, especially a mammal, which may be a human being, of at least one compound of formula (I) or an acid addition salt thereof.

The invention still further provides a pharmaceutical fungicide comprising a fungicidally effective amount of at least one compound of formula (I) or an acid addition salt thereof in admixture with an pharmaceutically acceptable carrier or diluent.

The invention also provides processes for preparing the compounds of the present invention, which in general terms comprise:

(a) reacting a compound of formula (IIIA):

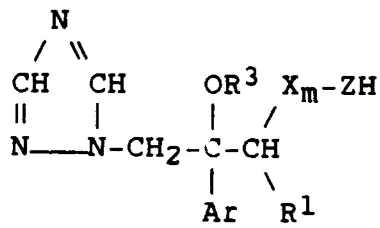

(IIIA)

[in which:

$R^1$, $R^3$, X, m and Ar are as defined above; and

Z represents an oxygen or sulphur atom or a group of formula -NH- or -N($CHR^8R^9$)-, in which $R^8$ and $R^9$ are the same or different and each represents a hydrogen atom or a methyl or ethyl group]

with a compound of formula (V):

$R^{2'}$-COOH (V)

(in which $R^{2'}$ represents any of the groups defined above for $R^2$ or represents a group of formula $R^2$-CH=CH-, and $R^2$ is as defined above) or with a reactive derivative thereof, to give a compound of formula (I'):

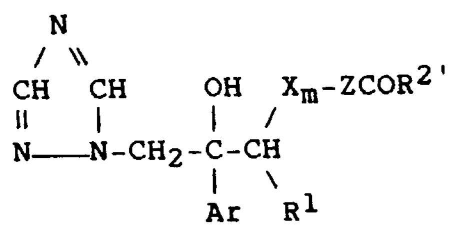

(I')

(in which $R^1$, $R^{2'}$, Ar, X, Z and m are as defined above);

or

(b) reacting a compound of formula (X'):

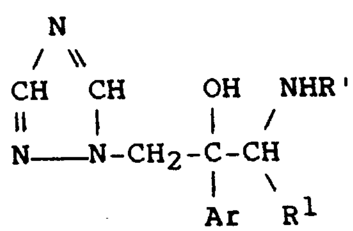

(X')

(in which: $R^1$ and Ar are as defined above, and R' represents a hydrogen atom or $R^2$)

with formaldehyde, to give a compound of formula (X''):

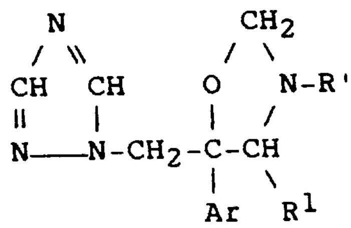

(X")

(in which $R^1$, Ar and R' are as defined above)
and, where R' represents a hydrogen atom, reacting said compound of formula (X") with a compound of formula (XVII):

$R^2$-(CH = CH)$_n$-COOH (XVII)

(in which $R^2$ is as defined above, and n is 0 or 1) or with a reactive derivative thereof, to give a compound of formula (Ig):

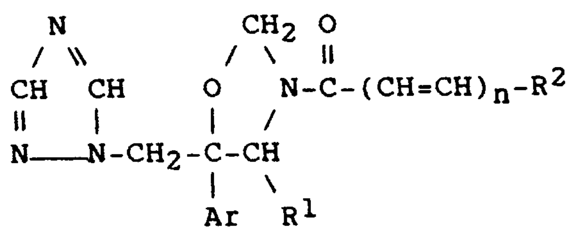

(Ig)

(in which $R^1$, $R^2$, Ar and n are as defined above);
or
(c) reacting said compound of formula (IIIA) in which Z represents an oxygen atom with a compound of formula (V'):

$R^4$-$SO_2$-OH (V')

(in which $R^4$ is as defined above) or with a reactive derivative thereof, to give a compound of formula (IIIB):

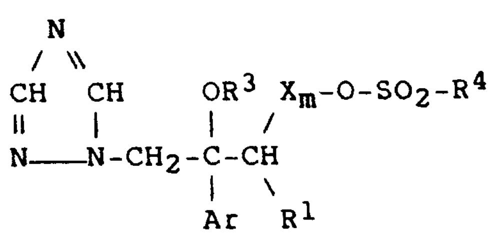

(IIIB)

(in which $R^1$, $R^3$, $R^4$, X, m and Ar are as defined above);
or
(d) reacting said compound of formula (IIIA) in which Z represents a group of formula -NH- with phthalic acid, a substituted phthalic acid in which the substituent is at least one of substituents (a), or a reactive derivative of said phthalic acid or substituted phthalic acid, to give a compound of formula (I) in which -Y-$R^2$ represents the phthalimido group or a substituted phthalimido group;
or

(e) reacting a compound of formula (IIIC):

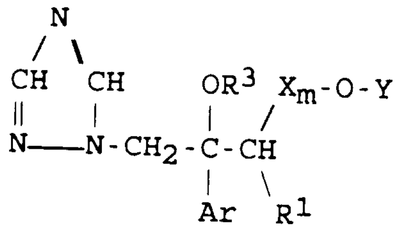

(IIIC)

(in which $R^1$, $R^3$, X, m and Ar are as defined above, and Y represents a hydrogen atom or a hydroxy-protecting group), or a corresponding oxirane compound in which m is 0, Y represents a hydrogen atom and the two hydroxy groups are condensed to form an oxirane group, with an alkali metal azide to give a compound of formula (IIID):

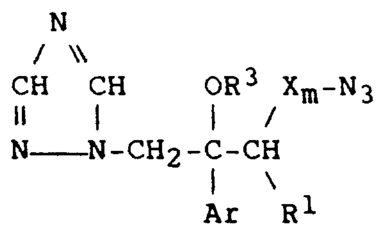

(IIID)

(in which $R^1$, $R^3$, X, m and Ar are as defined above),
and these are described in more detail hereinafter.

In the compounds of the present invention, Ar represents a phenyl group or a phenyl group having one or two halogen and/or trifluoromethyl substituents. The halogen atoms may be fluorine, chlorine, bromine or iodine atoms, of which the fluorine and chlorine atoms are preferred. There may be one or two substituents on the phenyl group, and, where there are two such substituents, these may be the same or different. Where the group is substituted, the substituents may be on any available position of the phenyl group, but are preferably on one or both of the 2- and 4- positions. Examples of preferred optionally substituted phenyl groups include the phenyl group itself, and the 4-(trifluoromethyl)phenyl, 4-fluorophenyl, 2-chloro-4-fluorophenyl, 4-chloro-2-fluorophenyl, 4-bromophenyl, 2-fluoro-4-iodophenyl, 2,4-dichlorophenyl, 2,4-difluorophenyl, 4-chlorophenyl and 2-fluoro-4-(trifluoromethyl)phenyl groups, of which the 4-fluorophenyl, 2-chloro-4-fluorophenyl, 4-chloro-2-fluorophenyl, 2,4-dichlorophenyl, 2,4-difluorophenyl and 4-chlorophenyl groups are more preferred and the 2,4-dichlorophenyl, 2,4-difluorophenyl and 4-chlorophenyl groups are especially preferred.

Where $R^1$ represents an alkyl group, this may be a straight or branched chain alkyl group containing from 1 to 6 carbon atoms, and examples include the methyl, ethyl, propyl isopropyl, butyl, isobutyl, sec-butyl, t-butyl, pentyl, isopentyl, 2-methylbutyl, hexyl, isohexyl and 2-methylpentyl groups, of which the methyl, ethyl, propyl and isopropyl groups are preferred, and the methyl and ethyl groups are most preferred.

Where X represents an alkylene group, this may be a straight or branched chain group, and, in the case of the branched chain groups, the two "free" valences may be attached to the same carbon atom or to different carbon atoms. Where these "free" valences are attached to the same carbon atom, the groups are sometimes referred to as "alkylidene" groups. Examples of such groups include the methylene, ethylene, ethylidene, trimethylene, propylidene, isopropylidene, 2-methylethylene, propylene (i.e. 1-methylethylene), tetramethylene, 2,2-dimethylethylene, 2-ethylethylene, 3-methyltrimethylene, pentamethylene, 2-propylethylene, 1-isopropylethylene and hexamethylene groups. Of these, the methylene and ethylidene groups are preferred.

Where X represents a divalent $C_2$ - $C_6$ aliphatic hydrocarbon group having one or two carbon-carbon double bonds, such a group may be a straight or branched chain group, and, in the case of the branched chain groups, the two "free" valences may be attached to the same carbon atom or to different carbon

atoms. Examples of such groups include the vinylene, 1-propenylene, 2-propenylene, 1-methylvinylene, 2-methylvinylene, 1-butenylene, 2-butenylene 3-butenylene, 1,3-butadienediyl, 1-pentenylene, 2-pentenylene, 3-pentenylene, 4-pentenylene, 1-hexenylene, 2-hexenylene, 3-hexenylene, 4-hexenylene and 5-hexenylene groups, of which the 1-propenylene, 2-methylvinylene and 2-butenylene groups are most preferred.

Where X represents a divalent $C_2$ - $C_6$ aliphatic hydrocarbon group having one or two carbon-carbon triple bonds, such a group may be a straight or branched chain group, and, in the case of the branched chain groups, the two "free" valences may be attached to the same carbon atom or to different carbon atoms. Examples of such groups include the ethynylidene, 1-propynylidene, 2-propynylidene, 1-butynylidene, 2-butynylidene, 3-butynylidene, 1-pentynylidene, 2-pentynylidene, 3-pentynylidene, 4-pentynylidene, 1-hexynylidene, 2-hexynylidene, 3-hexynylidene, 4-hexynylidene and 5-hexynylidene groups, of which the 1-propynylidene group is preferred.

Where X represents a divalent $C_3$ - $C_6$ cycloalkylene group, the two "free" valences may be attached to the same carbon atom or to different carbon atoms, but are preferably attached to the same carbon atom. Examples include the cyclopropylene, 1,2-cyclobutylene, 1,3-cyclobutylene, 1,2-cyclopentylene, 1,3-cyclopentylene, 1,2-cyclohexylene, 1,3-cyclohexylene and 1,4-cyclohexylene groups.

Where X represents a divalent $C_3$ - $C_6$ cycloalkylene group having one or two carbon-carbon double bonds, this may be any one of the cycloalkylene groups exemplified above, in which one or two of the carbon-carbon single bonds is replaced by a double bond. Examples include the cyclopropenylene, 1,2-cyclobutenylene, 1,3-cyclobutenylene, 1,2-cyclopentenylene, 1,3-cyclopentenylene, 2,3-cyclopentenylene, 3,4-cyclopentenylene, 1,2-cyclohexenylene, 1,3-cyclohexenylene, 1,4-cyclohexenylene, 2,3-cyclohexenylene and 3,4-cyclohexenylene groups.

Where X represents a divalent ($C_1$ - $C_3$ alkylene)-($C_3$ - $C_6$ cycloalkylene) group or a divalent ($C_3$ - $C_6$ cycloalkylene)-($C_1$ - $C_3$ alkylene) group, the alkylene and cycloalkylene parts of these groups may be any of the groups exemplified above. Preferred examples of such groups include the methylene-2-cyclopentylene and 2-cyclopropylenemethylene groups.

Of the above groups which may be represented by X, we most prefer the methylene, ethylene, ethylidene, 1-propenylene, 2-methylvinylene, 2-butenylene, 1-propynylidene, methylene-2-cyclopentylene and 2-cyclopropylenemethylene groups.

Where the group represented by the formula $-Y-R^2$ represents a group of formula $-OSO_2R^4$, and $R^4$ represents a $C_1$ - $C_4$ alkyl group, this may be a straight or branched chain alkyl group containing from 1 to 4 carbon atoms, and examples include the methyl, ethyl, propyl, isopropyl, butyl, isobutyl, sec-butyl and t-butyl groups, of which the methyl group is most preferred.

Where the group represented by the formula $-Y-R^2$ represents a group of formula $-OSO_2R^4$, and $R^4$ represents a $C_1$ - $C_4$ haloalkyl group, this may be a straight or branched chain haloalkyl group containing from 1 to 4 carbon atoms, and examples include the trifluoromethyl, trichloromethyl, fluoromethyl, chloromethyl, bromomethyl, iodomethyl, difluoromethyl, dichloromethyl, 2-chloroethyl, 2,2-dichloroethyl, 2,2,2-trichloroethyl, pentachloroethyl, 2-fluoroethyl, 2,2-difluoroethyl, 2,2,2-trifluoroethyl, pentafluoroethyl, 3-chloropropyl, 3,3-dichloropropyl, 3,3,3-trichloropropyl, 3,3,3,2,2-pentachloropropyl, 3-fluoropropyl, 3,3-difluoropropyl, 3,3,3-trifluoropropyl, pentafluoropropyl, 4-chlorobutyl, 4-fluorobutyl, 4-iodobutyl and 4-bromobutyl groups.

Where the group represented by the formula $-Y-R^2$ represents a group of formula $-OSO_2R^4$, and $R^4$ represents an optionally substituted phenyl group, the substituents are selected from nitro groups, halogen atoms and $C_1$ - $C_4$ alkyl groups. Examples of halogen atoms are the fluorine, chlorine, bromine or iodine atoms, whilst examples of the alkyl groups are as given above in relation to the same groups which may be represented by $R^4$. Examples of the substituted phenyl groups include the 4-butylphenyl, 2,4-difluorophenyl, 4-nitrophenyl, 4-chlorophenyl and tolyl groups. However, the unsubstituted phenyl group is preferred.

Where the group represented by the formula $-Y-R^2$ represents a protected hydroxy group, the protecting group may be any one commonly used in this field. Where the compounds of the present invention are intended for use as agrochemicals or as pharmaceuticals, it will, of course, be clear that the protecting group employed should be acceptable in either the agricultural or the pharmaceutical field, respectively, as is well known in the art. Where the compounds of the present invention are intended for use merely as intermediates in the preparation of others of the compounds of the invention, even this restriction on the types of protecting group does not apply, and the protecting group may be chosen simply having regard to its value for protection in the relevant reaction and its ease or otherwise of removal. However, the man skilled in the art would have no difficulty in determining what groups are employable. Purely by way of exemplification, such protecting groups include:

aliphatic acyl groups, such as: the alkanoyl groups, particularly $C_1$ - $C_{20}$, more preferably $C_1$ - $C_{18}$, alkanoyl groups, e.g. the formyl, acetyl, propionyl, butyryl, isobutyryl, pentanoyl, pivaloyl, valeryl, isovaleryl,

octanoyl, lauroyl, myristoyl, tridecanoyl, palmitoyl and stearoyl groups; halogenated alkanoyl groups, especially $C_2$ - $C_6$, more preferably $C_2$ - $C_4$, haloalkanoyl groups (in which there may be one or more halogen atoms, e.g. from 1 to 3 halogen atoms), and most preferably haloacetyl groups, e.g. the chloroacetyl, dichloroacetyl, trichloroacetyl and trifluoroacetyl groups; lower alkoxyalkanoyl groups in which the alkoxy part is preferably $C_1$ - $C_4$ and the alkanoyl part is preferably $C_2$ - $C_7$, more preferably $C_2$ - $C_5$, e.g. the methoxyacetyl group; and alkenoyl or alkynoyl groups, especially $C_3$ - $C_{18}$, preferably $C_3$ - $C_7$, and more preferably $C_3$ - $C_5$, alkenoyl and alkynoyl groups, e.g. the acryloyl, propioloyl, methacryloyl, crotonoyl, isocrotonoyl, oleoyl, elaidoyl, maleoyl, fumaroyl, citraconoyl, mesaconoyl and (E)-2-methyl-2-butenoyl groups;

aromatic acyl groups, such as arylcarbonyl groups, in which the aryl part is preferably a phenyl or naphthyl group which is unsubstituted or has at least one of substituents (c), as defined hereafter, for example: unsubstituted groups, such as the benzoyl, α-naphthoyl and β-naphthoyl groups; halogen-substituted arylcarbonyl groups, such as the 2-bromobenzoyl and 4-chlorobenzoyl groups; lower alkyl-substituted arylcarbonyl groups, such as the 2,4,6-trimethylbenzoyl and 4-toluoyl groups; lower alkoxy-substituted arylcarbonyl groups, such as the 4-anisoyl group; nitrated arylcarbonyl groups, such as the 4-nitrobenzoyl and 2-nitrobenzoyl groups; lower alkoxycarbonylated arylcarbonyl groups, in which the alkoxy group is preferably $C_1$ - $C_6$, more preferably $C_1$ - $C_4$, such as the 2-(methoxycarbonyl)benzoyl group; and aryl-substituted arylcarbonyl groups, such as the 4-phenylbenzoyl group;

tetrahydropyranyl or tetrahydrothiopyranyl groups, which may be unsubstituted or have at least one of substituents (d), defined below, for example the tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl groups;

tetrahydrofuranyl or tetrahydrothienyl groups, which may be unsubstituted or have at least one of substituents (d), defined below, for example the tetrahydrofuran-2-yl and tetrahydrothien-2-yl groups, which may be substituted or unsubstituted;

silyl groups, such as: the tri-lower-alkylsilyl groups in which each alkyl part, which may be the same or different, is a $C_1$ - $C_6$, preferably $C_1$ - $C_4$, alkyl group, e.g. the trimethylsilyl, triethylsilyl, isopropyldimethylsilyl, t-butyldimethylsilyl, methyldiisopropylsilyl, methyl-di-t-butylsilyl and triisopropylsilyl groups, and tri-lower-alkylsilyl groups in which 1 or 2 of the alkyl groups have been replaced by an aryl group [preferably a phenyl or naphthyl group, more preferably phenyl, which may be unsubstituted or have at least one of substituents (c), defined below] e.g. the diphenylmethylsilyl, diphenylbutylsilyl, diphenylisopropylsilyl and phenyldiisopropylsilyl groups;

alkoxymethyl groups, in which the alkoxy part is preferably $C_1$ - $C_6$, more preferably $C_1$ - $C_4$, and in which the alkoxy part is unsubstituted or has at least one of substituents (e), defined below, for example: the lower unsubstituted alkoxymethyl groups, such as the methoxymethyl, 1,1-dimethyl-1-methoxymethyl, ethoxymethyl, propoxymethyl, isopropoxymethyl, butoxymethyl and t-butoxymethyl groups; lower alkoxy-substituted lower alkoxymethyl groups, such as the 2-methoxyethoxymethyl group; and halogenated lower alkoxymethyl groups, such as the 2,2,2-trichloroethoxymethyl and bis(2-chloroethoxy)methyl groups;

substituted ethyl groups such as: the lower (preferably $C_1$ - $C_6$, more preferably $C_1$ - $C_4$) alkoxylated ethyl groups, such as the 1-ethoxyethyl and 1-isopropoxyethyl groups; halogen-substituted ethyl groups, such as the 2,2,2-trichloroethyl group; and arylselenylated ethyl groups, such as the 2-(phenylselenenyl)ethyl group;

aralkyl groups, particularly those in which a lower alkyl group (preferably $C_1$ - $C_6$, more preferably $C_1$ - $C_4$, most preferably $C_1$ - $C_3$) is substituted with from 1 to 3 $C_6$ - $C_{14}$ aryl groups [preferably a phenyl, naphthyl or anthryl group which may be unsubstituted or have at least one of substituents (c), defined below], for example: unsubstituted groups, e.g. the benzyl, α-naphthylmethyl, β-naphthylmethyl, diphenylmethyl, triphenylmethyl, α-naphthyldiphenylmethyl and 9-anthrylmethyl groups; lower (preferably $C_1$ - $C_6$, more preferably $C_1$ - $C_4$) alkyl-substituted groups, e.g the 4-methylbenzyl, 2,4,6-trimethylbenzyl and 3,4,5-trimethylbenzyl groups; lower (preferably $C_1$ - $C_6$, more preferably $C_1$ - $C_4$) alkoxy-substituted groups, e.g. the 4-methoxybenzyl and (4-methoxyphenyl)diphenylmethyl groups; nitro-substituted groups, e.g. the 2-nitrobenzyl, 4-nitrobenzyl and bis(2-nitrophenyl)methyl groups; halogen-substituted groups, e.g. the 4-chlorobenzyl and 4-bromobenzyl groups; cyano-substituted groups, e.g. the 4-cyanobenzyl and 4-cyanobenzyldiphenylmethyl groups; and $C_1$ - $C_3$ alkylenedioxy-substituted groups, such as the piperonyl group;

alkoxycarbonyl groups, such as the unsubstituted lower alkoxycarbonyl groups (in which the alkoxy group is preferably $C_1$ - $C_6$, more preferably $C_1$ - $C_4$), e.g. the methoxycarbonyl, ethoxycarbonyl, t-butoxycarbonyl and isobutoxycarbonyl groups; and such lower alkoxycarbonyl groups which are substituted with one or more halogen atoms, tri-lower alkylsilyl groups, arylalkylsilyl groups or alkanoyloxy groups, such as the 2,2,2-trichloroethoxycarbonyl, 2-trimethylsilylethoxycarbonyl and pivaloyloxymethoxycarbonyl groups;

alkenyloxycarbonyl groups, in which the alkenyl part is preferably $C_2$ - $C_7$, more preferably $C_2$ - $C_5$, such as

the vinyloxycarbonyl and allyloxycarbonyl groups; and
aralkyloxycarbonyl groups, in which the aralkyl part is preferably as defined above, and more preferably the aryl ring is unsubstituted or is substituted with 1 or 2 of substituents (c), defined below, preferably lower alkoxy or nitro groups, such as the benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl groups.

In the above examples, substituents (c) are: lower (preferably $C_1$ - $C_6$, more preferably $C_1$ - $C_4$) alkyl groups, e.g. the methyl, ethyl, propyl, isopropyl, butyl, isobutyl, sec-butyl and t-butyl groups; lower (preferably $C_1$ - $C_6$, more preferably $C_1$ - $C_4$) alkoxy groups, e.g. the methoxy, ethoxy, propoxy, isopropoxy, butoxy, isobutoxy, sec-butoxy, t-butoxy, pentyloxy, isopentyloxy, 2-methylbutoxy, neopentyloxy, hexyloxy, 4-methylpentyloxy, 3-methylpentyloxy, 2-methylpentyloxy, 3,3-dimethylbutoxy, 2,2-dimethylbutoxy, 1,1-dimethylbutoxy, 1,2-dimethylbutoxy, 1,3-dimethylbutoxy and 2,3-dimethylbutoxy groups; the nitro group; halogen atoms, such as the fluorine, chlorine, bromine and iodine atoms; the cyano group; aryl groups, e.g. as exemplified above, provided that, where any such aryl group is a substituent on an aryl group, it is not itself further substituted by another aryl group; $C_1$ - $C_3$, preferably $C_1$ or $C_2$ alkylenedioxy groups, e.g. the methylenedioxy group; the hydroxy group; the amino group; mono- and di- alkylamino groups, in which the or each alkyl group is preferably $C_1$ - $C_6$, more preferably $C_1$ - $C_4$, and examples are as given for the alkyl substituents above; the carbamoyl group; mono- and di- alkylcarbamoyl groups, in which the or each alkyl group is preferably $C_1$ - $C_6$, more preferably $C_1$ - $C_4$, and examples are as given for the alkyl substituents above; $C_1$ - $C_{18}$ aliphatic acyl groups, such as those defined above as protecting groups; halogenated lower (e.g. $C_1$ - $C_6$, especially $C_1$ - $C_4$) alkyl groups, such as the trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl and 2,2-dibromoethyl groups; the carboxy group; and esterified derivatives of the carboxy group, e.g. alkoxycarbonyl groups, in which the alkoxy group is preferably $C_1$ - $C_6$, more preferably $C_1$ - $C_4$ (such as the methoxycarbonyl, ethoxycarbonyl, propoxycarbonyl, isopropoxycarbonyl, butoxycarbonyl, isobutoxycarbonyl, sec-butoxycarbonyl or t-butoxycarbonyl groups), halogenated alkoxycarbonyl groups in which the halogenated alkyl part is as defined above (such as the trifluoromethoxycarbonyl group) or aralkyloxycarbonyl groups, in which the aryl part is preferably as defined above, e.g. the benzyloxycarbonyl group and substituted derivatives thereof.

Substituents (d), which are optional substituents on heterocyclic groups are as exemplified above for substituents (c), but additionally include the oxygen atom, to form an oxo group.

Substituents (e) include those groups and atoms exemplified above for substituents (c), other than alkyl groups and substituted alkyl groups.

Of the above protecting groups, the most preferred are the tetrahydropyranyl, tetrahydrofuranyl and ethoxymethyl groups.

Where Y represents a group of formula $-N(R^5)CO-$ or $-N(R^5)CO-CH=CH-$, $R^5$ represents a hydrogen atom or a $C_1$ - $C_4$ alkyl group; in the case of the alkyl groups, examples are as given above in relation to the same groups which may be represented by $R^4$.

Where $R^2$ represents a $C_1$ - $C_6$ alkyl group or a $C_1$ - $C_6$ haloalkyl group, the alkyl groups may be as exemplified above in relation to the same groups which may be represented by $R^1$. The haloalkyl groups may be any of these alkyl groups having at least one halogen (e.g. fluorine, chlorine, bromine or iodine) substituent, and examples include, inter alia, those given in relation to $R^4$.

Where $R^2$ represents an optionally substituted phenyl group, the substituent(s) are selected from substituents (a), defined above and exemplified below, and the number of such substituents may be from 1 to 5, although the preferred number is from 1 to 3, more preferably 1 or 2. Examples of such substituted phenyl groups include the 4-fluorophenyl, 4-chlorophenyl, 4-bromophenyl, 2,4-difluorophenyl, 3,4-difluorophenyl, 2,4-dichlorophenyl, pentafluorophenyl, 3-(trifluoromethyl)phenyl, 4-(trifluoromethyl)phenyl, 4-(trichloromethyl)phenyl, 2-fluoro-5-(trifluoromethyl)phenyl, 2-fluoro-4-(trifluoromethyl)phenyl, 3-fluoro-4-(trifluoromethyl)phenyl, 4-(difluoromethoxy)phenyl, 4-(trifluoromethoxy)phenyl, 4-(2,2,2-trifluoroethoxy)-phenyl, 4-(2,2,3,3-tetrafluoropropoxy)phenyl, 3-nitrophenyl, 4-nitrophenyl, 2-fluoro-4-nitrophenyl, 3-cyanophenyl, 4-cyanophenyl, 2-chloro-4-cyanophenyl, 4-(methoxycarbonyl)phenyl, 2-fluoro-4-(ethoxycarbonyl)phenyl, 4-(methylthio)phenyl, 4-(methylsulphinyl)phenyl, 4-(methylsulphonyl)phenyl and 4-(dichloroacetamido)phenyl groups, in particular the 4-chlorophenyl, 4-fluorophenyl, 4-cyanophenyl, 4-(trifluoromethyl)phenyl, 3-(trifluoromethyl)phenyl, 2,4-difluorophenyl, 3,4-difluorophenyl and 2-fluoro-4-(trifluoromethyl)phenyl groups.

Where $R^2$ represents a heterocyclic group having 5 or 6 ring atoms, from 1 to 3 of these are nitrogen and/or oxygen and/or sulphur hetero-atoms, and said heterocyclic group is unsubstituted or has from 1 to 3 of substituents (b), defined above and exemplified below, and the number of such substituents may be from 1 to 3, although the preferred number is 1 or 2. Examples of such heterocyclic groups include the thienyl,

furyl, pyranyl, pyrrolyl, imidazolyl, pyrazolyl, thiazolyl, isothiazolyl, oxazolyl, isoxazolyl, pyridyl, pyrazinyl, pyrimidinyl, pyridazinyl, furazanyl, pyrrolidinyl, pyrrolinyl, imidazolidinyl, imidazolinyl, pyrazolidinyl, pyrazolinyl, piperidyl, piperazinyl, morpholinyl and thiomorpholinyl groups. Examples of such substituted heterocyclic groups include the 6-chloro-3-pyridyl, 6-(trifluoromethyl)-3-pyridyl, 5-chloro-2-pyridyl, 5-(trifluoromethyl)-2-furyl and 5-(trifluoromethyl)-2-thienyl and 5-chloro-2-thienyl groups.

Where $R^3$ and $-X_m-Y-R^2$ together represent said group of formula (II), $R^2$, $R^3$, m and n are as defined above, and $R^2$ and $R^3$ may be as exemplified above.

Examples of the groups and atoms which may be represented by substituents (a) and/or (b) include:

$C_1$ - $C_4$ alkyl groups, such as the methyl, ethyl, propyl, isopropyl, butyl, isobutyl, sec-butyl and t-butyl groups;

$C_1$ - $C_4$ haloalkyl groups, such as the trifluoromethyl, trichloromethyl, fluoromethyl, chloromethyl, bromomethyl, iodomethyl, difluoromethyl, dichloromethyl, 2-chloroethyl, 2,2-dichloroethyl, 2,2,2-trichloroethyl, pentachloroethyl, 2-fluoroethyl, 2,2-difluoroethyl, 2,2,2-trifluoroethyl, pentafluoroethyl, 3-chloropropyl, 3,3-dichloropropyl, 3,3,3-trichloropropyl, 3,3,3,2,2-pentachloropropyl, 3,3,2,2-tetrachloropropyl, 3-fluoropropyl, 3,3-difluoropropyl, 3,3,3-trifluoropropyl, pentafluoropropyl, 4-chlorobutyl, 4-fluorobutyl, 4-iodobutyl and 4-bromobutyl groups;

$C_1$ - $C_4$ alkoxy groups, such as the methoxy, ethoxy, propoxy, isopropoxy, butoxy, isobutoxy, sec-butoxy and t-butoxy groups;

$C_1$ - $C_4$ haloalkoxy groups, such as the trifluoromethoxy, trichloromethoxy, fluoromethoxy, chloromethoxy, bromomethoxy, iodomethoxy, difluoromethoxy, dichloromethoxy, 2-chloroethoxy, 2,2-dichloroethoxy, 2,2,2-trichloroethoxy, pentachloroethoxy, 2-fluoroethoxy, 2,2-difluoroethoxy, 2,2,2-trifluoroethoxy, pentafluoroethoxy, 3-chloropropoxy, 3,3-dichloropropoxy, 3,3,3-trichloropropoxy, 3,3,3,2,2-pentachloropropoxy, 3,3,2,2-tetrachloropropoxy, 3-fluoropropoxy, 3,3-difluoropropoxy, 3,3,3-trifluoropropoxy, pentafluoropropoxy, 4-chlorobutoxy, 4-fluorobutoxy, 4-iodobutoxy and 4-bromobutoxy groups;

halogen atoms, e.g as exemplified above;

nitro groups;

cyano groups;

hydroxy groups;

benzyloxy groups;

hydroxymethyl groups;

groups of formula $-CH_2-OCO-R^7$, in which $R^7$ represents a $C_1$ - $C_6$ alkyl group, such as those exemplified above in relation to the same groups which may be represented by $R^1$, especially the acetoxymethyl and propionyloxymethyl groups;

groups of formula $-CO-R^6$, in which $R^6$ represents a $C_1$ - $C_6$ alkyl group or a $C_1$ - $C_6$ haloalkyl group, such as those exemplified above in relation to the same groups which may be represented by $R^2$, especially the acetyl, chloroacetyl, fluoroacetyl, trichloroacetyl, trifluoroacetyl, propionyl and 3-chloropropionyl groups;

groups of formula $-COOR^7$, in which $R^7$ represents a $C_1$ - $C_6$ alkyl group, such as those exemplified above in relation to the same groups which may be represented by $R^1$, especially the ethoxycarbonyl and methoxycarbonyl groups;

groups of formula $-SO_rR^7$, in which $R^7$ represents a $C_1$ - $C_6$ alkyl group, such as those exemplified above in relation to the same groups which may be represented by $R^1$, especially the methanesulphonyl and ethanesulphonyl groups, and the corresponding sulphinyl and thio groups;

amino groups, and mono- and di- alkylamino groups in which each alkyl group is $C_1$ - $C_4$, such as the methylamino, ethylamino, propylamino, isopropylamino, butylamino, isobutylamino, sec-butylamino, t-butylamino, dimethylamino, diethylamino, dipropylamino, diisopropylamino and dibutylamino groups; and

carboxylic acylamino groups, in which the acyl part is preferably: a $C_1$ - $C_8$ alkanoyl group, a $C_1$ - $C_8$ haloalkanoyl group, a $C_3$ - $C_8$ alkenoyl group, a benzoyl group, a benzoyl group having at least one of substituents (a), other than a substituted benzoyl group, a naphthoyl group, a cinnamoyl group or a heterocyclic carboxylic acyl group in which the heterocyclic part has 5 or 6 ring atoms of which 1 or 2 are nitrogen and/or oxygen and/or sulphur hetero-atoms; specific examples of such groups include the formamido, acetamido, propionamido, butyrylamino, isobutyrylamino, amylamino, isoamylamino, hexanoylamino, heptanoylamino, octanoylamino, benzoylamino, p-chlorobenzoylamino, p-nitrobenzoylamino, p-cyanobenzoylamino, $\alpha$-naphthoylamino, $\beta$-naphthoylamino, cinnamoylamino, nicotinoylamino, picolinoylamino, isonicotinoylamino, furoylamino and thenoylamino groups.

Of the compounds of the present invention described above, the preferred classes of compound are those of formula (I) in which:

(A) Ar represents a phenyl group having one or two halogen substituents;

(B) Ar represents a phenyl group having one or two fluorine and/or chlorine substituents;

(C) $R^1$ represents a $C_1$ - $C_3$ alkyl group;

(D) $R^1$ represents a methyl or ethyl group;

(E) m is 0 or X represents a $C_1$ or $C_2$ alkylene group, which may be a straight or branched chain group;

(F) $-Y_n-R^2$ represents the azido group;

(G) Y represents a group of formula $-N(R^5)CO-$, $-N(R^5)CO-CH=CH-$, -S-CO- or -S-CO-CH=CH-, in which:

$R^5$ represents a hydrogen atom or a $C_1$ - $C_4$ alkyl group;

(H) $R^2$ represents a phenyl group having one or two substituents selected from halogen atoms and trifluoromethyl, trifluoromethoxy and cyano groups.

The more preferred compounds are those in which Ar is as defined in (A), $R^1$ is as defined in (C), X is as defined in (E), Y is as defined in (G) and $R^2$ is as defined in (H), and the most preferred compounds are those in which Ar is as defined in (B), $R^1$ is as defined in (D), X is as defined in (E), Y is as defined in (G) and $R^2$ is as defined in (H). Still more preferred compounds are those in which $R^1$ is as defined in (D), X is as defined in (E), Y is as defined in (G) and $R^2$ is as defined in (H) and Ar represents a 4-chlorophenyl group, a 4-fluorophenyl group, a 2,4-dichlorophenyl group, a 2,4-difluorophenyl group, a 2-chloro-4-fluorophenyl group or a 4-chloro-2-fluorophenyl group.

The compounds of the present invention necessarily contain several basic nitrogen atoms and can, therefore, form acid addition salts and such salts also form part of the present invention. There is no limitation upon the nature of such salts, provided that, where they are to be used for agricultural or horticultural purposes, the salts do not or do not to a significant extent increase the phytotoxicity of the compounds beyond that of the free base, or, where they are to be used for therapeutic purposes, that they are pharmaceutically acceptable, which, as is well-known in the art, means that they do not have reduced activity (or unacceptably reduced activity) or increased toxicity (or unacceptably increased toxicity) compared with the free compound of formula (I). Where, however, they are to be used for non-agricultural, non-horticultural or non-therapeutic purposes, e.g. as intermediates in the preparation of other compounds, even this limitation does not apply. Examples of suitable salts include: salts with an inorganic acid, such as a hydrogen halide (e.g. hydrofluoric acid, hydrochloric acid, hydrobromic acid or hydroiodic acid), or nitric acid, perchloric acid, sulphuric acid or phosphoric acid; and salts with an organic acid, such as a lower alkylsulphonic acid (e.g. methanesulphonic acid, trifluoromethanesulphonic acid or ethanesulphonic acid), an arylsulphonic acid (e.g. benzenesulphonic acid or p-toluenesulphonic acid), or a carboxylic acid (e.g. fumaric acid, oxalic acid or maleic acid).

The compounds of the present invention can exist in the form of various optical isomers and diastereomers because of the existence of asymmetric carbon atoms in the molecule. Those compounds having a double bond in the group defined as $-X_m-Y_n-R^2$ can also exist in the form of geometrical isomers. i.e. the E and Z forms. The optical isomers can be resolved using conventional techniques of optical resolution to give optically active compounds. The diasteroisomers and geometrical isomers can be resolved using conventional techniques for separation such as recrystallization or chromatography. The present invention covers both the individual isomers and mixtures (e.g. racemic mixtures) thereof, whether as obtained by the synthesis reaction or by mixing. If individual isomers are required, these may be prepared from mixtures, as explained above, or they may be prepared by stereospecific or enantiospecific synthesis techniques, as explained in more detail hereafter.

Examples of specific compounds of the invention are given in the following formulae (I-1) and (I-2), in which the substituents are as defined in the corresponding one of Tables 1, 1A and 2 [i.e. Tables 1 and 1A relate to formula (I-1) and Table 2 relates to formula (I-2)]. In the Tables, where a dash (-) is shown under the column for X, this means X is absent, i.e. m in formula (I) is 0. Also, the following abbreviations are used:

| | |
|---|---|
| Ac | acetyl |
| iBu | isobutyl |
| tBu | t-butyl |
| Bz | benzyl |
| Et | ethyl |
| Etc | ethoxycarbonyl |
| Fur | furyl |
| Hx | hexyl |
| Me | methyl |
| Moc | methoxycarbonyl |
| Np | naphthyl |

| | |
|---|---|
| Ph | phenyl |
| Phtm | phthalimido |
| Pr | propyl |
| cPr | cyclopropyl |
| iPr | isopropyl |
| Pyaz | pyrazolyl |
| Pym | pyrimidinyl |
| Pyr | pyridyl |
| Tfm | trifluoromethyl |
| Thi | thienyl |
| Thiz | thiazolyl |
| Thp | tetrahydropyranyl |

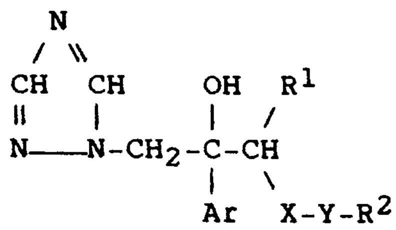

(I-1)

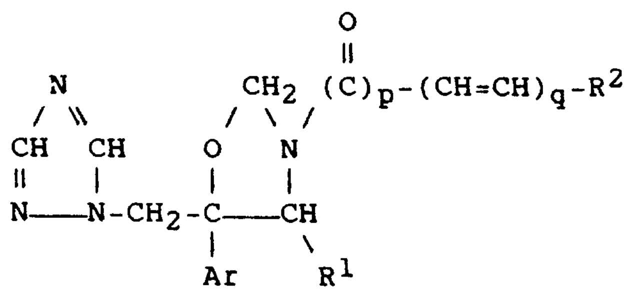

(I-2)

Table 1

| Cpd No. | Ar | $R^1$ | X | $R^2$-Y |
|---|---|---|---|---|
| 1-1 | 2,4-diCℓPh | Me | - | (4-FPh)-CONH- |
| 1-2 | 2,4-diCℓPh | Me | - | (4-CℓPh)-CONH- |
| 1-3 | 2,4-diCℓPh | Me | - | (2,4-diCℓPh)-CONH- |
| 1-4 | 2,4-diCℓPh | Me | - | (4-BrPh)-CONH- |
| 1-5 | 2,4-diCℓPh | Me | - | (4-TfmPh)-CONH- |
| 1-6 | 2,4-diCℓPh | Me | - | (2,4-diFPh)-CONH- |
| 1-7 | 2,4-diCℓPh | Me | - | (2-F-4-TfmPh)-CONH- |
| 1-8 | 2,4-diCℓPh | Me | - | (4-$F_2$CHOPh)-CONH- |
| 1-9 | 2,4-diCℓPh | Me | - | [4-($F_3CCH_2O$)Ph]-CONH- |
| 1-10 | 2,4-diCℓPh | Me | - | (2-F-4-TfmOPh)-CONH- |
| 1-11 | 2,4-diCℓPh | Me | - | (4-$NO_2$Ph)-CONH- |
| 1-12 | 2,4-diCℓPh | Me | - | (4-NCPh)-CONH- |
| 1-13 | 2,4-diCℓPh | Me | - | (4-MocPh)-CONH- |
| 1-14 | 2,4-diCℓPh | Me | - | (2-Cℓ-4-MocPh)-CONH- |
| 1-15 | 2,4-diCℓPh | Me | - | (4-MeSPh)-CONH- |
| 1-16 | 2,4-diCℓPh | Me | - | [4-MeS(O)Ph]-CONH- |
| 1-17 | 2,4-diCℓPh | Me | - | (4-$MeSO_2$Ph)-CONH- |
| 1-18 | 2,4-diCℓPh | Me | - | [4-EtS(O)Ph]-CONH- |
| 1-19 | 4-CℓPh | Me | - | (4-CℓPh)-CONH- |
| 1-20 | 4-CℓPh | Me | - | (4-TfmPh)-CONH- |
| 1-21 | 2,4-diCℓPh | Et | - | (4-TfmPh)-CONH- |
| 1-22 | 2,4-diFPh | Me | - | (4-FPh)-CONH- |
| 1-23 | 2,4-diFPh | Me | - | (4-CℓPh)-CONH- |
| 1-24 | 2,4-diFPh | Me | - | (4-TfmPh)-CONH- |
| 1-25 | 2,4-diFPh | Me | - | (2,4-diFPh)-CONH- |
| 1-26 | 2,4-diFPh | Me | - | (2-F-4-TfmPh)-CONH- |
| 1-27 | 2,4-diFPh | Me | - | (4-TfmOPh)-CONH- |

Table 1 (cont)

| Cpd No. | Ar | $R^1$ | X | $R^2$-Y |
|---|---|---|---|---|
| 1-28 | 2,4-diFPh | Me | - | [4-($CHF_2CF_2CH_2O$)Ph]-CONH- |
| 1-29 | 2,4-diFPh | Me | - | (4-$NO_2$Ph)-CONH- |
| 1-30 | 2,4-diFPh | Me | - | (2,3,4,5,6-pentaFPh)-CONH- |
| 1-31 | 2,4-diFPh | Me | - | (2-F-4-$NO_2$Ph)-CONH- |
| 1-32 | 2,4-diFPh | Me | - | (4-NCPh)-CONH- |
| 1-33 | 2,4-diFPh | Me | - | (4-MocPh)-CONH- |
| 1-34 | 2,4-diFPh | Me | - | (2-F-4-EtcPh)-CONH- |
| 1-35 | 2,4-diFPh | Me | - | [4-MeS(O)Ph]-CONH- |
| 1-36 | 2,4-diFPh | Me | - | (4-$MeSO_2$Ph)-CONH- |
| 1-37 | 2,4-diFPh | Me | - | [2-Cl-4-MeS(O)Ph]-CONH- |
| 1-38 | 2,4-diFPh | Et | - | (4-TfmPh)-CONH- |
| 1-39 | 2,4-diFPh | Pr | - | (4-FPh)-CONH- |
| 1-40 | 2,4-diFPh | <u>i</u>Pr | - | (4-$F_2$CHOPh)-CONH- |
| 1-41 | 2-F-4-TfmPh | Me | - | Ph-CONH- |
| 1-42 | 2-F-4-TfmPh | Me | - | (4-FPh)-CONH- |
| 1-43 | 4-TfmPh | Me | - | (4-ClPh)-CONH- |
| 1-44 | 2-F-4-TfmPh | Me | - | (4-TfmPh)-CONH- |
| 1-45 | 2-F-4-TfmPh | Me | - | (4-$F_2$CHOPh)-CONH- |
| 1-46 | 2-F-4-TfmPh | Me | - | (4-NCPh)-CONH- |
| 1-47 | 2-F-4-ClPh | Me | - | (4-ClPh)-CONH- |
| 1-48 | 2,4-diClPh | Me | - | (6-Cl-3-Pyr)-CONH- |
| 1-49 | 2,4-diClPh | Me | - | (6-Tfm-3-Pyr)-CONH- |
| 1-50 | 2,4-diFPh | Me | - | (5-Tfm-2-Pyr)-CONH- |
| 1-51 | 2,4-diClPh | Me | - | (5-Tfm-2-Fur)-CONH- |
| 1-52 | 2,4-diClPh | Me | - | (5-Tfm-2-Thi)-CONH- |
| 1-53 | 2,4-diClPh | Me | - | (5-$NO_2$-2-Thi)-CONH- |
| 1-54 | Ph | Me | - | (4-TfmPh)-CONH- |

Table 1 (cont)

| Cpd No. | Ar | $R^1$ | X | $R^2$-Y |
|---|---|---|---|---|
| 1-55 | 2,4-diCℓPh | Me | - | [4-(AcNH)Ph]-CONH- |
| 1-56 | 2,4-diCℓPh | Me | - | [4-(triFAc)Ph]-CONH- |
| 1-57 | 2,4-diFPh | Me | - | (2-Cℓ-4-AcPh)-CONH- |
| 1-58 | 4-FPh | Me | - | (4-TfmPh)-CONH- |
| 1-59 | 4-FPh | Me | - | (2-F-4-TfmPh)-CONH- |
| 1-60 | 4-FPh | Me | - | (4-NCPh)-CONH- |
| 1-61 | 4-FPh | Me | - | (4-CℓPh)-CH=CH-CONH- |
| 1-62 | 4-FPh | Me | - | (4-TfmPh)-CO-S- |
| 1-63 | 4-FPh | Me | - | (4-TfmPh)-CH=CH-CO-S- |
| 1-64 | 4-FPh | Me | - | (4-TfmPh)-CONMe- |
| 1-65 | 4-FPh | Me | - | (2-F-4-TfmPh)-CONMe- |
| 1-66 | 4-CℓPh | Me | - | (4-NCPh)-CONH- |
| 1-67 | 4-CℓPh | Me | - | (3-TfmPh)-CONH- |
| 1-68 | 4-CℓPh | Me | - | (2-F-4-TfmPh)-CONH- |
| 1-69 | 4-CℓPh | Me | - | (4-CℓPh)-CH=CH-CONH- |
| 1-70 | 4-CℓPh | Me | - | (4-TfmPh)-CH=CH-CONH- |
| 1-71 | 4-CℓPh | Me | - | (4-CℓPh)-CO-S- |
| 1-72 | 4-CℓPh | Me | - | (4-TfmPh)-CO-S- |
| 1-73 | 4-CℓPh | Me | - | (3-TfmPh)-CO-S- |
| 1-74 | 4-CℓPh | Me | - | (2-F-4-TfmPh)-CO-S- |
| 1-75 | 4-CℓPh | Me | - | (2-F-5-TfmPh)-CO-S- |
| 1-76 | 4-CℓPh | Me | - | (4-CℓPh)-CH=CH-CO-S- |
| 1-77 | 4-CℓPh | Me | - | (4-TfmPh)-CH=CH-CO-S- |
| 1-78 | 4-CℓPh | Me | - | (4-CℓPh)-CONMe- |
| 1-79 | 4-CℓPh | Me | - | (2,4-diFPh)-CONMe- |
| 1-80 | 4-CℓPh | Me | - | (3,4-diFPh)-CONMe- |
| 1-81 | 4-CℓPh | Me | - | (4-TfmPh)-CONMe- |
| 1-82 | 4-CℓPh | Me | - | (3-TfmPh)-CONMe- |

Table 1 (cont)

| Cpd No. | Ar | $R^1$ | X | $R^2$-Y |
|---|---|---|---|---|
| 1-83 | 4-CℓPh | Me | - | (2-F-4-TfmPh)-CONMe- |
| 1-84 | 4-CℓPh | Me | - | (4-CℓPh)-CH=CH-CONMe- |
| 1-85 | 4-CℓPh | Me | - | (4-TfmPh)-CH=CH-CONMe- |
| 1-86 | 2,4-diCℓPh | Me | - | (4-CℓPh)-CO-O- |
| 1-87 | 2,4-diCℓPh | Me | - | (4-CℓPh)-CH=CH-CO-O- |
| 1-88 | 2,4-diCℓPh | Me | - | (4-CℓPh)-CO-S- |
| 1-89 | 2,4-diCℓPh | Me | - | (4-TfmPh)-CO-S- |
| 1-90 | 2,4-diCℓPh | Me | - | (3-TfmPh)-CO-S- |
| 1-91 | 2,4-diCℓPh | Me | - | (2-F-4-TfmPh)-CO-S- |
| 1-92 | 2,4-diCℓPh | Me | - | (4-CℓPh)-CH=CH-CO-S- |
| 1-93 | 2,4-diCℓPh | Me | - | (4-TfmPh)-CH=CH-CO-S- |
| 1-94 | 2,4-diCℓPh | Me | - | (4-NCPh)-CONMe- |
| 1-95 | 2,4-diCℓPh | Me | - | (4-TfmPh)-CONMe- |
| 1-96 | 2,4-diCℓPh | Me | - | (3-TfmPh)-CONMe- |
| 1-97 | 2,4-diCℓPh | Me | - | (2-F-4-TfmPh)-CONMe- |
| 1-98 | 2,4-diCℓPh | Me | - | (4-CℓPh)-CH=CH-CONMe- |
| 1-99 | 2,4-diCℓPh | Me | - | (4-TfmPh)-CH=CH-CONMe- |
| 1-100 | 2,4-diCℓPh | Me | - | (6-Tfm-3-Pyr)-CONMe- |
| 1-101 | 2,4-diCℓPh | Me | - | (5-Tfm-2-Thi)-CONMe- |
| 1-102 | 2,4-diCℓPh | Me | - | (6-Cℓ-3-Pyr)-CH=CH-CONMe- |
| 1-103 | 2,4-diCℓPh | Me | - | (5-Tfm-2-Thi)-CH=CH-CONMe- |
| 1-104 | 2,4-diCℓPh | Me | - | (4-CℓPh)-CH=CH-CONH- |
| 1-105 | 2,4-diCℓPh | Me | - | (4-TfmPh)-CH=CH-CONH- |
| 1-106 | 2,4-diCℓPh | Me | - | (4-NCPh)-CH=CH-CONH- |
| 1-107 | 2,4-diFPh | Me | - | (4-CℓPh)-CH=CH-CONH- |
| 1-108 | 2,4-diFPh | Me | - | (4-TfmPh)-CH=CH-CONH- |
| 1-109 | 2,4-diFPh | Me | - | (4-CℓPh)-CH=CH-CO-O- |
| 1-110 | 2,4-diFPh | Me | - | (4-CℓPh)-CO-S- |

Table 1 (cont)

| Cpd No. | Ar | $R^1$ | X | $R^2$-Y |
|---|---|---|---|---|
| 1-111 | 2,4-diFPh | Me | - | (3,4-diFPh)-CO-S- |
| 1-112 | 2,4-diFPh | Me | - | (4-NCPh)-CO-S- |
| 1-113 | 2,4-diFPh | Me | - | (3,5-diFPh)-CO-S- |
| 1-114 | 2,4-diFPh | Me | - | (4-TfmPh)-CO-S- |
| 1-115 | 2,4-diFPh | Me | - | (3-TfmPh)-CO-S- |
| 1-116 | 2,4-diFPh | Me | - | (2-F-4-TfmPh)-CO-S- |
| 1-117 | 2,4-diFPh | Me | - | (4-CℓPh)-CH=CH-CO-S- |
| 1-118 | 2,4-diFPh | Me | - | (4-TfmPh)-CH=CH-CO-S- |
| 1-119 | 2,4-diFPh | Me | - | (4-CℓPh)-CONMe- |
| 1-120 | 2,4-diFPh | Me | - | (2,4-diFPh)-CONMe- |
| 1-121 | 2,4-diFPh | Me | - | (3,4-diFPh)-CONMe- |
| 1-122 | 2,4-diFPh | Me | - | (4-NCPh)-CONMe- |
| 1-123 | 2,4-diFPh | Me | - | (4-$NO_2$Ph)-CONMe- |
| 1-124 | 2,4-diFPh | Me | - | (4-TfmPh)-CONMe- |
| 1-125 | 2,4-diFPh | Me | - | (3-TfmPh)-CONMe- |
| 1-126 | 2,4-diFPh | Me | - | (3-F-4-TfmPh)-CONMe- |
| 1-127 | 2,4-diFPh | Me | - | (2-F-4-TfmPh)-CONMe- |
| 1-128 | 2,4-diFPh | Me | - | (4-CℓPh)-CH=CH-CONMe- |
| 1-129 | 2,4-diFPh | Me | - | (4-TfmPh)-CH=CH-CONMe- |
| 1-130 | 2,4-diFPh | Me | - | (4-TfmPh)-CONEt- |
| 1-131 | 2,4-diFPh | Me | - | (3-TfmPh)-CONEt- |
| 1-132 | 2,4-diFPh | Me | - | (4-CℓPh)-CH=CH-CONEt- |
| 1-133 | 2,4-diFPh | Me | - | (3-FPh)-CONH- |
| 1-134 | 2,4-diFPh | Me | - | (3,4-diFPh)-CONH- |
| 1-135 | 2,4-diFPh | Me | - | (2,5-diFPh)-CONH- |
| 1-136 | 2,4-diFPh | Me | - | (3,5-diFPh)-CONH- |
| 1-137 | 2,4-diFPh | Me | - | (2,6-diFPh)-CONH- |
| 1-138 | 2,4-diFPh | Me | - | (2-TfmPh)-CONH- |

Table 1 (cont)

| Cpd No. | Ar | $R^1$ | X | $R^2$-Y |
|---|---|---|---|---|
| 1-139 | 2,4-diFPh | Me | - | *t*Bu-CONH- |
| 1-140 | 2,4-diFPh | Me | - | $Cl_2$CH-CONH- |
| 1-141 | 2,4-diFPh | Me | - | (2-Fur)-CONH- |
| 1-142 | 2,4-diFPh | Me | - | (2-Thi)-CONH- |
| 1-143 | 2,4-diFPh | Me | - | (4-Pyr)-CONH- |
| 1-144 | 2,4-diFPh | Me | - | (2-Pyr)-CONH- |
| 1-145 | 2,4-diFPh | Et | - | (4-ClPh)-CONH- |
| 1-146 | 2-F-4-ClPh | Me | - | (4-TfmPh)-CONH- |
| 1-147 | 2-F-4-ClPh | Me | - | (2-F-4-TfmPh)-CONH- |
| 1-148 | 2,4-diClPh | Me | - | (2,3-diClPh)-CONH- |
| 1-149 | 2,4-diClPh | Me | - | *t*Bu-CONH- |
| 1-150 | 4-FPh | Me | - | (4-ClPh)-CONH- |
| 1-151 | 4-ClPh | Me | - | (2-F-4-NCPh)-CONH- |
| 1-152 | 2,4-diFPh | Me | - | (4-*t*BuPh)-CONH- |
| 1-153 | 2,4-diFPh | Me | - | (4-MeOPh)-CONH- |
| 1-154 | 2,4-diFPh | Me | - | (4-MePh)-CONH- |
| 1-155 | 2,4-diFPh | Me | - | 4-($NMe_2$)Ph-CONH- |
| 1-156 | 2,4-diFPh | Me | - | (2-Np)-CONH- |
| 1-157 | 2,4-diFPh | Et | - | (2,5-diMe-3-Fur)-CONH- |
| 1-158 | 2,4-diFPh | Et | - | (2,3,5-triMe-4-Pyaz)-CONH- |
| 1-159 | 2,4-diFPh | Me | - | (4-BzOPh)-CONH- |
| 1-160 | 2,4-diFPh | Me | - | (4-HOPh)-CONH- |
| 1-161 | 2,4-diFPh | Me | - | Ph-CONH- |
| 1-162 | 2,4-diFPh | Me | - | 2-($HOCH_2$)Ph-CONH- |
| 1-163 | 2,4-diFPh | Me | - | 2-($AcOCH_2$)Ph-CONH- |
| 1-164 | 2,4-diFPh | Me | - | 4-($CHF_2CF_2O$)Ph-CONH- |
| 1-165 | 2-F-4-ClPh | Et | - | (4-ClPh)-CONH- |
| 1-166 | 4-F-2-ClPh | Et | - | (4-ClPh)-CONH- |

Table 1 (cont)

| Cpd No. | Ar | $R^1$ | X | $R^2$-Y |
|---|---|---|---|---|
| 1-167 | 2,4-diClPh | iBu | - | (4-ClPh)-CONH- |
| 1-168 | 2,4-diFPh | Me | - | 4-ClPhtm |
| 1-169 | 2,4-diFPh | Me | - | (4-ClPh)-CO-O- |
| 1-170 | 2,4-diFPh | Me | - | (4-TfmPh)-CO-O- |
| 1-171 | 2,4-diFPh | Et | - | (4-TfmPh)-CONMe- |
| 1-172 | 2,4-diFPh | Me | - | 1-oxo-2,3-dihydro--2-isoindolyl |
| 1-173 | 2,4-diFPh | Me | $-(CH_2)_2-$ | (4-ClPh)-CONH- |
| 1-174 | 2,4-diClPh | Me | $-CH_2-$ | (4-TfmPh)-CONH- |
| 1-175 | 2,4-diClPh | Me | $-CH_2-$ | (4-ClPh)-CH=CH-CONH- |
| 1-176 | 2,4-diFPh | Et | $-CH=CH-CH_2-$ | (4-TfmPh)-CONH- |
| 1-177 | 2,4-diClPh | Me | $-C\equiv C-CH_2-$ | (4-TfmPh)-CONH- |
| 1-178 | 2,4-diFPh | Me | $-CH_2-CMe_2-$ | (4-TfmPh)-CO-O- |
| 1-179 | 2,4-diClPh | Me | - | $-N_3$ |
| 1-180 | 4-ClPh | Me | - | $-N_3$ |
| 1-181 | 2,4-diFPh | Me | - | $-N_3$ |
| 1-182 | 2-F-4-ClPh | Me | - | $-N_3$ |
| 1-183 | 4-F-2-ClPh | Me | - | $-N_3$ |
| 1-184 | 4-FPh | Me | - | $-N_3$ |
| 1-185 | 4-BrPh | Me | - | $-N_3$ |
| 1-186 | 2-F-4-IPh | Et | - | $-N_3$ |
| 1-187 | 2,4-diFPh | Et | - | $-N_3$ |
| 1-188 | 2-F-4-ClPh | Et | - | $-N_3$ |
| 1-189 | 4-F-2-ClPh | Et | - | $-N_3$ |
| 1-190 | 2,4-diFPh | Me | -CH(Me)- | $-N_3$ |
| 1-191 | 2,4-diFPh | Me | $-CH_2-$ | $-N_3$ |
| 1-192 | 2,4-diFPh | Me | $-(CH_2)_2-$ | $-N_3$ |
| 1-193 | 2,4-diClPh | Me | $-CH_2-$ | $-N_3$ |

Table 1 (cont)

| Cpd No. | Ar | $R^1$ | X | $R^2$-Y |
|---|---|---|---|---|
| 1-194 | 2,4-diFPh | Me | -CH(iPr)-$CH_2$- | -$N_3$ |
| 1-195 | 2,4-diClPh | Me | -$CH_2$-CH(Et)- | -$N_3$ |
| 1-196 | 2,4-diClPh | Me | -$CH_2$-cPn- | 3-$N_3$ |
| 1-197 | 2,4-diFPh | Et | -C≡C-$CH_2$- | -$N_3$ |
| 1-198 | 2,4-diClPh | Me | - | (2-Cl-4-TfmOPh)-CONH- |
| 1-199 | 2,4-diFPh | Me | - | (2-Cl-4-CNPh)-CONH- |
| 1-200 | 4-ClPh | Me | - | (2-Br-4-FPh)-CONH- |
| 1-201 | 2,4-diFPh | Et | - | (2-F-4-IPh)-CONH- |
| 1-202 | 2,4-diFPh | Me | -$(CH_2)_2$- | (2-Br-4-TfmPh)-CONH- |
| 1-203 | 2,4-diFPh | Me | -CH(Me)- | (2-Cl-4-MeSPh)-CONH- |
| 1-204 | 4-ClPh | Et | -$CH_2$-CH=CH-$CH_2$- | (4-CNPh)-CONH- |
| 1-205 | 2,4-diFPh | iPr | -$CH_2$-CH(Pr)- | (2-F-4-TfmPh)-CONH- |
| 1-206 | 2,4-diFPh | Me | -C≡C-$CH_2$- | (4-$NO_2$Ph)-CONH- |
| 1-207 | 4-FPh | Me | -CH=C(Me)- | (4-ClPh)-CONH- |
| 1-208 | 2,4-diFPh | Et | - | (2,4-diClPh)-CONH- |
| 1-209 | 2,4-diFPh | Me | -C≡C-$CH_2$- | (2-$NO_2$-4-FPh)-CONH- |
| 1-210 | 2,4-diClPh | Et | 2-cPr-$CH_2$- | (4-TfmPh)-CONH- |
| 1-211 | 2,4-diFPh | Me | - | [(4-AcNH)Ph]-CONH- |
| 1-212 | 2,4-diClPh | Me | - | (4-AcPh)-CONH- |
| 1-213 | 2,4-diFPh | Me | - | (3-TfmPh)-CH=CH-CONH- |
| 1-214 | 2,4-diFPh | Me | - | (3-TfmPh)-CONH- |
| 1-215 | 2,4-diFPh | Me | - | (5-Cl-2-Thi)-CONH- |

Table 1 (cont)

| Cpd No. | Ar | $R^1$ | X | $R^2$-Y |
|---|---|---|---|---|
| 1-216 | 2,4-diFPh | Me | $-CH(Me)CH_2-$ | (4-TfmPh)-CONH- |
| 1-217 | 2,4-diFPh | Me | -CH(Me)- | (4-TfmPh)-CONH- |
| 1-218 | 2,4-diFPh | Hx | - | $-N_3$ |
| 1-219 | 2,4-diFPh | Me | $-(CH_2)_3-$ | $-N_3$ |
| 1-220 | 2,4-diCℓPh | Me | $-(CH_2)_2CH(Me)-$ | $-N_3$ |
| 1-221 | 2-F-4-CℓPh | Et | $-(CH_2)_4-$ | $-N_3$ |
| 1-222 | 2,4-diFPh | Me | $-(CH_2)_2CH(Me)-$ | $-N_3$ |
| 1-223 | 2,4-diFPh | Et | $-CH_2CH(Me)-$ | $-N_3$ |
| 1-224 | 2-F-4-CℓPh | iPr | $-CH(Me)CH_2-$ | $-N_3$ |
| 1-225 | 2-F-4-CℓPh | Et | - | (4-TfmPh)-CONH- |
| 1-226 | 2-Cℓ-4-FPh | Me | - | (4-CℓPh)-CONH- |
| 1-227 | 4-TfmPh | Me | $-CH_2CH_2-$ | $-N_3$ |
| 1-228 | 2,4-diCℓPh | Et | - | (4-CℓPh)-CONH- |
| 1-229 | 2,4-diCℓPh | Et | - | (4-TfmPh)-CONH- |
| 1-230 | 2,4-diCℓPh | Et | - | (2-F-4-TfmPh)-CONH- |
| 1-231 | 2,4-diCℓPh | Et | $-CH_2-$ | $-N_3$ |
| 1-232 | 2,4-diCℓPh | Et | $-CH_2CH_2-$ | $-N_3$ |
| 1-233 | 2,4-diCℓPh | Et | $-CH_2CH(Me)-$ | $-N_3$ |
| 1-234 | 2,4-diCℓPh | Et | -CH(Me)- | $-N_3$ |
| 1-235 | 2,4-diCℓPh | Et | - | $-N_3$ |
| 1-236 | 4-FPh | Et | - | $-N_3$ |
| 1-237 | 4-BrPh | Et | - | $-N_3$ |

Table 1A

| Cpd No. | Ar | $R^1$ | X | $R^2$-Y |
|---|---|---|---|---|
| 1-238 | 2,4-diFPh | Me | - | 2-Thp-O- |
| 1-239 | 2,4-diFPh | Me | - | $-O-SO_2Me$ |
| 1-240 | 4-FPh | Me | - | 2-Thp-O- |
| 1-241 | 4-FPh | Me | - | $-O-SO_2Me$ |
| 1-242 | 4-BrPh | Me | - | 2-Thp-O- |
| 1-239 | 4-BrPh | Me | - | $-O-SO_2Me$ |

Table 2

| Cpd No. | Ar | $R^1$ | $R^2$ | p | q |
|---|---|---|---|---|---|
| 2-1 | 2,4-diClPh | H | 4-FPh | 0 | 0 |
| 2-2 | 2,4-diFPh | H | 4-ClPh | 0 | 0 |
| 2-3 | 2,4-diClPh | H | 4-TfmPh | 0 | 0 |
| 2-4 | 2,4-diFPh | H | 4-$F_2$CHOPh | 0 | 0 |
| 2-5 | 2,4-diClPh | H | 4-$NO_2$Ph | 0 | 0 |
| 2-6 | 2-F-4-TfmPh | H | 4-NCPh | 0 | 0 |
| 2-7 | 2,4-diClPh | H | 4-MeSPh | 0 | 0 |
| 2-8 | 2,4-diClPh | H | 4-MeS(O)Ph | 0 | 0 |
| 2-9 | 2,4-diFPh | H | 4-Me$SO_2$Ph | 0 | 0 |
| 2-10 | 4-TfmPh | H | 4-TfmPh | 0 | 0 |
| 2-11 | 2,4-diFPh | H | 4-MocPh | 0 | 0 |
| 2-12 | 2,4-diClPh | H | 4-ClPh | 1 | 0 |
| 2-13 | 2,4-diClPh | H | 4-TfmPh | 1 | 0 |
| 2-14 | 2,4-diClPh | H | 4-MeSPh | 1 | 0 |
| 2-15 | 2,4-diFPh | H | 4-MeS(O)Ph | 1 | 0 |
| 2-16 | 2-F-4-TfmPh | H | 4-Me$SO_2$Ph | 1 | 0 |
| 2-17 | 4-ClPh | H | 4-NCPh | 1 | 0 |
| 2-18 | 2,4-diFPh | H | 2-F-4-TfmPh | 1 | 0 |
| 2-19 | 2,4-diFPh | H | 4-$NO_2$Ph | 1 | 0 |
| 2-20 | 2,4-diClPh | H | 4-MocPh | 1 | 0 |
| 2-21 | 2,4-diFPh | Me | 4-ClPh | 0 | 0 |
| 2-22 | 2,4-diFPh | Me | 2,4-diFPh | 0 | 0 |
| 2-23 | 2,4-diFPh | Me | 4-TfmPh | 0 | 0 |
| 2-24 | 2,4-diFPh | Me | 4-TfmOPh | 0 | 0 |
| 2-25 | 2,4-diFPh | Me | 4-NCPh | 0 | 0 |
| 2-26 | 2,4-diClPh | Me | 4-ClPh | 0 | 0 |
| 2-27 | 2,4-diClPh | Me | 4-TfmPh | 0 | 0 |

Table 2 (cont)

| Cpd No. | Ar | $R^1$ | $R^2$ | p | q |
|---|---|---|---|---|---|
| 2-28 | 2,4-diClPh | Me | 4-NCPh | 0 | 0 |
| 2-29 | 2,4-diClPh | Me | 4-MeSPh | 0 | 0 |
| 2-30 | 2,4-diClPh | Me | 4-MeS(O)Ph | 0 | 0 |
| 2-31 | 2,4-diFPh | Me | 4-$MeSO_2$Ph | 0 | 0 |
| 2-32 | 2,4-diClPh | Me | 4-MocPh | 0 | 0 |
| 2-33 | 2,4-diClPh | Me | 4-$NO_2$Ph | 0 | 0 |
| 2-34 | 2,4-diClPh | Me | 4-$F_2$CHOPh | 0 | 0 |
| 2-35 | 2,4-diClPh | Me | 4-($F_3CCH_2O$)Ph | 0 | 0 |
| 2-36 | 2-F-4-TfmPh | Me | 4-TfmPh | 0 | 0 |
| 2-37 | 4-ClPh | Me | 4-FPh | 0 | 0 |
| 2-38 | 2,4-diFPh | Me | 4-ClPh | 1 | 0 |
| 2-39 | 2,4-diFPh | Me | 4-FPh | 1 | 0 |
| 2-40 | 2,4-diFPh | Me | 4-TfmPh | 1 | 0 |
| 2-41 | 2,4-diFPh | Me | 2-F-4-TfmPh | 1 | 0 |
| 2-42 | 2,4-diFPh | Me | 2,4-diFPh | 1 | 0 |
| 2-43 | 2,4-diFPh | Me | 4-NCPh | 1 | 0 |
| 2-44 | 2,4-diFPh | Me | 4-$F_2$CHOPh | 1 | 0 |
| 2-45 | 2,4-diFPh | Me | 4-MocPh | 1 | 0 |
| 2-46 | 2,4-diFPh | Me | 4-MeSPh | 1 | 0 |
| 2-47 | 2,4-diFPh | Me | 4-MeS(O)Ph | 1 | 0 |
| 2-48 | 2,4-diFPh | Me | 4-$EtSO_2$Ph | 1 | 0 |
| 2-49 | 2,4-diClPh | Me | 2,4-diClPh | 1 | 0 |
| 2-50 | 2,4-diClPh | Me | 4-FPh | 1 | 0 |
| 2-51 | 2,4-diClPh | Me | 4-TfmPh | 1 | 0 |
| 2-52 | 2,4-diClPh | Me | 4-TfmOPh | 1 | 0 |
| 2-53 | 2,4-diClPh | Me | 2-F-4-$F_2$CHOPh | 1 | 0 |
| 2-54 | 2,4-diClPh | Me | 2-Cl-4-NCPh | 1 | 0 |

Table 2 (cont)

| Cpd No. | Ar | $R^1$ | $R^2$ | p | q |
|---|---|---|---|---|---|
| 2-55 | 2,4-diCℓPh | Me | 4-$NO_2$Ph | 1 | 0 |
| 2-56 | 2,4-diCℓPh | Me | 3,4-diFPh | 1 | 0 |
| 2-57 | 2,4-diCℓPh | Me | 4-MocPh | 1 | 0 |
| 2-58 | 2,4-diCℓPh | Me | 3-F-4-TfmPh | 1 | 0 |
| 2-59 | 2,4-diCℓPh | Me | 4-MeSPh | 1 | 0 |
| 2-60 | 2,4-diCℓPh | Me | 4-MeS(O)Ph | 1 | 0 |
| 2-61 | 2,4-diCℓPh | Me | 4-$MeSO_2$Ph | 1 | 0 |
| 2-62 | 2,4-diCℓPh | Me | 4-(diCℓAcNH)Ph | 1 | 0 |
| 2-63 | 2,4-diCℓPh | Me | 5-Tfm-2-Fur | 1 | 0 |
| 2-64 | 2,4-diCℓPh | Me | 5-Cℓ-2-Thi | 0 | 0 |
| 2-65 | 2,4-diFPh | Me | 5-Tfm-2-Thi | 1 | 0 |
| 2-66 | 2,4-diFPh | Me | 5-Tfm-2-Thiz | 1 | 0 |
| 2-67 | 2,4-diCℓPh | Me | 2-Tfm-5-Thiz | 1 | 0 |
| 2-68 | 2,4-diFPh | Me | 6-Cℓ-3-Pyr | 1 | 0 |
| 2-69 | 2,4-diCℓPh | Me | 6-Tfm-3-Pyr | 0 | 0 |
| 2-70 | 2,4-diCℓPh | Me | 6-Tfm-3-Pyr | 1 | 0 |
| 2-71 | 2,4-diFPh | Me | 6-TfmO-3-Pyr | 1 | 0 |
| 2-72 | 2,4-diCℓPh | Me | 5-Tfm-2-Pyr | 1 | 0 |
| 2-73 | 2,4-diCℓPh | Me | 5-Cℓ-2-Pym | 1 | 0 |
| 2-74 | 2,4-diFPh | Me | 5-Tfm-2-Pym | 1 | 0 |
| 2-75 | 2,4-diCℓPh | Me | 2-TfmO-5-Pym | 1 | 0 |
| 2-76 | 4-FPh | Me | 4-TfmPh | 1 | 0 |
| 2-77 | 4-CℓPh | Me | 4-TfmPh | 1 | 0 |
| 2-78 | 4-CℓPh | Me | 3-TfmPh | 1 | 0 |
| 2-79 | 4-CℓPh | Me | 4-CℓPh | 1 | 0 |
| 2-80 | 4-CℓPh | Me | 2-F-4-TfmPh | 1 | 0 |
| 2-81 | 4-CℓPh | Me | 3,4-diFPh | 1 | 0 |

Table 2 (cont)

| Cpd No. | Ar | $R^1$ | $R^2$ | p | q |
|---|---|---|---|---|---|
| 2-82 | 4-CℓPh | Me | 3,5-diFPh | 1 | 0 |
| 2-83 | 4-CℓPh | Me | 4-CℓPh | 1 | 1 |
| 2-84 | 4-CℓPh | Me | 4-TfmPh | 1 | 1 |
| 2-85 | 4-CℓPh | Me | 3-TfmPh | 1 | 1 |
| 2-86 | 4-CℓPh | Me | 2-F-4-TfmPh | 1 | 1 |
| 2-87 | 4-CℓPh | Me | 4-NCPh | 1 | 1 |
| 2-88 | 4-CℓPh | Me | 4-$NO_2$Ph | 1 | 1 |
| 2-89 | 4-CℓPh | Me | 6-Cℓ-3-Pyr | 1 | 1 |
| 2-90 | 4-CℓPh | Me | 5-Tfm-2-Thi | 1 | 1 |
| 2-91 | 4-CℓPh | Me | 5-Tfm-2-Thiz | 1 | 1 |
| 2-92 | 4-CℓPh | Me | 5-Cℓ-2-Pym | 1 | 1 |
| 2-93 | 2,4-diCℓPh | Me | 4-CℓPh | 1 | 1 |
| 2-94 | 2,4-diCℓPh | Me | 4-TfmPh | 1 | 1 |
| 2-95 | 2,4-diCℓPh | Me | 3-TfmPh | 1 | 1 |
| 2-96 | 2,4-diCℓPh | Me | 3,4-diFPh | 1 | 1 |
| 2-97 | 2,4-diCℓPh | Me | 2-F-4-TfmPh | 1 | 1 |
| 2-98 | 2,4-diCℓPh | Me | 5-Tfm-2-Fur | 1 | 1 |
| 2-99 | 2,4-diFPh | Me | 4-CℓPh | 1 | 1 |
| 2-100 | 2,4-diFPh | Me | 4-TfmPh | 1 | 1 |
| 2-101 | 2,4-diFPh | Me | 3-TfmPh | 1 | 1 |
| 2-102 | 2,4-diFPh | Me | 2-F-4-TfmPh | 1 | 1 |
| 2-103 | 2,4-diFPh | Me | 3-F-4-TfmPh | 1 | 1 |
| 2-104 | 2,4-diFPh | Me | 6-Cℓ-3-Pyr | 1 | 1 |
| 2-105 | 2,4-diFPh | Me | 6-Tfm-3-Pyr | 1 | 1 |
| 2-106 | 2,4-diFPh | Me | 5-Tfm-2-Thi | 1 | 1 |
| 2-107 | 4-TfmPh | Me | 3-CℓPh | 1 | 1 |
| 2-108 | 2-F-4-TfmPh | Me | 3-TfmPh | 1 | 1 |

Of the compounds listed above, the following are preferred, that is to say Compounds No. 1-2, 1-5, 1-20, 1-23, 1-24, 1-25, 1-26, 1-27, 1-32, 1-66, 1-68, 1-89, 1-117, 1-124, 1-127, 1-145, 1-154, 1-168, 1-179, 1-180, 1-181, 1-182, 1-183, 1-187, 1-189, 1-190, 1-191, 1-193, 2-40, 2-41, 2-80, 2-99 and 2-100. The following are more preferred, especially for agrochemical use, that is Compounds No. 1-2, 1-5, 1-23, 1-25, 1-26, 1-27, 1-127, 1-145, 1-154, 1-168, 1-179, 1-180, 1-181, 1-182, 1-183, 1-187, 1-189, 1-190, 1-191, 1-193, 1-228, 1-229, 1-230, 1-231, 1-232, 1-233, 1-234 and 1-235, and the following are more preferred, especially for pharmaceutical use, that is Compounds No. 1-2, 1-20, 1-24, 1-26, 1-32, 1-66, 1-68, 1-89, 1-117, 1-124, 1-127, 2-40, 2-41, 2-80, 2-99 and 2-100.

The most preferred compounds for agrochemical use are:

1-5. 2-(2,4-Dichlorophenyl)-1-(1H-1,2,4-triazol-1-yl)-3-[4-(trifluoromethyl)benzoylamino]-2-butanol;
1-26. 2-(2,4-Difluorophenyl)-3-[2-fluoro-4-(trifluoromethyl)benzoylamino]-1-(1H-1,2,4-triazol-1-yl)-2-butanol;
1-145. 3-(4-Chlorobenzoylamino)-2-(2,4-difluorophenyl)-1-(1H-1,2,4-triazol-1-yl)-2-pentanol;
1-181. 3-Azido-2-(2,4-difluorophenyl)-1-(1H-1,2,4-triazol-1-yl)-2-butanol;
1-182. 3-Azido-2-(4-chloro-2-fluorophenyl)-1-(1H-1,2,4-triazol-1-yl)-2-butanol;
1-187. 3-Azido-2-(2,4-difluorophenyl)-1-(1H-1,2,4-triazol-1-yl)-2-pentanol;
1-189. 3-Azido-2-(2-chloro-4-fluorophenyl)-1-(1H-1,2,4-triazol-1-yl)-2-pentanol;
1-190. 4-Azido-2-(2,4-difluorophenyl)-3-methyl-1-(1H-1,2,4-triazol-1-yl)-2-pentanol;
1-191. 4-Azido-2-(2,4-difluorophenyl)-3-methyl-1-(1H-1,2,4-triazol-1-yl)-2-butanol;
1-193. 4-Azido-2-(2,4-dichlorophenyl)-3-methyl-1-(1H-1,2,4-triazol-1-yl)-2-butanol; and
1-235. 3-Azido-2-(2,4-dichlorophenyl)-1-(1H-1,2,4-triazol-1-yl)-2-pentanol.

The most preferred compounds for pharmaceutical use are:

1-20. 2-(4-Chlorophenyl)-1-(1H-1,2,4-triazol-1-yl)-3-[4-(trifluoromethyl)benzoylamino]-2-butanol;
1-24. 2-(2,4-Difluorophenyl)-1-(1H-1,2,4-triazol-1-yl)-3-[4-(trifluoromethyl)benzoylamino]-2-butanol;
1-26. 2-(2,4-Difluorophenyl)-3-[2-fluoro-4-(trifluoromethyl)benzoylamino]-1-(1H-1,2,4-triazol-1-yl)-2-butanol;
1-32. 3-(4-Cyanobenzoylamino)-2-(2,4-difluorophenyl)-1-(1H-1,2,4-triazol-1-yl)-2-butanol;
1-68. 2-(4-Chlorophenyl)-3-[2-fluoro-4-(trifluoromethyl)benzoylamino]-1-(1H-1,2,4-triazol-1-yl)-2-butanol;
1-89. 2-(2,4-Dichlorophenyl)-1-(1H-1,2,4-triazol-1-yl)-3-[4-(trifluoromethyl)benzoylthio]-2-butanol;
1-117. 3-(4-Chlorocinnamoylamino)-2-(2,4-difluorophenyl)-1-(1H-1,2,4-triazol-1-yl)-2-butanol;
1-124. 2-(2,4-Difluorophenyl)-1-(1H-1,2,4-triazol-1-yl)-3-{N-methyl-N-[4-(trifluoromethyl)benzoyl]amino}-2-butanol; and
1-127. 2-(2,4-Difluorophenyl)-3-{N-[2-fluoro-4-(trifluoromethyl)benzoyl]-N-methylamino}-1-(1H-1,2,4-triazol-1-yl)-2-butanol.

Also preferred are the acid addition salts, especially the oxalates, nitrates and hydrochlorides of the above compounds.

The compounds of the present invention may be prepared by a variety of methods known for the preparation of this type of compound. In general, the precise details of the method chosen for their preparation will vary depending on the nature of the compound. Examples of preferred preparative methods are illustrated in the following Methods A to L.

Compounds of formula (I) in which -Y-$R^2$ represents a protected hydroxy group may be prepared by protecting the hydroxy group of a compound of formula (III):

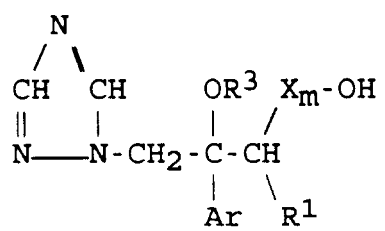

(III)

(in which $R^1$, $R^3$, Ar, X and m are as defined above).

The reaction employed will, of course, depend on the nature of the protecting group, and such reactions are well known to those skilled in the art. Thus, reactions for introducing protecting groups are described, for example, by T.W. Greene, "Protective Groups in Organic Synthesis", published by Wiley-Interscience Publications, especially at Chapter 2, "Protection of the Hydroxy Group, including 1,2- and 1,3-diols", the disclosure of which is incorporated herein by reference.

## Method A

Those compounds of formula (I) in which Y represents a group of formula -O-CO- or -O-CO-CH=CH-, that is to say compounds of formula (Ia), shown below, can, for example, be prepared by reacting a compound of formula (IV):

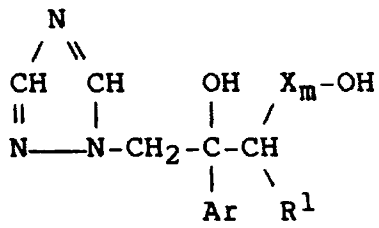

(IV)

(in which $R^1$, Ar, X and m are as defined above) with a carboxylic acid of formula (V):

$R^{2'}$-COOH (V)

(in which $R^{2'}$ represents any of the groups defined above for $R^2$ or represents a group of formula $R^2$-CH=CH-, and $R^2$ is as defined above) or with a reactive derivative thereof, to give said compound of formula (Ia):

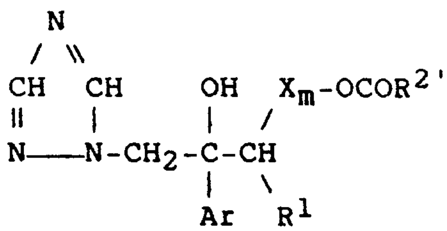

(Ia)

(in which $R^1$, $R^{2'}$, Ar, X and m are as defined above).

The reaction of the diol compound of formula (IV) with the carboxylic acid of formula (V) or with the reactive derivative thereof is preferably effected in an inert solvent. There is no particular restriction on the nature of the solvent to be employed, provided that it has no adverse effect on the reaction or on the reagents involved. Examples of suitable solvents include: hydrocarbons, especially aromatic hydrocarbons, such as benzene; halogenated hydrocarbons, especially halogenated aliphatic hydrocarbons, such as methylene chloride; and ethers, such as tetrahydrofuran. Where the acid itself is employed, we prefer to use an appropriate condensing agent such as dicyclohexylcarbodiimide in the presence of a basic catalyst, preferably an amine, such as pyridine, triethylamine or 4-(N,N-dimethylamino)pyridine. Alternatively, the compound of formula (Ia) can be prepared by reacting an acid chloride, which can be prepared by reacting the carboxylic acid of formula (V) with a halogenating agent, such as thionyl chloride, with the compound of formula (IV) in the presence of a base. The reaction can take place over a wide range of temperatures, and the precise reaction temperature is not critical to the invention. In general, we find it convenient to carry out the reaction at a temperature from -20°C to ambient. The time required for the reaction may also vary widely, depending on many factors, notably the reaction temperature and the nature of the reagents. However, provided that the reaction is effected under the preferred conditions outlined above, a period of from 10 minutes to 1 hour will usually suffice.

Method B

Those compounds of formula (I) in which Y represents a sulphur atom and m is 0, that is to say compounds of formula (Ib), can, for example, be prepared as illustrated in the following Reaction Scheme B:

Reaction Scheme B:

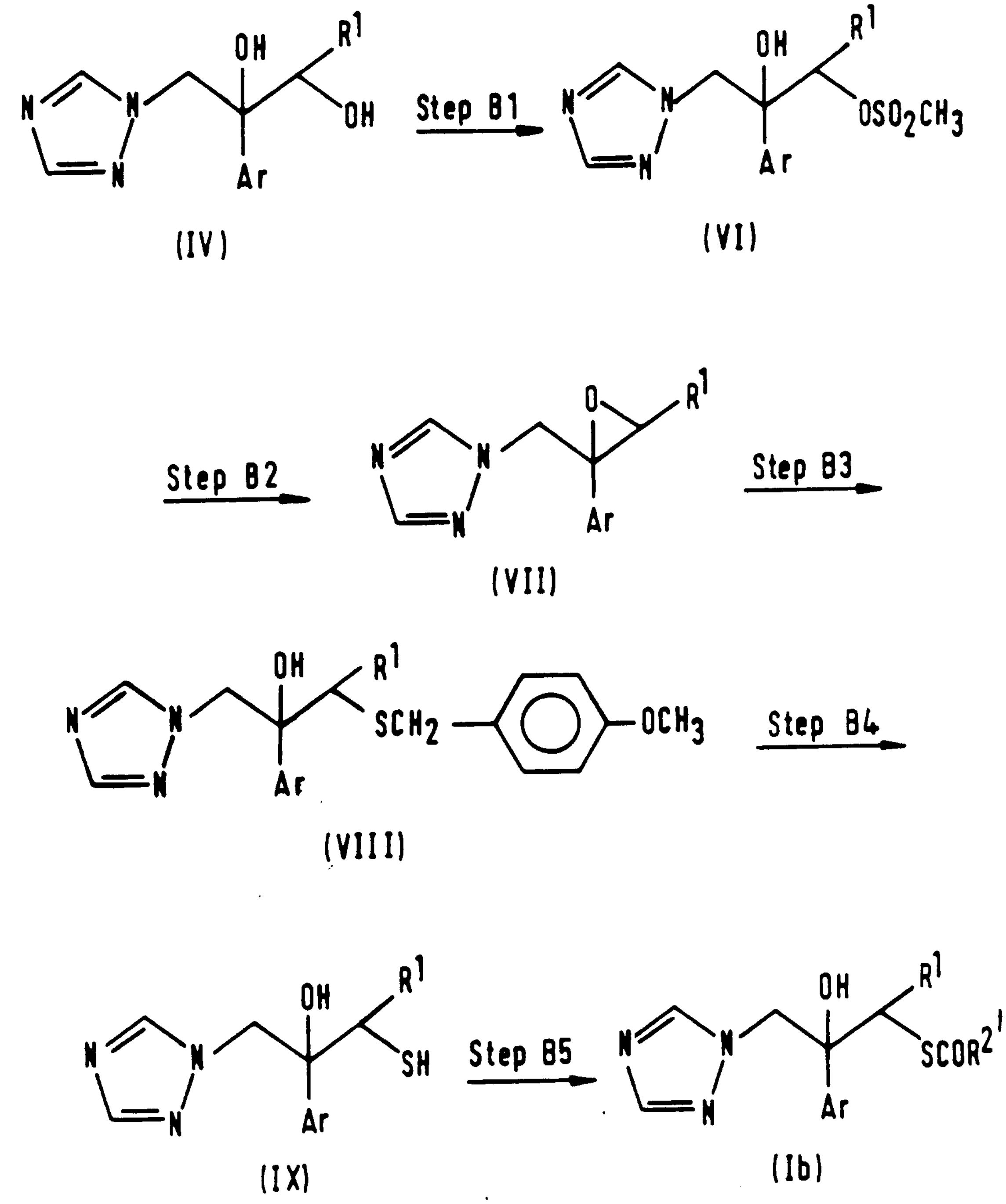


(IV) (VI) (VII) (VIII) (IX) (Ib)

In the above formulae, $R^1$, $R^{2'}$ and Ar are as defined above.

Thus, in Step B1 of this reaction scheme, a methanesulphonate of formula (VI) is prepared by reacting a diol compound of formula (IV) with methanesulphonyl chloride in an inert solvent in the presence of a base. There is no particular restriction on the nature of the solvent to be employed, provided that it has no adverse effect on the reaction or on the reagents involved. Examples of suitable solvents include: halogenated hydrocarbons, especially halogenated aliphatic hydrocarbons, such as methylene chloride; and nitriles, such as acetonitrile. There is likewise no particular limitation on the base to be employed, as its function is essentially to bind to the hydrogen chloride formed during the reaction, but examples of suitable bases include such amines as triethylamine and pyridine.

The reaction can take place over a wide range of temperatures, and the precise reaction temperature is not critical to the invention. In general, we find it convenient to carry out the reaction at a temperature from

-20°C to 50°C. The time required for the reaction may also vary widely, depending on many factors, notably the reaction temperature and the nature of the reagents. However, provided that the reaction is effected under the preferred conditions outlined above, a period of from 8 minutes to 3 hours will usually suffice.

In Step B2, the resulting methanesulphonate of formula (VI) is reacted with a base to remove the methanesulphonyloxy group and the adjacent hydroxy group as methanesulphonic acid, and produce the oxirane of formula (VII). There is no particular restriction on the base employed in this reaction, provided that it does not affect other parts of the molecule. Examples include such basic alkali metal compounds as hydroxides and hydrides, e.g. sodium hydroxide or sodium hydride. There is no particular restriction on the nature of the solvent to be employed, provided that it has no adverse effect on the reaction or on the reagents involved. Examples of suitable solvents include: ethers such as tetrahydrofuran; fatty acid amides, such as dimethylformamide; and sulphoxides, such as dimethyl sulphoxide.

In Step B3, the oxirane of formula (VII) is reacted with sodium p-methoxybenzylthiolate to prepare the thioether derivative of formula (VIII). The reaction is preferably carried out by heating a solution of sodium p-methoxybenzylthiolate (which may be produced by mixing equimolar amounts of sodium hydride and p-methoxybenzylmercaptan) in a suitable solvent with the oxirane of formula (VII). There is no particular restriction on the nature of the solvent to be employed, provided that it has no adverse effect on the reaction or on the reagents involved. Examples of suitable solvents include: ethers, such as tetrahydrofuran; fatty acid amides, such as dimethylformamide; and sulphoxides, such as dimethyl sulphoxide. The reaction can take place over a wide range of temperatures, and the precise reaction temperature is not critical to the invention. In general, we find it convenient to carry out the reaction at a temperature from 40 to 100°C. The time required for the reaction may also vary widely, depending on many factors, notably the reaction temperature and the nature of the reagents. However, provided that the reaction is effected under the preferred conditions outlined above, a period of from 30 minutes to 5 hours will usually suffice. We prefer to employ from 1 to 3 moles of sodium p-methoxybenzylthiolate for each mole of the compound of formula (VII). The thioether compound of formula (VIII) can be produced without isolation of the oxirane intermediate of formula (VII) when more than two moles of sodium p-methoxybenzylthiolate are employed for each mole of the methanesulphonate of formula (VI).

In Step B4, a mercaptan derivative of formula (IX) is prepared from the thioether derivative of formula (VIII) by contacting the thioether derivative of formula (VIII) with a strong acid to remove the p-methoxybenzyl group. The reaction may be effected by treating the compound of formula (VIII) with trifluoromethanesulphonic acid in trifluoroacetic acid in the presence of a cation scavenger, such as anisole. The reaction can take place over a wide range of temperatures, and the precise reaction temperature is not critical to the invention. In general, we find it convenient to carry out the reaction at a temperature from -20 to 50°C. The time required for the reaction may also vary widely, depending on many factors, notably the reaction temperature and the nature of the reagents. However, provided that the reaction is effected under the preferred conditions outlined above, a period of from 10 minutes to 3 hours will usually suffice, but it is usually complete at room temperature within 30 minutes.

In Step B5, a thioester compound of formula (Ib) is prepared from the mercaptan compound of formula (IX). This reaction can be carried out by reacting the compound of formula (IX) with a carboxylic acid of formula (V):

$R^{2'}$-COOH (V)

(in which $R^{2'}$ is as defined above) in an inert solvent in the presence of a basic catalyst, e.g. an organic amine, such as pyridine, triethylamine or 4-(N,N-dimethylamino)pyridine. There is no particular restriction on the nature of the solvent to be employed, provided that it has no adverse effect on the reaction or on the reagents involved. Examples of suitable solvents include: aromatic hydrocarbons, such as benzene; halogenated hydrocarbons, particularly halogenated aliphatic hydrocarbons, such as methylene chloride; and ethers, such as tetrahydrofuran. When the carboxylic acid itself is employed, we prefer also to use an appropriate condensing agent, for example, dicyclohexylcarbodiimide. The thioester compound of formula (Ib) can also be prepared by reacting the compound of formula (IX) with an acid chloride, which may be produced by reaction of the compound of formula (V) with thionyl chloride, in the presence of a base.

Method C:

Compounds of formula (I) in which -Y-$R^2$ represents an azido group and m is 0, that is to say compounds of formula (Ic), and compounds of formula (I) in which Y represents a group of formula -NHCO-and m is 0, that is to say compounds of formula (Id), may be prepared, for example, as shown in the following Reaction Scheme C:

Reaction Scheme C:

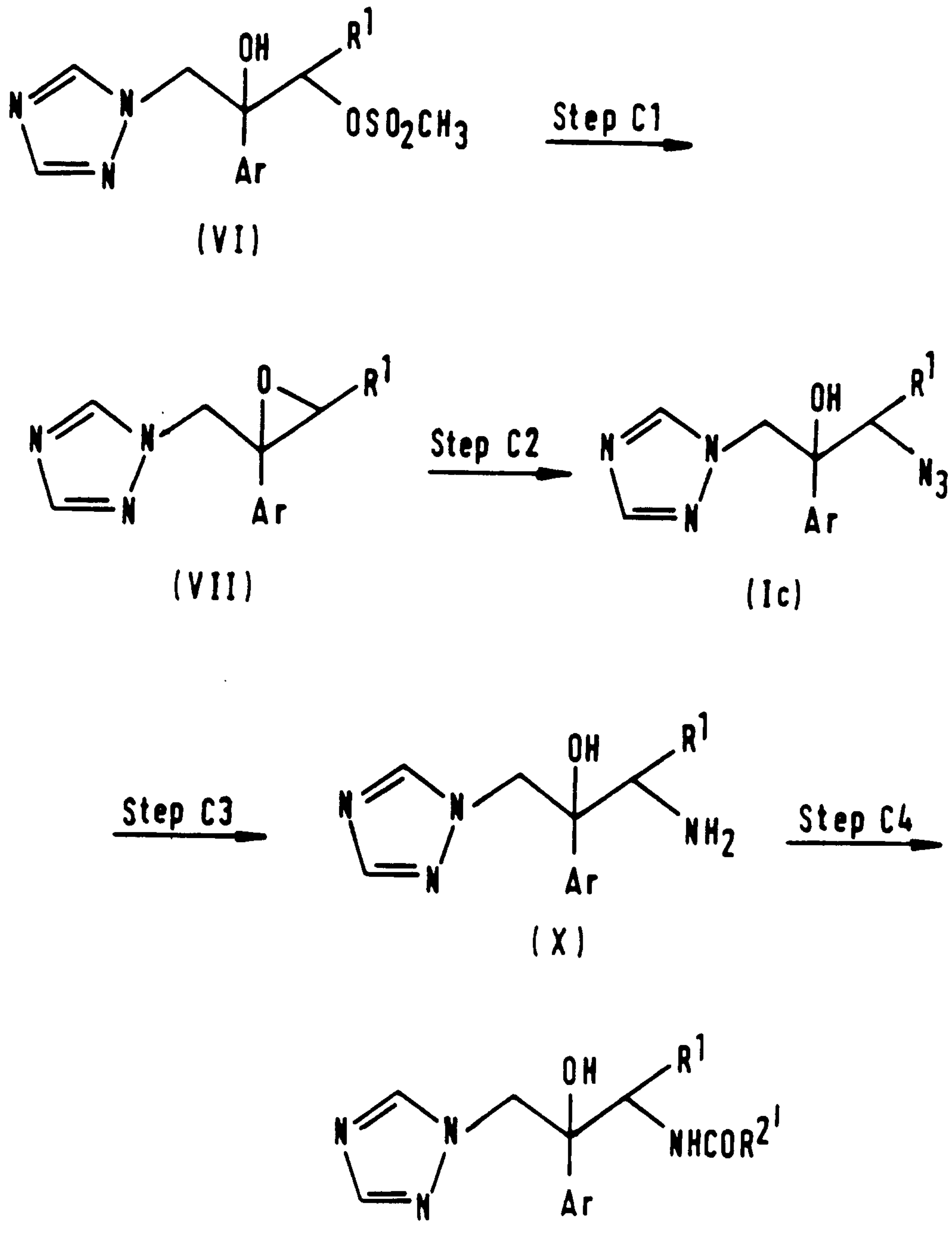


(VI) (VII) (Ic) (X) (Id)

In the above formulae, $R^1$, $R^{2'}$ and Ar are as defined above.

In this reaction scheme, the methanesulphonate derivative of formula (VI) described in Step B1 above is reacted with sodium azide or lithium azide to prepare the azido-alcohol derivative of formula (Ic) via an oxirane intermediate of formula (VII). The amide compound of formula (Id) can then, if required, be prepared by reduction of the azide compound of formula (Ic) to afford an aminoalcohol derivative of formula (X), which is then reacted with a carboxylic acid of formula (V) or reactive derivative thereof.

In Steps C1 and C2, in which a compound of formula (Ic) is prepared from a methanesulphonate compound of formula (VI) via an oxirane compound of formula (VII), the reaction is preferably effected in the presence of a solvent. There is no particular restriction on the nature of the solvent to be employed, provided that it has no adverse effect on the reaction or on the reagents involved. Examples of suitable solvents include: polar solvents, especially fatty acid amides, such as dimethylformamide or dimethylacetamide; and sulphoxides, such as dimethyl sulphoxide. The reaction can take place over a wide range of temperatures, and the precise reaction temperature is not critical to the invention. In general, we find it convenient to carry out the reaction at a temperature from room temperature to the boiling point of the solvent employed and more preferably at a temperature of from 70 to 130 °C. The time required for the reaction may also vary widely, depending on many factors, notably the reaction temperature and the nature of the reagents. However, provided that the reaction is effected under the preferred conditions outlined above, a period of from 1 to 15 hours will usually suffice. We prefer to employ from 2 to 5, more preferably from 2 to 3, moles of sodium azide or lithium azide for each mole of the compound of formula (VI). The reaction is effected in the presence of an ammonium halide, preferably ammonium chloride, more preferably from about 1 to 1.5 moles of the ammonium halide per mole of said compound of formula (VI). If the reaction is discontinued shortly after its initiation, or if only 1 mole of the azide is employed or if an oxirane intermediate of formula (VII) can be recovered, this oxirane intermediate may be reacted again with sodium azide or lithium azide in the presence of ammonium chloride to give the compound of formula (Ic).

Step C3, in which the compound of formula (X) is prepared from the compound of formula (Ic), can be, and usually is, carried out by catalytic reduction in the presence of hydrogen. Any catalyst commonly used for this type of reaction can be employed in the reaction, and examples include palladium-on-charcoal, platinum or Raney nickel. The reaction is normally effected in the presence of a solvent. There is no particular restriction on the nature of the solvent to be employed, provided that it has no adverse effect on the reaction or on the reagents involved. Examples of suitable solvents include: alcohols, such as methanol or ethanol; esters, such as ethyl acetate; and ethers, such as tetrahydrofuran. The reaction can take place over a wide range of temperatures, and the precise reaction temperature is not critical to the invention. In general, we find it convenient to carry out the reaction at about room temperature. The time required for the reaction may also vary widely, depending on many factors, notably the reaction temperature and the nature of the reagents. However, provided that the reaction is effected under the preferred conditions outlined above, a period of from 30 minutes to 5 hours, preferably from 30 minutes to 2 hours, will usually suffice. Alternatively, the compound of formula (Ic) may be reacted with hydrogen sulphide or 1,3-propanedithiol in a solvent (for example methylene chloride) and in the presence of an organic base, such as triethylamine.

In Step C4, the amide compound of formula (Id) is prepared by reacting the compound of formula (X) with a carboxylic acid of formula (V) or a reactive derivative thereof. This reaction is essentially the same as, and may be carried out under the same conditions as, that described above in Method A.

Those compounds of formula (I) in which Y represents a group of formula $-N(R^5)CO-$ or $-N(R^5)CO-CH=CH-$, where $R^5$ represents an alkyl group, and m is 0, that is to say compounds of formula (Ie), can be prepared, for example, as shown in the following Reaction Scheme D:

Reaction Scheme D:

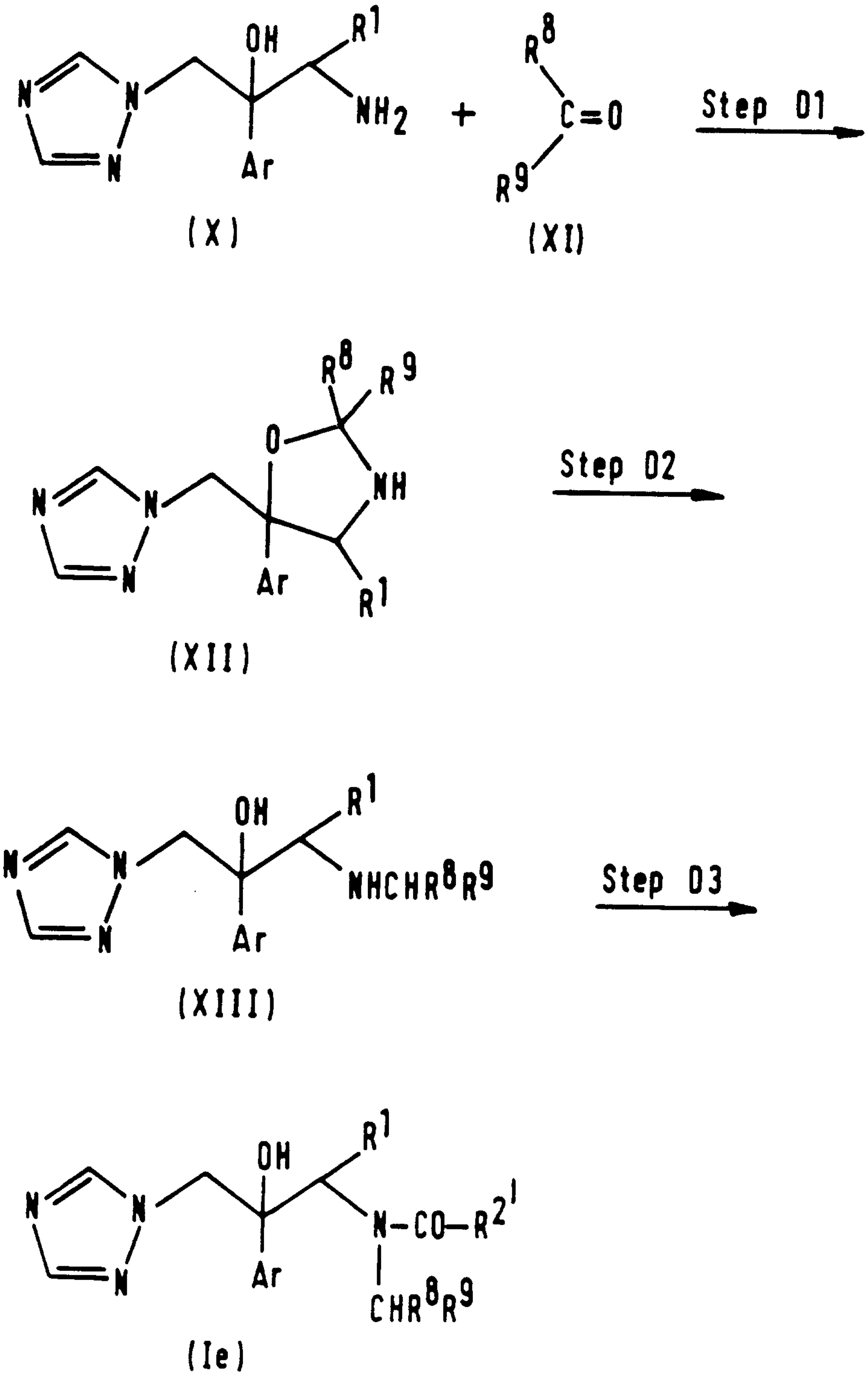


(X) (XI) (XII) (XIII) (Ie)

In the above formulae:

$R^1$, $R^{2'}$ and Ar are as defined above; and

$R^8$ and $R^9$ are the same or different and each represents a hydrogen atom or a methyl or ethyl group.

In this reaction scheme, the N-alkylamide compound of formula (Ie) may be prepared by reacting the amino-alcohol compound of formula (X) (described in Step C3 of Method C) with an aldehyde or ketone compound of formula (XI) to produce a cyclic oxazolidine compound of formula (XII) (Step D1), which is reduced to cause ring opening and thereby afford an N-alkylamino compound of formula (XIII) (Step D2),

and then reacting the resulting compound of formula (XIII) with a carboxylic acid of formula (V) or a reactive derivative thereof, if necessary, in the presence of an appropriate condensing agent (Step D3).

In Step D1, an oxazolidine compound of formula (XII) is prepared by reacting an amino-alcohol compound of formula (X) with an aldehyde or ketone compound of formula (XI). Examples of suitable aldehyde or ketone compounds include formaldehyde, acetaldehyde and propionaldehyde, but the exact nature of this compound will depend on the final compound which it is desired to prepare. We prefer to employ from 1 to 1.5 moles of the carbonyl compound of formula (XI) per mole of the compound of formula (X). The reaction is preferably effected with heating and preferably in the presence of a solvent. There is no particular restriction on the nature of the solvent, if it is employed, provided that it has no adverse effect on the reaction or on the reagents involved. Examples of suitable solvents include: aromatic hydrocarbons, such as benzene or toluene. The reaction can take place over a wide range of temperatures, and the precise reaction temperature is not critical to the invention. In general, we find it convenient to carry out the reaction at a temperature from 50 °C to the boiling point of the solvent employed. The time required for the reaction may also vary widely, depending on many factors, notably the reaction temperature and the nature of the reagents. However, provided that the reaction is effected under the preferred conditions outlined above, a period of from 30 minutes to 3 hours will usually suffice.

In Step D2, a compound of formula (XIII) is prepared from the cyclic oxazolidine compound of formula (XII) by reacting the compound of formula (XII) with an excess of lithium aluminium hydride in an inert solvent. There is no particular restriction on the nature of the solvent to be employed, provided that it has no adverse effect on the reaction or on the reagents involved. Examples of suitable solvents include: ethers, such as diethyl ether, tetrahydrofuran or dioxane.

In Step D3, the resulting amino-alcohol of formula (XIII) is subjected to acylation of its amino group using a carboxylic acid of formula (V) or a reactive derivative thereof. This reaction is essentially the same as, and may be carried out under the same conditions as, that described above in Method A.

The compound of formula (IV) used as the starting material in the above reaction schemes may readily be prepared by a known method [see the Abstract Papers of the 8th Symposium on Medicinal Chemistry, Osaka, page 9 (1986)].

The resulting compounds prepared by the processes described above will normally be racemic mixtures. These may, if desired, be employed as such. Alternatively, optical resolution may be carried out by addition of an optically active acid, such as ℓ-camphorsulphonic acid to the racemate of formula (I), (IV), (VI) - (X), (XII) or (XIII) to form a crystalline salt, followed by separation of the optically active compound.

Method E:

Those compounds of formula (I) in which $R^3$ and and $-X_m-Y-R^2$ together represent a group of formula (II):

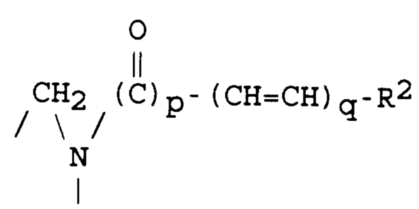

(II)

(in which $R^2$ is as defined above, and p and q are both 0), that is to say compounds of formula (If), can be prepared, for example, by the method shown in the following Reaction Scheme E:

## Reaction Scheme E:

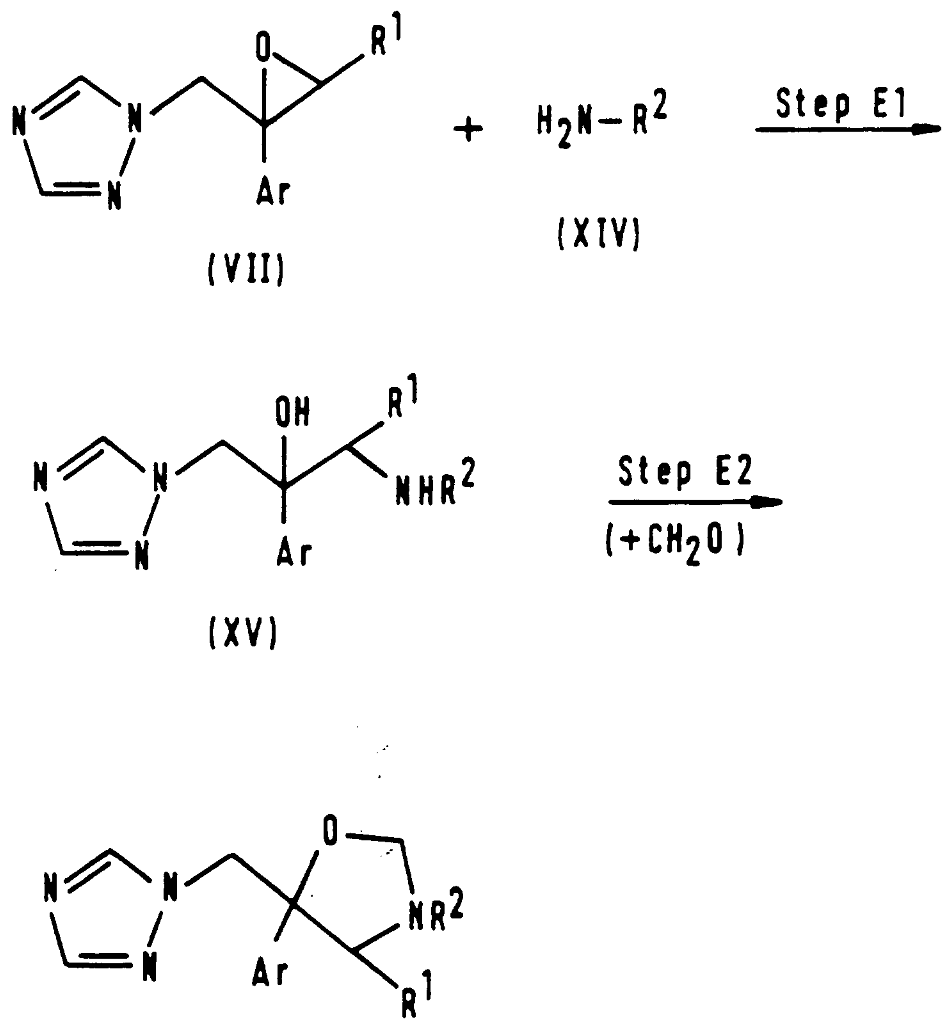


(VII) (XIV) (XV)

(If)

In the above formulae, $R^1$, $R^2$ and Ar are as defined above.

Thus, an oxazolidine compound of formula (If) can be prepared by reacting an oxirane compound of formula (VII) with an amino compound of formula (XIV) and then reacting the resulting amino-alcohol of formula (XV) with formaldehyde.

In Step E1, a compound of formula (XV) is prepared by reacting the oxirane compound of formula (VII) with the amino compound of formula (XIV). This reaction is preferably carried out in the presence of an inert solvent. There is no particular restriction on the nature of the solvent to be employed, provided that it has no adverse effect on the reaction or on the reagents involved. Examples of suitable solvents include: aromatic hydrocarbons, such as benzene, toluene or xylene. It may also be conducted in the absence of a solvent, if required. The reaction can take place over a wide range of temperatures, and the precise reaction temperature is not critical to the invention. In general, we find it convenient to carry out the reaction at a temperature from 70° to 150°C. The time required for the reaction may also vary widely, depending on many factors, notably the reaction temperature and the nature of the reagents. We prefer to employ from 1 to 5 moles of the amino compound of formula (XIV) for each mole of the oxirane compound of formula (VII).

In Step E2, the oxazolidine compound of formula (If) is prepared by reacting the amino-alcohol compound of formula (XV) with formaldehyde. The reaction is preferably effected in the presence of a solvent. There is no particular restriction on the nature of the solvent to be employed, provided that it has no adverse effect on the reaction or on the reagents involved. Examples of suitable solvents include: aromatic hydrocarbons, such as benzene or toluene, but others may also be used. The formaldehyde

employed in the reaction is preferably used in an amount of from 1 to 3 moles, more preferably from 1 to 1.5 moles, for each mole of the compound of formula (XV). The formaldehyde may be employed as such or, more preferably, as formalin or paraformaldehyde. The reaction can take place over a wide range of temperatures, and the precise reaction temperature is not critical to the invention. In general, we find it convenient to carry out the reaction at a temperature from 50 °C to boiling point of the solvent employed, and water produced in the reaction can be removed azeotropically. The time required for the reaction may also vary widely, depending on many factors, notably the reaction temperature and the nature of the reagents. However, provided that the reaction is effected under the preferred conditions outlined above, a period of from 30 minutes to 3 hours will usually suffice.

Method F:

Those N-acyloxazolidine compounds of formula (I) in which $R^3$ and and $-X_m-Y-R^2$ together represent a group of formula (II), defined above, (in which: $R^2$ is as defined above; p is 1; and q is 0 or 1), that is to say compounds of formula (Ig), can be prepared, for example, by the method shown in the following Reaction Scheme F:

**Reaction Scheme F:**

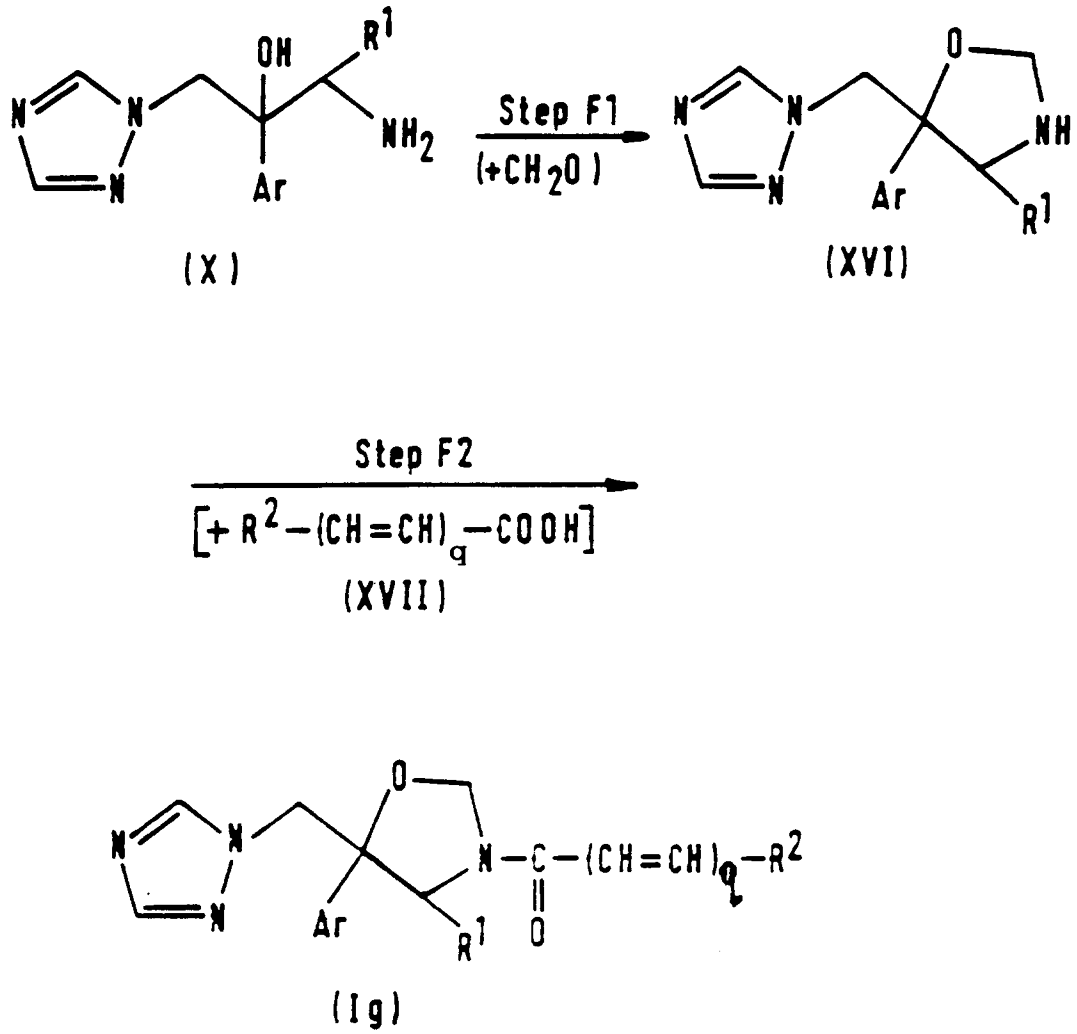


(X) (XVI) (Ig)

In the above formulae, $R^1$, $R^2$, q and Ar are as defined above.

The starting material for this reaction scheme is the compound of formula (X), which may have been prepared as described in Step C3 of Reaction Scheme C. This amino-alcohol compound of formula (X) is then condensed with formaldehyde, to give the cyclic compound of formula (XVI), which is then reacted with a carboxylic acid of formula (XVII), or a reactive derivative thereof, if necessary, in the presence of an appropriate condensing agent.

In Step F1 of this reaction scheme, formaldehyde is reacted with the amino-alcohol compound of formula (X), to prepare the oxazolidine of formula (XVI). The reaction is essentially the same as, and may be carried out under the same conditions as, that described above as Step E2 of Reaction Scheme E.

Reaction of the resulting compound of formula (XVI) with the carboxylic acid of formula (XVII) or reactive derivative thereof, in Step F2, gives an N-acyloxazolidine compound of formula (Ig). This reaction is preferably carried out in the presence of a condensing agent such as dicyclohexylcarbodiimide. We prefer to employ from 1 to 2 moles of the carboxylic acid of formula (XVII) and of the condensing agent for each mole of the compound of formula (XVI). The reaction is preferably effected in the presence of a solvent. There is no particular restriction on the nature of the solvent to be employed, provided that it has no adverse effect on the reaction or on the reagents involved. Examples of suitable solvents include: aromatic hydrocarbons, such as benzene; ethers, such as tetrahydrofuran; and halogenated hydrocarbons, especially halogenated aliphatic hydrocarbons, such as methylene chloride. The reaction can take place over a wide range of temperatures, and the precise reaction temperature is not critical to the invention. In general, we find it convenient to carry out the reaction at a temperature from -20°C to boiling point of the solvent employed, preferably 0°C to room temperature. The time required for the reaction may also vary widely, depending on many factors, notably the reaction temperature and the nature of the reagents.

Alternatively, the compound of formula (Ig) can be prepared by reacting the compound of formula (XVI) with an acid chloride, which may be prepared by reacting the carboxylic acid of formula (XVII) with thionyl chloride or phosphorus pentachloride.

Where benzene, tetrahydrofuran, methylene chloride or the like is used as the solvent for either of the alternative reactions of Step F2, the reaction is preferably carried out in the presence of a base, preferably an organic amine, such as triethylamine. Pyridine can be also used as a solvent in the reaction, in which case an additional amine is not necessary.

Those compounds of formula (I) in which $R^2$ represents a phenyl or aromatic heterocyclic group having a $-SO_rR^7$ substituent (r and $R^7$ are as defined above, other than r is 0) can be prepared by oxidation of the corresponding compound in which $R^2$ represents a phenyl or aromatic heterocyclic group having a corresponding $-SR^7$ substituent, using a peracid (for example, peracetic acid or 3-chloroperbenzoic acid) according to conventional means.

Those compounds of formula (I) in which the group of formula $-Y-R^2$ represents a group of formula $-OSO_2R^4$ can be prepared by a reaction analogous to that described above in Step B1 of Reaction Scheme B, but employing an alternative substituted sulphonyl halide, especially chloride, in which the substituent corresponds to the group $R^4$, in place of the methanesulphonyl chloride.

Method G:

Compounds of formula (I) in which m is 1 can be prepared as illustrated by the following Reaction Scheme G:

Reaction Scheme 6:

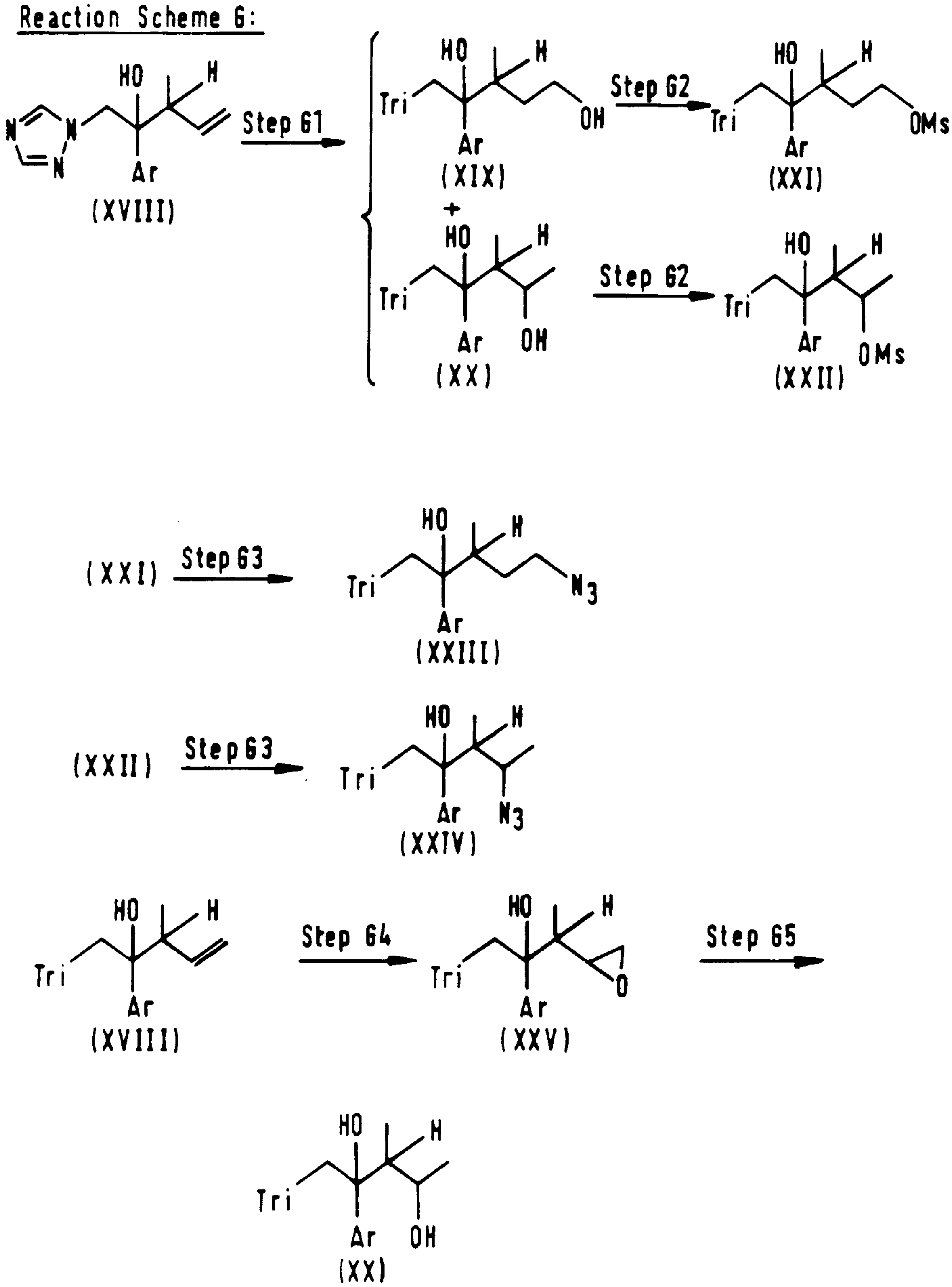


(XVIII) (XIX) + (XX) (XXI) (XXII)

(XXI) (XXIII)

(XXII) (XXIV)

(XVIII) (XXV)

(XX)

## Reaction Scheme G (cont.)

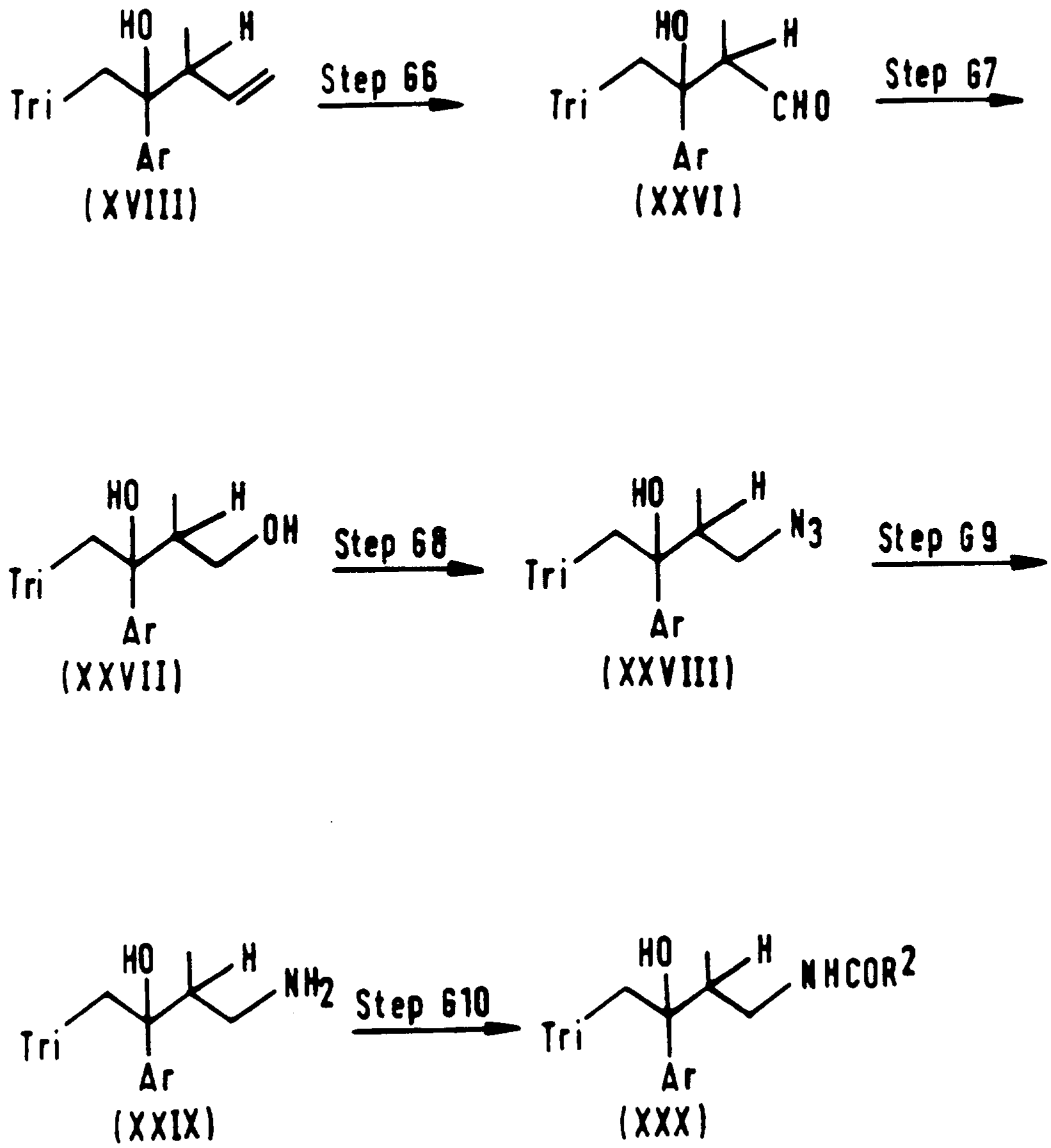


(XVIII) (XXVI) (XXVII) (XXVIII) (XXIX) (XXX)

In the above formulae:
Tri represents the 1H-1,2,4-triazol-1-yl group;
Ms represents the methanesulphonyl group; and
Ar and $R^2$ are as defined above.

The first half of Step G1 consists in the hydroboration of the compound of formula (XVIII), namely the addition to the double bond of this compound of a reactant, which may be, for example diborane ($B_2H_6$), a borane/dimethyl sulphide complex ($BH_3.SMe_2$) or 9-borabicyclononane (9-BBN). The reaction is normally performed in a solvent. There is no particular restriction on the nature of the solvent to be employed, provided that it has no adverse effect on the reaction or on the reagents involved. Examples of suitable solvents include: ethers, such as tetrahydrofuran. The reaction can take place over a wide range of temperatures, and the precise reaction temperature is not critical to the invention. In general, we find it convenient to carry out the reaction at a temperature from -20 to 60 °C. The time required for the reaction

may also vary widely, depending on many factors, notably the reaction temperature and the nature of the reagents. However, provided that the reaction is effected under the preferred conditions outlined above, a period of from 15 minutes to 5 hours will usually suffice.

The second half of this Step G1 consists in the oxidative elimination of boron to give the alcohol of formula (XIX) or (XX). This may be performed by treating the reaction product of the first half of the step with hydrogen peroxide under alkaline conditions. The reaction can take place over a wide range of temperatures, and the precise reaction temperature is not critical to the invention. In general, we find it convenient to carry out the reaction at a temperature from 0 to 60 °C. The time required for the reaction may also vary widely, depending on many factors, notably the reaction temperature and the nature of the reagents. However, provided that the reaction is effected under the preferred conditions outlined above, a period of from 1 to 5 hours will usually suffice.

The product of this step may easily be purified by column chromatography.

In Step G2, the alcohol of formula (XIX) or (XX) is reacted with methanesulphonyl chloride, to prepare the methanesulphonate of formula (XXI) or (XXII), respectively. This reaction may be performed in a solvent. There is no particular restriction on the nature of the solvent to be employed, provided that it has no adverse effect on the reaction or on the reagents involved. Examples of suitable solvents include: halogenated hydrocarbons, especially halogenated aliphatic hydrocarbons, such as methylene chloride; nitriles, such as acetonitrile; and organic amines, such as pyridine. The reaction is preferably effected in the presence of an acid-binding agent, e.g. triethylamine (although this is not necessary where the solvent is pyridine or another similar organic amine). We prefer to use from 1 to 1.5 equivalents of methanesulphonyl chloride per mole of the compound of formula (XIX) or (XX). The reaction can take place over a wide range of temperatures, and the precise reaction temperature is not critical to the invention. In general, we find it convenient to carry out the reaction at a temperature from -20 °C to room temperature. The time required for the reaction may also vary widely, depending on many factors, notably the reaction temperature and the nature of the reagents. However, provided that the reaction is effected under the preferred conditions outlined above, a period of from 5 minutes to 2 hours will usually suffice.

Step G3 consists in the reaction of the methanesulphonate of formula (XXI) or (XXII) with lithium azide or sodium azide to form the azido compound of formula (XXIII) or (XXIV). This reaction is preferably effected using from 1 to 3 equivalents of the azide per mole of the methanesulphonate of formula (XXI) or (XXII). The reaction is preferably effected in the presence of a solvent. There is no particular restriction on the nature of the solvent to be employed, provided that it has no adverse effect on the reaction or on the reagents involved. Examples of suitable solvents include: fatty acid amides, such as dimethylformamide or dimethylacetamide; and sulphoxides, such as dimethyl sulphoxide. The reaction can take place over a wide range of temperatures, and the precise reaction temperature is not critical to the invention. In general, we find it convenient to carry out the reaction at a temperature from 50 to 100 °C. The time required for the reaction may also vary widely, depending on many factors, notably the reaction temperature and the nature of the reagents. However, provided that the reaction is effected under the preferred conditions outlined above, a period of from 1 to 3 hours will usually suffice.

Steps G4 and G5 provide an alternative method of preparing the compound of formula (XX). Thus, Step G4 consists of the epoxidation of the compound of formula (XVIII) using an oxidizing agent, such as a peroxide, to give the compound of formula (XXV). This reaction is preferably effected in a solvent. There is no particular restriction on the nature of the solvent to be employed, provided that it has no adverse effect on the reaction or on the reagents involved. Examples of suitable solvents include: halogenated hydrocarbons, especially halogenated aliphatic hydrocarbons, such as methylene chloride. We prefer to employ from 1 to 1.5 equivalents of a peroxide, such as peracetic acid or m-chloroperbenzoic acid, per mole of the compound of formula (XXV). The reaction can take place over a wide range of temperatures, and the precise reaction temperature is not critical to the invention. In general, we find it convenient to carry out the reaction at a temperature from 0 to 50 °C, preferably at room temperature. The time required for the reaction may also vary widely, depending on many factors, notably the reaction temperature and the nature of the reagents. However, provided that the reaction is effected under the preferred conditions outlined above, a period of from 30 minutes to 3 hours will usually suffice.

Step G5 consists in the reduction of the epoxy compound of formula (XXV), to give the diol of formula (XX). This reaction is preferably effected in a solvent. There is no particular restriction on the nature of the solvent to be employed, provided that it has no adverse effect on the reaction or on the reagents involved. Examples of suitable solvents include: ethers, such as diethyl ether, tetrahydrofuran or dioxane. A preferred reducing agent is lithium aluminium hydride, preferably employed in an amount of from 0.5 to 2 molar equivalents, based on the compound of formula (XXV). The reaction can take place over a wide range of temperatures, and the precise reaction temperature is not critical to the invention. In general, we find it

convenient to carry out the reaction at a temperature from 0°C to the boiling point of the solvent employed. The time required for the reaction may also vary widely, depending on many factors, notably the reaction temperature and the nature of the reagents. However, provided that the reaction is effected under the preferred conditions outlined above, a period of from 30 minutes to 3 hours will usually suffice.

Step G6 consists of the oxidation of the compound of formula (XVIII) to convert the vinyl group to a formyl group in the compound of formula (XXVI). This may be achieved, for example, by preparation of an ozonide, and then decomposition of the ozonide to give the compound of formula (XXVI). The reaction with ozone may be performed in a suitable solvent, first by introducing ozone into the system. There is no particular restriction on the nature of the solvent to be employed, provided that it has no adverse effect on the reaction or on the reagents involved. Examples of suitable solvents include: halogenated hydrocarbons, especially halogenated aliphatic hydrocarbons, such as methylene chloride; and esters, such as ethyl acetate. The reaction can take place over a wide range of temperatures, and the precise reaction temperature is not critical to the invention. In general, we find it convenient to carry out the reaction at a temperature from -78°C to room temperature. The time required for the reaction may also vary widely, depending on many factors, notably the reaction temperature and the nature of the reagents. The resulting ozonide is then subjected to reductive decomposition, e.g. with dimethyl sulphide.

Alternatively, the starting material of formula (XVIII) may be dissolved in a suitable solvent, such as a mixture of dioxane and water, and then oxidized with from 2 to 4 equivalents of sodium metaperiodate and a catalytic amount of osmium tetraoxide. The reaction can take place over a wide range of temperatures, and the precise reaction temperature is not critical to the invention. In general, we find it convenient to carry out the reaction at a temperature from 0 to 50°C. The time required for the reaction may also vary widely, depending on many factors, notably the reaction temperature and the nature of the reagents. However, provided that the reaction is effected under the preferred conditions outlined above, a period of from 3 to 10 hours will usually suffice.

Step G7 consists of the reduction of the formyl group in the compound of formula (XXVI) to a hydroxymethyl group in a compound of formula (XXVII). This reaction is preferably effected in a suitable solvent. There is no particular restriction on the nature of the solvent to be employed, provided that it has no adverse effect on the reaction or on the reagents involved. Examples of suitable solvents include: alcohols, such as methanol or ethanol, using a suitable reducing agent, e.g. sodium borohydride. The reaction can take place over a wide range of temperatures, and the precise reaction temperature is not critical to the invention. In general, we find it convenient to carry out the reaction at a temperature from -30°C to room temperature. The time required for the reaction may also vary widely, depending on many factors, notably the reaction temperature and the nature of the reagents. However, provided that the reaction is effected under the preferred conditions outlined above, a period of from 5 to 30 minutes will usually suffice.

Step G8 consists of the conversion of the hydroxy group of the compound of formula (XXVII) to an azido group in a compound of formula (XXVIII). This reaction is a combination of Steps G2 and G3, and may be carried out under the same conditions and using the same reagents.

Step G9 consists of the catalytic hydrogenation of the compound of formula (XXVIII) using a catalyst, such as palladium-on-charcoal, platinum or Raney nickel, to give a compound of formula (XXIX). The reaction is essentially the same as and may be performed under the same conditions as that described in Step C3 of Reaction Scheme C.

Finally, the amino compound of formula (XXIX) obtained in Step G9 may be acylated in Step G10 with from 1 to 2 equivalents of an acid chloride to give a compound of formula (XXX). This reaction is essentially the same as and may be performed under the same conditions as that described in Step C4 of Method C.

Method H

This illustrates the stereospecific and enantiospecific synthesis of compounds of the present invention in which $-Y-R^2$ represents an azido group, to give a compound of formula (XLVII), which may then, if desired, be converted to corresponding compounds in which the azido group is replaced by an acylamino group, i.e. a compound of formula (IL), as shown below in Reaction Scheme H:

Reaction Scheme H:

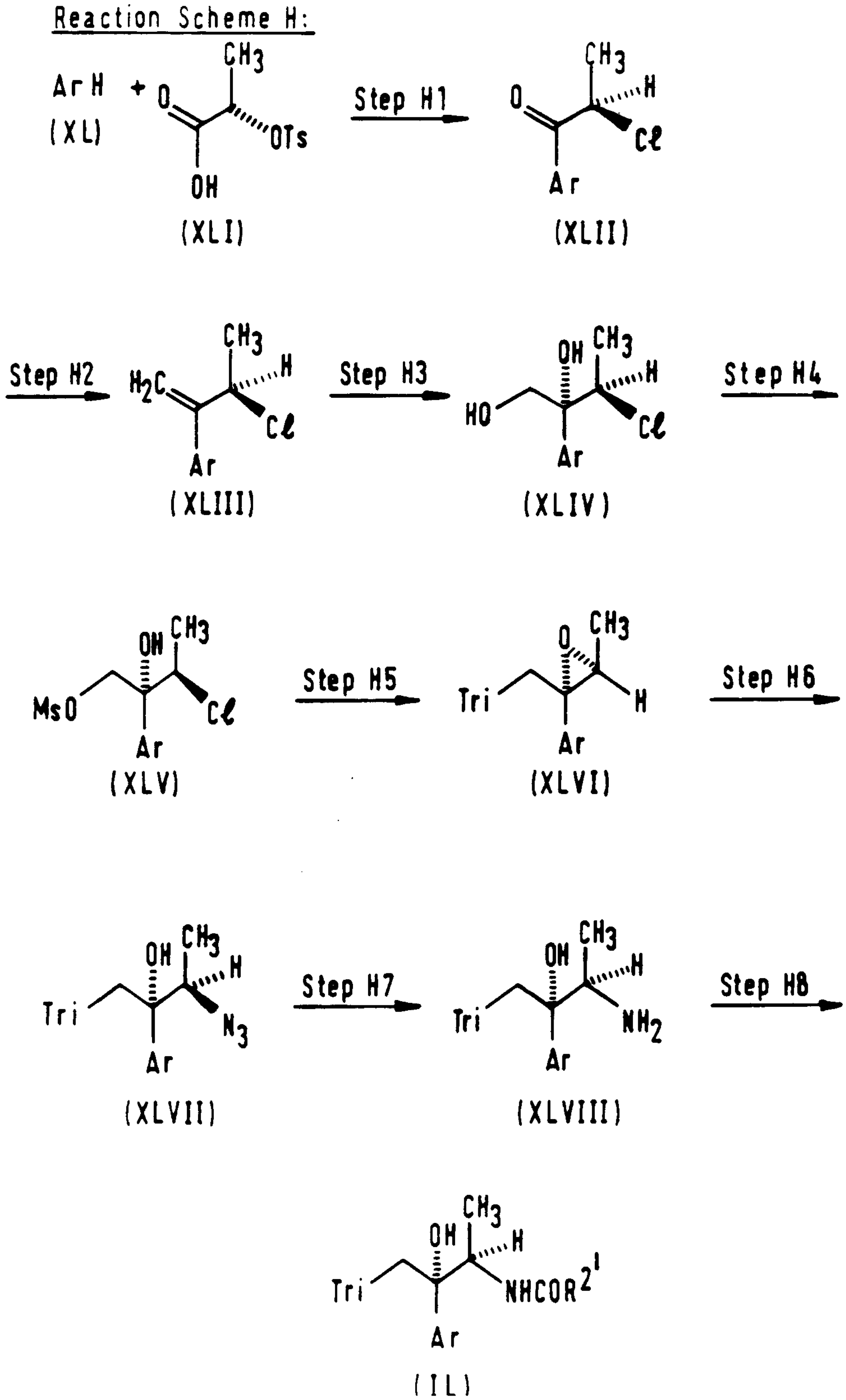


(XLI) (XLII) (XLIII) (XLIV) (XLV) (XLVI) (XLVII) (XLVIII) (IL)

In the above formulae:

Tri represents the 1H-1,2,4-triazol-1-yl group;
Ms represents the methanesulphonyl group;
Ts represents the p-toluenesulphonyl group; and
Ar and $R^{2'}$ are as defined above.

Step H1 is a Friedel-Craft's reaction, in which the p-toluenesulphonyloxy group is eliminated and replaced by a chlorine atom in a configuration opposite to that of the eliminated group. First, the optically active carboxylic acid of formula (XLI) (derived from L-lactic acid) is reacted with oxalyl chloride to give the corresponding acid chloride, which is then reacted with from 1 to 2 equivalents of the compound of formula (XL), e.g. fluorobenzene, m-chloro-fluorobenzene, the difluorobenzenes, especially 1,3-difluorobenzene, or chlorobenzene, in the presence of from 2.5 to 3 equivalents of aluminium chloride. The reaction can take place over a wide range of temperatures, and the precise reaction temperature is not critical to the invention. In general, we find it convenient to carry out the reaction at a temperature from 0°C to room temperature. The time required for the reaction may also vary widely, depending on many factors, notably the reaction temperature and the nature of the reagents. However, provided that the reaction is effected under the preferred conditions outlined above, a period of from 5 to 20 hours will usually suffice. The reaction product may then be worked up and, if required, purified by distillation under reduced pressure.

The position on the benzene ring of the compound of formula Ar-H (XL) which attaches to the ketone of formula (XLII) will depend primarily on the positioning of the halogen substituents on the compound of formula (XL). For example, where the aromatic compound of formula (XL) is 1,3-difluorobenzene, the predominant product will be a compound of formula (XLII) in which the group Ar is the 2,4-difluorophenyl group, whereas, where the aromatic compound of formula (XL) is chlorobenzene, the predominant product will be a compound of formula (XLII) in which the group Ar is the 4-chlorophenyl group.

In Step H2, the ketonic oxygen atom of the compound of formula (XLII) obtained in Step H1 is replaced by a methylene group, to give the compound of formula (XLIII). This may be effected as follows. First, a Grignard reagent is prepared from chloromethyltrimethylsilane in a suitable solvent. There is no particular restriction on the nature of the solvent to be employed, provided that it has no adverse effect on the reaction or on the reagents involved. Examples of suitable solvents include: ethers, such as diethyl ether, tetrahydrofuran and dibutyl ether. Next, the Grignard reagent is reacted with the ketone of formula (XLII), preferably at a temperature of from 0°C to ambient, to give an intermediate silyl alcohol. The silyl alcohol is then treated with from 0.1 to 1 equivalent of a boron trifluoride/diethyl ether complex in a suitable solvent, e.g. methylene chloride, preferably at a temperature of from 0°C to ambient and suitably for a period of from 30 minutes to 3 hours to eliminate the silyl alcohol and thus give the olefin compound of formula (XLIII).

Step H3 is an oxidation reaction in which the olefin compound of formula (XLIII) is oxidised to a diol of formula (XLIV). The reaction is preferably effected using from 1 to 1.5 equivalents of N-methylmorpholine oxide and an equivalent of water in the presence of osmium tetraoxide as a catalyst. The reaction can take place over a wide range of temperatures, and the precise reaction temperature is not critical to the invention. In general, we find it convenient to carry out the reaction at a temperature from 0 to 50°C. The time required for the reaction may also vary widely, depending on many factors, notably the reaction temperature and the nature of the reagents. However, provided that the reaction is effected under the preferred conditions outlined above, a period of from 5 to 20 hours will usually suffice. A small amount of the stereoisomer formed in this Step may be eliminated by column chromatography, if required.

Step H4 consists of the methanesulphonylation of the compound of formula (XLIV), to give the selectively sulphonylated compound of formula (XLV). This reaction is normally effected in a solvent. There is no particular restriction on the nature of the solvent to be employed, provided that it has no adverse effect on the reaction or on the reagents involved. Examples of suitable solvents include: halogenated hydrocarbons, preferably halogenated aliphatic hydrocarbons, such as methylene chloride. We prefer to use from 1 to 1.5 equivalents of methanesulphonyl chloride in the presence of from 1 to 1.5 equivalents of a suitable acid-binding agent, e.g. triethylamine. The reaction can take place over a wide range of temperatures, and the precise reaction temperature is not critical to the invention. In general, we find it convenient to carry out the reaction at a temperature from -20°C to ambient. The time required for the reaction may also vary widely, depending on many factors, notably the reaction temperature and the nature of the reagents. However, provided that the reaction is effected under the preferred conditions outlined above, a period of from 5 to 30 minutes will usually suffice.

The first half of Step H5 involves the preparation of the sodium salt of triazole by mixing equimolar amounts of sodium hydride and triazole in a suitable solvent, such as dimethylformamide. The second half of this Step consists of reacting the sodium salt of triazole with the alcohol of formula (XLV), to give the oxirane of formula (XLVI). We prefer to employ from 2 to 4 equivalents of the sodium salt per equivalent of the alcohol of formula (XLV). The reaction can take place over a wide range of temperatures, and the precise reaction temperature is not critical to the invention. In general, we find it convenient to carry out the reaction at a temperature from ambient to 100°C. The time required for the reaction may also vary widely, depending on many factors, notably the reaction temperature and the nature of the reagents. However,

provided that the reaction is effected under the preferred conditions outlined above, a period of from 1 to 5 hours will usually suffice.

Step H6 converts the oxirane of formula (XLVI) to the azide of formula (XLVII) by reaction with lithium azide or sodium azide, and may be performed in a suitable solvent. There is no particular restriction on the nature of the solvent to be employed, provided that it has no adverse effect on the reaction or on the reagents involved. Examples of suitable solvents include: fatty acid amides, such as dimethylformamide and dimethylacetamide; and sulphoxides, such as dimethyl sulphoxide. We prefer to use from 1 to 3 equivalents of lithium azide or sodium azide per equivalent of the oxirane of formula (XLVI). The reaction is effected in the presence of an ammonium halide, e.g. ammonium chloride, preferably in an amount of from 1 to 1.5 equivalents per equivalent of the oxirane of formula (XLVI). The reaction can take place over a wide range of temperatures, and the precise reaction temperature is not critical to the invention. In general, we find it convenient to carry out the reaction at a temperature from 50 to 120°C. The time required for the reaction may also vary widely, depending on many factors, notably the reaction temperature and the nature of the reagents. However, provided that the reaction is effected under the preferred conditions outlined above, a period of from 2 to 10 hours will usually suffice.

Steps H7 [in which the azide compound of formula (XLVII) is subjected to catalytic hydrogenation to give the amino compound of formula (XLVIII)] and H8 [in which the resulting amino compound is acylated] are essentially the same as, and may be carried out under the same conditions as, Steps C3 and C4, respectively, of Reaction Scheme C.

Alternatively, if desired, the compound of formula (XLVIII) may be treated as described in Reaction Schemes D or F, to give corresponding products. The compound of formula (XLVI) may also be treated as described in Reaction Scheme B [replacing the compound of formula (VII)], to give optically active compounds corresponding to the compound of formula (Ib).

Method J:

This provides an alternative stereospecific and enantiospecific reaction sequence for preparing the compound of formula (XLVII) (see Reaction Scheme H), which may then be treated as in Steps H7 and H8, to give the optically active compound of formula (IL). The reactions involved are illustrated in Reaction Scheme J:

Reaction Scheme J:

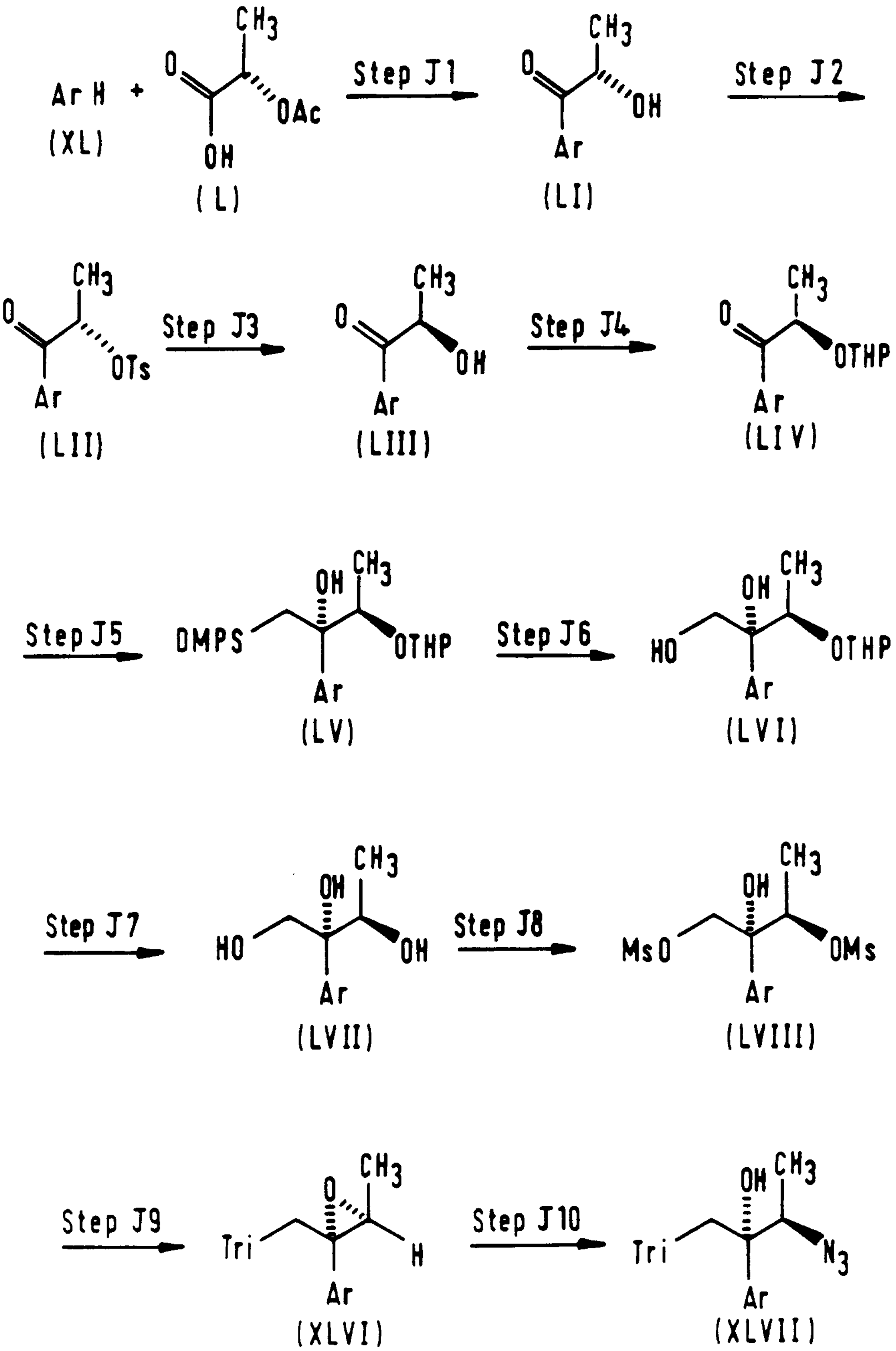


In the above formulae:

Ar, Tri, Ms and Ts are as defined above;
THP represents a tetrahydropyranyl group;
DMPS represents a dimethylisopropoxysilyl group; and
Ac represents an acetyl group.

Step J1 is Friedel-Craft's reaction, and may be carried out under conditions well known for this type of reaction. For example, first, the optically active carboxylic acid of formula (L) (derived from L-lactic acid) is reacted with oxalyl chloride to give an acid chloride, which is then reacted with the compound of formula Ar-H (XL), to give the ketonic compound of formula (LI). The compound of formula (XL) is preferably employed in an amount of from 1 to 2 equivalents per equivalent of the acid of formula (L), and the reaction is preferably effected in the presence of aluminium chloride, e.g. from 2 to 3 equivalents per equivalent of the acid of formula (L). The reaction may be effected in the presence or absence of an added solvent. There is no particular restriction on the nature of the solvent, if one is employed, provided that it has no adverse effect on the reaction or on the reagents involved. Examples of suitable solvents include: halogenated hydrocarbons, especially halogenated aliphatic hydrocarbons, such as methylene chloride. In most cases, however, the compound of formula Ar-H is liquid and the aluminium chloride dissolves in it, and so a solvent is not necessary. The reaction can take place over a wide range of temperatures, and the precise reaction temperature is not critical to the invention. In general, we find it convenient to carry out the reaction at a temperature from 0°C to room temperature. The time required for the reaction may also vary widely, depending on many factors, notably the reaction temperature and the nature of the reagents. However, provided that the reaction is effected under the preferred conditions outlined above, a period of from 5 to 20 hours will usually suffice. The reaction mixture may then be worked up to give a crude product, which is then deacetylated, e.g. using methanol/sulphuric acid, to give the compound of formula (LI).

Step J2 is a tosylation reaction in which the compound of formula (LI), obtained as described in Step J1, is reacted with p-toluenesulphonic acid or a reactive derivative thereof, e.g. a halide, such as the chloride, or the anhydride, to give the compound of formula (LII), in which the hydroxy group is protected. We prefer to employ from 1 to 2 equivalents of the p-toluenesulphonic acid or reactive derivative thereof per equivalent of the ketone of formula (LI). The reaction is preferably performed in a suitable solvent. There is no particular restriction on the nature of the solvent to be employed, provided that it has no adverse effect on the reaction or on the reagents involved. Examples of suitable solvents include: organic amines, such as pyridine. The reaction can take place over a wide range of temperatures, and the precise reaction temperature is not critical to the invention. In general, we find it convenient to carry out the reaction at a temperature from -15 to -10°C. The time required for the reaction may also vary widely, depending on many factors, notably the reaction temperature and the nature of the reagents. However, provided that the reaction is effected under the preferred conditions outlined above, a period of from 3 to 7 hours will usually suffice.

In Step J3, the configuration of the product of Step J2 is inverted by a substitution reaction, in which the p-toluenesulphonyloxy group is again converted to a hydroxy group by a dropwise addition, normally slow, of an equivalent of a suitable hydrolysing agent, for example an aqueous solution of lithium hydroxide. The reaction is performed in a suitable solvent. There is no particular restriction on the nature of the solvent to be employed, provided that it has no adverse effect on the reaction or on the reagents involved. Examples of suitable solvents include: fatty acid amides, such as dimethylformamide. The reaction can take place over a wide range of temperatures, and the precise reaction temperature is not critical to the invention, although at higher temperatures racemization may take place, which is undesirable when an enantiospecific synthesis is required. In general, we find it convenient to carry out the reaction at a relatively low temperature, e.g. a temperature of around -15°C. The time required for the reaction may also vary widely, depending on many factors, notably the reaction temperature and the nature of the reagents. However, provided that the reaction is effected under the preferred conditions outlined above, a period of from 1 to 3 hours will usually suffice, and this will, in most cases, be about the time required for the dropwise addition, so that no additional reaction time is required.

In Step J4, the hydroxy group of the compound of formula (LIII) is protected by conversion to a tetrahydropyranyl group. This may be achieved by reacting dihydropyran with the compound of formula (LIII), normally in a suitable solvent, e.g. methylene chloride, in the presence of pyridinium p-toluenesulphonate. The reaction can take place over a wide range of temperatures, and the precise reaction temperature is not critical to the invention. In general, we find it convenient to carry out the reaction at about room temperature. The time required for the reaction may also vary widely, depending on many factors, notably the reaction temperature and the nature of the reagents. However, provided that the reaction is effected under the preferred conditions outlined above, a period of from 3 to 15 hours will usually suffice.

In Step J5, first, a Grignard reagent is prepared from chloromethyldimethylisopropoxysilane, by a reaction well known per se, in a suitable solvent, preferably an ether, such as diethyl ether, tetrahydrofuran or dibutyl ether, and then the Grignard reagent is reacted with the ketone of formula (LIV) to give the compound of formula (LV). The reaction can take place over a wide range of temperatures, and the precise reaction temperature is not critical to the invention. In general, we find it convenient to carry out the reaction

at a temperature from -20 to 0 ° C. The time required for the reaction may also vary widely, depending on many factors, notably the reaction temperature and the nature of the reagents. However, provided that the reaction is effected under the preferred conditions outlined above, a period of from 10 to 30 minutes will usually suffice.

In Step J6, the silyl group in the compound of formula (LV) is converted to a hydroxy group, to give a compound of formula (LVI). The reaction may be performed by reacting the compound of formula (LV) with a suitable oxidizing agent, e.g. 35% hydrogen peroxide in the presence of sodium carbonate or sodium bicarbonate. The reaction is performed in a suitable solvent. There is no particular restriction on the nature of the solvent to be employed, provided that it has no adverse effect on the reaction or on the reagents involved. The solvent is preferably water-miscible, and examples of suitable solvents include: ethers and/or alcohols, such as a mixture of tetrahydrofuran and methanol, and mixtures of one or more of these solvents with water. The reaction can take place over a wide range of temperatures, and the precise reaction temperature is not critical to the invention. In general, we find it convenient to carry out the reaction at a temperature of from ambient to 70 ° C. The time required for the reaction may also vary widely, depending on many factors, notably the reaction temperature and the nature of the reagents. However, provided that the reaction is effected under the preferred conditions outlined above, a period of from 30 minutes to 2 hours will usually suffice.

Step J7 consists in the deprotection of the compound of formula (LVI) to give the compound of formula (LVII). This reaction may be performed in a suitable solvent. There is no particular restriction on the nature of the solvent to be employed, provided that it has no adverse effect on the reaction or on the reagents involved. Examples of suitable solvents include: alcohols, such as methanol or ethanol. The reaction should take place in the presence of catalytic amount of p-toluenesulphonic acid. The reaction can take place over a wide range of temperatures, and the precise reaction temperature is not critical to the invention. In general, we find it convenient to carry out the reaction at a temperature of from 0 ° C to ambient. The time required for the reaction may also vary widely, depending on many factors, notably the reaction temperature and the nature of the reagents. However, provided that the reaction is effected under the preferred conditions outlined above, a period of from 15 minutes to 1 hour will usually suffice.

Step J8 consists in a conventional mesylation reaction to protect two of the hydroxy groups of the compound of formula (LVII) and thus give the compound of formula (LVIII). The reaction is preferably effected using from 2 to 3 equivalents of methanesulphonyl chloride per equivalent of the compound of formula (LVII). The reaction is performed in a suitable solvent. There is no particular restriction on the nature of the solvent to be employed, provided that it has no adverse effect on the reaction or on the reagents involved. Examples of suitable solvents include: halogenated hydrocarbons, especially halogenated aliphatic hydrocarbons, such as methylene chloride. The reaction is carried out in the presence of an acid-binding agent, such as triethylamine. The reaction can take place over a wide range of temperatures, and the precise reaction temperature is not critical to the invention. In general, we find it convenient to carry out the reaction at a temperature from -20 to 0 ° C.

Steps J9 [in which the compound of formula (LVIII) is converted to a compound of formula (XLVI)] and J10 [in which the resulting compound of formula (XLVI) is converted to a compound of formula (XLVII)] are essentially the same as, and may be carried out under the same conditions as, Steps H6 and H7 of Reaction Scheme H.

Method K

This provides an alternative stereospecific and enantiospecific route for the preparation of the compound of formula (XLVI) (see Reaction Scheme H), as illustrated in the following Reaction Scheme K:

Reaction Scheme K:

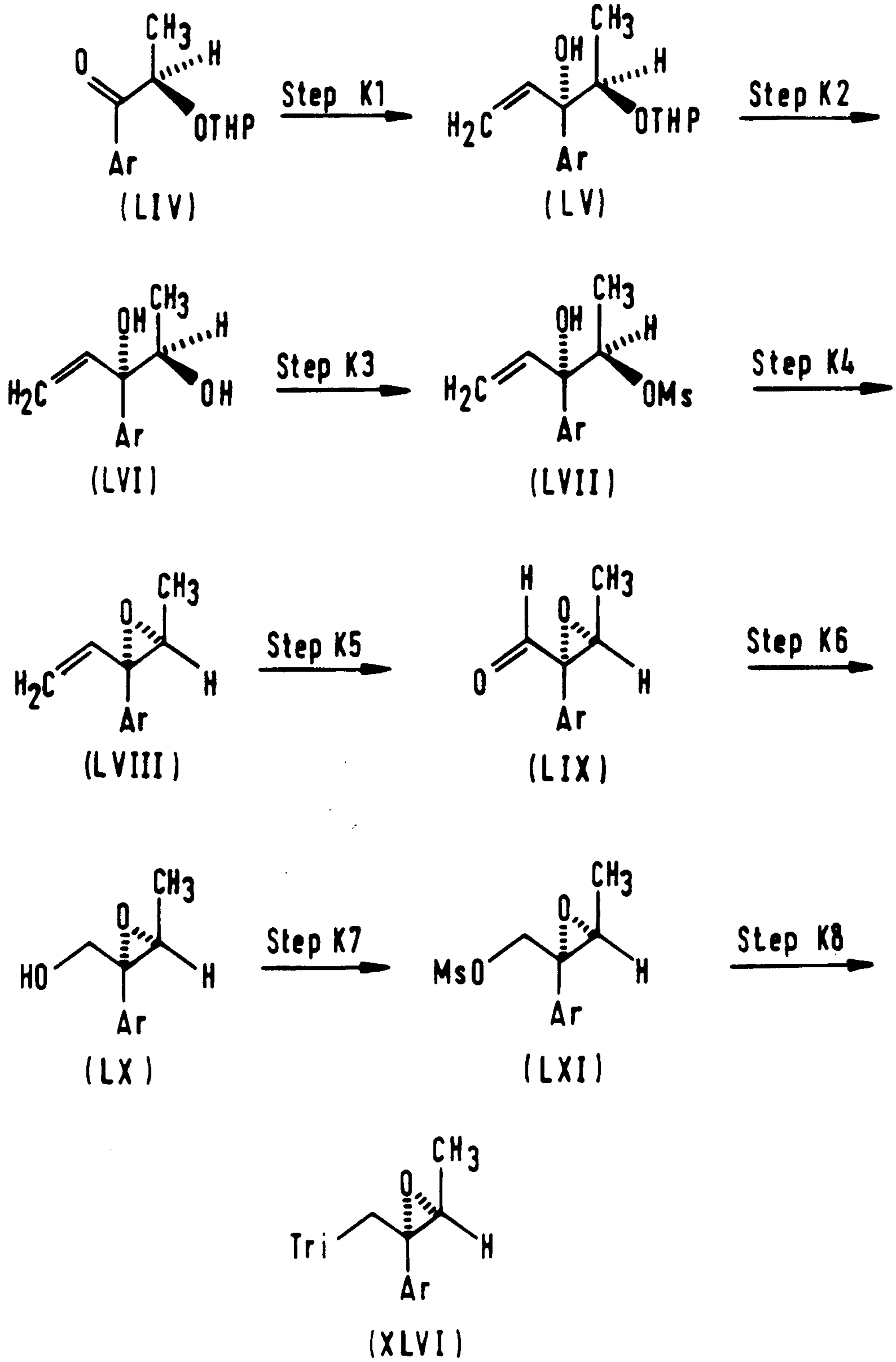


(LIV) (LV) (LVI) (LVII) (LVIII) (LIX) (LX) (LXI) (XLVI)

In the above formulae, Ar, Tri, Ms and THP are as defined above.

Step K1 consists of a Grignard reaction to convert the ketonic oxygen atom of the compound of formula (LIV) to a vinyl group. The reaction may be performed by reacting the starting material, the compound of formula (LIV) (see Reaction Scheme J), with vinylmagnesium bromide in a suitable solvent, preferably an ether, such as diethyl ether or tetrahydrofuran. The reaction is preferably effected at a relatively low

temperature, e.g. at a temperature of from -78 to -30 ° C The time required for the reaction may vary widely, depending on many factors, notably the reaction temperature and the nature of the reagents. However, provided that the reaction is effected under the preferred conditions outlined above, a period of from 10 to 30 minutes will usually suffice.

The reaction in Step K2 consists of the removal of the tetrahydropyranyl protecting group from the compound of formula (LV), to give the compound of formula (LVI). This reaction may be performed by treating the compound of formula (LV) with a catalytic amount of p-toluenesulphonic acid in a suitable solvent. There is no particular restriction on the nature of the solvent to be employed, provided that it has no adverse effect on the reaction or on the reagents involved. Examples of suitable solvents include: alcohols, such as methanol or ethanol. The reaction can take place over a wide range of temperatures, and the precise reaction temperature is not critical to the invention. In general, we find it convenient to carry out the reaction at a temperature from 0 ° C to ambient. The time required for the reaction may also vary widely, depending on many factors, notably the reaction temperature and the nature of the reagents. However, provided that the reaction is effected under the preferred conditions outlined above, a period of from 10 to 30 minutes will usually suffice.

The reaction in Step K3 consists of the methanesulphonylation of the compound of formula (LVI), to give the compound of formula (LVII). We prefer to employ from 1 to 1.5 equivalents of methanesulphonyl chloride per equivalent of the compound of formula (LVI), in a suitable solvent. There is no particular restriction on the nature of the solvent to be employed, provided that it has no adverse effect on the reaction or on the reagents involved. Examples of suitable solvents include: halogenated hydrocarbons, preferably halogenated aliphatic hydrocarbons, such as methylene chloride. The reaction is effected in the presence of an organic base, e.g. an amine, such as triethylamine, preferably from 1 to 2 equivalents per equivalent of the compound of formula (LVI). The reaction can take place over a wide range of temperatures, and the precise reaction temperature is not critical to the invention. In general, we find it convenient to carry out the reaction at a relatively low temperature, e.g. from -20 to 0 ° C. The time required for the reaction may also vary widely, depending on many factors, notably the reaction temperature and the nature of the reagents. However, provided that the reaction is effected under the preferred conditions outlined above, a period of from 5 to 15 minutes will usually suffice.

The reaction in Step K4 consists of the elimination of methanesulphonic acid from the compound of formula (LVII), to give the oxirane compound of formula (LVIII). This reaction may be performed by reacting the compound of formula (LVII) with sodium hydride, preferably in an amount of from 1 to 1.5 equivalents per equivalent of the compound of formula (LVII), in a suitable solvent. There is no particular restriction on the nature of the solvent to be employed, provided that it has no adverse effect on the reaction or on the reagents involved. Examples of suitable solvents include: ethers, such as tetrahydrofuran; fatty acid amides, such as dimethylformamide; and sulphoxides, such as dimethyl sulphoxide. The reaction can take place over a wide range of temperatures, and the precise reaction temperature is not critical to the invention. In general, we find it convenient to carry out the reaction at a temperature from 0 ° C to ambient. The time required for the reaction may also vary widely, depending on many factors, notably the reaction temperature and the nature of the reagents. However, provided that the reaction is effected under the preferred conditions outlined above, a period of from 1 to 10 hours will usually suffice.

The reaction of Step K5 consists of the oxidation of the vinyl group of the compound of formula (LVIII), to give a carbonyl group in the compound of formula (LIX). The reaction may be performed by reacting the compound of formula (LVIII) with sodium m-periodate, preferably in an amount of from 2 to 4 equivalents per equivalent of the compound of formula (LVIII), in the presence of a catalytic amount of osmium tetraoxide. The reaction is effected in a suitable aqueous solvent, the nature of which is not critical to the invention, such as aqueous methanol or aqueous tetrahydrofuran. The reaction can take place over a wide range of temperatures, and the precise reaction temperature is not critical to the invention. In general, we find it convenient to carry out the reaction at a temperature from 0 ° C to ambient. The time required for the reaction may also vary widely, depending on many factors, notably the reaction temperature and the nature of the reagents. However, provided that the reaction is effected under the preferred conditions outlined above, a period of from 5 to 15 hours will usually suffice.

The reaction of Step K6 consists of the reduction of the carbonyl group formed in Step K5 to a hydroxymethyl group. The reaction may be performed by reacting the compound of formula (LIX) with a suitable reducing agent, such as sodium borohydride in a suitable solvent, the nature of which is not critical to the invention, such as methanol or ethanol. The reaction can take place over a wide range of temperatures, and the precise reaction temperature is not critical to the invention. In general, we find it convenient to carry out the reaction at a relatively low temperature, e.g. from -20 ° C to ambient. The time required for the reaction may also vary widely, depending on many factors, notably the reaction temperature and the nature of the reagents. However, provided that the reaction is effected under the preferred conditions outlined above, a period of from 5 to 30 minutes will usually suffice.

The reaction of Step K7 consists of the methanesulphonylation of the compound of formula (LX), prepared in Step K6) to give a compound of formula (LXI). It is essentially the same as, and may be carried out under the same conditions as, the reaction described in Step K3.

Step K8 involves the replacement of the methanesulphonyloxy group in the compound of formula (LXI) by a 1H-1,2,4-triazol-1-yl group. The reaction may be performed by reacting the compound of formula (LXI) with the sodium salt of triazole (which may be prepared by mixing triazole and sodium hydride in dimethylformamide), preferably in an amount of from 1 to 3 equivalents per equivalent of the compound of formula (LXI). The reaction can take place over a wide range of temperatures, and the precise reaction temperature is not critical to the invention. In general, we find it convenient to carry out the reaction at a temperature from 50 to 120 °C. The time required for the reaction may also vary widely, depending on many factors, notably the reaction temperature and the nature of the reagents. However, provided that the reaction is effected under the preferred conditions outlined above, a period of from 30 minutes to 3 hours will usually suffice.

Method L

This describes an alternative method of preparing a compound of formula (XLVI) (see Reaction Scheme H), as illustrated in Reaction Scheme L:

## Reaction Scheme L:

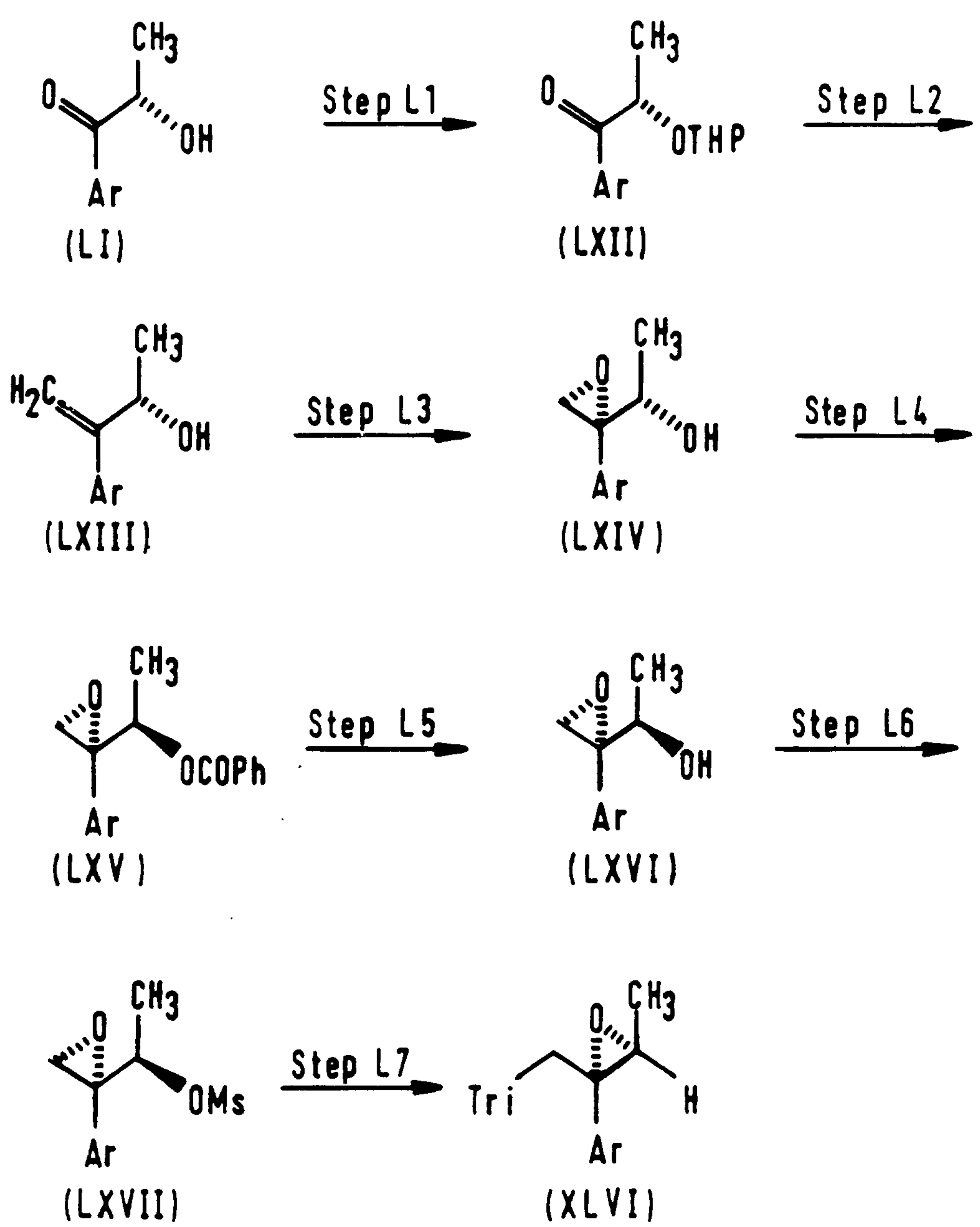


(LI) (LXII) (LXIII) (LXIV) (LXV) (LXVI) (LXVII) (XLVI)

In the above formulae:

Ar, Tri, Ms and THP are as defined above; and

Ph represents a phenyl group.

In Step L1, the hydroxy group of the compound of formula (LI) (see Reaction Scheme J) is protected by a tetrahydropyranyl group to give a compound of formula (LXII). This reaction is essentially the same as, and may be carried out under the same conditions as, that described in Step J4 of Reaction Scheme J.

In Step L2, a Grignard reagent is prepared from chloromethyltrimethylsilane in diethyl ether. The Grignard reagent is then reacted with the compound of formula (LXII), to give a silyl alcohol intermediate, which is then treated in methanol with a catalytic amount of p-toluenesulphonic acid at a temperature of from 0 to 50°C for a period of from 1 to 5 hours. The reaction consists in the deprotection of the hydroxy group and the formation of a double bond by elimination of the silyl alcohol.

Step L3 consists in a stereospecific oxidation using a vanadium catalyst and from 1 to 2 equivalents of t-butyl hydroperoxide per equivalent of the compound of formula (LXIII). The reaction may be performed in

a solvent. There is no particular restriction on the nature of the solvent to be employed, provided that it has no adverse effect on the reaction or on the reagents involved. Examples of suitable solvents include: aromatic hydrocarbons, such as benzene. The reaction can take place over a wide range of temperatures, and the precise reaction temperature is not critical to the invention. In general, we find it convenient to carry out the reaction at about room temperature. The time required for the reaction may also vary widely, depending on many factors, notably the reaction temperature and the nature of the reagents. However, provided that the reaction is effected under the preferred conditions outlined above, a period of from 2 to 10 hours will usually suffice.

Step L4 consists in the inversion of the stereo-chemistry at the carbon atom bearing the hydroxy group by the Mitsunobu reaction. The reaction may be performed by reacting the compound of formula (LXIV), prepared as described in Step L3, with from 1 to 2 equivalents each of triphenylphosphine, benzoic acid and diethyl diazocarboxylate, per equivalent of the compound of formula (LXIV). The reaction is effected in a suitable solvent, the nature of which is not critical, such as tetrahydrofuran. The reaction can take place over a wide range of temperatures, and the precise reaction temperature is not critical to the invention. In general, we find it convenient to carry out the reaction at a temperature from 0°C to ambient. The time required for the reaction may also vary widely, depending on many factors, notably the reaction temperature and the nature of the reagents. However, provided that the reaction is effected under the preferred conditions outlined above, a period of from 30 minutes to 3 hours will usually suffice.

The reaction of Step L5 may be performed by treating the compound of formula (LXV), prepared as described in Step L4, in methanol with a catalytic amount of sodium methoxide. The reaction can take place over a wide range of temperatures, and the precise reaction temperature is not critical to the invention. In general, we find it convenient to carry out the reaction at a temperature below ambient. The time required for the reaction may also vary widely, depending on many factors, notably the reaction temperature and the nature of the reagents. However, provided that the reaction is effected under the preferred conditions outlined above, a period of from 2 to 10 hours will usually suffice.

The reaction of Step L6 is essentially the same as, and may be carried out under the same conditions as, that of Step K3 of Reaction Scheme K, to give a compound of formula (LXVII).

The reaction of Step L7 may be performed by reacting the product of Step L6 with from 1 to 3 equivalents of the sodium salt of triazole. The reaction may, for example, be effected in dimethylformamide at a temperature of from ambient to 100°C for a period of from 30 minutes to 10 hours.

Acid addition salts of the compound of formula (I) may be prepared from, e.g. hydrochloric acid, hydrobromic acid, sulphuric acid, nitric acid, oxalic acid or methanesulphonic acid by well known conventional methods.

It is belived that all of the compounds of formula (I) and acid addition salts thereof are useful as fungicides for agricultural, horticultural and similar uses. For such uses, they may be formulated in conventional preparations for such use and may be applied to plants, parts of plants, reproductive matter from plants, or to the environment containing such plants, parts of plants or reproductive matter from plants as is well known for conventional fungicides and other agrochemicals.

Reflecting the activity of the present compounds, the invention further provides compositions which contain one or more of the compounds of the invention, together with a carrier and optionally other auxiliary agents, if necessary. The present compositions may be formulated as preparations of the type commonly employed for agricultural or horticultural use, for instance as dusts, coarse dusts, microgranules, fine microgranules, wettable powders, emulsifiable concentrates, aqueous or oily suspensions, and aerosols. It is, of course, not necessary to use a completely pure form of the compound of the invention in the composition and, of course, purification can be suspended at any stage and the resulting crude substance may be used as the active ingredient of the composition.

The carrier employed in such compositions may be natural or synthetic and organic or inorganic; it is generally employed to assist the active ingredient to reach the substrate to be treated, and to make it easier to store, transport or handle the active compound. It may be solid, liquid or gaseous.

Suitable solid carriers include:

inorganic substances, such as clays (examples of which are bentonite, kaolinite, montmorillonite and attapulgite), talc, mica, pyrophyllite, pumice, vermiculite, gypsum, calcium carbonate, dolomite, diatomaceous earth, magnesium carbonate, apatite, zeolite, silicic anhydride and synthetic calcium silicate; vegetable organic substances, such as nut shells (e.g. of walnuts or other nuts), soybean meal, tobacco powder, walnut powder, wheat flour, wood meal, starch and crystalline cellulose; synthetic or natural high molecular weight polymers, especially resins, such as cumarone resins, petroleum resins, alkyd resins, polyvinyl chloride, polyalkylene glycols, ketone resins, ester gums, xanthan gum, copal gum and dammar gum; waxes such as carnauba wax and beeswax; or urea.

Examples of suitable liquid carriers include:
paraffinic or naphthenic hydrocarbons, such as kerosene, mineral oil, spindle oil and white oil; aromatic hydrocarbons, such as benzene, toluene, xylene, solvent naphtha, ethylbenzene, cumene and methylnaphthalene; chlorinated hydrocarbons, such as carbon tetrachloride, chloroform, trichloroethylene, monochlorobenzene and o-chlorotoluene; ethers such as dioxane and tetrahydrofuran; ketones, such as acetone, methyl ethyl ketone, diisobutyl ketone, cyclohexanone, acetophenone and isophorone; esters such as ethyl acetate, amyl acetate, ethylene glycol acetate, diethylene glycol acetate, dibutyl maleate and diethyl succinate; alcohols such as methanol, ethanol, isopropanol, hexanol, ethylene glycol, diethylene glycol, cyclohexanol, and benzyl alcohol; ether alcohols, such as ethylene glycol monoethyl ether, ethylene glycol monophenyl ether, diethylene glycol monoethyl ether and diethylene glycol monobutyl ether; other polar solvents, such as dimethylformamide and dimethyl sulphoxide; and water.

Suitable gaseous carriers include:
air, nitrogen, carbon dioxide and fluorocarbon propellants such as those sold under the Trade Mark "Freon"; they may be mixed in a known manner to give a propellant.

The compositions of the invention may contain one or more surface active agents and/or polymers to improve the properties of the compositions and help them to disperse, emulsify, spread, penetrate and bind or to control disintegration, improve fluidity or impart corrosion resistance to the composition, or to stabilize the active compound. Any of the conventional classes of surface active agent (non-ionic, anionic, cationic or amphoteric) may be employed, but it is preferred to employ non-ionic and/or anionic surface active agents whereby wetting, adhesion and absorption and desired effects may be improved.

Examples of suitable non-ionic surface active agents include:
the polymerization adducts of ethylene oxide with higher alcohols, such as lauryl alcohol, stearyl alcohol and oleyl alcohol; the polymerization adducts of ethylene oxide with alkylphenols, such as isooctylphenol or nonylphenol; the polymerization adducts of ethylene oxide with alkylnaphthols, such as butylnaphthol or octylnaphthol; the polymerization adducts of ethylene oxide with higher fatty acids, such as palmitic acid, stearic acid or oleic acid; the polymerization adducts of ethylene oxide with mono- or dialkylphosphoric acids, such as stearylphosphoric acid or dilaurylphosphoric acid; the polymerization adducts of ethylene oxide with amines, such as dodecylamine; amides or ethoxylated amides of higher fatty acids, such as stearamide; higher fatty acid esters of polyhydric alcohols, such as sorbitan, and the polymerization adducts of ethylene oxide therewith; higher fatty acid esters of glycerol borates or of ethoxylated glycerol borates; glycerides and sucrose esters of fatty acids; and the polymerization adducts of ethylene oxide with propylene oxide.

Examples of suitable anionic surface active agents include:
salts of higher fatty acids, i.e. soaps, e.g. sodium oleate; salts, e.g. sodium and calcium salts, of sulphonic acids and the acids themselves, e.g. ligninsulphonic acid, and aryl sulphonate salts, such as sodium isopropylnaphthalenesulphonate, sodium methylenebisnaphthalenesulphonate, sodium ligninsulphonate or sodium dodecylbenzenesulphonate, or alkyl sulphonate salts, especially sodium dialkyl sulphosuccinates, such as sodium dioctyl sulphosuccinate or sodium 2-ethylhexenesulphonate; salts, e.g. sodium, ammonium and amine salts, of polyoxyethylene alkyl aryl ether sulphates or of polyoxyethylene alkyl ether sulphates or the free acids; or salts of polyoxyethylene alkyl aryl ether phosphates or of polyoxyethylene alkyl phosphates; alkyl sulphate salts, such as sodium lauryl sulphate or oleyl sulphate amine salt;
Examples of suitable cationic surfactants include:
the higher aliphatic amines and ethylene oxide condensates with such amines; quaternary ammonium salts, e.g. chlorides; N-alkylamine acetates; and N-alkylamine oxides;
Examples of amphoteric surfactants include betaines and amino acid-type surfactants.

Moreover, the compositions of the present invention may be used in combination with high molecular weight compounds or other formulation agents, for example: protective colloids, such as casein, gelatin, gum arabic, albumin, glue, sodium alginate, carboxymethylcellulose, methylcellulose, hydroxyethylcellulose or polyvinyl alcohol; dispersing agents, such as sodium polyphosphate; inorganic dispersing agents, such as bentonite or veegum; stabilizers; binding agents; and anti-freezing agents. For wider applicability and labor saving, the composition of the invention can, if desired, be combined with one or more other agrochemicals, e.g. fungicides, insecticides, herbicides, plant growth regulators and fertilizers.

The above-mentioned carriers and various auxiliary agents may be used alone or in any desired combination, depending upon the type of preparation, the application and other factors.

For example, dusts may conveniently contain from 1 to 25% by weight of the active compound, the remainder being a solid carrier.

Wettable powders may conveniently contain, for example, from 25 to 90% by weight of the compound, the remainder being a solid carrier and a dispersing and wetting agent, if required, together with a

protective colloidal agent, a thixotropic agent and an anti-foaming agent.

Granules may conveniently contain from 1 to 35% by weight of the active compound, a major portion of the remainder being a solid carrier. The active compound is homogeneously admixed with the solid carrier or is adhered to or adsorbed onto the carrier surface; the diameter of each granule is preferably from 0.2 to 1.5 mm.

Emulsifiable concentrates may conveniently contain, for example, from 5 to 50% by weight of the active compound and from 5 to 20% by weight of an emulsifying agent, the remainder being a liquid carrier, together with, if required, a corrosion inhibitor.

Oil preparations may conveniently contain from 0.5 to 5% by weight of the active compound, the remainder being a liquid carrier such as kerosene.

Aerosols may conveniently contain from 0.1 to 5% by weight of the active compound and optionally a perfume, the remainder being an oily and/or aqueous carrier, and a propellant such as liquified petroleum gas, a fluorocarbon or carbon dioxide.

The compositions of the invention may be applied, for example, to paddy or other fields before or after emergence of disease in plants or to plants already infected with harmful fungi; a concentration of from 10 to 500 ppm for the active ingredient is usually suitable, especially for application to leaves and stems of plants and to soil, whereby effective control may be attained.

The composition of the invention may conveniently be blended with other fungicide for a broader fungicidal spectrum and, in some case, a synergistic effect may be expected. Suitable other fungicides include:

carbamate-type fungicides;

such as 3,3'-ethylenebis(tetrahydro-4,6-dimethyl-2H-1,3,5-thiadiazine-2-thione, zinc or manganese ethylenebisdithiocarbamate, bis(dimethyldithiocarbamoyl)disulphide, zinc propylenebisdithiocarbamate, methyl 1-(butylcarbamoyl)-2-benzimidazolcarbamate, 1,2-bis(3-methoxycarbonyl-2-thioureido)benzene and bisdimethyldithiocarbamoyl zinc ethylenebisdithiocarbamate;

dicarboximide-type fungicides;

such as N-trichloromethylthio-4-cyclohexene-1,2-dicarboximide and N-tetrachloroethylthio-4-cyclohexene-1,2-dicarboximide;

oxazine-type fungicides;

such as 5,6-dihydro-2-methyl-1,4-oxazine-3-carboxanilide-4,4-dioxide;

naphthoquinone-type fungicides;

such as 2,3-dichloro-1,4-naphthoquinone;

and other fungicides;

such as 3-hydroxy-5-methylisoxazole, 5-ethoxy-3-trichloromethyl-1,2,4-thiadiazole, 2,4-dichloro-6-(o-chloroanilino)-1,3,5-triazine, 2,3-dicyano-1,4-dithioanthraquinone, copper 8-quinolate, polyoxin, validamycin, tetrachloroisophthalonitrile, 2-(1-methylpropyl)-4,6-dinitrophenol β,β-dimethylacrylate, triphenyltin hydroxide, phytomycin, dinitromethylheptylphenyl crotonate, 5-butyl-2-dimethylamino-6-methylpyrimidin-4-ol, 6-(3,5-dichloro-4-methylphenyl)-3-(2H)pyridazinone, 6-(3-bromophenyl)-3-(2H)pyridazinone, N-(2,6-dimethylphenyl)-N-methoxyacetylalanine methyl ester and bis(8-guanidinooctyl)amine acetate.

The composition of the invention may be blended with insecticides. Suitable insecticides include:

phosphorus-containing insecticides;

such as O,O-diethyl O-(2-isopropyl-4-methyl-6-pyrimidinyl)phosphorothioate, O,O-diethyl S-[2-(ethylthio)-ethyl]phosphorodithioate, O,O-dimethyl O-(3-methyl-4-nitrophenyl)thiophosphate, O,O-dimethyl S-(N-methylcarbamoylmethyl)phosphorodithioate, O,O-dimethyl S-(N-methyl-N-formylcarbamoylmethyl)-phosphorodithioate, O,O-dimethyl S-[2-(ethylthio)ethyl]phosphorodithioate, O,O-dimethyl-1-hydroxy-2,2,2-trichloroethylphosphonate, O,O-diethyl-O-(5-phenyl-3-isoxazolyl)phosphorothioate, O,O-dimethyl-O-(3-methyl-4-methylmercaptophenyl)thiophosphate, O-ethyl-O-p-cyanophenyl phenylphosphonothioate, O,O-dimethyl S-(1,2-dicarboethoxyethyl)phosphorodithioate, 2-chloro-1-(2,4,5-trichlorophenyl)vinyldimethyl phosphate, 2-chloro-1-(2,4-dichlorophenyl)vinyldimethyl phosphate, O,O-dimethyl O-p-cyanophenyl phosphorothioate, 2,2-dichlorovinyldimethylphosphate, ethyl mercaptophenylacetate O,O-dimethyl phosphorodithioate, S-[(6-chloro-2-oxo-3-benzooxazolinyl)methyl]-O,O-diethylphosphorodithioate, 4-methylthiophenyl dipropylphosphate, 2-chloro-1-(2,4-dichlorophenyl)vinyldiethylphosphate, O,O-diethyl O-(3-oxo-2-phenyl-2H-pyridazin-6-yl)phosphorothioate, O,O-dimethyl S-(1-methyl-2-ethylsulphinyl)ethyl phosphorothioate, O,O-dimethyl S-phthalimidomethyl phosphorodithioate, dimethylmethylcarbamoylethylthioethyl thiophosphorothioate, O,O-diethyl S-(N-ethoxycarbonyl-N-methylcarbamoylmethyl)-phosphorodithioate, O,O-dimethyl-S-[2-methoxy-1,3,4-thiadiazol-5(4H)-onyl-(4)-methyl]dithiophosphate, 2-methoxy-4H-1,3,2-benzodioxaphosphorin 2-sulphide, O,O-diethyl-O-(3,5,6-trichloro-2-pyridyl)-phosphorothioate, O,S-dimethyl-N-acetyl phosphoroamidothioate, O-2,4-dichlorophenyl O-ethyl S-propylphosphorodithioate, O,O-diethyl S-(2-chloro-1-phthalimidoethyl)phosphorodithioate and O-6-ethoxy-2-ethylpyrimidin-4-yl O,O-dimethylphosphorothioate;

carbamate-type insecticides;

such as 1-naphthyl N-methylcarbamate, S-methyl-N-[methylcarbamoyloxy]thioacetoimidate, 2-sec-butylphenyl-N-methylcarbamate, 2-isopropoxyphenyl-N-methylcarbamate, 1,3-bis(carbamoylthio)-2-(N,N-dimethylamino)propane hydrochloride and 2-dimethylamino-5,6-dimethylpyrimidin-4-yl dimethylcarbamate;

and other insecticides;

such as nicotine sulphate, milbemycin D, 6-methyl-2,3-quinoxalinedithiocyclic S,S-dithiocarbonate, 2,4-dinitro-6-sec-butylphenyldimethylacrylate, 1,1-bis(p-chlorophenyl)-2,2,2-trichloroethanol, azoxybenzene, di(p-chlorophenyl)cyclopropyl carbinol, isopropyl 4,4'-dichlorobenzilate, ethyl 4,4'-dichlorobenzilate, ethyl O-benzoyl-3-chloro-2,6-dimethoxybenzohydroxymate, isopropyl 4,4'-dibromobenzilate, tricyclohexyltin hydroxide, hexakis(β,β-dimethylphenethyl)distanoxane, 2-(4-t-butylphenoxy)cyclohexylpropinylsulphide, 3-methyl-1,5-bis(2,4-xylyl)-1,3,5-triazapenta-1,4-diene, 2,4,5,4'-tetrachlorodiphenyl sulphone, hexachlorohexahydromethanobenzodioxathiepine oxide, 5-dimethylamino-1,2,3-trithiane hydrogen oxalate and machine oil.

However, the nature of any such additional insecticide is not critical.

Certain of the compounds of the present invention can also be used as pharmaceuticals for the treatment of fungal infections, whether of the skin, in which case they are normally administered topically, or internal, in which case they may be administered orally or parenterally.

Those compounds of the present invention which are currently thought to be of especial value as pharmaceuticals are the compounds of formula (I) in which:

Ar, $R^1$, X and m are as defined above;

Y represents a group of formula $-N(R^5)CO-$, $-N(R^5)CO-CH=CH-$, -O-CO-, -S-CO- or -S-CO-CH=CH-, in which:

$R^5$ represents a hydrogen atom or a $C_1$ - $C_4$ alkyl group;

$R^2$ represents a $C_1$ - $C_6$ alkyl group, a $C_1$ - $C_6$ haloalkyl group, a phenyl group, a phenyl group having at least one of substituents (a), defined above, a naphthyl group or a heterocyclic group having 5 or 6 ring atoms of which from 1 to 3 are nitrogen and/or oxygen and/or sulphur hetero-atoms, said heterocyclic group being unsubstituted or having from 1 to 3 of substituents (b), defined above;

or

$-Y-R^2$ represents an azido group; and

$R^3$ represents a hydrogen atom;

or

$R^3$ and $-X_m-Y-R^2$ together represent said group of formula (II), defined above, in which $R^2$, p and q are as defined above;

and acid addition salts thereof.

Where the compound of the present invention is employed for pharmaceutical use, it may be administered in the form of any conventional pharmaceutical formulation, the nature of which will, as is well known, depend on the route of administration and the nature of the condition to be treated. Thus, the compounds of the invention may be formulated in conventional dosage forms, normally in admixture with a pharmaceutical carrier or diluent. For oral administration, the compounds can be formulated, for example, as tablets, capsules, granules, powders or syrups. For parenteral administration, they may be formulated as injections in a suitable liquid or as suppositories. For topical administration, they may be formulated as

ointments, creams, powders, liquids or aerosols.

The dosage and frequency of dose may vary depending upon the symptoms, age and body weight of the patient, as well as upon the route of administration, but, in general, the compounds of the invention may be administered orally in a daily dose of from 50 to 2,000 mg for an adult, preferably a dosage of from 100 to 600 mg, which may be administered either as single dose or as divided doses.

The invention is further illustrated by the following Examples, which describe the preparation of various of the compounds of the invention, as well as the preparation of various of the starting materials which may be used in the Examples for the preparation of compounds of the present invention. In the Tables which appear in the Examples, the abbreviations used are as defined previously in relation to Tables 1 and 2. In the compound names used, the symbol R* indicates that the compound in question is a racemic mixture of the R and S isomers. Thus. (2R*, 3R*) means a 1 : 1 mixture of (2R, 3R) and (2S, 3S) and is the same as (2RS, 3RS) and the same as (2S*, 3S*), and (2R*, 3S*) means a 1 : 1 mixture of (2R, 3S) and (2S, 3R) and is the same as (2S*, 3R*).

## EXAMPLE 1

### (2R*, 3R*)-2-(2,4-Dichlorophenyl)-3-(methanesulphonyloxy)-1-(1H-1,2,4-triazol-1-yl)-2-butanol

4.8 g (42 mmoles) of methanesulphonyl chloride were added, whilst ice-cooling and stirring, to a solution of 10.0 g (33 mmoles) of (2R*, 3R*)-2-(2,4-dichlorophenyl)-1-(1H-1,2,4-triazol-1-yl)-2,3-butanediol [prepared by the procedure described in the Abstract Papers of the 8th Symposium on Medicinal Chemistry, Osaka (1986), page 9] dissolved in 400 ml of pyridine. The temperature of the reaction mixture was allowed to rise to room temperature, and then the mixture was stirred for 2 hours. At the end of this time, the pyridine was distilled off under reduced pressure, and the residue was mixed with a dilute aqueous solution of sodium bicarbonate. The resulting solution was then extracted twice with diethyl ether. The combined organic extracts were washed with a saturated aqueous solution of sodium chloride and then freed from the diethyl ether by distillation. The crystalline residue was recrystallized from a mixture of ethyl acetate and hexane, to afford 11.9 g of the title compound, melting at 157 - 159 °C.

## EXAMPLE 2

### (2R*, 3R*)-3-Azido-2-(2,4-dichlorophenyl)-1-(1H-1,2,4-triazol-1-yl)-2-butanol

9.69 g (149 mmoles) of sodium azide and 1.55 g (29 mmoles) of ammonium chloride were added to a solution of 11.0 g (29 mmoles) of (2R*, 3R*)-2-(2,4-dichlorophenyl)-1-(1H-1,2,4-triazol-1-yl)-3-(methanesulphonyloxy)-2-butanol(prepared as described in Example 1) dissolved in 160 ml of dimethylformamide. The mixture was then heated at 115 °C for 15 hours, whilst stirring. At the end of this time, the reaction mixture was freed from the solvent by evaporation under reduced pressure. The residue was diluted with 700 ml of benzene and washed with water. It was then dried and the solvent was distilled off under reduced pressure. The resulting crystalline residue was then recrystallized from diethyl ether, to afford 6.42 g of the title compound, melting at 113 - 114 °C.

## EXAMPLE 3

### (2R*, 3R*)-3-Amino-2-(2,4-dichlorophenyl)-1-(1H-1,2,4-triazol-1-yl)-2-butanol

A solution of 6.42 g (19.6 mmoles) of (2R*, 3R*)-3-azido-2-(2,4-dichlorophenyl)-1-(1H-1,2,4-triazol-1-yl)-2-butanol (prepared as described in Example 2) dissolved in 128 ml of ethanol was stirred for 1 hour in the presence of 1.67 g of 10% w/w palladium-on-charcoal in an atmosphere of hydrogen under atmospheric pressure. At the end of this time, the reaction mixture was filtered and the filtrate was concentrated by evaporation under reduced pressure. The resulting crystalline residue was recrystallized from diethyl ether, to afford 4.90 g of the title compound, melting at 104 - 105 °C.

EXAMPLE 4

(2R*, 3R*)-2-(2,4-Dichlorophenyl)-3-(4-fluorobenzoylamino)-1-(1H-1,2,4-triazol-1-yl)-2-butanol

50 mg (0.32 mmoles) of 4-fluorobenzoyl chloride were added at 0°C, whilst stirring, to a solution of 68 mg (0.23 mmoles) of (2R*, 3R*)-3-amino-2-(2,4-dichlorophenyl)-1-(1H-1,2,4-triazol-1-yl)-2-butanol (prepared as described in Example 3) dissolved in 1 ml of pyridine. The mixture was then kept at the same temperature for 50 minutes, after which 0.2 ml of methanol was added, and then the mixture was stirred for 10 minutes at the same temperature. At the end of this time, the solvent was removed by distillation under reduced pressure. The residue was mixed with a dilute aqueous solution of sodium bicarbonate, and then the mixture was extracted with ethyl acetate. The organic extract was washed with a saturated aqueous solution of sodium chloride, dried and then concentrated by evaporation under reduced pressure. The crystalline residue was then recrystallized from ethyl acetate, to give 59 mg of the title compound, melting at 217°C.

Infrared Absorption Spectrum (KBr) $\nu_{max}$ $cm^{-1}$:
3440, 3280, 1650, 1503.

Nuclear Magnetic Resonance Spectrum ($CDC\ell_3$) δ ppm:
0.95 (3H, doublet, J = 7 Hz);
4.41 (1H, doublet, J = 15 Hz);
5.45 (2H, multiplet);
5.64 (1H, doublet, J = 15 Hz);
6.76 (1H, broad doublet);
7.11 (1H, doublet of doublets, J = 9 & 2 Hz);
7.17 (2H, triplet, J = 8.5 Hz);
7.36 (1H, doublet, J = 2 Hz);
7.56 (1H, doublet, J = 9 Hz);
7.80 (1H, singlet);
7.84 (1H, singlet);
7.91 (2H, doublet of doublets, J = 8.5 & 6 Hz).

EXAMPLE 5

(2R*, 3R*)-3-(4-Chlorobenzoylamino)-2-(2,4-dichlorophenyl)-1-(1H-1,2,4-triazol-1-yl)-2-butanol and its oxalate

162 mg (0.925 mmoles) of 4-chlorobenzoyl chloride were added at 0°C, whilst stirring, to a solution of 197 mg (0.654 mmoles) of (2R*, 3R*)-3-amino-2-(2,4-dichlorophenyl)-1-(1H-1,2,4-triazol-1-yl)-2-butanol (prepared as described in Example 3) dissolved in 2.5 ml of pyridine. The mixture was then kept at the same temperature for 45 minutes, after which 0.5 ml of methanol was added, and the mixture was stirred at the same temperature for 10 minutes. At the end of this time, the reaction mixture was freed from the solvent by distillation under reduced pressure. The residue was mixed with a dilute aqueous solution of sodium bicarbonate and then extracted with ethyl acetate. The organic extract was washed with a saturated aqueous solution of sodium chloride, dried and then concentrated by evaporation under reduced pressure. The crystalline residue was recrystallized from a 3 : 1 by volume mixture of diethyl ether and hexane, to afford 221 mg of (2R*, 3R*)-3-(4-chlorobenzoylamino)-2-(2,4-dichlorophenyl)-1-(1H-1,2,4-triazol-1-yl)-2-butanol, melting at 217 - 219°C.

Infrared Absorption Spectrum ($CHC\ell_3$) $\nu_{max}$ $cm^{-1}$:
3430, 1655, 1585, 1505.

Nuclear Magnetic Resonance Spectrum ($CDC\ell_3$) δ ppm:
0.95 (3H, doublet, J = 7 Hz);
4.40 (1H, doublet, J = 15 Hz);
5.46 (2H, multiplet);
5.63 (1H, doublet, J = 15 Hz);
6.88 (1H, broad doublet);
7.11 (1H, doublet of doublets, J = 9 & 2 Hz);
7.35 (1H, doublet, J = 2 Hz);
7.45 (2H, doublet, J = 8 Hz);
7.54 (1H, doublet, J = 9 Hz);
7.78 (1H, singlet);

7.83 (2H, doublet, J = 8 Hz);
7.84 (1H, singlet).

203 mg of (2R*, 3R*)-3-(4-chlorobenzoylamino)-2-(2,4-dichlorophenyl)-1-(1H-1,2,4-triazol-1-yl)-2-butanol (prepared as described above) were dissolved in ethyl acetate, with heating, and 46 mg of oxalic acid were added to the resulting solution. The solution was then cooled to precipitate crystals, which were collected by filtration, giving 225 mg of the oxalate of the title compound, melting at 178 - 182°C.

EXAMPLE 6

(2R, 3R)-2-(2,4-Dichlorophenyl)-1-(1H-1,2,4-triazol-1-yl)-2,3-butanediol

4.39 g (14.5 mmoles) of (2R*, 3R*)-2-(2,4-dichlorophenyl)-1-(1H-1,2,4-triazol-1-yl)-2,3-butanediol (racemate) [prepared by the procedure described in the Abstract Papers of the 8th Symposium on Medicinal Chemistry, Osaka (1986), page 9] were dissolved in 100 ml of methanol, with heating. 3.54 g (15.2 mmoles) of ℓ-camphorsulphonic acid were then added to the resulting solution. 330 ml of diethyl ether were then added, and the resulting precipitated crystals were collected by filtration. The yield was 3.4 g.
Specific rotation: $[\alpha]_D^{25}$ -92° (c = 0.90, methanol).

The ℓ-camphorsulphonic acid salt of the title compound was recrystallized by adding 150 ml of diethyl ether to a solution of the crude crystals, obtained as described above, which had been dissolved in 50 ml of methanol, with heating, to afford a pure specimen, melting at 209 - 212°C. The yield was 1.95 g.
Specific rotation $[\alpha]_D^{25}$ -99° (c = 1.00, methanol).

A mixture of 1.9 g of the optically active ℓ-camphorsulphonic acid salt prepared as described above, 70 ml of ethyl acetate and 35 ml of a dilute aqueous solution of sodium bicarbonate were stirred together, and then the ethyl acetate extract was separated. The extract was then washed with water and with a saturated aqueous solution of sodium chloride, in that order, after which it was dried. The solvent was then removed by distillation under reduced pressure, to afford 1.05 g of the title compound as crystals, which were recrystallized from a mixture of ethyl acetate and hexane to afford a pure specimen melting at 110°C.
Specific rotation $[\alpha]_D^{25}$ = -111° (c = 0.74, methanol).

EXAMPLE 7

(2R, 3R)-2-(2,4-Dichlorophenyl)-3-(methanesulphonyloxy)-1-(1H-1,2,4-triazol-1-yl)-2-butanol

The procedure described in Example 1 was repeated, but using 2.1 g of (2R, 3R)-2-(2,4-dichlorophenyl)-1-(1H-1,2,4-triazol-1-yl)-2,3-butanediol [(-)-enantiomer] (prepared as described in Example 6), to afford 2.37 g of the title compound, melting at 140 - 143°C.
Specific rotation: $[\alpha]_D^{25}$ = -138° (c = 1.02, $CHCl_3$).

EXAMPLE 8

(2R, 3R)-3-Azido-2-(2,4-dichlorophenyl)-1-(1H-1,2,4-triazol-1-yl)-2-butanol

The procedure described in Example 2 was repeated, but using 2.36 g of (2R, 3R)-2-(2,4-dichlorophenyl)-3-(methanesulphonyloxy)-1-(1H-1,2,4-triazol-1-yl)-2-butanol [(-)-enantiomer] (prepared as described in Example 7), to afford 1.42 g of the title compound, melting at 154 - 156°C.
Specific rotation: $[\alpha]_D^{25}$ = -100° (c = 0.89, $CHCl_3$).

EXAMPLE 9

(2R, 3R)-3-Amino-2-(2,4-dichlorophenyl)-1-(1H-1,2,4-triazol-1-yl)-2-butanol

The procedure described in Example 3 was repeated, but using 1.20 g of (2R, 3R)-3-azido-2-(2,4-dichlorophenyl)-1-(1H-1,2,4-triazol-1-yl)-2-butanol [(-)-enantiomer] (prepared as described in Example 8), to afford 0.75 g of the title compound, melting at 238 - 242°C (with decomposition).
Specific rotation: $[\alpha]_D^{25}$ = -74° (c = 0.50, methanol).

EXAMPLE 10

(2R, 3R)-3-(4-Chlorobenzoylamino)-2-(2,4-dichlorophenyl)-1-(1H-1,2,4-triazol-1-yl)-2-butanol and its oxalate

A procedure similar to that described in Example 5 was repeated, except that 230 mg of (2R, 3R)-3-amino-2-(2,4-dichlorophenyl)-1-(1H-1,2,4-triazol-1-yl)-2-butanol [(-)-enantiomer] (prepared as described in Example 9) were used, to afford 283 mg of the title compound as an oil.
Specific rotation: $[\alpha]_D^{25}$ = -104° (c = 0.90, $CHC\ell_3$).

Melting point of the oxalate: 121 - 123°C.

EXAMPLE 11

(2R*, 3R*)-3-(2,4-Dichlorobenzoylamino)-2-(2,4-dichlorophenyl)-1-(1H-1,2,4-triazol-1-yl)-2-butanol

58 mg (0.28 mmoles) of 2,4-dichlorobenzoyl chloride were added at 0°C, whilst stirring, to a solution of 56.5 mg (0.188 mmoles) of (2R*, 3R*)-3-amino-2-(2,4-dichlorophenyl)-1-(1H-1,2,4-triazol-1-yl)-2-butanol (prepared as described in Example 3) dissolved in 1 ml of pyridine. The mixture was then kept at the same temperature for 50 minutes, after which 0.2 ml of methanol was added, and the mixture was stirred for 15 minutes. At the end of this time, the reaction mixture was freed from the solvent by distillation under reduced pressure. The residue was then mixed with a dilute aqueous solution of sodium bicarbonate and extracted with ethyl acetate. The organic extract was washed with a saturated aqueous solution of sodium chloride, dried and then concentrated by evaporation under reduced pressure. The residue was recrystallized from a mixture of hexane and ethyl acetate, to afford 48 mg of the title compound, melting at 180 - 181°C.

Infrared Absorption Spectrum (KBr) $\nu_{max}$ $cm^{-1}$:
3360, 1670, 1585, 1530.

Nuclear Magnetic Resonance Spectrum ($CDC\ell_3$) $\delta$ ppm:
0.98 (3H, doublet, J = 7 Hz);
4.58 (1H, doublet, J = 15 Hz);
5.50 (2H, multiplet);
5.68 (1H, doublet, J = 15 Hz);
6.97 (1H, broad doublet);
7.15 - 7.8 (6H, multiplet);
7.81 (1H, singlet);
7.85 (1H, singlet).

EXAMPLE 12

(2R*, 3R*)-2-(2,4-Dichlorophenyl)-1-(1H-1,2,4-triazol-1-yl)-3-(4-trifluoromethylbenzoylamino)-2-butanol

122.6 mg (0.588 mmoles) of 4-trifluoromethylbenzoyl chloride were added at 0°C, whilst stirring, to a solution of 126.5 mg (0.42 mmoles) of (2R*, 3R*)-3-amino-2-(2,4-dichlorophenyl)-1-(1H-1,2,4-triazol-1-yl)-2-butanol (prepared as described in Example 3) dissolved in 2 ml of pyridine. The mixture was then kept at the same temperature for 50 minutes, after which 0.3 ml of methanol was added, and the mixture was stirred for 15 minutes. At the end of this time, the reaction mixture was freed from the solvent by distillation under reduced pressure. The residue was mixed with a dilute aqueous solution of sodium bicarbonate and extracted with ethyl acetate. The organic extract was washed with a saturated aqueous solution of sodium chloride, dried and then concentrated by evaporation under reduced pressure. The crystalline residue was recrystallized from a mixture of ethyl acetate with a small quantity of hexane, to afford 134 mg of the title compound, melting at 186°C.

Infrared Absorption Spectrum (KBr) $\nu_{max}$ $cm^{-1}$:
3360, 1665, 1530.

Nuclear Magnetic Resonance Spectrum ($CDC\ell_3$) $\delta$ ppm:
0.99 (3H, doublet, J = 7 Hz);
4.40 (1H, doublet, J = 15 Hz);
5.49 (2H multiplet);
5.68 (1H, doublet, J = 15 Hz);
6.84 (1H, broad doublet);

7.10 (1H, doublet of doublets, J = 9 & 2 Hz);
7.37 (1H, doublet, J = 2 Hz);
7.56 (1H, doublet, J = 9 Hz);
7.79 (2H, doublet, J = 9 Hz);
7.83 (1H, singlet);
7.87 (1H, singlet);
8.04 (2H, doublet, J = 9 Hz).

EXAMPLE 13

(2R, 3R)-2-(2,4-Dichlorophenyl)-3-(4-trifluoromethylbenzoylamino)-1-(1H-1,2,4-triazol-1-yl)-2-butanol

A procedure similar to that described in Example 12 was repeated, except that 200 mg of (2R, 3R)-3-amino-2-(2,4-dichlorophenyl)-1-(1H-1,2,4-triazol-1-yl)-2-butanol [(-)-enantiomer] (prepared as described in Example 9) were used, to afford 221 mg of the title compound, melting at 88 - 91 °C.
Specific rotation: $[\alpha]_D^{25}$ = -114.5° (c = 1.01, $CHC\ell_3$).

EXAMPLE 14

(2R*, 3R*)-2-(2,4-Dichlorophenyl)-3-[4-(methylthio)benzoylamino]-1-(1H-1,2,4-triazol-1-yl)-2-butanol

247.9 mg (1.328 mmoles) of 4-(methylthio)benzoyl chloride were added at 0 °C, whilst stirring, to a solution of 200 mg (0.664 mmoles) of (2R*, 3R*)-3-amino-2-(2,4-dichlorophenyl)-1-(1H-1,2,4-triazol-1-yl)-2-butanol (prepared as described in Example 3) dissolved in 10 ml of pyridine. After the mixture had been allowed to stand at the same temperature for 30 minutes, the temperature of the reaction mixture was allowed to rise to room temperature, and then the mixture was stirred for 30 minutes. At the end of this time, the mixture was cooled to 0 °C, and 0.6 ml of methanol were added. After 10 minutes, the mixture was freed from the solvent by distillation under reduced pressure, and the residue was mixed with a dilute aqueous solution of sodium bicarbonate. It was then extracted with ethyl acetate. The organic extract was washed with a saturated aqueous solution of sodium chloride, dried and then concentrated by evaporation under reduced pressure. The residue was recrystallized from ethyl acetate to afford 121.7 mg of the title compound, melting at 167 - 168 °C.

Infrared Absorption Spectrum (KBr) $\nu_{max}$ $cm^{-1}$:
3300, 1627, 1596, 1545, 1508.

Nuclear Magnetic Resonance Spectrum ($CDC\ell_3$) δ ppm:
0.98 (3H, doublet, J = 7 Hz);
2.51 (3H, singlet);
4.42 (1H, doublet, J = 15 Hz);
5.47 (2H, multiplet);
5.67 (1H, doublet, J = 15 Hz);
6.83 (1H, broad doublet);
7.11 (1H, doublet of doublets, J = 9 & 2 Hz);
7.32 (2H, doublet, J = 8 Hz);
7.39 (1H, doublet, J = 2 Hz);
7.58 (1H, doublet, J = 9 Hz);
7.80 (1H, singlet);
7.82 (2H, doublet, J = 8 Hz);
7.86 (1H, singlet).

EXAMPLE 15

(2R*, 3R*)-2-(2,4-Difluorophenyl)-3-(4-fluorobenzoylamino)-1-(1H-1,2,4-triazol-1-yl)-2-butanol

165.5 mg (1.044 mmoles) of 4-fluorobenzoyl chloride were added at 0 °C, whilst stirring, to a solution of 200 mg (0.746 mmoles) of (2R*, 3R*)-3-amino-2-(2,4-difluorophenyl)-1-(1H-1,2,4-triazol-1-yl)-2-butanol (prepared by a procedure similar to that described in Example 3) dissolved in 2 ml of pyridine. After 1 hour, 0.5 ml of methanol was added to the mixture at the same temperature, and then the mixture was stirred for 10 minutes. At the end of this time, the reaction mixture was freed from the solvent by distillation under

reduced pressure. The residue was mixed with a dilute aqueous solution of sodium bicarbonate and extracted with ethyl acetate. The organic extract was washed with a saturated aqueous solution of sodium chloride, dried and then concentrated by evaporation under reduced pressure. The residue was recrystallized from ethyl acetate to afford 258.5 mg of the title compound, melting at 205 °C.

Infrared Absorption Spectrum (KBr) $\nu_{max}$ $cm^{-1}$:
3280, 1634, 1600, 1530, 1502.

Nuclear Magnetic Resonance Spectrum [$CDCl_3$ + $CD_3OD$ (10 : 1 by volume)] δ ppm:
1.04 (3H, doublet, J = 7 Hz);
4.47 (1H, doublet, J = 15 Hz);
4.96 (1H, multiplet);
5.08 (1H, doublet, J = 15 Hz);
6.5 - 7.1 (2H, multiplet);
7.1 - 7.9 (1H, multiplet);
7.19 (2H, triplet, J = 9 Hz);
7.78 (1H, singlet);
7.97 (2H, doublet of doublets, J = 9 & 6 Hz);
8.01 (1H, singlet).

EXAMPLE 16

(2R*, 3R*)-3-(4-Chlorobenzoylamino)-2-(2,4-difluorophenyl)-1-(1H-1,2,4-triazol-1-yl)-2-butanol

184 mg (1.05 mmoles) of 4-chlorobenzoyl chloride were added at 0 °C, whilst stirring, to a solution of 200 mg (0.746 mmoles) of (2R*, 3R*)-3-amino-2-(2,4-difluorophenyl)-1-(1H-1,2,4-triazol-1-yl)-2-butanol (prepared by a procedure similar to that described in Example 3) dissolved in 2 ml of pyridine. After 1 hour, 0.5 ml of methanol was added to the reaction mixture at the same temperature, and then the mixture was stirred for 10 minutes. At the end of this time, the reaction mixture was freed from the solvent by distillation under reduced pressure. The residue was mixed with a dilute aqueous solution of sodium bicarbonate and extracted with ethyl acetate. The organic extract was washed with a saturated aqueous solution of sodium chloride, dried and then concentrated by evaporation under reduced pressure. The crystalline residue was recrystallized from ethyl acetate, to afford 229 mg of the title compound, melting at 221 - 222 °C.

Infrared Absorption Spectrum (KBr) $\nu_{max}$ $cm^{-1}$:
3310, 1635, 1618, 1595, 1536, 1503.

Nuclear Magnetic Resonance Spectrum [$CDCl_3$ + $CD_3OD$ (10 : 1 by volume)] δ ppm:
1.02 (3H, doublet, J = 7 Hz);
4.50 (1H, doublet, J = 15 Hz);
4.95 (1H, multiplet);
5.06 (1H, doublet, J = 15 Hz);
6.5 - 7.1 (2H, multiplet);
7.1 - 7.9 (1H, multiplet);
7.50 (2H, doublet, J = 8 Hz);
7.77 (1H, singlet);
7.90 (2H, doublet, J = 8 Hz);
8.00 (1H, singlet).

EXAMPLE 17

(2R*, 3R*)-2-(2,4-Difluorophenyl)-1-(1H-1,2,4-triazol-1-yl)-3-(4-trifluoromethylbenzoylamino)-2-butanol

217.7 mg (1.044 mmoles) of 4-trifluoromethylbenzoyl chloride were added at 0 °C, whilst stirring, to a solution of 200 mg (0.746 mmoles) of (2R*, 3R*)-3-amino-2-(2,4-difluorophenyl)-1-(1H-1,2,4-triazol-1-yl)-2-butanol (prepared by a procedure similar to that described in Example 3) dissolved in 2 ml of pyridine. After 1.5 hours, 0.3 ml of methanol was added to the reaction mixture at the same temperature, and then the mixture was stirred for 10 minutes. The solvent was then removed from the reaction mixture by distillation under reduced pressure, and the residue was mixed with a dilute aqueous solution of sodium bicarbonate and then extracted with ethyl acetate. The organic extract was washed with a saturated aqueous solution of sodium chloride, dried and then concentrated by evaporation under reduced pressure. The crystalline residue was recrystallized from ethyl acetate, to afford 254 mg of the title compound as crystals, melting at

201 - 205 °C.

Infrared Absorption Spectrum (KBr) $\nu_{max}$ $cm^{-1}$:
3375, 1666, 1540, 1502.

Nuclear Magnetic Resonance Spectrum [$CDCl_3$ + CDOD (10 : 1 by volume)] δ ppm:
1.08 (3H, doublet, J = 7 Hz);
4.50 (1H, doublet, J = 15 Hz);
4.98 (1H, multiplet);
5.11 (1H, doublet, J = 15 Hz);
6.60 - 7.16 (2H, multiplet);
7.20 - 7.90 (1H, multiplet);
7.77 (1H, singlet);
7.80 (2H, doublet, J = 8 Hz);
8.10 (1H, singlet);
8.11 (2H, doublet, J = 8 Hz).

EXAMPLE 18

(2R*, 3R*)-2-(2,4-Difluorophenyl)-3-(pentafluorobenzoylamino)-1-(1H-1,2,4-triazol-1-yl)-2-butanol

250 mg (1.09 mmoles) of pentafluorobenzoyl chloride were added at 0 °C, whilst stirring, to a solution of 200 mg (0.75 mmoles) of (2R*, 3R*)-3-amino-2-(2,4-difluorophenyl)-1-(1H-1,2,4-triazol-1-yl)-2-butanol (prepared by a procedure similar to that described in Example 3) dissolved in 2.5 ml of pyridine. The reaction mixture was then treated in a similar manner to that described in Example 17, and the resulting crude product was recrystallized from a mixture of ethyl acetate, benzene and hexane, to afford 282 mg of the title compound, melting at 111 - 116 °C.

Infrared Absorption Spectrum ($CHCl_3$) $\nu_{max}$ $cm^{-1}$:
3425, 1680, 1500.

Nuclear Magnetic Resonance Spectrum ($CDCl_3$) δ ppm:
0.98 (3H, doublet, J = 7 Hz);
4.53 (1H, doublet, J = 14 Hz);
5.09 (1H, doublet, J = 14 Hz);
5.1 (2H, multiplet);
6.6 - 7.8 (4H, multiplet);
7.82 (1H, singlet);
7.85 (1H, singlet).

EXAMPLE 19

(2R*, 3R*)-3-(4-Acetylaminobenzoylamino)-2-(2,4-difluorophenyl)-1-(1H-1,2,4-triazol-1-yl)-2-butanol

A procedure similar to that described in Example 15 was repeated, except that 85.6 mg of (2R*, 3R*)-3-amino-2-(2,4-difluorophenyl)-1-(1H-1,2,4-triazol-1-yl)-2-butanol and 88 mg of 4-acetamidobenzoyl chloride were used, to afford 69.7 mg of the title compound, melting at 254 - 255.5 °C.

Nuclear Magnetic Resonance Spectrum [$CDCl_3$ + $CD_3OD$ (3 : 1 by volume)] δ ppm:
1.01 (3H, doublet, J = 7 Hz);
2.16 (3H, singlet);
4.43 (1H, doublet, J = 15 Hz);
5.5 (1H, multiplet);
5.61 (1H, doublet, J = 15 Hz);
7.12 (1H, doublet of doublets, J = 8 & 2 Hz);
7.39 (1H, doublet, J = 2 Hz);
7.57 (1H, doublet, J = 8 Hz);
7.67 (2H, doublet, J = 9 Hz);
7.74 (1H, singlet);
7.88 (2H, doublet, J = 9 Hz);
7.99 (1H, singlet).

EXAMPLE 20 - 23

Following a procedure similar to that described in Example 4, the compounds listed below were synthesized (all as racemates) by reacting (2R*, 3R*)-3-amino-2-(2,4-dichlorophenyl)-1-(1H-1,2,4-triazol-1-yl)-2-butanol (prepared as described in Example 3) with substituted benzoyl chlorides, in which the benzoyl group is substituted with various kinds of substituent, as shown in the following Table 3. The compounds employed are identified by the numbers assigned to them in the foregoing Table 1.

Table 3

| Ex No. | Cpd No. | stereochemistry | substituent | m.p. (°C) |
|---|---|---|---|---|
| 20 | 1-12 | (2R*, 3R*) | 4-CN | 246 - 248 |
| 21 | 1-11 | (2R*, 3R*) | 4-$NO_2$ | 257 - 258.5 |
| 22 | 1-6 | (2R*, 3R*) | 2,4-$F_2$ | 129 - 130 |
| 23 | 1-212 | (2R*, 3R*) | 4-$COCH_3$ | 169 - 172 |

EXAMPLE 24

(2R*, 3R*)-2-(2,4-Dichlorophenyl)-3-[4-(methanesulphinyl)benzoylamino]-1-(1H-1,2,4-triazol-1-yl)-2-butanol

35 mg (0.19 mmoles) of m-chloroperbenzoic acid (85% purity) were added at 0°, whilst stirring, to a solution of 88 mg (0.195 mmoles) of (2R*, 3R*)-2-(2,4-dichlorophenyl)-3-[4-(methylthio)benzoylamino]-1-(1H-1,2,4-triazol-1-yl)-2-butanol (prepared as described in Example 14) dissolved in 2 ml of methylene chloride. After 15 minutes, the mixture was diluted with ethyl acetate and then washed with a dilute aqueous solution of sodium bicarbonate and with a saturated aqueous solution of sodium chloride, in that order, after which it was dried and the solvent was removed by distillation under reduced pressure. The resulting crystalline residue was recrystallized from ethyl acetate, to afford 68 mg of the title compound, melting at 206.5 - 207.5°C.

Infrared Absorption Spectrum ($CHCl_3$) $\nu_{max}$ $cm^{-1}$:
3360 (broad), 1664, 1526.

Nuclear Magnetic Resonance Spectrum ($CDCl_3$) $\delta$ ppm:
1.00 (3H, doublet, J = 6.5 Hz);
2.76 (3H, singlet);
4.45 (1H, doublet, J = 15 Hz);
5.5 (2H, multiplet);
5.67 (1H, doublet, J = 15 Hz);
7.03 (1H, broad doublet);
7.13 (1H, doublet of doublets, J = 9 & 2 Hz);
7.39 (1H, doublet, J = 2 Hz);
7.58 (1H, doublet, J = 9 Hz);
7.77 (2H, doublet, J = 8 Hz);
7.80 (1H, singlet);
7.86 (1H, singlet);
8.08 (2H, doublet, J = 8 Hz).

EXAMPLE 25

1-{3-[N-(4-Chlorophenyl)amino]-2-(2,4-dichlorophenyl)-2-hydroxypropyl}-1H-1,2,4-triazole

A mixture of 140 mg (0.52 mmoles) of 1-[2-(2,4-dichlorophenyl)-2,3-epoxypropyl]-1H-1,2,4-triazole [prepared following the procedure described in Japanese Patent Application Kokai (i.e. as laid open to public inspection) No. 58-128 383], 133 mg (1.04 mmoles) of p-chloroaniline and 0.2 ml of xylene was heated at 150°C for 5 hours under an atmosphere of nitrogen. At the end of this time, the resulting crude product was purified by column chromatography through silica gel, using a gradient system of benzene and ethyl acetate. The fractions eluted with mixtures of benzene and ethyl acetate ranging from 3 : 2 to 2 : 3 by volume were evaporated under reduced pressure, to give a crystalline residue, which was then recrystallized from a mixture of benzene and hexane, to give 52 mg of the title compound, melting at 169 - 170 °C.

Nuclear Magnetic Resonance Spectrum [$CDCl_3$ + $CD_3OD$ (2 : 1 by volume)] δ ppm:
3.78 (2H, singlet);
4.70 (1H, doublet, J = 14 Hz);
5.19 (1H, doublet, J = 14 Hz);
6.54 (2H, doublet);
7.08 (2H, doublet);
7.15 (1H, doublet of doublets, J = 9 & 2 Hz);
7.38 (1H, doublet, J = 2 Hz);
7.62 (1H, doublet, J = 9 Hz);
7.79 (1H, singlet);
8.04 (1H, singlet).

EXAMPLE 26

3-(4-Chlorophenyl)-5-(2,4-dichlorophenyl)-5-[(1H-1,2,4-triazol-1-yl)methyl]oxazolidine and its oxalate

A mixture of 41 mg (0.103 mmoles) of 1-{3-[N-(4-chlorophenyl)amino]-2-(2,4-dichlorophenyl)-2-hydroxypropyl}-1H-1,2,4-triazole (prepared as described in Example 25) and 13.7 mg (0.45 mmoles) of paraformaldehyde dissolved in 4 ml of toluene was heated at 90 °C for 3 hours. At the end of this time, the solvent was distilled off, and then the resulting oily residue was purified by column chromatography through 2 g of silica gel, using a gradient elution system with mixtures of benzene and ethyl acetate ranging from 3 : 2 to 1 : 4 by volume, to afford 42 mg of the title compound as an oil.

Nuclear Magnetic Resonance Spectrum ($CDCl_3$) δ ppm:
3.68 (1H, doublet, J = 10 Hz);
4.09 (1H, doublet, J = 10 Hz);
4.77 (2H, singlet);
4.78 (1H, doublet, J = 2.5 Hz);
5.05 (1H, doublet, J = 2.5 Hz);
6.42 (2H, doublet);
7.0 - 7.5 (4H, multiplet);
7.53 (1H, doublet, J = 8 Hz);
7.77 (1H, singlet);
8.05 (1H, singlet).

42 mg of the oxazolidine compound obtained as described above were mixed with 20 mg of oxalic acid in diethyl ether, and the precipitated crystals were collected by filtration, to afford 38 mg of the oxalate melting at 155 - 161 °C (with decomposition).

EXAMPLE 27

1-{2-(2,4-Dichlorophenyl)-2-hydroxy-3-[N-(4-methylthiophenyl)amino]propyl}-1H-1,2,4-triazole

A mixture of 250 mg (0.93 mmoles) of 1-[2-(2,4-dichlorophenyl)-2,3-epoxypropyl]-1H-1,2,4-triazole [prepared following the procedure described in Japanese Patent Application Kokai No. 58-128 383], 260 mg (1.87 mmoles) of p-(methylthio)aniline and 0.3 ml of xylene was heated at 140 °C for 5 hours under an atmosphere of nitrogen. At the end of this time, the resulting crude product was purified by column chromatography through silica gel, using a gradient elution system with mixtures of benzene and ethyl acetate ranging from 3 : 2 to 2 : 3, to give 87 mg of the title compound as an oil.

Nuclear Magnetic Resonance Spectrum ($CDCl_3$) δ ppm:
2.40 (3H, singlet);
3.5 - 4.2 (3H, multiplet);
4.70 (1H, doublet, J = 14 Hz);
5.09 (1H, singlet);
5.23 (1H, doublet, J = 14 Hz);
6.56 (2H, doublet);
7.15 (1H, doublet of doublets, J = 9 & 2 Hz);
7.17 (2H, doublet);
7.35 (1H, doublet, J = 2 Hz);

7.65 (1H, doublet, J = 9 Hz);
7.82 (1H, singlet);
7.93 (1H, singlet).

EXAMPLE 28

5-(2,4-Dichlorophenyl)-3-[4-(methylthio)phenyl]-5-[(1H-1,2,4-triazol-1-yl)methyl]oxazolidine and its oxalate

A mixture of 234 mg (0.572 mmoles) of 1-{2-(2,4-dichlorophenyl)-2-hydroxy-3-[N-(4-methylthiophenyl)-amino]propyl}-1H-1,2,4-triazole (prepared as described in Example 27) and 76 mg (2.53 mmoles) of paraformaldehyde in toluene was heated at 90 °C for 2 hours. The solvent was then removed by distillation under reduced pressure, after which the residue was purified by column chromatography through silica gel, using a gradient elution system with mixtures of benzene and ethyl acetate ranging from 3 : 2 to 1 : 4 by volume, to afford 240 mg of the title compound as an oil.

Infrared Absorption Spectrum ($CHC\ell_3$) $\nu_{max}$ $cm^{-1}$:
1600, 1500.

Nuclear Magnetic Resonance Spectrum ($CDC\ell_3$) $\delta$ ppm:
2.40 (3H, singlet);
3.70 (1H, doublet, J = 10 Hz);
4.10 (1H, doublet, J = 10 Hz);
4.79 (2H, singlet);
4.80 (1H, doublet, J = 2.5 Hz);
5.09 (1H, doublet, J = 2.5 Hz);
6.46 (2H, doublet);
7.1 - 7.5 (4H, multiplet);
7.53 (1H, doublet, J = 8 Hz);
7.76 (1H, singlet);
8.06 (1H, singlet).

80 mg (0.19 mmoles) of the oxazolidine compound obtained as described above were mixed with 34 mg (0.38 mmoles) of oxalic acid in diethyl ether, and the precipitated crystals were collected by filtration, to afford 76 mg of the oxalate of the title compound, melting at 162 - 165 °C.

EXAMPLE 29

5-(2,4-Dichlorophenyl)-3-[4-(methanesulphinyl)phenyl]-5-[(1H-1,2,4-triazol-1-yl)methyl]oxazolidine

14.2 mg (0.082 mmoles) of m-chloroperbenzoic acid were added at 0 °C, whilst stirring, to a solution of 31.5 mg (0.075 mmoles) of 5-(2,4-dichlorophenyl)-3-[4-(methylthio)phenyl)-5-[(1H-1,2,4-triazol-1-yl)methyl]-oxazolidine (prepared as described in Example 28) dissolved in 0.8 ml of methylene chloride, and the mixture was allowed to stand for 30 minutes. At the end of this time, an aqueous solution of sodium sulphite was added to the reaction mixture, and the mixture was then extracted with chloroform. The extract was washed with a saturated aqueous solution of sodium chloride and dried. The solvent was then removed by distillation under reduced pressure, and the residue was purified by column chromatography through silica gel, using ethyl acetate containing progressively from 10 to 30% by volume of ethanol as the eluent, to afford 31 mg of the title compound, which was recrystallized from a mixture of benzene and ethyl acetate, to afford 13 mg of a diastereomer melting at 181 - 186 °C.

Nuclear Magnetic Resonance Spectrum ($CDC\ell_3$) $\delta$ ppm:
2.68 (3H, singlet);
3.83 (1H, doublet, J = 10 Hz);
4.19 (1H, doublet, J = 10 Hz);
4.70 (1H, doublet, J = 14 Hz);
4.92 (1H, doublet, J = 2.5 Hz);
4.95 (1H, doublet, J = 14 Hz);
5.14 (1H, doublet, J = 2.5 Hz);
6.60 (2H, doublet);
7.25 (1H, doublet of doublets, J = 9 & 2 Hz);
7.50 (1H, doublet, J = 2 Hz);
7.55 (2H, doublet);

7.58 (1H, doublet, J = 9 Hz);
7.79 (1H, singlet);
8.09 (1H, singlet).

EXAMPLE 30

5-(2,4-Dichlorophenyl)-3-[4-(methanesulphonyl)phenyl]-5-[(1H-1,2,4-triazol-1-yl)methyl]oxazolidine

37.8 mg (0.22 mmoles) of m-chloroperbenzoic acid were added, whilst stirring and ice-cooling, to a solution of 80 mg (0.18 mmoles) of 5-(2,4-dichlorophenyl)-3-[4-(methanesulphinyl)phenyl]-5-[(1H-1,2,4-triazol-1-yl)methyl]oxazolidine (prepared as described in Example 29) dissolved in 2.5 ml of methylene chloride, and the mixture was stirred for 1 hour. At the end of this time, an aqueous solution of sodium sulphite was added to the reaction mixture, which was then extracted with chloroform. The extract was washed with an aqueous solution of sodium bicarbonate and with a saturated aqueous solution of sodium chloride, in that order, and dried. The solvent was then removed by distillation under reduced pressure, and the crystalline residue was recrystallized from ethyl acetate, to afford 60 mg of the title compound melting at 193 - 196°C.

Nuclear Magnetic Resonance Spectrum ($CDCl_3$) δ ppm:
2.98 (3H, singlet);
3.88 (1H, doublet, J = 10 Hz);
4.20 (1H, doublet, J = 10 Hz);
4.68 (1H, doublet, J = 15 Hz);
4.94 (1H, doublet, J = 2.5 Hz);
4.95 (1H, doublet, J = 15 Hz);
5.13 (1H, doublet, J = 2.5 Hz);
6.50 (2H, doublet);
7.23 (1H, doublet of doublets, J = 9 & 2 Hz);
7.49 (1H, doublet, J = 2 Hz);
7.55 (1H, doublet, J = 9 Hz);
7.75 (2H, doublet);
7.77 (1H, singlet);
8.05 (1H, singlet).

EXAMPLE 31

5-(2,4-Difluorophenyl)-5-[(1H-1,2,4-triazol-1-yl)methyl]oxazolidine

A mixture of 400 mg (1.57 mmoles) of 1-amino-2-(2,4-difluorophenyl)-3-(1H-1,2,4-triazol-1-yl)-2-propanol [prepared following a procedure similar to that described in Japanese Patent Application Kokai No. 59-62 574], 78 mg (2.6 mmoles) of paraformaldehyde and 15 ml of toluene was stirred at 80°C for 2 hours. At the end of this time, the mixture was cooled, the remaining paraformaldehyde was filtered off and the filtrate was freed from the solvent by evaporation under reduced pressure, to give 420 mg of the title compound as an oil.

EXAMPLE 32

3-(4-Chlorobenzoyl)-5-(2,4-difluorophenyl)-5-[(1H-1,2,4-triazol-1-yl)methyl]oxazolidine and its oxalate

53 mg (0.53 mmoles) of triethylamine and 92 mg (0.53 mmoles) of 4-chlorobenzoyl chloride were added, whilst ice-cooling, to a solution of 140 mg (0.33 mmoles) of 5-(2,4-difluorophenyl)-5-[(1H-1,2,4-triazol-1-yl)methyl]oxazolidine (prepared as described in Example 31) dissolved in 1 ml of methylene chloride, and the mixture was stirred, whilst ice-cooling, for 30 minutes. At the end of this time, 0.2 ml of methanol was added, and the reaction mixture was stirred for 20 minutes, whilst ice-cooling. The reaction mixture was then partitioned between ethyl acetate and an aqueous solution of sodium bicarbonate. The separated organic layer was washed with a saturated aqueous solution of sodium chloride, dried and then concentrated by evaporation under reduced pressure. The residue was recrystallized from a mixture of chloroform and hexane, to afford 106 mg of the title compound melting at 152 - 155°C.

| Elemental analysis: | | | |
|---|---|---|---|
| Calculated for $C_{19}H_{15}C\ell F_2O_2N_4$:<br>Found: | C, 56.38%;<br>C, 56.37%; | H, 3.73%;<br>H, 3.68%; | N, 13.84%.<br>N, 13.40%. |

Nuclear Magnetic Resonance Spectrum ($CDC\ell_3$) δ ppm:
4.00 (1H, doublet, J = 12 Hz);
4.31 (1H, doublet, J = 12 Hz);
4.48 (1H, doublet, J = 15 Hz);
4.68 (1H, doublet, J = 15Hz),
5.0 - 5.3 (2H, multiplet);
6.5 - 7.5 (3H, multiplet);
7.40 (4H, singlet);
7.82 (1H, singlet);
8.03 (1H, singlet).

108 mg of the compound obtained as described above were dissolved in ethyl acetate, and 25 mg of oxalic acid were added to the solution. Concentration of the mixture by evaporation under reduced pressure, gave 107 mg of the oxalate of the title compound, melting at 150 - 155 °C.

EXAMPLE 33

3-(2,4-Dichlorobenzoyl)-5-(2,4-difluorophenyl)-5-[(1H-1,2,4-triazol-1-yl)methyl]oxazolidine and its oxalate

150 mg (0.72 mmoles) of 2,4-dichlorobenzoyl chloride were added, whilst ice-cooling, to a solution of 138 mg (0.52 mmoles) of 5-(2,4-difluorophenyl)-5-[(1H-1,2,4-triazol-1-yl)methyl]oxazolidine (prepared as described in Example 31) dissolved in 2 ml of pyridine. The mixture was then stirred at the same temperature for 30 minutes, after which 1 ml of ethanol was added to it, and the mixture was stirred at room temperature for 10 minutes. At the end of this time, the solvent was removed by distillation under reduced pressure. The residue was partitioned between ethyl acetate and an aqueous solution of sodium bicarbonate. The organic layer was washed with a saturated aqueous solution of sodium chloride, dried and then concentrated by evaporation under reduced pressure. The residue was purified by preparative thin layer chromatography using ethyl acetate as the developing solvent, to give 98 mg of the title compound as an oil.

Nuclear Magnetic Resonance Spectrum ($CDC\ell_3$) δ ppm:
3.76 (2 x 0.5H, singlet);
3.9 - 4.4 (2 x 0.5H, multiplet);
4.5 - 4.7 (2H, multiplet);
4.82 (2 x 0.5H, singlet);
5.31 (2 x 0.5H, singlet);
6.6 - 7.6 (6H, multiplet);
7.86 (1H, singlet);
8.04 (0.5H, singlet);
8.09 (0.5H, singlet).

98 mg of the compound obtained as described above were dissolved in ethyl acetate, and 23 mg of oxalic acid were added to the solution. After distilling off the solvent under reduced pressure, 96 mg of the oxalate of the title compound were obtained as a foam.

EXAMPLE 34

(4R*, 5R*)-5-(2,4-Dichlorophenyl)-4-methyl-5-[(1H-1,2,4-triazol-1-yl)methyl]oxazolidine

A mixture of 4.00 g (13.3 mmoles) of (2R*, 3R*)-3-amino-2-(2,4-dichlorophenyl)-1-(1H-1,2,4-triazol-1-yl)-2-butanol (prepared as described in Example 3) and 400 mg (13.3 mmoles) of paraformaldehyde was heated under reflux in 400 ml of benzene for 3 hours. At the end of this time, the solvent was removed by distillation under reduced pressure, to give 4.16 g of the title compound as an oil.

EXAMPLE 35

(4R*, 5R*)-3-(4-Chlorobenzoyl)-5-(2,4-dichlorophenyl)-4-methyl-5-[(1H-1,2,4-triazol-1-yl)methyl]oxazolidine and its oxalate

350 mg (2.00 mmoles) of 4-chlorobenzoyl chloride were added, whilst ice-cooling and stirring, to a solution of 420 mg (1.34 mmoles) of (4R*, 5R*)-5-(2,4-dichlorophenyl)-4-methyl-5-[(1H-1,2,4-triazol-1-yl)-methyl]oxazolidine (prepared as described in Example 34) dissolved in 10 ml of pyridine. After 1 hour, 0.8 ml of methanol was added to the mixture, which was then stirred for 10 minutes. At the end of this time, the solvent was removed by distillation under reduced pressure, and a dilute aqueous solution of sodium bicarbonate was added to the residue. The resulting mixture was then extracted with ethyl acetate. The extract was dried and then concentrated by evaporation under reduced pressure. The residue was purified by column chromatography through silica gel, using a gradient elution system with mixtures of benzene and ethyl acetate ranging from 6 : 1 to 1 : 1 by volume, to give the title compound as crystals. These were recrystallized from a mixture of ethyl acetate and hexane, to give 360 mg of a pure sample melting at 166 - 167 °C.

Infrared Absorption Spectrum ($CHCl_3$) $\nu_{max}$ $cm^{-1}$:
1640.

Nuclear Magnetic Resonance Spectrum ($CDCl_3$) $\delta$ ppm:
1.03 (3H, doublet, J = 6.5 Hz);
4.50 (1H, doublet, J = 16 Hz);
4.95 (1H, doublet, J = 16 Hz);
5.25 (3H, broad);
7.0 - 7.7 (9H, multiplet).

150 mg of the amide compound obtained as described above and 35 mg of oxalic acid were dissolved in ethyl acetate, and the solution was concentrated by evaporation under reduced pressure, to give 157 mg of the oxalate of the title compound, melting at 138 - 140 °C.

EXAMPLE 36

(4R, 5R)-5-(2,4-Dichlorophenyl)-4-methyl-5-[(1H-1,2,4-triazol-1-yl)methyl]oxazolidine

Following a procedure similar to that described in Example 34, 420 mg of the title compound were obtained as an oil from 400 mg of (2R, 3R)-3-amino-2-(2,4-dichlorophenyl)-1-(1H-1,2,4-triazol-1-yl)-2-butanol [(-)-enantiomer] (prepared as described in Example 9) and 40 mg of paraformaldehyde.

EXAMPLE 37

(4R, 5R)-3-(4-Chlorobenzoyl)-5-(2,4-dichlorophenyl)-4-methyl-5-[(1H-1,2,4-triazol-1-yl)methyl]oxazolidine and its oxalate

A procedure similar to that described in Example 35 was repeated, except that 420 mg of (4R, 5R)-5-(2,4-dichlorophenyl)-4-methyl-5-[(1H-1,2,4-triazol-1-yl)methyl]oxazolidine [(-)-enantiomer] (prepared as described in Example 36) and 350 mg of 4-chlorobenzoyl chloride were employed, to give 370 mg of the title compound as an oil.
Specific rotation: $[\alpha]_D^{25}$ = +5.42° (c = 1.07, $CHCl_3$)

205 mg of the optically active amide obtained as described above were mixed with 41 mg of oxalic acid in ethyl acetate to give 150 mg of the oxalate of the title compound, melting at 122 - 126 °C.

EXAMPLE 38

(4R*, 5R*)-5-(2,4-Difluorophenyl)-4-methyl-5-[(1H-1,2,4-triazol-1-yl)methyl]oxazolidine

Following a procedure similar to that described in Example 34, 1.10 g of the title compound were obtained as an oil from 1.00 g of (2R*, 3R*)-3-amino-2-(2,4-difluorophenyl)-1-(1H-1,2,4-triazol-1-yl)-2-butanol (prepared by a procedure similar to that described in Example 3) and 0.13 g of paraformaldehyde.

EXAMPLE 39

(4R*, 5R*)-3-(4-Chlorobenzoyl)-5-(2,4-difluorophenyl)-4-methyl-5-[(1H-1,2,4-triazol-1-yl)methyl]oxazolidine and its oxalate

144 mg (0.82 mmoles) of 4-chlorobenzoyl chloride were added at 0°C, whilst stirring, to a solution of 155 mg (0.55 mmoles) of (4R*, 5R*)-5-(2,4-difluorophenyl)-4-methyl-5-[(1H-1,2,4-triazol-1-yl)methyl]-oxazolidine (prepared as described in Example 38) dissolved in 2 ml of pyridine. The mixture was then treated in the same way as described in Example 35, to give a crude product. This was purified by column chromatography through silica gel, using a 1 : 3 by volume mixture of hexane and ethyl acetate as eluent, to give 204 mg of the title compound as an oil.

Infrared Absorption Spectrum ($CHC\ell_3$) $\nu_{max}$ $cm^{-1}$:
1638.

Nuclear Magnetic Resonance Spectrum ($CDC\ell_3$) $\delta$ ppm:
1.01 (3H, doublet, J = 7 Hz);
4.55 (2H, singlet);
4.8 - 5.5 (3H, multiplet);
6.6 - 8.0 (9H, multiplet).

40 mg of oxalic acid were added to a solution of 150 mg of the amide compound obtained as described above dissolved in ethyl acetate. The mixture was cooled to produce a precipitate, which was collected by filtration, to give 158 mg of the oxalate of the title compound, melting at 161 - 164°C.

EXAMPLE 40

(4R*, 5R*)-5-(2,4-Difluorophenyl)-4-methyl-3-(pentafluorobenzoyl)-5-[(1H-1,2,4-triazol-1-yl)methyl]oxazolidine and its oxalate

245 mg (1.06 mmoles) of pentafluorobenzoyl chloride were added at 0°C, whilst stirring, to a solution of 200 mg (0.71 mmoles) of (4R*, 5R*)-5-(2,4-difluorophenyl)-4-methyl-5-[(1H-1,2,4-triazol-1-yl)methyl]-oxazolidine (prepared as described in Example 38) dissolved in 2.5 ml of pyridine, and the mixture was allowed to stand for 1 hour. At the end of this time, 0.5 ml of methanol was added, and the mixture was allowed to stand at 0°C for 10 minutes. The solvent was then distilled from the reaction mixture under reduced pressure, and the residue was dissolved in ethyl acetate. The resulting solution was washed with a saturated aqueous solution of sodium chloride, dried and then concentrated by evaporation under reduced pressure. The resulting residue was purified by column chromatography through silica gel, using a 3 : 1 by volume mixture of benzene and ethyl acetate as eluent, to give 291 mg of the title compound as an oil.

Nuclear Magnetic Resonance Spectrum ($CDC\ell_3$) $\delta$ ppm:
0.92 (1.5H, doublet, J = 7 Hz);
1.11 (1.5H, doublet, J = 7 Hz);
4.2 (0.5H, multiplet);
4.60 (2H, singlet);
5.0 (0.5H, multiplet);
5.14 (1H, singlet);
5.45 (0.5H, doublet, J = 6 Hz);
5.75 (0.5H, doublet, J = 6 Hz);
6.6 - 7.5 (3H, multiplet);
7.62 (0.5H, singlet);
7.68 (0.5H, singlet);
7.71 (0.5H, singlet);
7.77 (0.5H, singlet).

50 mg of oxalic acid were added to a solution of 200 mg of the amide compound obtained as described above dissolved in ethyl acetate, and the mixture was cooled to produce a precipitate, which was collected by filtration, to give 225 mg of the oxalate of the title compound, melting at 140°C (with decomposition).

EXAMPLE 41

(4R*, 5R*)-5-(2,4-Difluorophenyl)-4-methyl-5-[(1H-1,2,4-triazol-1-yl)methyl]-3-[4-(trifluoromethyl)benzoyl]-oxazolidine and its oxalate

206 mg (0.99 mmoles) of 4-(trifluoromethyl)benzoyl chloride were added at 0 °C, whilst stirring, to a solution of 185 mg (0.66 mmoles) of (4R*, 5R*)-5-(2,4-difluorophenyl)-4-methyl-5-[(1H-1,2,4-triazol-1-yl)-methyl]oxazolidine (prepared as described in Example 38) dissolved in 2.5 ml of pyridine. The reaction mixture was then treated in the same manner as described in Example 40, to give a crude product. This was purified by column chromatography through silica gel, using a 3 : 1 by volume mixture of benzene and ethyl acetate as eluent, to afford 245 mg of the title compound as an oil.

Infrared Absorption Spectrum ($CHC\ell_3$) $\nu_{max}$ $cm^{-1}$:
1645.

Nuclear Magnetic Resonance Spectrum ($CDC\ell_3$) $\delta$ ppm:
1.01 (3H, doublet, J = 6 Hz);
4.55 (2H, singlet);
4.9 - 5.5 (3H, multiplet);
6.6 - 7.6 (3H, multiplet);
7.6 - 7.9 (6H, multiplet).

50 mg of oxalic acid were added to a solution of 220 mg of the amide compound obtained as described above dissolved in ethyl acetate. The mixture was then cooled to produce a precipitate, which was collected by filtration, to afford 242 mg of the oxalate of the title compound, melting at 176 - 179 °C.

EXAMPLE 42

(2R*, 3R*)-3-(4-Chlorobenzoyloxy)-2-(2,4-dichlorophenyl)-1-(1H-1,2,4-triazol-1-yl)-2-butanol

118 mg (0.67 mmole) of 4-chlorobenzoyl chloride were added, whilst ice-cooling, to a suspension of 164 mg (0.54 mmole) of (2R*, 3R*)-2-(2,4-dichlorophenyl)-1-(1H-1,2,4-triazol-1-yl)-2,3-butanediol [prepared by the procedure described in the Abstract Papers of the 8th Symposium on Medicinal Chemistry, Osaka (1986), page 9] in 4 ml of pyridine, and the mixture was stirred at room temperature for 40 minutes. During this period the starting material gradually dissolved to form a solution. The solvent was then removed by distillation under reduced pressure, and the residue was partitioned between ethyl acetate and an aqueous solution of sodium bicarbonate. The organic layer was washed with a saturated aqueous solution of sodium chloride, dried and then freed from the solvent by evaporation under reduced pressure. The residue was recrystallized from a mixture of ethyl acetate and hexane, to afford 207 mg of the title compound, melting at 214 - 215 °C.

Infrared Absorption Spectrum ($CHC\ell_3$) $\nu_{max}$ $cm^{-1}$:
3400, 1715, 1502, 1270.

Nuclear Magnetic Resonance Spectrum ($CDC\ell_3$) $\delta$ ppm:
1.13 (3H, doublet, J = 7 Hz);
4.54 (1H, doublet, J = 15 Hz);
5.51 (1H, doublet, J = 15 Hz);
6.27 (1H, quartet, J = 7 Hz);
7.0 - 8.2 (9H, multiplet).

EXAMPLE 43

(2R*, 3S*)-2-(2,4-Dichlorophenyl)-3-methyl-2-[(1H-1,2,4-triazol-1-yl)methyl]oxirane

535 mg (12.3 mmoles) of sodium hydride (as a 55% w/w suspension in mineral oil) were washed with dry hexane and then suspended in 6 ml of dimethylformamide. The suspension was then cooled with ice. A solution of 4.77 g (12.5 mmoles) of (2R*, 3R*)-2-(2,4-dichlorophenyl)-3-(methanesulphonyloxy)-1-(1H-1,2,4-triazol-1-yl)-2-butanol (prepared as described in Example 1) in 48 ml of dimethylformamide was then added dropwise, whilst stirring, to the suspension. The reaction mixture was then stirred at the same temperature for 30 minutes, after which it was poured into 300 ml of ice-water, and extracted with benzene. The organic extract was washed with a saturated aqueous solution of sodium chloride and then evaporated under reduced pressure, to remove the solvent and afford 3.50 g of the title compound as an oil.

Nuclear Magnetic Resonance Spectrum ($CDC\ell_3$) $\delta$ ppm:
1.63 (3H, doublet, J = 6 Hz);
3.18 (1H, quartet, J = 6 Hz);
4.37 (1H, doublet, J = 15 Hz);
4.99 (1H, doublet, J = 15 Hz);
6.90 (1H, doublet, J = 8 Hz);
7.11 (1H, doublet of doublets, J = 8 & 2 Hz);
7.38 (1H, doublet, J = 2 Hz);
7.83 (1H, singlet);
7.99 (1H, singlet).

EXAMPLE 44

(2R*, 3R*)-2-(2,4-Dichlorophenyl)-3-(4-methoxybenzylthio)-1-(1H-1,2,4-triazol-1-yl)-2-butanol

1.09 g of sodium hydride (as a 55% w/w suspension in mineral oil) was washed with dry hexane, after which it was suspended in 31 ml of dimethylformamide and the suspension was ice-cooled. 4.86 g (31.5 mmoles) of 4-methoxy-α-toluenethiol were then added dropwise to the suspension, and the resulting reaction mixture was stirred for 7 minutes, whilst ice-cooling, after which it was stirred at room temperature for 20 minutes. At the end of this time, a solution of 3.58 g (12.6 mmoles) of (2R*, 3R*)-2-(2,4-dichlorophenyl)-3-methyl-2-[(1H-1,2,4-triazol-1-yl)methyl]oxirane (prepared as described in Example 43) in 26 ml of dimethylformamide was added to the mixture, and the mixture was stirred at 60 °C for 45 minutes. It was then allowed to return to room temperature, was poured into 340 ml of ice-water, and was then extracted with benzene. The organic extract was washed with water and with a saturated aqueous solution of sodium chloride, in that order, dried and then freed from the solvent by evaporation under reduced pressure. The resulting crude product was purified by column chromatography through 75 g of silica gel, using a gradient elution system with mixtures of ethyl acetate and hexane ranging from 3 : 7 to 1 : 1 by volume, to afford 4.10 g of the title compound as an oil.

Infrared Absorption Spectrum (liquid film) $\nu_{max}$ $cm^{-1}$:
3400, 1615, 1586, 1517.

Nuclear Magnetic Resonance Spectrum ($CDC\ell_3$) $\delta$ ppm:
1.03 (3H, doublet, J = 7 Hz);
3.64 (1H, quartet, J = 7 Hz);
3.82 (5H, singlet);
4.85 (1H, singlet);
4.94 (2H, singlet);
6.90 (2H, doublet, J = 9 Hz);
7.05 (1H, doublet of doublets, J = 9 & 2 Hz);
7.23 (1H, doublet, J = 2 Hz);
7.33 (2H, doublet, J = 9 Hz);
7.49 (1H, doublet, J = 9 Hz);
7.72 (2H, singlet).

EXAMPLE 45

(2R*, 3R*)-2-(2,4-Dichlorophenyl)-3-mercapto-1-(1H-1,2,4-triazol-1-yl)-2-butanol

A mixture of 2.19 g (5.00 mmoles) of (2R*, 3R*)-2-(2,4-dichlorophenyl)-3-(4-methoxybenzylthio)-1-(1H-1,2,4-triazol-1-yl)-2-butanol (prepared as described in Example 44), 5.4 g of anisole, 28.5 g of trifluoroacetic acid and 1.65 g of trifluoromethanesulphonic acid was stirred at room temperature for 17 minutes. At the end of this time, the reaction mixture was concentrated by evaporation under reduced pressure, and the residue was mixed with a dilute aqueous solution of sodium bicarbonate and then extracted with ethyl acetate. The organic extract was washed with a saturated aqueous solution of sodium chloride, dried and then freed from the solvent by distillation under reduced pressure, to afford 2.67 g of an oily mixture containing the title compound.

EXAMPLE 46

(2R*, 3R*)-2-(2,4-Dichlorophenyl)-1-(1H-1,2,4-triazol-1-yl)-3-[4-(trifluoromethyl)benzoylthio]-2-butanol

1.40 g (6.7 mmoles) of 4-(trifluoromethyl)benzoyl chloride was added, whilst ice-cooling and stirring, to a solution of 2.67 g of the mixture containing (2R*, 3R*)-2-(2,4-dichlorophenyl)-3-mercapto-1-(1H-1,2,4-triazol-1-yl)-2-butanol described in Example 45 (corresponding to 5.0 mmoles of the aforementioned compound) dissolved in 26 ml of pyridine. The mixture was then stirred at the same temperature for 30 minutes and at room temperature for 50 minutes, after which it was cooled with ice, and 2.5 ml of methanol were added to it. It was then stirred for 10 minutes. At the end of this time, the reaction mixture was freed from the solvent by distillation under reduced pressure, and the residue was mixed with a dilute aqueous solution of sodium bicarbonate and extracted with ethyl acetate. The organic extract was washed with a saturated aqueous solution of sodium chloride, dried and then evaporated to remove the solvent. The crystalline residue was recrystallized from methanol, to afford 0.85 g of the title compound, melting at 161 - 164 °C.

Infrared Absorption Spectrum ($CHCl_3$) $\nu_{max}$ $cm^{-1}$:
3350, 1650.

Nuclear Magnetic Resonance Spectrum ($CDCl_3$) $\delta$ ppm:
1.18 (3H, doublet, J = 7 Hz);
4.67 (1H, doublet, J = 14 Hz);
5.15 (1H, quartet, J = 7 Hz);
5.4 (1H, broad);
5.60 (1H, doublet, J = 14 Hz);
7.12 (1H, doublet of doublets, J = 8 & 2 Hz);
7.40 (1H, doublet, J = 2 Hz);
7.58 (1H, doublet, J = 8 Hz);
7.78 (2H, doublet, J = 8 Hz);
7.87 (2H, singlet);
8.2 (2H, doublet, J = 8 Hz).

EXAMPLE 47

(2R*, 3R*)-2-(2,4-Difluorophenyl)-3-(methanesulphonyloxy)-1-(1H-1,2,4-triazol-1-yl)-2-butanol

8.0 g (70 mmoles) of methanesulphonyl chloride were added at 0 °C over a period of 3 minutes, whilst stirring, to a solution of 15.0 g (55.7 mmoles) of (2R*, 3R*)-2-(2,4-difluorophenyl)-1-(1H-1,2,4-triazol-1-yl)-2,3-butanediol [prepared by the procedure described in the Abstract Papers of the 8th Symposium on Medicinal Chemistry, Osaka (1986), page 9] dissolved in 350 ml of pyridine, and the mixture was stirred for 1.5 hours at the same temperature. At the end of this time, the reaction mixture was freed from pyridine by distillation under reduced pressure. The residue was mixed with a dilute aqueous solution of sodium bicarbonate and extracted with diethyl ether. The extract was dried and then concentrated by evaporation under reduced pressure, to afford 18.0 g of the title compound, melting at 122 - 125 °C.

Infrared Absorption Spectrum ($CHCl_3$) $\nu_{max}$ $cm^{-1}$:
3400, 1620, 1500.

Nuclear Magnetic Resonance Spectrum ($CDCl_3$) $\delta$ ppm:
1.27 (3H, doublet, J = 6.5 Hz);
3.09 (3H, singlet);
4.70 (1H, doublet, J = 14 Hz);
5.03 (1H, broad doublet, J = 14 Hz);
5.35 (1H, quartet, J = 6.5 Hz);
6.5 - 7.6 (3H, multiplet);
7.80 (1H, singlet);
7.96 (1H, singlet).

EXAMPLE 48

(2R*, 3R*)-2-(2,4-Difluorophenyl)-3-(4-methoxybenzylthio)-1-(1H-1,2,4-triazol-1-yl)-2-butanol

985 mg (22.6 mmoles) of sodium hydride (as a 55% w/w suspension in mineral oil) were washed with dry hexane and then suspended in 20 ml of dimethylformamide. 2.72 g (17.6 mmoles) of 4-methoxy-α-toluenethiol were then added to the suspension, whilst ice-cooling, and the resulting mixture was stirred at the same temperature for 5 minutes and then at room temperature for 15 minutes. At the end of this time, a solution of 2.45 g (7.05 mmoles) of (2R*, 3R*)-2-(2,4-difluorophenyl)-3-(methanesulphonyloxy)-1-(1H-1,2,4-triazol-1-yl)-2-butanol (prepared as described in Example 47) in 25 ml of dimethylformamide was added to the mixture, and the mixture was stirred at room temperature for 1 hour. The reaction mixture was then poured into 200 ml of ice-water and extracted with benzene. The organic extract was washed with ice-water and with a saturated aqueous solution of sodium chloride, in that order, after which it was dried and then freed from the solvent by distillation under reduced pressure. The resulting residue was purified by column chromatography through 40 g of silica gel, using a 2 : 3 by volume mixture of ethyl acetate and hexane as eluent, to afford 1.75 g of the title compound as an oil.

Infrared Absorption Spectrum ($CHC\ell_3$) $\nu_{max}$ $cm^{-1}$:
3420, 1612, 1510, 1500.

Nuclear Magnetic Resonance Spectrum ($CDC\ell_3$) δ ppm:
1.12 (3H, doublet, J = 7 Hz);
3.18 (1H, quartet, J = 7 Hz);
3.79 (5H, singlet);
4.37 (1H, doublet, J = 14 Hz);
4.98 (1H, doublet, J = 14 Hz);
6.5 - 7.6 (3H, multiplet);
7.75 (2H, singlet).

EXAMPLE 49

(2R*, 3R*)-2-(2,4-Difluorophenyl)-3-mercapto-1-(1H-1,2,4-triazol-1-yl)-2-butanol

A mixture of 300 mg (0.74 mmoles) of (2R*, 3R*)-2-(2,4-difluorophenyl)-3-(4-methoxybenzylthio)-1-(1H-1,2,4-triazol-1-yl)-2-butanol (prepared as described in Example 48), 0.8 g of anisole, 4.2 g of trifluoroacetic acid and 244 mg of trifluoromethanesulphonic acid was stirred for 25 minutes, whilst ice-cooling. At the end of this time, the mixture was concentrated by evaporation under reduced pressure, and the resulting residue was mixed with a dilute aqueous solution of sodium bicarbonate and then extracted with ethyl acetate. The organic extract was washed with a saturated aqueous solution of sodium chloride, dried and then freed from the solvent by distillation under reduced pressure, to afford 337 mg of an oily substance containing the title compound.

Nuclear Magnetic Resonance Spectrum ($CDC\ell_3$) δ ppm:
1.18 (3H, doublet, J = 7 Hz);
1.94 (1H, doublet of doublets, J = 10 & 1.5 Hz);
3.46 (1H, doublet of quartets, J = 10 & 7 Hz);
4.79 (1H, doublet, J = 14 Hz);
4.8 (1H, broad);
5.02 (1H, doublet, J = 14 Hz);
6.5 - 7.0 (2H, multiplet);
7.1 - 7.6 (1H, multiplet);
7.78 (1H, singlet);
7.80 (1H, singlet).

EXAMPLE 50

(2R*, 3R*)-3-(4-Chlorobenzoylthio)-2-(2,4-difluorophenyl)-1-(1H-1,2,4-triazol-1-yl)-2-butanol

91 mg (0.52 mmoles) of 4-chlorobenzoyl chloride were added, whilst ice-cooling and stirring, to a solution of 180 mg of the mixture containing (2R*, 3R*)-2-(2,4-difluorophenyl)-3-mercapto-1-(1H-1,2,4-triazol-1-yl)-2-butanol described in Example 49 (corresponding to 0.37 mmole of the aforementioned compound)

dissolved in 2 ml of pyridine. The mixture was then stirred at the same temperature for 30 minutes and at room temperature for 30 minutes, after which it was cooled again with ice. 0.2 ml of methanol was then added and the resulting mixture was stirred for 10 minutes. The reaction mixture was then freed from the solvent by distillation under reduced pressure. The resulting residue was mixed with a dilute aqueous solution of sodium bicarbonate and extracted with ethyl acetate. The organic extract was washed with a saturated aqueous solution of sodium chloride, dried and then evaporated under reduced pressure, to remove the solvent. The resulting crystalline residue was recrystallized from a 1 : 1 by volume mixture of diethyl ether and hexane, to afford 79 mg of the title compound as crystals, melting at 139 - 140 °C.

Infrared Absorption Spectrum (KBr) $\nu_{max}$ $cm^{-1}$:
3400, 1667.

Nuclear Magnetic Resonance Spectrum ($CDC\ell_3$) $\delta$ ppm:
1.19 (3H, doublet, J = 7 Hz);
4.60 (1H, broad quartet, J = 7 Hz);
4.69 (1H, doublet, J = 15 Hz);
5.04 (1H, doublet, J = 15 Hz);
5.20 (1H, doublet, J = 1.5 Hz);
6.6 - 7.1 (2H, multiplet);
7.2 - 7.7 (1H, multiplet);
7.47 (2H, doublet, J = 9 Hz);
7.80 (2H, singlet);
8.01 (2H, doublet, J = 9 Hz).

## EXAMPLES 51 TO 54

Following a procedure similar to that described in Example 50, the compounds (racemates) shown in the following Table 4 were prepared by the reaction of (2R*, 3R*)-2-(2,4-difluorophenyl)-3-mercapto-1-(1H-1,2,4-triazol-1-yl)-2-butanol (prepared as described in Example 49) with various substituted benzoyl chloride derivatives or substituted cinnamoyl chloride derivatives. The compounds prepared are identified by the numbers assigned to them in the foregoing Table 1.

Table 4

| Ex No. | Cpd No. | stereochemistry | m.p. (°C) |
|---|---|---|---|
| 51 | 1-114 | (2R*, 3R*) | 118.5 - 120 |
| 52 | 1-115 | (2R*, 3R*) | 110 - 113 |
| 53 | 1-116 | (2R*, 3R*) | 113 - 114.5 |
| 54 | 1-117 | (2R*, 3R*) | 143 - 144 |

## EXAMPLE 55

(2R*, 3R*)-2-(2,4-Difluorophenyl)-1-(1H-1,2,4-triazol-1-yl)-3-[4-(trifluoromethyl)cinnamoylamino]-2-butanol

242 mg (1.12 mmole) of 4-(trifluoromethyl)cinnamoyl chloride were added to a suspension of 200 mg (0.75 mmoles) of (2R*, 3R*)-3-amino-2-(2,4-difluorophenyl)-1-(1H-1,2,4-triazol-1-yl)-2-butanol (prepared by a procedure similar to that described in Example 3) in 2 ml of pyridine, and the mixture was heated under reflux for 30 minutes. At the end of this time, the mixture was cooled, 0.2 ml of methanol was added to it, and it was then stirred for 15 minutes. The reaction mixture was then freed from the solvent by distillation under reduced pressure, and the resulting residue was mixed with a dilute aqueous solution of sodium bicarbonate and extracted with ethyl acetate. The solvent was removed from the organic extract by distillation under reduced pressure, and the residue was purified by column chromatography through silica gel, using a 5 : 1 by volume mixture of ethyl acetate and hexane as eluent, to afford the title compound as crystals. 260 mg of a purified sample, melting at 166 - 167 °C, were obtained by recrystallization from a mixture of ethyl acetate and hexane.

Infrared Absorption Spectrum (KBr) $\nu_{max}$ $cm^{-1}$:
3300, 1760, 1617.

Nuclear Magnetic Resonance Spectrum ($CDC\ell_3$) $\delta$ ppm:

1.00 (3H, doublet, J = 7 Hz);
4.51 (1H, doublet, J = 14 Hz);
~5.0 (1H, multiplet);
5.08 (1H, doublet, J = 14 Hz);
5.39 (1H, broad singlet);
6.64 (1H, doublet, J = 16 Hz);
6.4 - 7.1 (3H, multiplet);
7.2 - 7.4 (1H, multiplet);
7.65 (4H, singlet);
7.77 (1H, doublet, J = 16 Hz);
7.84 (2H, singlet).

EXAMPLES 56 + 57

Following a procedure similar to that described in Example 55, the compounds (racemates) shown in the following Table 5 were prepared by the reaction of (2R*, 3R*)-3-amino-2-(2,4-difluorophenyl)-1-(1H-1,2,4-triazol-1-yl)-2-butanol (prepared by a procedure similar to that described in Example 3) with 3-(trifluoromethyl)cinnamoyl chloride or 4-chlorocinnamoyl chloride. The compounds prepared are identified by the numbers assigned to them in the foregoing Table 1.

Table 5

| Ex No. | Cpd No. | stereochemistry | m.p. (°C) |
|---|---|---|---|
| 56 | 1-213 | (2R*, 3R*) | 171 |
| 57 | 1-107 | (2R*, 3R*) | 204 - 206 |

EXAMPLE 58

(2R*, 3R*)-2-(2,4-Difluorophenyl)-3-(methylamino)-1-(1,2,4-triazol-1-yl)-2-butanol

127 mg (3.36 mmoles) of lithium aluminium hydride were suspended in 8.4 ml of tetrahydrofuran, and a solution of 941 mg (3.36 mmoles) of (4R*, 5R*)-5-(2,4-difluorophenyl)-4-methyl-5-[(1H-1,2,4-triazol-1-yl)-methyl]oxazolidine (prepared as described in Example 38) dissolved in 16.8 ml of tetrahydrofuran was added dropwise to the suspension, whilst ice-cooling and stirring. The mixture was then stirred at the same temperature for 30 minutes, after which 0.51 ml of a 1N aqueous solution of sodium hydroxide was added to the mixture, which was then stirred for a further 1 hour. At the end of this time, the mixture was filtered using a Celite (trade mark) filter aid, and the filtrate was evaporated under reduced pressure, to remove the solvent and to afford 868 mg of the title compound. A pure sample, melting at 141 - 145°C, was obtained by recrystallization from a mixture of ethyl acetate and hexane.

Infrared Absorption Spectrum (KBr) $\nu_{max}$ $cm^{-1}$:
3210, 1610, 1580, 1499.

Nuclear Magnetic Resonance Spectrum ($CDCl_3$) $\delta$ ppm:
0.90 (3H, doublet of doublets, J = 7 & 2 Hz);
2.48 (3H, singlet);
2.96 (1H, quartet of doublets, J = 7 & 2 Hz);
4.80 (2H, singlet);
6.55 - 7.1 (2H, multiplet);
7.1 - 7.7 (1H, multiplet);
7.77 (1H, singlet);
7.96 (1H, singlet).

EXAMPLE 59

(2R*, 3R*)-2-(2,4-Difluorophenyl)-3-{N-methyl-N-[4-(trifluoromethyl)benzoyl]amino}-1-(1H-1,2,4-triazol-1-yl)-2-butanol

428 mg (1.50 mmoles) of (2R*, 3R*)-2-(2,4-difluorophenyl)-3-(methylamino)-1-(1H-1,2,4-triazol-1-yl)-2-butanol (prepared as described in Example 58) were dissolved in 8 ml of pyridine, and 450 mg (2.16 mmoles) of 4-(trifluoromethyl)benzoyl chloride were then added to the solution, after which it was stirred at room temperature for 20 hours. At the end of this time, it was ice-cooled, and 0.5 ml of methanol were added. The reaction mixture was then stirred for 15 minutes, after which the solvent was removed by evaporation under reduced pressure. The residue was mixed with a dilute aqueous solution of sodium bicarbonate and extracted with ethyl acetate. The organic extract was dried and the solvent was removed by distillation under reduced pressure. The residue was recrystallized from diethyl ether, to afford 380 mg of the title compound, melting at 144 - 146 °C.

Infrared Absorption Spectrum (KBr) $\nu_{max}$ $cm^{-1}$:
3110, 1638, 1610.

EXAMPLES 60 + 61

Following a procedure similar to that described in Example 59, but using (2R*, 3R*)-2-(2,4-difluorophenyl)-3-(methylamino)-1-(1H-1,2,4-triazol-1-yl)-2-butanol (prepared as described in Example 58) and 2-fluoro-4-(trifluoromethyl)benzoylchloride or 4-chlorocinnamoyl chloride, the compounds (racemates) shown in the following Table 6 were prepared. The compounds prepared are identified by the numbers assigned to them in the foregoing Table 1.

Table 6

| Ex No. | Cpd No. | stereochemistry | m.p. (°C) |
|---|---|---|---|
| 60 | 1-127 | (2R*, 3R*) | 157 - 159 |
| 61 | 1-128 | (2R*, 3R*) | 169 - 171 |

EXAMPLE 62

(2R*, 3R*)-2-(4-Chlorophenyl)-3-(methanesulphonyloxy)-1-(1H-1,2,4-triazol-1-yl)-2-butanol

1.11 g (9.73 mmoles) of methanesulphonyl chloride was added to a solution of 2.00 g (7.49 mmoles) of (2R*, 3R*)-2-(4-chlorophenyl)-1-(1H-1,2,4-triazol-1-yl)-2,3-butanediol [prepared in a manner similar to that described for the preparation of (2R*, 3R*)-2-(2,4-dichlorophenyl)-1-(1H-1,2,4-triazol-1-yl)-2,3-butanediol in the Abstract Papers of the 8th Symposium on Medicinal Chemistry, Osaka (1986), page 9] dissolved in 45 ml of pyridine, whilst ice-cooling and stirring. The reaction temperature was allowed to rise to room temperature, after which the mixture was stirred for a further 1 hour. The pyridine was then removed by distillation under reduced pressure, and the resulting residue was mixed with a dilute aqueous solution of sodium bicarbonate and extracted twice with diethyl ether. The combined extracts were washed with a saturated aqueous solution of sodium chloride, after which the diethyl ether was removed by distillation under reduced pressure. The resulting crude product was purified by chromatography through silica gel, using a 2 : 1 by volume mixture of ethyl acetate and hexane as eluent, to afford 2.05 g of the title compound as an oil.

Nuclear Magnetic Resonance Spectrum ($CDC\ell_3$) δ ppm:
1.27 (3H, doublet, J = 7 Hz);
3.03 (3H, singlet);
4.70 (2H, singlet);
5.11 (1H, quartet, J = 7 Hz);
5.6 (1H, broad);
7.29 (4H, singlet);
7.76 (1H, singlet);
7.84 (1H, singlet).

EXAMPLE 63

(2R*, 3R*)-3-Azido-2-(4-chlorophenyl)-1-(1H-1,2,4-triazol-1-yl)-2-butanol and its oxalate

1.95 g (30 mmoles) of sodium azide and 380 mg (7.13 mmoles) of ammonium chloride were added to a solution of 2.05 g (5.94 mmoles) of (2R*, 3R*)-2-(4-chlorophenyl)-3-(methanesulphonyloxy)-1-(1H-1,2,4-triazol-1-yl)-2-butanol (prepared as described in Example 62) dissolved in 30 ml of dimethylformamide, and the reaction mixture was stirred at 100°C for 2 hours. At the end of this time, it was freed from the solvent by distillation under reduced pressure. Benzene was added to the residue, and the resulting solution was washed with water and dried. The solvent was then removed by evaporation under reduced pressure, and the residue was subjected to column chromatography through silica gel. 1.52 g of the title compound was obtained from the fractions eluted with a 3 : 1 by volume mixture of ethyl acetate and hexane. Recrystallization from a mixture of benzene and hexane gave crystals melting at 103.5 - 104°C.

Nuclear Magnetic Resonance Spectrum ($CDCl_3$) δ ppm:
1.11 (3H, doublet, J = 7 Hz);
3.68 (1H, quartet, J = 7 Hz);
4.50 (1H, doublet, J = 14 Hz);
4.71 (1H, doublet, J = 14 Hz);
5.10 (1H, singlet);
7.35 (4H, singlet);
7.72 (1H, singlet);
7.74 (1H, singlet).

The oxalate of the title compound was obtained by adding a molar equivalent of oxalic acid to a solution of the title compound in ethyl acetate. Its melting point was 153 - 155°C.

EXAMPLE 64

(2R*, 3R*)-3-Amino-2-(4-chlorophenyl)-1-(1H-1,2,4-triazol-1-yl)-2-butanol

A solution of 1.52 g of (2R*, 3R*)-3-azido-2-(4-chlorophenyl)-1-(1H-1,2,4-triazol-1-yl)-2-butanol (prepared as described in Example 63) dissolved in 45 ml of ethanol was stirred for 1 hour under an atmosphere's pressure of hydrogen in the presence of 430 mg of 10% w/w palladium-on-charcoal. At the end of this time, the reaction mixture was filtered using a Celite (trade mark) filter aid, and the filtrate was concentrated by evaporation under reduced pressure, to afford 1.26 g of the title compound as an oil.

Nuclear Magnetic Resonance Spectrum ($CDCl_3$) δ ppm:
0.88 (3H, doublet, J = 7 Hz);
2.8 - 3.6 (4H, broad);
4.56 (2H, singlet);
7.26 (4H, singlet);
7.80 (1H, singlet);
7.94 (1H, singlet).

EXAMPLES 65 - 70

Following a procedure similar to that described in Example 55, but using (2R*, 3R*)-3-amino-2-(2,4-difluorophenyl)-1-(1H-1,2,4-triazol-1-yl)-2-butanol (prepared by a procedure similar to that described in Example 3) or (2R*, 3R*)-3-amino-2-(4-chlorophenyl)-1-(1H-1,2,4-triazol-1-yl)-2-butanol (prepared as described in Example 64) and a substituted benzoyl chloride having various kinds of substituent(s) on the benzoyl group or 5-chloro-2-thienyl chloride, the compounds shown in the following Table 7 were also prepared. The compounds prepared are identified by the numbers assigned to them in the foregoing Table 1.

Table 7

| Ex No. | Cpd No. | stereochemistry | m.p. (°C) |
|---|---|---|---|
| 65 | 1-26 | (2R*, 3R*) | 176 - 177 |
| 66 | 1-214 | (2R*, 3R*) | 105 - 106 |
| 67 | 1-32 | (2R*, 3R*) | 206 |
| 68 | 1-215 | (2R*, 3R*) | 191 - 193 |
| 69 | 1-20 | (2R*, 3R*) | 204 - 207 |
| 70 | 1-68 | (2R*, 3R*) | 219 - 220 |

EXAMPLE 71

(4R*, 5R*)-3-(4-Chlorocinnamoyl)-5-(2,4-difluorophenyl)-4-methyl-5-[(1H-1,2,4-triazol-1-yl)methyl]oxazolidine and its oxalate

202 mg (1.06 mmoles) of 4-chlorocinnamoyl chloride were added to a solution of 197 mg (0.70 mmoles) of (4R*, 5R*)-5-(2,4-difluorophenyl)-4-methyl-5-[(1H-1,2,4-triazol-1-yl)methyl]oxazolidine (prepared as described in Example 38) dissolved in 2 ml of pyridine, and the mixture was stirred at room temperature for 1 hour. At the end of this time, 0.5 ml of methanol was added to the mixture, which was then stirred for a further 10 minutes. The pyridine was then removed by distillation under reduced pressure, and the resulting residue was mixed with a dilute aqueous solution of sodium bicarbonate. The resulting mixture was then extracted with ethyl acetate. The crude product obtained from the extract was purified by column chromatography through silica gel, using a 5 : 1 by volume mixture of ethyl acetate and hexane as eluent, to afford 250 mg of the title compound as an oil.

Infrared Absorption Spectrum ($CHCl_3$) $\nu_{max}$ $cm^{-1}$:
1645, 1615.

Nuclear Magnetic Resonance Spectrum ($CDCl_3$) $\delta$ ppm:
1.01 (3H, doublet, J = 7 Hz);
4.55 (2H, singlet);
4.75 (1H, broad);
5.33 (1H, broad doublet, J = 5 Hz);
5.56 (1H, doublet, J = 5 Hz);
6.2 - 8.1 (11H, multiplet).

238 mg of the oxalate, melting at 161 - 165°C, were prepared by dissolving 250 mg of the free amide obtained as described above and 51 mg of oxalic acid in ethyl acetate and then adding hexane to form crystals.

EXAMPLE 72

(4R*, 5R*)-5-(2,4-Difluorophenyl)-4-methyl-5-[(1H-1,2,4-triazol-1-yl)methyl]-3-[4-(trifluoromethyl)cinnamoyl]-oxazolidine

260 mg (1.11 mmole) of 4-(trifluoromethyl)cinnamoyl chloride were added at 0°C, whilst stirring, to a solution of 207 mg (0.74 mmoles) of (4R*, 5R*)-5-(2,4-difluorophenyl)-4-methyl-5-[(1H-1,2,4-triazol-1-yl)-methyl]oxazolidine (prepared as described in Example 38) in 2 ml of pyridine. The reaction mixture was treated in a similar manner to that described in Example 71, and the resulting crude product was purified by column chromatography through silica gel. A crystalline product was obtained from the fractions eluted with a 5 : 1 by volume mixture of ethyl acetate and benzene, and this was recrystallized from a mixture of ethyl acetate and hexane to afford 206 mg of the title compound, melting at 168 - 169°C.

Infrared Absorption Spectrum ($CHCl_3$) $\nu_{max}$ $cm^{-1}$:
1655, 1618.

Nuclear Magnetic Resonance Spectrum ($CDCl_3$) $\delta$ ppm:
1.03 (3H, doublet, J = 7 Hz);
4.59 (2H, singlet);
4.8 (1H, broad);
5.34 (1H, broad doublet, J = 5 Hz);

5.58 (1H, doublet, J = 5 Hz);
6.3 - 8.0 (11H, multiplet).

EXAMPLE 73

(4R*, 5R*)-5-(2,4-Difluorophenyl)-4-methyl-5-[(1H-1,2,4-triazol-1-yl)methyl]-3-[3-(trifluoromethyl)cinnamoyl]-oxazolidine

A procedure similar to that described in Example 71 was repeated, except that 119 mg (0.43 mmoles) of (4R*, 5R*)-5-(2,4-difluorophenyl)-4-methyl-5-[(1H-1,2,4-triazol-1-yl)methyl]oxazolidine (prepared as described in Example 38) and 150 mg (0.64 mmoles) of 3-(trifluoromethyl)cinnamoyl chloride were employed, to afford 100 mg of the title compound, melting at 160 - 162 °C.

Infrared Absorption Spectrum ($CHCl_3$) $\nu_{max}$ $cm^{-1}$:
1655, 1616.

Nuclear Magnetic Resonance Spectrum ($CDCl_3$) $\delta$ ppm:
1.04 (3H, doublet, J = 7 Hz);
4.61 (2H, singlet);
4.8 (1H, broad);
5.36 (1H, broad doublet, J = 5 Hz);
5.60 (1H, doublet, J = 5 Hz);
6.3 - 8.0 (11H, multiplet).

EXAMPLE 74

(4R*, 5R*)-5-(4-Chlorophenyl)-4-methyl-5-[(1H-1,2,4-triazol-1-yl)methyl]oxazolidine

A mixture of 592 mg (2.22 mmoles) of (2R*, 3R*)-3-amino-2-(4-chlorophenyl)-1-(1H-1,2,4-triazol-1-yl)-2-butanol (prepared as described in Example 64) and 81 mg (2.67 mmoles) of paraformaldehyde in 50 ml of benzene was heated under reflux for 2 hours. At the end of this time, the reaction mixture was distilled to remove the benzene and to afford 650 mg of the title compound as an oil.

EXAMPLE 75

(4R*, 5R*)-5-(4-Chlorophenyl)-4-methyl-5-[(1H-1,2,4-triazol-1-yl)methyl]-3-[4-(trifluoromethyl)cinnamoyl]-oxazolidine

427 mg (1.82 mmoles) of 4-(trifluoromethyl)cinnamoyl chloride were added at 0 °C, whilst stirring, to a solution of 321 mg (1.21 mmoles) of (4R*, 5R*)-5-(4-chlorophenyl)-4-methyl-5-[(1H-1,2,4-triazol-1-yl)methyl]-oxazolidine (prepared as described in Example 74) in 3 ml of pyridine, and the mixture was stirred at room temperature for 1 hour. At the end of this time, 0.5 ml of methanol was added to the mixture, which was then stirred for 10 minutes. The reaction mixture was then concentrated by evaporation under reduced pressure, to remove the pyridine, and the resulting residue was mixed with a dilute aqueous solution of sodium bicarbonate and then extracted with ethyl acetate. The crude product produced from the extract was subjected to column chromatography through silica gel, and a crystalline product was obtained from the fractions eluted with a 4 : 1 by volume mixture of ethyl acetate and hexane. 310 mg of the title compound, melting at 180 - 181 °C, were then obtained by recrystallization of this crystalline product from a mixture of ethyl acetate and hexane.

Infrared Absorption Spectrum
($CHCl_3$) $\nu_{max}$ $cm^{-1}$: 1651, 1611.

Nuclear Magnetic Resonance Spectrum ($CDCl_3$) $\delta$ ppm:
0.91 (3H, doublet, J = 6.5 Hz);
4.53 (2H, singlet);
4.88 (1H, quartet, J = 6.5 Hz);
5.37 (1H, broad doublet, J = 5 Hz);
5.57 (1H, doublet, J = 5 Hz);
6.6 - 8.0 (12H, multiplet).

EXAMPLE 76

(4R*, 5R*)-5-(4-Chlorophenyl)-3-[2-fluoro-4-(trifluoromethyl)benzoyl]-4-methyl-5-[(1H-1,2,4-triazol-1-yl)-methyl]oxazolidine and its oxalate

A procedure similar to that described in Example 71 was repeated, except that 430 mg (1.61 mmoles) of (4R*, 5R*)-5-(4-chlorophenyl)-4-methyl-5-[(1H-1,2,4-triazol-1-yl)methyl]oxazolidine (prepared as described in Example 74) and 547 mg (2.42 mmoles) of 2-fluoro-4-(trifluoromethyl)benzoyl chloride were employed, to afford 233 mg of the title compound as an oil.

Nuclear Magnetic Resonance Spectrum ($CDC\ell_3$) δ ppm:
0.76, 1.00 (2 : 3, 3H, doublet, J = 7 Hz);
4.3 (1H, multiplet);
4.59 (2H, singlet);
4.8 - 5.9 (2H, multiplet);
6.8 - 7.9 (9H, multiplet).

237 mg of the oxalate of the title compound, melting at 175 - 177 °C, were prepared by dissolving 230 mg of the free amide obtained as described above and 45 mg of oxalic acid in ethyl acetate and then adding hexane to form crystals.

EXAMPLE 78

(4R*, 5R*)-5-(2,4-Difluorophenyl)-3-[2-fluoro-4-(trifluoromethyl)benzoyl]-4-methyl-5-[(1H-1,2,4-triazol-1-yl)-methyl]oxazolidine and its oxalate

A procedure similar to that described in Example 71 was repeated, except that 200 mg (0.714 mmoles) of (4R*, 5R*)-5-(2,4-difluorophenyl)-4-methyl-5-[(1H-1,2,4-triazol-1-yl)methyl]oxazolidine (prepared as described in Example 38) and 242 mg (1.07 mmoles) of 2-fluoro-4-(trifluoromethyl)benzoyl chloride were employed, to afford 193 mg of the title compound as an oil.

Infrared Absorption Spectrum ($CHC\ell_3$) $\nu_{max}$ $cm^{-1}$:
1650, 1620.

Nuclear Magnetic Resonance Spectrum ($CDC\ell_3$) δ ppm:
0.84, 1.10 (2 : 3, 3H, doublet, J = 7 Hz);
4.3 (1H, multiplet);
4.61, 1.67 (3 : 2, 2H, singlet);
4.9 - 5.9 (2H, multiplet);
6.6 - 7.9 (8H, multiplet).

188 mg of the oxalate of the title compound, melting at 167 - 168 °C, were prepared by dissolving 193 mg of the free amide obtained as described above and 37 mg of oxalic acid in ethyl acetate and then adding hexane to form crystals.

EXAMPLE 79

(3S*, 4R*)-4-(2,4-Difluorophenyl)-3-methyl-5-(1H-1,2,4-triazol-1-yl)-1,4-pentanediol and (2R*, 3S*)-2-(2,4-difluorophenyl)-3-methyl-1-(1H-1,2,4-triazol-1-yl)-2,4-pentanediol

2.2 ml (4.4 mmoles) of a 2M suspension of a borane-dimethyl sulphide complex in tetrahydrofuran were added, whilst stirring and ice-cooling, to a solution of 200 mg (0.72 mmole) of (2R*, 3S*)-2-(2,4-difluorophenyl)-3-methyl-1-(1H-1,2,4-triazol-1-yl)-4-penten-2-ol (prepared by a procedure similar to that described in Japanese Patent Application Kokai No. 60-36468) in 5 ml of tetrahydrofuran. The mixture was warmed to room temperature and stirred at the same temperature for 15 minutes and then at 50 °C for a further 20 minutes. After this, the mixture was again ice-cooled, and then 1 ml of a 15% w/v aqueous solution of sodium hydroxide and 1 ml of a 30% w/v aqueous solution of hydrogen peroxide were added. The mixture was stirred at room temperature for 30 minutes then at 50 - 60 °C for a further 2 hours. At the end of this time, the mixture was diluted with ethyl acetate, washed with a saturated aqueous solution of sodium chloride and dried. The solvent was then distilled off under reduced pressure, to leave an oil, which was purified by column chromatography through silica gel, eluted with a gradient solvent system (ranging from 5 : 1 to 10 : 1 by volume) of ethyl acetate and hexane, to give 30 ml of the (2R*, 3S*)-2,4-pentanediol isomer of the title compound as an oil. Analysis of the nuclear magnetic resonance spectrum of this product

revealed that it was about a 1 : 1 mixture of two isomers with respect to the carbon atom at the 4-position.

Isomer A

Nuclear Magnetic Resonance Spectrum ($CDCl_3$) δ ppm:
0.80 (3H, doublet of doublets, J = 7 & 3.5 Hz);
1.18 (3H, doublet, J = 6 Hz);
4.90 (2H, singlet);
5.97 (1H, singlet);
7.79 (1H, singlet);
8.05 (1H, singlet).

Isomer B

Nuclear Magnetic Resonance Spectrum (inter alia $CDCl_3$) δ ppm:
0.76 (3H, doublet of doublets, J = 7 & 1.5 Hz);
1.27 (3H, doublet, J = 6 Hz);
5.47 (1H, singlet);
7.70 (1H, singlet);
7.93 (1H, singlet).

A further elution was effected with ethyl acetate and with 1% v/v methanol in ethyl acetate, to give 139 mg of the (3S*, 4R*)-1,4-pentanediol isomer of the title compound, which was recrystallized from a mixture of ethyl acetate and hexane to give crystals melting at 121 - 122 °C.

Nuclear Magnetic Resonance Spectrum of the (3S*, 4R*)-1,4-pentanediol isomer ($CDCl_3$) δ ppm:
0.78 (3H, doublet of doublets, J = 7 & 1 Hz);
1.6 - 2.0 (2H, multiplet);
2.4 (1H, multiplet);
3.3 - 4.0 (3H, multiplet);
4.62 (1H, doublet, J = 14 Hz);
4.93 (1H, doublet of doublets, J = 14 & 1 Hz);
5.52 (1H, broad);
6.5 - 7.0 (2H, multiplet);
7.43 (1H, triplet of doublets, J = 9 & 7 Hz);
7.73 (1H, singlet);
7.97 (1H, singlet).

EXAMPLE 80

(2R*, 3S*)-2-(2,4-Difluorophenyl)-5-(methanesulphonyloxy)-3-methyl-1-(1H-1,2,4-triazol-1-yl)-2-pentanol

25 mg (0.25 mmole) of triethylamine and 25 mg (0.22 mmole) of methanesulphonyl chloride were added, whilst stirring at -15 °C, to a solution of 30 mg (0.1 mmole) of (3S*, 4R*)-4-(2,4-difluorophenyl)-3-methyl-5-(1H-1,2,4-triazol-1-yl)-1,4-pentanediol (prepared as described in Example 79) in 1 ml of methylene chloride. After 15 minutes a dilute aqueous solution of sodium bicarbonate was added, and then the reaction mixture was extracted with ethyl acetate. The extract was dried and the solvent was distilled off under reduced pressure, to give 38 mg of the title compound, as an oil.

Nuclear Magnetic Resonance Spectrum ($CDCl_3$) δ ppm:
0.82 (3H, doublet of doublets, J = 6.5 & 1 Hz);
1.6 - 2.6 (3H, multiplet);
3.03 (3H, singlet);
4.0 - 4.7 (2H, multiplet);
4.59 (1H, doublet, J = 14 Hz);
4.70 (1H, singlet);
5.00 (1H, doublet, J = 14 Hz);
6.5 - 7.0 (2H, multiplet);
7.2 - 7.7 (1H, multiplet);
7.83 (1H, singlet);
8.17 (1H, singlet).

EXAMPLE 81

(2R*, 3S*)-5-Azido-2-(2,4-difluorophenyl)-3-methyl-1-(1H-1,2,4-triazol-1-yl)-2-pentanol and its oxalate

33 mg (0.51 mmole) of sodium azide were added to a solution of 38 mg (0.101 mmole) of (2R*, 3S*)-2-(2,4-difluorophenyl)-5-(methanesulphonyloxy)-3-methyl-1-(1H-1,2,4-triazol-1-yl)-2-pentanol (prepared as described in Example 80) in 1 ml of dimethylformamide, and then the mixture was stirred at 100°C for 2 hours. After the mixture had been cooled, ethyl acetate was added and the mixture was then washed with a saturated aqueous solution of sodium chloride. The solvent was then distilled off under reduced pressure, to leave an oil, which was purified by column chromatogarphy through silica gel, eluted with a 7 : 1 by volume mixture of ethyl acetate and hexane, to give 13 mg of the title compound as an oil.

Infrared Absorption Spectrum ($CHC\ell_3$) $\nu_{max}$ $cm^{-1}$:
3420, 2100.

Nuclear Magnetic Resonance Spectrum ($CDC\ell_3$) $\delta$ ppm:
0.77 (3H, doublet of doublets, J = 6.5 & 0.5 Hz);
1.5 - 2.5 (3H, multiplet);
3.2 - 3.7 (2H, multiplet);
4.53 (1H, doublet, J = 14 Hz)
4.85 (1H, singlet);
4.97 (1H, doublet of doublets, J = 14 & 1 Hz);
6.5 - 7.0 (2H, multiplet);
7.2 - 7.7 (1H, multiplet);
7.79 (1H, singlet);
7.87 (1H, singlet).

13 mg of the azide alcohol obtained by the procedure described above were dissolved in ethyl acetate. 4 mg of oxalic acid were added to the solution, and then the mixture was caused to crystallize by the addition of hexane, to give 13 mg of the oxalate melting at 140 - 144°C.

EXAMPLE 82

(2R*, 3S*)-2-(2,4-Difluorophenyl)-4-(methanesulphonyloxy)-3-methyl-1-(1H-1,2,4-triazol-1-yl)-2-pentanol

140 mg (1.22 mmoles) of methanesulphonyl chloride were added at 0°C to a solution of 213 mg (0.72 mmole) of (2R*, 3S*)-2-(2,4-difluorophenyl)-3-methyl-1-(1H-1,2,4-triazol-1-yl)-2,4-pentanediol (prepared as described in Example 79 - about a 2 : 1 mixture of isomers in respect of the carbon atom at the 4-position) in 4 ml of pyridine, and then the mixture was stirred for 2.5 hours. At the end of this time, the pyridine was distilled off under reduced pressure to give a residue, which was mixed with a dilute aqueous solution of sodium bicarbonate and then extracted with ethyl acetate. The extract was dried and the solvent was distilled off under reduced pressure, to give 270 mg of the title compound as an oil, which was about a 2 : 1 mixture of the isomers in respect of the carbon atom at the 4-position.

The major product:

Nuclear Magnetic Resonance Spectrum ($CDC\ell_3$) $\delta$ ppm:
0.76 (3H, doublet of doublets, J = 6.5 & 0.5 Hz);
1.46 (3H, doublet, J = 6.5 Hz);
2.5 (1H, multiplet);
3.05 (3H, singlet);
4.65 (1H, doublet, J = 14 Hz);
5.05 (1H, doublet of doublets, J = 14 & 1 Hz);
5.35 (1H, multiplet);
6.5 - 7.0 (2H, multiplet);
7.1 - 7.6 (1H, multiplet);
7.77 (1H, singlet);
7.85 (1H, singlet).

The minor product:

Nuclear Magnetic Resonance Spectrum ($CDCl_3$) δ ppm:
0.87 (3H, doublet of doublets, J = 6.5 & 0.5 Hz);
1.57 (3H, doublet, J = 6.5 Hz);
3.02 (3H, singlet);
4.71 (1H, doublet, J = 14 Hz);
5.05 (1H, doublet of doublets, J = 14 & 0.5 Hz);
7.77 (1H, singlet);
7.89 (1H, singlet).

EXAMPLE 83

(2R*, 3S*)-4-Azido-2-(2,4-difluorophenyl)-3-methyl-1-(1H-1,2,4-triazol-1-yl)-2-pentanol and its oxalate

116 mg (1.79 mmoles) of sodium azide were added to a solution of 168 mg (0.45 mmole) of (2R*, 3S*)-2-(2,4-difluorophenyl)-4-(methanesulphonyloxy)-3-methyl-1-(1H-1,2,4-triazol-1-yl)-2-pentanol (prepared as described in Example 82) in 5 ml of dimethylformamide, and then the mixture was stirred at 100°C for 3 hours. After the mixture had been cooled, the solvent was distilled off under reduced pressure, and ethyl acetate was added to the residue. The resulting mixture was washed with a saturated aqueous solution of sodium chloride and then dried, after which the solvent was distilled off under reduced pressure, to leave an oil, which was purified by column chromatography through silica gel, eluted with a 2 : 1 by volume mixture of ethyl acetate and hexane, to give 35 mg of isomer (1) in the form of crystals (melting at 99 - 100°C after washing with hexane). 70 mg of the other isomer (2) were obtained from the other fraction of the eluate in the form of an oil after a further purification by preparative thin layer chromatography developed with a 1 : 1 by volume mixture of ethyl acetate and hexane.

Isomer (1):

Infrared Absorption Spectrum ($CHCl_3$) $\nu_{max}$ $cm^{-1}$:
3420, 2100.
Nuclear Magnetic Resonance Spectrum ($CDCl_3$) δ ppm:
0.72 (3H, doublet of doublets, J = 7 & 1 Hz);
1.34 (3H, doublet, J = 6.5 Hz);
2.24 (1H, broad quintet, J = 7 Hz);
3.98 (1H, quintet, J = 6.5 Hz);
4.69 (1H, doublet, J = 14 Hz);
4.97 (1H, doublet, J = 1.5 Hz);
5.00 (1H, doublet of doublets, J = 14 & 1 Hz);
6.5 - 7.0 (2H, multiplet);
7.32 (1H, triplet of doublets, J = 9 & 7 Hz);
7.77 (1H, singlet);
7.81 (1H, singlet).

Isomer (2):

Infrared Absorption Spectrum ($CHCl_3$) $\nu_{max}$ $cm^{-1}$:
3420, 2100.
Nuclear Magnetic Resonance Spectrum ($CDCl_3$) δ ppm:
0.84 (3H, doublet of doublets, J = 6.5 & 0.5 Hz);
1.37 (3H, doublet, J = 6.5 Hz);
2.23 (1H, quartet of doublets, J = 6.5 & 2 Hz);
4.13 (1H, quartet of doublets, J = 6.5 & 2 Hz);
4.53 (1H, doublet, J = 14 Hz);
4.73 (1H, singlet);
5.00 (1H, doublet of doublets, J = 14 & 1 Hz);
6.5 - 7.0 (2H, multiplet);
7.2 - 7.7 (1H, multiplet);

7.71 (1H, singlet);
8.01 (1H, singlet).

62 mg of the isomer (2) obtained by the procedure described above were dissolved in ethyl acetate. 17 mg of oxalic acid were added to the solution, and then the mixture was caused to crystallize by the addition of hexane, to give 46 mg of the oxalate of the title compound, melting at 130 °C.

EXAMPLE 84

(2R*, 3R*)-2-(2,4-Difluorophenyl)-4,5-epoxy-3-methyl-1-(1H-1,2,4-triazol-1-yl)-2-pentanol

635 mg (3.13 mmoles) of m-chloroperbenzoic acid (of 85% purity) were added at 0 °C to a solution of 514 mg (1.84 mmoles) of (2R*, 3R*)-2-(2,4-difluorophenyl)-3-methyl-1-(1H-1,2,4-triazol-1-yl)-4-penten-2-ol (prepared by a procedure similar to that described in Japanese Patent Application Kokai No. 60-36468) in 15 ml of methylene chloride. 5 minutes later, the mixture was warmed to room temperature and stirred for 2 hours. At the end of this time, the reaction mixture was diluted with ethyl acetate and washed, in turn, with an aqueous solution of sodium sulphite, an aqueous solution of sodium bicarbonate and a saturated aqueous solution of sodium chloride. After the solution had been dried, the solvent was distilled off under reduced pressure, to leave a residue, which was purified by column chromatography through silica gel, eluted with a 2 : 1 by volume mixture of ethyl acetate and hexane, to give 472 mg or the title compound as a solid. This product was about a 3 : 1 mixture of isomers in respect of the carbon atom at the 4-position. The mixed isomers were then recrystallized from a mixture of ethyl acetate and hexane to separate the major isomer, melting at 106 - 109 °C.

Nuclear Magnetic Resonance Spectrum ($CDCl_3$) δ ppm:
1.30 (3H, doublet, J = 6 Hz);
1.90 (1H, broad quintet);
2.00 (1H, doublet of doublets, J = 4 & 3 Hz);
2.35 (1H, triplet, J = 4 Hz);
2.85 (1H, multiplet);
4.53 (1H, doublet, J = 14 Hz);
4.89 (1H, doublet of doublets, J = 14 & 1.5 Hz);
4.9 (1H, broad);
6.5 - 7.0 (2H, multiplet);
7.3 - 7.7 (1H, multiplet);
7.79 (1H, singlet);
7.91 (1H, singlet).

EXAMPLE 85

(2R*, 3R*)-2-(2,4-Difluorophenyl)-3-methyl-1-(1H-1,2,4-triazol-1-yl)-2,4-pentanediol

53 mg (1.40 mmoles) of lithium aluminium hydride were added, whilst stirring and ice-cooling and under an atmosphere of nitrogen, to a solution of 207 mg (0.70 mmole) of (2R*, 3R*)-2-(2,4-difluorophenyl)-4,5-epoxy-3-methyl-(1H-1,2,4-triazol-1-yl)-2-pentanol (prepared as described in Example 84 - about a 3 : 1 mixture of isomers in respect of the carbon atom at the 4-position) in 4 ml of diethyl ether. 10 minutes after the addition, the mixture was heated under reflux for 1 hour. The mixture was then cooled, after which 1 ml of water was added slowly, and the mixture was stirred for 10 minutes. Insoluble matter was filtered off using a Celite (trade mark) filter aid, and the filtrate was extracted with ethyl acetate. The extract was dried and the solvent was distilled off to leave an oil, which was purified by column chromatography through silica gel, eluted with a 5 : 5 : 1 by volume mixture of ethyl acetate, chloroform and hexane, to give 160 mg of the title compound. The product, which was about a 3 : 1 mixture of the two isomers with respect to the carbon atom at the 4-position, was recrystallized from a mixture of benzene and hexane to give the major isomer, melting at 145 - 146 °C.

Nuclear Magnetic Resonance Spectrum ($CDCl_3$) δ ppm:
1.05 (3H, doublet, J = 6.5 Hz);
1.25 (3H, doublet, J = 6.5 Hz);
2.20 (1H, quartet of doublets, J = 6.5 & 1 Hz);
3.03 (1H, broad singlet);
3.74 (1H, broad quartet, J = 6.5 Hz);

4.51 (1H, doublet, J = 14 Hz);
4.77 (1H, doublet of doublets, J = 14 & 1 Hz);
5.33 (1H, singlet);
6.5 - 7.0 (2H, multiplet);
7.1 - 7.6 (1H, multiplet);
7.65 (1H, singlet);
7.89 (1H, singlet).

EXAMPLE 86

(2R*, 3R*)-4-Azido-2-(2,4-difluorophenyl)-3-methyl-1-(1H-1,2,4-triazol-1-yl)-2-pentanol

25 mg (0.25 mmole) of triethylamine and 27 mg (0.24 mmole) of methanesulphonyl chloride were added, whilst stirring and ice-cooling, to a solution of 56 mg (0.19 mmole) of (2R*, 3R*)-2-(2,4-difluorophenyl)-3-methyl-1-(1H-1,2,4-triazol-1-yl)-2,4-pentanediol (prepared as described in Example 85) in 1 ml of methylene chloride. After 15 minutes, an aqueous solution of sodium bicarbonate was added to the reaction mixture, which was then extracted with ethyl acetate. The extract was washed with a saturated aqueous solution of sodium chloride and the solvent was distilled off under reduced pressure. 71 mg of the oil thus obtained were dissolved in 2 ml of dimethylformamide, 49 mg (0.76 mmole) of sodium azide were added to the resulting solution, and then the mixture was stirred at 100°C for 1 hour. After it had been cooled, the reaction mixture was diluted with ethyl acetate, and washed with a saturated aqueous solution of sodium chloride, and the solvent was distilled off under reduced pressure, to leave an oil. This oil was purified by column chromatography through silica gel, eluted with a 2 : 1 by volume mixture of ethyl acetate and hexane, to give 25 mg of the title compound in a crystalline form. The resulting crystals were recrystallized from a mixture of benzene and hexane to give crystals having a double melting point of 82 - 84°C and of 91 - 92°C.

Infrared Absorption Spectrum ($CHCl_3$) $\nu_{max}$ $cm^{-1}$:
3425, 2110.

Nuclear Magnetic Resonance Spectrum ($CDCl_3$) $\delta$ ppm:
1.16 (3H, doublet, J = 6.5 Hz);
1.20 (3H, doublet of doublets, J = 6.5 & 0.5 Hz);
2.4 (1H, multiplet);
3.27 (1H, quartet of doublets, J = 6.5 & 3 Hz);
4.49 (1H, doublet, J = 14 Hz);
4.81 (1H, doublet of doublets, J = 14 & 1 Hz);
4.81 (1H, doublet, J = 2 Hz);
6.5 - 7.0 (2H, multiplet);
7.1 - 7.6 (1H, multiplet);
7.74 (2H, singlet).

EXAMPLE 87

(2R*, 3R*)-3-(2,4-Dichlorophenyl)-3-hydroxy-2-methyl-4-(1H-1,2,4-triazol-1-yl)butanal and its (2S*, 3R*) isomer

290 mg (1.32 mmoles) of sodium metaperiodate and 1 mg of osmium tetraoxide were added to a solution of 139 mg (0.45 mmole) of a 1 : 1 mixture of (2R*, 3S*)-and (2R*, 3R*)-2-(2,4-dichlorophenyl)-3-methyl-1-(1H-1,2,4-triazol-1-yl)-4-penten-2-ol (prepared as described in Japanese Patent Application Kokai No. 60-36468) in 2.8 ml of a 5 : 2 by volume mixture of tetrahydrofuran and water, and then the mixture was stirred overnight at room temperature. At the end of this time, the reaction mixture was diluted with ethyl acetate, washed with a saturated aqueous solution of sodium chloride and dried. The solvent was then distilled off under reduced pressure, to leave an oil, which was purified by column chromatography through 4 g of silica gel, eluted with a 4 : 5 by volume mixture of ethyl acetate and hexane, to give 27 mg of the (2R*, 3R*) isomer of the title compound, 25 mg of a mixture of the (2R*, 3R*) isomer and the (2S*, 3R*) isomer of the title compound, and 31 mg of the (2S*, 3R*) isomer of the title compound. The (2R*, 3R*) isomer was recrystallized from benzene to give crystals melting at 150 - 151°C. The (2S*, 3R*) isomer was recrystallized from a mixture of benzene and ethyl acetate to give crystals melting at 155 - 157°C.

The (2R*, 3R*) isomer

Infrared Absorption Spectrum ($CHCl_3$) $\nu_{max}$ $cm^{-1}$:
3400, 1715.

Nuclear Magnetic Resonance Spectrum ($CDCl_3$) δ ppm:
0.96 (3H, doublet, J = 7 Hz);
3.47 (1H, quartet of doublets, J = 7 & 3 Hz);
4.64 (1H, doublet, J = 14 Hz);
5.30 (1H, broad);
5.42 (1H, doublet, J = 14 Hz);
7.11 (1H, doublet of doublets, J = 8 & 2 Hz);
7.31 (1H, doublet, J = 2 Hz);
7.52 (1H, doublet, J = 8 Hz);
7.77 (1H, singlet);
7.85 (1H, singlet);
9.88 (1H, doublet, J = 3 Hz).

The (2S*, 3R*) isomer

Nuclear Magnetic Resonance Spectrum ($CDCl_3$) δ ppm:
1.40 (3H, doublet, J = 7 Hz);
3.52 (1H, quartet of doublets, J = 7 & 1.5 Hz);
4.50 (1H, doublet, J = 14 Hz);
5.42 (1H, doublet, J = 14 Hz);
5.5 (1H, singlet);
7.11 (1H, doublet of doublets, J = 8 & 2 Hz);
7.35 (1H, doublet, J = 2 Hz);
7.54 (1H, doublet, J = 8 Hz);
7.77 (1H, singlet);
7.86 (2H, singlet);
9.39 (1H, doublet, J = 1.5 Hz).

EXAMPLE 88

(2S*, 3R*)-3-(2,4-Dichlorophenyl)-2-methyl-4-(1H-1,2,4-triazol-1-yl)-1,3-butanediol and its (2R*, 3R*) isomer

15 mg of sodium borohydride were added, whilst stirring and ice-cooling, to a solution of 85 mg of a 1 : 1 mixture of the (2R*, 3R*)- and (2S*, 3R*)- isomers of 3-(2,4-dichlorophenyl)-3-hydroxy-2-methyl-4-(1H-1,2,4-triazol-1-yl)butanal (prepared as described in Example 87) in 1.5 ml of methanol. 10 minutes after the addition, the mixture was diluted with ethyl acetate and washed with a saturated aqueous solution of sodium chloride. The solvent was then distilled off under reduced pressure, and the residue was purified by column chromatography through 3 g of silica gel, eluted with ethyl acetate, to give 31 mg of the (2R*, 3R*)-isomer of the title compound. This compound was recrystallized from a mixture of benzene and hexane to give crystals melting at 120 - 122 °C. A further elution was effected using 7% v/v methanol in ethyl acetate to give 33 mg of the (2S*, 3R*) isomer of the title compound. This compound was recrystallized from a mixture of benzene and hexane to give crystals melting at 176 - 177 °C.

The (2R*, 3R*) isomer:

Nuclear Magnetic Resonance Spectrum ($CDCl_3$) δ ppm:
1.39 (3H, doublet, J = 7 Hz);
2.85 (1H, multiplet);
3.40 (1H, singlet);
3.45 (1H, singlet);
4.50 (1H, doublet, J = 14 Hz);
5.31 (1H, doublet, J = 14 Hz);
7.05 (1H, doublet of doublets, J = 8 & 2 Hz);
7.25 (1H, doublet, J = 2 Hz);

7.52 (1H, doublet, J = 8 Hz);
7.66 (1H, singlet);
7.91 (1H, singlet).

The (2S*, 3R*) isomer

Infrared Absorption Spectrum (Nujol) $\nu_{max}$ $cm^{-1}$:
3400, 3140.

Nuclear Magnetic Resonance Spectrum [$CDCl_3$ + $CD_3OD$ (1 : 1 by volume)] $\delta$ ppm:
0.77 (3H, doublet, J = 7 Hz);
2.9 (1H, multiplet);
3.6 - 4.3 (2H, multiplet);
4.74 (1H, doublet, J = 14.5 Hz);
5.44 (1H, doublet, J = 14.5 Hz);
7.04 (1H, doublet of doublets, J = 9 & 2 Hz);
7.30 (1H, doublet, J = 2 Hz);
7.48 (1H, doublet, J = 9 Hz);
7.67 (1H, singlet);
8.07 (2H, singlet).

EXAMPLE 89

(2R*, 3S*)-2-(2,4-Dichlorophenyl)-4-(methanesulphonyloxy)-3-methyl-1-(1H-1,2,4-triazol-1-yl)-2-butanol

61 mg (0.61 mmole) of triethylamine and 64 mg (0.56 mmole) of methanesulphonyl chloride were added, whilst stirring at 0 °C, to a solution of 74 mg (0.231 mmole) of (2S*, 3R*)-3-(2,4-dichlorophenyl)-2-methyl-4-(1H-1,2,4-triazol-1-yl)-1,3-butanediol (prepared as described in Example 88) in 2 ml of methylene chloride. After 15 minutes, a dilute aqueous solution of sodium bicarbonate was added, and then the reaction mixture was extracted with ethyl acetate. The extract was washed with a saturated aqueous solution of sodium chloride, and the solvent was distilled off under reduced pressure, to give 92 mg of the title compound as a crude product.

Nuclear Magnetic Resonance Spectrum ($CDCl_3$) $\delta$ ppm:
0.70 (3H, doublet, J = 7 Hz);
2.9 - 3.4 (1H, multiplet);
3.07 (3H, singlet);
4.22 (1H, doublet of doublets, J = 10 & 5 Hz);
4.59 (1H, doublet, J = 14.5 Hz);
4.71 (1H, doublet of doublets, J = 10 & 7 Hz);
5.18 (1H, broad);
5.51 (1H, doublet, J = 14.5 Hz);
7.04 (1H, doublet of doublets, J = 8 & 2 Hz);
7.27 (1H, doublet, J = 2 Hz);
7.43 (1H, doublet, J = 8 Hz);
7.73 (1H, singlet);
7.81 (1H, singlet).

EXAMPLE 90

(2R*, 3S*)-4-Azido-2-(2,4-dichlorophenyl)-3-methyl-1-(1H-1,2,4-triazol-1-yl)-2-butanol

119 mg (1.82 mmoles) of sodium azide were added to a solution of 180 mg (0.46 mmole) of (2R*, 3S*)-2-(2,4-dichlorophenyl)-4-(methanesulphonyloxy)-3-methyl-1-(1H-1,2,4-triazol-1-yl)-2-butanol (prepared as described in Example 89) in 3 ml of dimethylformamide, and then the mixture was stirred at 100 °C for 3 hours. After it had been cooled, the reaction mixture was diluted with ethyl acetate and washed with a saturated aqueous solution of sodium chloride. The solvent was then distilled off under reduced pressure, to leave an oil, which was purified by column chromatogarphy through silica gel, eluted with a 2 : 1 by volume mixture of ethyl acetate and hexane, to give 108 mg of the title compound as crystals. This product was recrystallized from a mixture of benzene and hexane, to give crystals melting at 116 - 117 °C.

Infrared Absorption Spectrum ($CHCl_3$) $\nu_{max}$ $cm^{-1}$:
3430, 2100.
Nuclear Magnetic Resonance Spectrum ($CDCl_3$) $\delta$ ppm:
0.71 (3H, doublet, J = 7 Hz);
2.6 - 3.2 (1H, multiplet);
3.2 - 4.1 (2H, multiplet);
4.59 (1H, doublet, J = 14 Hz);
5.00 (1H, singlet);
5.50 (1H, doublet, J = 14 Hz);
7.05 (1H, doublet of doublets, J = 8 & 2 Hz);
7.28 (1H, doublet, J = 2 Hz);
7.43 (1H, doublet, J = 8 Hz);
7.74 (1H, singlet);
7.80 (1H, singlet).

## EXAMPLE 91

### (2R*, 3S*)-4-Amino-2-(2,4-dichlorophenyl)-3-methyl-1-(1H-1,2,4-triazol-1-yl)-2-butanol

30 mg of 10% w/w palladium-on-charcoal were added to a solution of 108 mg of (2R*, 3S*)-4-azido-2-(2,4-dichlorophenyl)-3-methyl-1-(1H-1,2,4-triazol-1-yl)-2-butanol (prepared as described in Example 90) in 4 ml of ethanol, and then the mixture was stirred for 1.5 hours under ambient pressure and under an atmosphere of hydrogen. The reaction mixture was then filtered using a Celite filter aid, and the filtrate was concentrated by evaporation under reduced pressure, to give 100 mg of the title compound as crystals. This product was recrystallized from a mixture of ethyl acetate and benzene to give crystals melting at 154 - 156 °C.

Nuclear Magnetic Resonance Spectrum ($CDCl_3$) $\delta$ ppm:
0.76 (3H, doublet, J = 7 Hz);
2.5 - 3.5 (3H, multiplet);
3.80 (3H, broad singlet);
4.69 (1H, doublet, J = 14.5 Hz);
5.24 (1H, doublet, J = 14.5 Hz);
7.07 (1H, doublet of doublets, J = 9 & 2 Hz);
7.31 (1H, doublet, J = 2 Hz);
7.60 (1H, doublet, J = 9 Hz);
7.68 (1H, singlet);
8.09 (1H, singlet).

## EXAMPLE 92

### (2R*, 3S*)-2-(2,4-Dichlorophenyl)-3-methyl-1-(1H-1,2,4-triazol-1-yl)-4-[4-(trifluoromethyl)benzoylamino]-2-butanol and its oxalate

37 mg (0.176 mmole) of 4-(trifluoromethyl)benzoyl chloride were added, whilst stirring at 0 °C, to a solution of 46 mg (0.146 mmole) of (2R*, 3S*)-4-amino-2-(2,4-dichlorophenyl)-3-methyl-1-(1H-1,2,4-triazol-1-yl)-2-butanol (prepared as described in Example 91) in 1 ml of pyridine. After 30 minutes, a dilute aqueous solution of sodium bicarbonate was added, and then the mixture was extracted with ethyl acetate. The extract was dried, and the solvent was distilled off under reduced pressure, to leave an oil, which was purified by column chromatography through silica gel, eluted with a 2 : 1 by volume mixture of ethyl acetate and hexane, to give 53 mg of the title compound as an oil.

Infrared Absorption Spectrum ($CHCl_3$) $\nu_{max}$ $cm^{-1}$:
3350, 1665, 1525.
Nuclear Magnetic Resonance Spectrum ($CDCl_3$) $\delta$ ppm:
0.78 (3H, doublet, J = 7 Hz);
2.7 - 3.2 (1H, multiplet);
3.3 - 4.4 (2H, multiplet);
4.69 (1H, doublet, J = 14 Hz);
5.64 (1H, doublet, J = 14 Hz);

7.11 (1H, doublet of doublets, J = 8 & 2 Hz);
7.31 (1H, doublet, J = 2 Hz);
7.53 (1H, doublet, J = 8 Hz);
7.79 (1H, singlet);
7.92 (1H, singlet);
7.6 - 8.1 (4H, multiplet).

10 mg of oxalic acid were added to a solution of the amide compound, obtained as described above, in ethyl acetate. A precipitate was then obtained by the addition of hexane to the solution. This precipitate was collected by filtration to give 58 mg of the oxalate of the title compound, melting at 81 °C, with decomposition.

EXAMPLE 93

(2R*, 3S*)-4-(4-Chlorocinnamoylamino)-2-(2,4-dichlorophenyl)-3-methyl-1-(1H-1,2,4-triazol-1-yl)-2-butanol and its oxalate

Following substantially the same procedure as described in Example 92, but using 46 mg (0.146 mmole) of (2R*, 3S*)-4-amino-2-(2,4-dichlorophenyl)-3-methyl-1-(1H-1,2,4-triazol-1-yl)-2-butanol and 30 mg (0.161 mmole) of 4-chlorocinnamoyl chloride in pyridine, 33 mg of the title compound were obtained as an oil. This oil was then dissolved in ethyl acetate, and 6 mg of oxalic acid were added. The crystals which precipitated upon the addition of hexane to the solution were collected by filtration, to give the 33 mg of the oxalate of the title compound, melting at 190 - 191 °C.

EXAMPLE 94

(R)-(2,4-Difluorophenyl) 1-chloroethyl ketone

55 ml (0.63 mole) of oxalyl chloride and 12 ml of dimethylformamide were added to a solution of 120 g (0.491 mole) of (S)-2-(p-toluenesulphonyloxy)propionic acid (J. Kenyon et al., J. Chem. Soc., 1925, 399) in 700 ml of methylene chloride, and then the mixture was stirred at room temperature for 2 hours, after which the solvent was distilled off under reduced pressure to leave an oil, to which 112 g (0.98 mole) of 1,3-difluorobenzene were added. The mixture was then ice-cooled, and 196.4 g (1.474 moles) of aluminium chloride were added, whilst stirring and ice-cooling. After 1 hour, the mixture was warmed to room temperature and then stirred overnight. The reaction mixture was then poured into ice, 300 ml of concentrated aqueous hydrochloric acid were added, and then the mixture was stirred for 15 minutes. At the end of this time, the mixture was extracted with benzene and washed, in turn, with water, with a dilute aqueous solution of sodium bicarbonate and then with a saturated aqueous solution of sodium chloride. After this, the solvent was distilled off to leave an oil, which was distilled to give 80.6 g (yield 80%) of the title compound, boiling at 60 - 62 °C at 1 mmHg (133 Pascals).

Specific Rotation $[\alpha]_D^{25}$ : -27.3 ° (c = 0.95, $CHC\ell_3$).

Infrared Absorption Spectrum ($CHC\ell_3$) $\nu_{max}$ $cm^{-1}$:
1690, 1610.

Nuclear Magnetic Resonance Spectrum ($CDC\ell_3$) $\delta$ ppm:
1.70 (3H, doublet, J = 6.5 Hz);
5.15 (1H, quartet, J = 6.5 Hz);
6.7 - 7.2 (2H, multiplet);
7.75 - 8.25 (1H, multiplet).

EXAMPLE 95

(R)-3-Chloro-2-(2,4-difluorophenyl)-1-butene

17.1 g (0.704 mole) of magnesium and 10 g (0.082 mole) of chloromethyltrimethylsilane were added to 500 ml of diethyl ether, and the mixture was heated under reflux under an atmosphere of nitrogen. A suspension of a small amount of magnesium (which had been activated with methyl iodide) in diethyl ether was then added to start the reaction, and then a solution of 76.3 g (0.622 mole) of chloromethyltrimethylsilane in 800 ml of diethyl ether was added dropwise over 30 minutes. When the addition was complete, the mixture was heated under reflux for a further 30 minutes and then cooled to -10 °C. A solution of 72 g

(0.352 mole) of (R)-(2,4-difluorophenyl) 1-chloroethyl ketone (prepared as described in Example 94) in 720 ml of diethyl ether was added dropwise over 20 minutes, and then the mixture was stirred at 0°C for 1 hour. At the end of this time, the mixture was washed with a saturated aqueous solution of ammonium chloride and extracted with hexane. The extract was washed with a saturated aqueous solution of sodium chloride, and then the solvent was distilled off under reduced pressure, to leave 105 g of an oil. The whole of this oil was dissolved in 850 ml of methylene chloride, and then 28 g (0.200 mole) of a boron trifluoride - diethyl ether complex were added, whilst stirring and ice-cooling. After 1 hour, 1 litre of hexane was added, and then the mixture was washed, in turn, with a dilute aqueous solution of sodium bicarbonate and with a saturated aqueous solution of sodium chloride. The solvent was distilled off to leave an oil, which was then distilled under reduced pressure to give 62 g (yield 82%) of the title compound, boiling at 80 - 82°C at 11 mmHg (1466 Pascals).

Specific Rotation $[\alpha]_D^{25}$ : +39.7° (c = 1.04, $CHCl_3$).

Infrared Absorption Spectrum ($CHCl_3$) $\nu_{max}$ $cm^{-1}$:
1610, 1595, 1500.

Nuclear Magnetic Resonance Spectrum ($CDCl_3$) $\delta$ ppm:
1.65 (3H, doublet, J = 6.5 Hz);
4.92 (1H, quartet, J = 6.5 Hz);
5.25 (1H, singlet);
5.64 (1H, singlet);
6.5 - 7.1 (2H, multiplet);
7.1 - 7.6 (1H, multiplet).

## EXAMPLE 96

### (2R, 3R)-3-Chloro-2-(2,4-difluorophenyl)-1,2-butanediol

51.8 g (443 mmoles) of N-methylmorpholine oxide, 10 ml (556 mmoles) of water and 600 mg of osmium tetraoxide were added to a solution of 55.4 g (273 mmoles) of (R)-3-chloro-2-(2,4-difluorophenyl)-1-butene (prepared as described in Example 95) in 550 ml of tetrahydrofuran, and then the mixture was allowed to stand overnight at room temperature. At the end of this time, the reaction mixture was diluted with ethyl acetate and washed with a saturated aqueous solution of sodium chloride. The solvent was then distilled off under reduced pressure, to give a crystalline residue, which was recrystallized from a mixture of benzene and hexane, to give 46.8 g of the title compound, melting at 71.5 - 72.5°C.

Specific Rotation $[\alpha]_D^{25}$ : +6.3° (c = 1.12, $CHCl_3$).

Infrared Absorption Spectrum ($CHCl_3$) $\nu_{max}$ $cm^{-1}$:
3550, 3400 (broad).

Nuclear Magnetic Resonance Spectrum ($CDCl_3$) $\delta$ ppm:
1.25 (3H, doublet, J = 6.5 Hz);
2.15 (1H, broad);
3.34 (1H, singlet);
3.7 - 4.4 (2H, multiplet);
4.64 (1H, quartet, J = 6.5 Hz);
6.6 - 7.2 (2H, multiplet);
7.5 - 8.0 (1H, multiplet).

The mother liquor was concentrated by evaporation under reduced pressure, and the condensate was purified by column chromatography, eluted with 20% by volume ethyl acetate in hexane, to give the title compound and 7.9 g (yield 12%) of its (2S, 3R) isomer.

6.5 g (82% total yield) of the title compound was obtained as crystals, melting at 70 - 72°C, by recrystallization from a mixture of benzene and hexane.

Specific Rotation $[\alpha]_D^{25}$ : +6.1° (c = 1.22, $CHCl_3$).

## EXAMPLE 97

### (2R, 3R)-3-Chloro-2-(2,4-difluorophenyl)-1-(methanesulphonyloxy)-2-butanol

23 g (227 mmoles) of triethylamine were added, whilst stirring and ice-cooling, to a solution of 44.3 g (187 mmoles) of (2R, 3R)-3-chloro-(2,4-difluorophenyl)-1,2-butanediol (prepared as described in Example 96) in 440 ml of methylene chloride. 25.8 g (225 mmoles) of methanesulphonyl chloride were then added

dropwise over a period of 10 minutes. After completion of the addition, the mixture was stirred at the same temperature for a further 30 minutes. Ice-water was then added to the mixture, and the organic phase was separated and washed with a saturated aqueous solution of sodium chloride. The solvent was then distilled off under reduced pressure, to give 59 g of the title compound.

Infrared Absorption Spectrum ($CHCl_3$) $\nu_{max}$ $cm^{-1}$:
3560.

Nuclear Magnetic Resonance Spectrum ($CDCl_3$) $\delta$ ppm:
1.26 (3H, doublet, J = 6.5 Hz);
2.89 (3H, singlet);
3.17 (1H, singlet);
4.63 (1H, quartet, J = 6.5 Hz);
4.78 (2H, singlet);
6.6 - 7.2 (2H, multiplet);
7.4 - 8.13 (1H, multiplet).

EXAMPLE 98

(2R, 3S)-2-(2,4-Difluorophenyl)-3-methyl-2-[(1H-1,2,4-triazol-1-yl)methyl]oxirane

24 g (550 mmoles) of sodium hydride (as a 55% w/w suspension in mineral oil) were washed with dry hexane and suspended in 600 ml of dimethylformamide. 40.1 g (580 mmoles) of triazole were slowly added to the suspension, whilst stirring and ice-cooling. When the hydrogen gas ceased to evolve, 59 g (187 mmoles) of (2R, 3R)-3-chloro-2-(2,4-difluorophenyl)-1-(methanesulphonyloxy)-2-butanol (prepared as described in Example 97) were added. The mixture was then stirred at room temperature for 10 minutes, after which it was heated at 60°C for 2.5 hours. At the end of this time, the mixture was cooled, and water was added to the reaction mixture, which was then extracted with benzene. The extract was dried, and the solvent was distilled off under reduced pressure, to give 38.5 g (yield 82%) of the title compound as crystals. The spectroscopic data of this compound coincided with those of the compound prepared as described in Example 179.

EXAMPLE 99

(2R, 3R)-3-Azido-2-(2,4-difluorophenyl)-1-(1H-1,2,4-triazol-1-yl)-2-butanol

23.5 g (360 mmoles) of sodium azide and 10.6 g (195 mmoles) of ammonium chloride were added to a solution of 30 g (119 mmoles) of (2R, 3S)-2-(2,4-difluorophenyl)-3-methyl-2-[(1H-1,2,4-triazol-1-yl)methyl]-oxirane (prepared as described in Example 98) in 400 ml of dimethylformamide, and then the mixture was stirred at 105°C for 4 hours. At the end of this time, the mixture was cooled, after which it was diluted with benzene, washed, in turn, with water and with a saturated aqueous solution of sodium chloride and dried. The solvent was then distilled off under reduced pressure, to leave 32.7 g of a crystalline residue, which was recrystallized from a mixture of benzene and hexane to give 25.8 g (yield 73%) of the title compound, melting at 139 - 140°C.

Specific Rotation $[\alpha]_D^{25}$ : -88° (c = 1.12, $CHCl_3$).

Infrared Absorption Spectrum (KBr) $\nu_{max}$ $cm^{-1}$:
3195 (broad), 2120, 2090.

Nuclear Magnetic Resonance Spectrum ($CDCl_3$) $\delta$ ppm:
1.16 (3H, doublet, J = 6.5 Hz);
3.78 (1H, quartet, J = 6.5 Hz);
4.78 (2H, singlet);
4.95 (1H, singlet);
6.5 - 7.0 (2H, multiplet);
7.2 - 7.6 (1H, multiplet);
7.76 (1H, singlet);
7.82 (1H, singlet).

EXAMPLE 100

(2R, 3R)-3-Amino-2-(2,4-difluorophenyl)-1-(1H-1,2,4-triazol-1-yl)-2-butanol

1.3 g of 10% w/w palladium-on-charcoal was added to a solution of 5.00 g of (2R, 3R)-3-azido-2-(2,4-difluorophenyl)-1-(1H-1,2,4-triazol-1-yl)-2-butanol (prepared as described in Example 99) in 100 ml of ethanol, and then the mixture was stirred at ambient pressure for 1 hour under an atmosphere of hydrogen. At the end of this time, the reaction mixture was filtered using a Celite filter aid, and the filtrate was evaporated under reduced pressure. 4.56 g (yield 100%) of a crystalline residue were recrystallized from benzene to give the title compound, melting at 154 - 155 °C.

Specific Rotation $[\alpha]_D^{25}$ : -73 ° (c = 1.06, CHCℓ$_3$).

Infrared Absorption Spectrum (KBr) $\nu_{max}$ cm$^{-1}$:
3210, 1620, 1600.

Nuclear Magnetic Resonance Spectrum (CDCℓ$_3$) δ ppm:
0.84 (3H, doublet, J = 6.5 Hz);
1.04 - 1.91 (2H, broad);
3.61 (1H, quartet, J = 6.5 Hz);
4.3 - 5.5 (1H, broad);
4.68 (2H, singlet);
6.5 - 7.1 (2H, multiplet);
7.2 - 7.84 (1H, multiplet);
7.81 (1H, singlet);
8.01 (1H, singlet).

EXAMPLE 101

(2R, 3R)-2-(2,4-Difluorophenyl)-1-(1H-1,2,4-triazol-1-yl)-3-(4-cyanobenzoylamino)-2-butanol

890 mg (5.37 mmoles) of 4-cyanobenzoyl chloride were added, whilst stirring at 0 °C, to a solution of 1.20 g (4.47 mmoles) of (2R, 3R)-3-amino-2-(2,4-difluorophenyl)-1-(1H-1,2,4-triazol-1-yl)-2-butanol (prepared as described in Example 100) in 13 ml of pyridine, and then the mixture was stirred at the same temperature for 1 hour. At the end of this time, the solvent was distilled off under reduced pressure, and the residue was mixed with an aqueous solution of sodium bicarbonate and extracted with ethyl acetate. The organic extract was washed with a saturated aqueous solution of sodium chloride and dried. The solvent was then distilled from the dried organic extract, to leave an oil, which was purified by column chromatography through silica gel, eluted with benzene containing ethyl acetate in amounts ranging from 35 to 40% by volume, to give 1.70 g of a crystalline product. This product was recrystallized from a mixture of ethyl acetate and hexane to give 1.33 g of the title compound, melting at 156 - 157 °C.

Specific Rotation $[\alpha]_D^{25}$ : -122 ° (c = 1.01, CHCℓ$_3$).

Infrared Absorption Spectrum (KBr) $\nu_{max}$ cm$^{-1}$:
3450, 2230, 1659.

Nuclear Magnetic Resonance Spectrum (CDCℓ$_3$) δ ppm:
1.02 (3H, doublet, J = 6.5 Hz);
4.46 (1H, doublet, J = 14 Hz)
5.08 (1H, doublet, J = 14 Hz);
5.40 (1H, doublet, J = 1.8 Hz);
6.6 - 7.13 (2H, multiplet);
7.18 - 7.6 (2H, multiplet);
7.78 (2H, doublet, J = 8 Hz):
7.83 (1H, singlet);
7.87 (1H, singlet);
8.02 (2H, doublet, J = 8 Hz).

EXAMPLE 102

(2R, 3R)-3-Amino-2-(4-chlorophenyl)-1-(1H-1,2,4-triazol-1-yl)-2-butanol

(a) (2R, 3S)-2-(4-Chlorophenyl)-3-methyl-2-[(1H-1,2,4-triazol-1-yl)methyl]oxirane

Following a procedure similar to that described in Example 98, the title compound was prepared as an oil.

Specific Rotation $[\alpha]_D^{25}$ : -7.1° (c = 1.16, $CHCl_3$).

Nuclear Magnetic Resonance Spectrum ($CDCl_3$) δ ppm:
1.60 (3H, doublet, J = 5.5 Hz);
3.11 (1H, quartet, J = 5.5 Hz);
4.39 (1H, doublet, J = 15 Hz);
4.82 (1H, doublet, J = 15 Hz);
6.95 - 7.35 (4H, multiplet);
7.86 (1H, singlet);
7.95 (1H, singlet).

(b) (2R, 3R)-3-Azido-2-(4-chlorophenyl)-1-(1H-1,2,4-triazol-1-yl)-2-butanol

Following a procedure similar to that described in Example 99, the title compound was prepared as crystals, melting at 121 - 122.5°C.

Specific Rotation $[\alpha]_D^{25}$ : -71.3° (c = 0.80, $CHCl_3$).

Infrared Absorption Spectrum (KBr) $\nu_{max}$ $cm^{-1}$:
3200 (broad), 2120, 2080.

(c) (2R, 3R)-3-Amino-2-(4-chlorophenyl)-1-(1H-1,2,4-triazol-1-yl)-2-butanol

Following a procedure similar to that described in Example 64, the title compound was prepared. Its nuclear magnetic resonance spectrum coincided with that of the compound prepared as described in Example 64.

EXAMPLE 103

(2R, 3R)-2-(4-Chlorophenyl)-1-(1H-1,2,4-triazol-1-yl)-3-[(4-trifluoromethyl)benzoylamino]-2-butanol

Following a procedure similar to that described in Example 101, the title compound was prepared as crystals, melting at 200 - 201°C.

Specific Rotation $[\alpha]_D^{25}$ : -139° (c = 0.49, $CHCl_3$).

Infrared Absorption Spectrum (KBr) $\nu_{max}$ $cm^{-1}$:
3370, 1647, 1528.

Nuclear Magnetic Resonance Spectrum ($CDCl_3$) δ ppm:
1.04 (3H, doublet, J = 6.5 Hz);
4.47 (1H, doublet, J = 14 Hz);
4.72 (1H, doublet, J = 14 Hz);
~4.6 (1H, multiplet);
5.30 (1H, broad singlet);
6.86 (1H, broad doublet, J = 9 Hz);
7.30 (4H, singlet);
7.69 (1H, singlet);
7.85 (1H, singlet);
7.75 (2H, broad doublet);
8.02 (2H, broad doublet).

EXAMPLE 104

(2R, 3R)-2-(4-Chlorophenyl)-3-[2-fluoro-4-(trifluoromethyl)benzoylamino]-1-(1H-1,2,4-triazol-1-yl)-2-butanol and its hydrochloride

Following a procedure similar to that described in Example 101, the title compound was prepared as an oil.

Infrared Absorption Spectrum ($CHCl_3$) $\nu_{max}$ $cm^{-1}$:
3440, 1660.

Nuclear Magnetic Resonance Spectrum ($CDCl_3$) $\delta$ ppm:
1.03 (3H, doublet, J = 6.5 Hz);
4.5 - 5.5 (4H, multiplet);
7.30 (4H, singlet);
7.0 - 7.8 (3H, multiplet);
7.69 (1H, singlet);
7.84 (1H, singlet);
8.25 (1H, broad triplet, J = 8 Hz).

Specific Rotation $[\alpha]_D^{25}$ : -107° (c = 0.54, $CHCl_3$).

1.30 g of the title compound were dissolved in ethyl acetate, and then crystallization was effected by adding 0.7 ml of 4N hydrogen chloride in dioxane to give 1.16 g of the hydrochloride, melting at 157 - 160°C.

Specific Rotation $[\alpha]_D^{25}$ : -79° (c = 0.62, $CH_3OH$).

EXAMPLES 105 TO 132

In addition, by following procedures essentially as described above, the compounds shown in the following Table 8 were also prepared. The Example No. given in the last column of the following Table 8 gives the number of the foregoing Example whose procedure was most closely followed in the preparation of each compound. In the following Table, where the product was an oil and the property given is its Rf value, this was obtained by thin layer chromatography using a Merck, Art 5717, silica gel plate and the developing solvent specified in the Table.

Table 8

| Example No. | Cpd No. | stereochemistry | melting point or other property | prep as in Ex. |
|---|---|---|---|---|
| 105 | 1-133 | 2R*, 3R* | 164 - 165°C | 8 |
| 106 | 1-134 | 2R*, 3R* | 168 - 169°C | 8 |
| 107 | 1-135 | 2R*, 3R* | 150 - 151°C | 8 |
| 108 | 1-136 | 2R*, 3R* | 178°C | 8 |
| 109 | 1-137 | 2R*, 3R* | 224 - 225°C | 8 |
| 110 | 1-138 | 2R*, 3R* | 184 - 186°C | 8 |
| 111 | 1-139 | 2R*, 3R* | 159 - 160°C | 8 |
| 112 | 1-140 (as oxalate) | 2R*, 3R* | 88 - 89°C | 8 |
| 113 | 1-141 (as oxalate) | 2R*, 3R* | 174 - 176°C | 8 |
| 114 | 1-142 | 2R*, 3R* | 166 - 167°C | 8 |
| 115 | 1-143 | 2R*, 3R* | 219 - 223°C | 8 |
| 116 | 1-144 | 2R*, 3R* | oil, Rf=0.67 (ethyl acetate:methanol = 2:1 by volume) | 8 |
| 117 | 1-145 | 2R*, 3R* | amorphous | 1 |
| 118 | 1-146 | 2R*, 3R* | 177 - 179°C | 1 |
| 119 | 1-147 | 2R*, 3R* | 175 - 177°C | 1 |
| 120 | 1-148 | 2R*, 3R* | 155 - 157°C | 8 |
| 121 | 1-149 (as oxalate) | 2R*, 3R* | 156 - 160°C | 8 |

Table 8 (cont)

| Example No. | Cpd No. | stereochemistry | melting point or other property | prep as in Ex. |
|---|---|---|---|---|
| 122 | 1-150 | 2R*, 3R* | 201 - 205°C | 1 |
| 123 | 1-152 | 2R*, 3R* | 164 - 166°C | 8 |
| 124 | 1-153 | 2R*, 3R* | 173 - 174°C | 8 |
| 125 | 1-154 | 2R*, 3R* | 186 - 188°C | 8 |
| 126 | 1-155 | 2R*, 3R* | 222 - 223°C | 8 |
| 127 | 1-156 | 2R*, 3R* | 187 - 189°C | 8 |
| 128 | 1-157 | 2R*, 3R* | 204°C | 1 |
| 129 | 1-158 | 2R*, 3R* | amorphous | 1 |
| 130 | 1-159 | 2R*, 3R* | 165 - 166°C | 8 |
| 131 | 1-160 | 2R*, 3R* | 225 - 227°C | 8 |
| 132 | 1-161 | 2R*, 3R* | 133 - 134°C | 8 |

EXAMPLE 133

(2R*, 3R*)-2-(2,4-Difluorophenyl)-3-[2-(hydroxymethyl)benzoylamino]-1-(1H-1,2,4-triazol-1-yl)-2-butanol

130 mg (2.99 mmoles) of sodium hydride (as a 55% w/w suspension in mineral oil) were washed with hexane and suspended in 8 ml of dimethyl sulphoxide. 400 mg (1.49 mmoles) of (2R*, 3R*)-3-amino-2-(2,4-difluorophenyl)-1-(1H-1,2,4-triazol-1-yl)-2-butanol (prepared by a procedure similar to that described in Example 3) were then added, whilst ice-cooling, to the suspension, and then the mixture was stirred for 15 minutes. After this, 600 mg (4.48 mmole) of phthalide were added, and then the mixture was stirred at 60 °C for 1 hour. At the end of this time, the reaction mixture was cooled, mixed with water and extracted with ethyl acetate. The extract was dried, and the solvent was distilled off under reduced pressure to leave a crystalline residue. The residue was recrystallized from ethyl acetate to give 270 mg of the title compound, melting at 204 - 205 °C.

Infrared Absorption Spectrum (KBr) $\nu_{max}$ $cm^{-1}$:
3450, 3300, 1655, 1620.

Nuclear Magnetic Resonance Spectrum ($CD_3OD$) $\delta$ ppm:
1.06 (3H, doublet, J = 6.5 Hz);
4.4 - 5.3 (5H, multiplet);
6.6 - 7.8 (8H, multiplet);
8.1 (1H, broad).

EXAMPLE 134

(2R*, 3R*)-2-(2,4-Difluorophenyl)-3-[2-(acetoxymethyl)benzoylamino)-1-(1H-1,2,4-triazol-1-yl)-2-butanol

0.5 ml of pyridine, 11 mg (0.11 mmole) of triethylamine, 12 mg (0.11 mmole) of acetic anhydride and 1 mg of 4-(dimethylamino)pyridine were added, whilst stirring and at room temperature, to a suspension of (2R*, 3R*)-3-[2-(hydroxymethyl)benzoylamino]-2-(2,4-difluorophenyl)-1-(1H-1,2,4-triazol-1-yl)-2-butanol (prepared as described in Example 133) in 1 ml of methylene chloride, and then the mixture was stirred for 30 minutes. After this, the reaction mixture was diluted with ethyl acetate and washed with a saturated aqueous solution of sodium chloride. The solvent was then distilled off under reduced pressure to leave a crystalline residue, which was recrystallized from a mixture of ethyl acetate and hexane to give 25 mg of the title compound, melting at 162 - 163 °C.

Nuclear Magnetic Resonance Spectrum ($CDC\ell_3$) $\delta$ ppm:
1.04 (3H, doublet, J = 6.5 Hz);
2.07 (3H, singlet);
4.56 (1H, doublet, J = 14.5 Hz);
5.11 (1H, doublet, J = 14.5 Hz);
5.34 (2H, singlet);
4.7 - 5.5 (2H, multiplet);
6.5 - 7.7 (8H, multiplet);
7.78 (1H, singlet);
7.84 (1H, singlet).

EXAMPLE 135

(2R*, 3R*)-2-(2,4-Difluorophenyl)-3-(4-chlorophthalimido)-1-(1H-1,2,4-triazol-1-yl)-2-butanol and its nitrate

A solution of 150 mg (0.56 mmole) of (2R*, 3R*)-3-amino-2-(2,4-difluorophenyl)-1-(1H-1,2,4-triazol-1-yl)-2-butanol (prepared by a procedure similar to that described in Example 3), 124 mg (0.68 mmole) of 4-chlorophthalic acid anhydride and 5 mg (0.05 mmole) of triethylamine in 4 ml of toluene was heated under reflux for 4 hours, while removing the water formed in situ through a Dean Stark tube packed with molecular sieve 4A. At the end of this time, the reaction mixture was mixed with ethyl acetate and with a dilute aqueous solution of sodium bicarbonate. The organic phase was dried and the solvent was distilled off under reduced pressure. The residue was purified by preparative thin layer chromatography (silica gel, developed with ethyl acetate) to give 238 mg of the title compound as a colourless foam.

Infrared Absorption Spectrum ($CHC\ell_3$) $\nu_{max}$ $cm^{-1}$:
3380, 1770, 1705, 1615, 1500, 1350, 1270, 1130, 1040, 960, 850.

Nuclear Magnetic Resonance Spectrum ($CDC\ell_3$) $\delta$ ppm:
1.35 (3H, doublet, J = 7 Hz);
4.40 (1H, doublet, J = 15 Hz);
4.90 (1H, doublet, J = 15 Hz);
5.16 (1H, quartet, J = 7 Hz);
5.3 (1H, broad);
6.6 - 7.1 (2H, multiplet);
7.54 (1H, singlet);
8.13 (1H, singlet);
7.4 - 8.1 (4H, multiplet).

127 mg of the compound obtained as described above were dissolved in diethyl ether, and then about 20 mg of fuming nitric acid were added, to give 108 mg of the nitrate of the title compound, melting at 143 - 150 °C.

EXAMPLE 136

(2R*, 3R*)-2-(2,4-Difluorophenyl)-3-(1-oxo-2,3-dihydro-2-isoindolyl)-1-(1H-1,2,4-triazol-1-yl)-2-butanol

182 mg (0.70 mmole) of triphenylphosphine and 121 mg (0.70 mmole) of diethyl azodicarboxylate were added, whilst stirring and ice-cooling, to a solution of 140 mg (0.35 mmole) of (2R*, 3R*)-3-[2-(hydroxymethyl)benzoylamino]-2-(2,4-difluorophenyl)-1-(1H-1,2,4-triazol-1-yl)-2-butanol (prepared as de-scribed in Example 133) in 3 ml of tetrahydrofuran, and then the mixture was stirred for 10 minutes. At the end of this time, water was added to the reaction mixture, which was then extracted with ethyl acetate. The extract was dried, and the solvent was distilled off under reduced pressure to give an oil, which was subjected to column chromatography through silica gel, eluted with a 3 : 1 by volume mixture of benzene and ethyl acetete, to give about 200 mg of a crude product. This product was subjected twice to preparative thin layer chromatography (silica gel, eluted with a 10 : 10: 1 by volume mixture of chloroform, ethyl acetate and ethanol), to give crude crystals, which were recrystallized from a mixture of ethyl acetate and hexane to give 62 mg of the title compound, melting at 171 - 173 °C.

Infrared Absorption Spectrum ($CHC\ell_3$) $\nu_{max}$ $cm^{-1}$:
3400, 1673.

Nuclear Magnetic Resonance Spectrum ($CDC\ell_3$) $\delta$ ppm:
1.18 (3H, doublet, J = 6.5 Hz);
4.27 (1H, doublet, J = 14.5 Hz);
4.56 (1H, doublet, J = 18 Hz);
4.99 (1H, doublet, J = 18 Hz);
5.04 (1H, quartet, J = 6.5 Hz);
5.17 (1H, doublet of doublets, J = 14.5 & 0.5 Hz);
5.7 (1H, broad);
6.6 - 7.0 (2H, multiplet);
7.2 - 8.0 (5H, multiplet);
7.68 (1H, singlet);
7.90 (1H, singlet).

EXAMPLES 137 TO 170

In addition, by following procedures essentially as described above, the compounds shown in the following Table 9 were also prepared. The Example No. given in the last column of the following Table 9 gives the number of the foregoing Example whose procedure was most closely followed in the preparation of each compound. In the following Table, where the product was an oil and the property given is its Rf value, this was obtained by thin layer chromatography using a Merck, Art 5717, silica gel plate and the developing solvent specified in the Table.

Table 9

| Example No. | Cpd No. | stereochemistry | melting point or other property | prep as in Ex. |
|---|---|---|---|---|
| 137 | 1-164 | 2R*, 3R* | 128 - 129°C | 8 |
| 138 | 1-165 | 2R*, 3R* | 183 - 185°C | 8 |
| 139 | 1-169 | 2R*, 3R* | 165 - 168°C | 29 |
| 140 | 1-170 | 2R*, 3R* | 155 - 156°C | 29 |
| 141 | 1-180 (as oxalate) | 2R*, 3S* | 168 - 170°C | 55 |
| 142 | 1-181 | 2R*, 3R* | 106 - 108°C | 55 |
| 143 | 1-181 | 2R*, 3S* | 76 - 77°C | 55 |
| 144 | 1-181 | 2S, 3R | 125 - 126°C | 55 |
| 145 | 1-182 | 2R*, 3R* | 134 - 136°C | 55 |
| 146 | 1-183 | 2R*, 3R* | 98 - 99°C | 55 |
| 147 | 1-184 | 2R*, 3R* | 68 - 73°C | 55 |
| 148 | 1-185 | 2R*, 3R* | oil, Rf=0.27 (ethyl acetate:hexane = 1:1 by volume) | 55 |
| 149 | 1-187 | 2R*, 3R* | 86 - 87°C | 55 |
| 150 | 1-187 | 2R*, 3S* | 125°C | 55 |
| 151 | 1-188 | 2R*, 3R* | 115 - 117°C | 55 |
| 152 | 1-189 | 2R*, 3R* | 120 - 121°C | 55 |
| 153 | 1-191 | 2R*, 3S* | 103 - 104°C | 69 |
| 154 | 1-193 | 2R*, 3R* | 114 - 115°C | 69 |
| 155 | 1-25 | 2R*, 3R* | 165 - 166°C | 8 |
| 156 | 1-24 (as oxalate) | 2R, 3R | 192 - 197°C | 73 |
| 157 | 1-24 (as hydrochloride) | 2R, 3R | 124 - 138°C | 73 |
| 158 | 1-26 | 2R, 3R | 153 - 157°C | 73 |
| 158A | 1-66 | 2R, 3R | 230 - 232°C | 73 |
| 159 | 1-27 | 2R*, 3R* | 174 - 177°C | 8 |

Table 9 (cont)

| Example No. | Cpd No. | stereochemistry | melting point or other property | prep as in Ex. |
|---|---|---|---|---|
| 160 | 1-27 | 2R*, 3S* | 138 - 148°C | 8 |
| 161 | 1-48 | 2R*, 3R* | 188 - 190°C | 1 |
| 162 | 1-117 | 2R, 3R | 63°C (with decomp.) | 31 |
| 163 | 1-47 | 2R*, 3R* | 197 - 199°C | 1 |
| 164 | 1-2 (as oxalate) | 2R*, 3S* | 165 - 166°C | 1 |
| 165 | 1-2 (as oxalate) | 2S, 3S | 115 - 122°C (with decomp.) | 1 |
| 166 | 1-13 | 2R*, 3R* | 122 - 130°C (with decomp.) | 8 |
| 167 | 1-55 | 2R*, 3R* | 188 - 189°C | 8 |
| 168 | 1-125 | 2R*, 3R* | 155 - 157.5°C | 39 |
| 169 | 1-226 | 2R*, 3R* | 218 - 220°C | 8 |
| 170 | 1-225 | 2R*, 3R* | 157 - 159°C | 8 |

EXAMPLE 171

(S)-(2,4-Difluorophenyl) 1-hydroxyethyl ketone

16.9 ml (0.197 mole) of oxalyl chloride and 1.0 ml of dimethylformamide were added to a solution of 20 g (0.15 mole) of (S)-2-acetoxypropionic acid [S. G. Cohen et al., J. Am. Chem. Soc., 85, 1685 (1963)] in 100 ml of methylene chloride, and then the mixture was stirred at room temperature for 1.5 hours. At the end of this time, the solvent was distilled off to leave an oil, to which 25.9 g (0.227 mole) of m-difluorobenzene were added. 50.5 g (0.379 mole) of aluminium chloride were then added, whilst stirring and ice-cooling, and then the mixture was warmed to room temperature and stirred overnight. 60 ml of methylene chloride were then added to the reaction mixture, after which it was added slowly to ice-water and diluted with 400 ml of ethyl acetate. The organic phase was separated and washed, in turn, with water, with a dilute aqueous solution of sodium bicarbonate and with a saturated aqueous solution of sodium chloride. The solvent was then distilled off to leave 36 g of an oil, which was dissolved in 180 ml of methanol. A mixture of 18 ml of water and 15 ml of concentrated sulphuric acid was added, whilst stirring and ice-cooling, to the previous mixture, and then the whole mixture was warmed to room temperature and stirred overnight. At the end of this time, the reaction mixture was concentrated by evaporation under reduced pressure, 400 ml of ethyl acetate were added to the residue, and the mixture was washed, in turn, with water, with a dilute aqueous solution of sodium bicarbonate and with a saturated aqueous solution of sodium chloride. The solvent was then distilled off under reduced pressure, to leave an oil, which was distilled in vacuo to give 18.65 g (yield 66.8%) of the title compound, boiling at 65 - 67°C at 3 mmHg (400 Pascals).

Specific Rotation $[\alpha]_D^{25}$ : -67.1° (c = 1.17, $CHCl_3$).

Infrared Absorption Spectrum ($CHCl_3$) $\nu_{max}$ $cm^{-1}$:
3500, 1680.

Nuclear Magnetic Resonance Spectrum ($CDCl_3$ + $D_2O$) $\delta$ ppm:
1.42 (3H, doublet of doublets, J = 6.5 & 2 Hz);
5.0 (1H, quartet of doublets, J = 6.5 & 2 Hz)
6.7 - 7.3 (2H, multiplet);
7.8 - 8.2 (1H, multiplet).

## EXAMPLE 172

### (S)-(2,4-Difluorophenyl) 1-(p-toluenesulphonyloxy)ethyl ketone

10.24 g (53.7 mmoles) of p-toluenesulphonyl chloride were added, whilst stirring at -10 °C, to a solution of 5.00 g (26.9 mmoles) of (S)-(2,4-difluorophenyl) 1-hydroxyethyl ketone (prepared as described in Example 171) in 25 ml of pyridine, and then the mixture was stirred at the same temperature for 6 hours. At the end of this time, the reaction mixture was cooled to -20 °C and mixed with a dilute aqueous solution of sodium bicarbonate and 100 ml of ethyl acetate. The organic phase was separated and washed, in turn, with a dilute aqueous solution of sodium bicarbonate and with a saturated aqueous solution of sodium chloride. The solvent was then distilled off under reduced pressure, to leave a crystalline residue, which was recrystallized from a mixture of acetone and cyclohexane to give 5.49 g (yield 60%) of the title compound, melting at 88 - 90 °C.

Specific Rotation $[\alpha]_D^{25}$ : -19.3 ° (c = 0.92, $CHCl_3$).

Infrared Absorption Spectrum ($CHCl_3$) $\nu_{max}$ $cm^{-1}$:
1695.

Nuclear Magnetic Resonance Spectrum ($CDCl_3$) $\delta$ ppm:
1.53 (3H, doublet of doublets, J = 7 & 2 Hz);
2.42 (3H, singlet);
5.67 (1H, quartet, J = 7 Hz)
6.7 - 7.1 (2H, multiplet);
7.27 (2H, doublet, J = 9 Hz);
7.78 (2H, doublet, J = 9 Hz);
7.6 - 8.1 (1H, multiplet).

## EXAMPLE 173

### (R)-(2,4-Difluorophenyl) 1-hydroxyethyl ketone

A solution of 352 mg (14.7 mmoles) of lithium hydroxide in 20 ml of water was added dropwise, whilst stirring at -15 °C, to a solution of 5,00 g (14.7 mmoles) of (S)-(2,4-difluorophenyl) 1-(p-toluenesulphonyloxy)-ethyl ketone (prepared as described in Example 172) in 50 ml of dimethylformamide over a period of 2 hours. After completion of the addition, the mixture was stirred at the same temperature for 1 hour and then mixed with 20 ml of a 5% w/v aqueous solution of ammonium chloride. The mixture was then extracted with 100 ml of ethyl acetate and the extract was washed, in turn, with a dilute aqueous solution of ammonium chloride and with a saturated aqueous solution of sodium chloride. The solvent was then distilled off under reduced pressure, to leave an oil, which was purified by column chromatogarphy through 75 g of silica gel, eluted with a 20 : 1 by volume mixture of benzene and ethyl acetate, to give 2.00 g (yield 73.2%) of the title compound as an oil.

Specific Rotation $[\alpha]_D^{25}$ : +64.2 ° (c = 0.74, $CHCl_3$).

The infrared absorption spectrum and the nuclear magnetic resonance spectrum of this compound coincided with those of the compound prepared as described in Example 171.

## EXAMPLE 174

### (R)-(2,4-Difluorophenyl) 1-(2-tetrahydropyranyloxy)ethyl ketone

1.17 g (13.9 mmoles) of 2,3-dihydropyran and 135 mg (0.54 mmole) of pyridinium p-toluenesulphonate were added, whilst stirring and ice-cooling, to a solution of 2.00 g (10.7 mmoles) of (R)-(2,4-difluorophenyl) 1-hydroxyethyl ketone (prepared as described in Example 173) in 20 ml of methylene chloride. The mixture

was then warmed to room temperature and allowed to stand overnight. At the end of this time, the mixture was diluted with 50 ml of ethyl acetate and washed with a saturated aqueous solution of sodium chloride. The solvent was then distilled off under reduced pressure, to leave an oil, which was purified by column chromatography through 100 ml of silica gel, eluted with a 5 : 1 by volume mixture of benzene and hexane, to give 2.54 g (yield 87.5%) of the title compound as an oil.

Specific Rotation $[\alpha]_D^{25}$ : +51.1° (c = 1.35, $CHCl_3$).

Infrared Absorption Spectrum ($CHCl_3$) $\nu_{max}$ $cm^{-1}$:
1695.

Nuclear Magnetic Resonance Spectrum ($CDCl_3$) δ ppm:
1.42 (1.5H, doublet of doublets, J = 6.5 & 3 Hz):
1.45 (1.5H, doublet of doublets, J = 6.5 & 1.5 Hz);
1.3 - 2.0 (6H);
3.1 - 4.1 (2H, multiplet);
3.6 - 3.8 (1H, multiplet);
4.86 (0.5H, quartet of doublets, J = 6.5 & 2 Hz);
5.10 (0.5H, quartet of doublets, J = 6.5 & 2 Hz);
6.7 - 7.2 (2H, multiplet);
7.7 - 8.2 (1H, multiplet).

EXAMPLE 175

(2S, 3R)-2-(2,4-Difluorophenyl)-1-(dimethylisopropoxysilyl)-3-(2-tetrahydropyranyloxy)-2-butanol

0.50 g (20.6 mmoles) of magnesium and 3.03 g (18.2 mmoles) of chloromethyldimethylisopropoxysilane were added to 60 ml of diethyl ether, and then the mixture was heated under reflux under an atmosphere of nitrogen. A suspension of a small amount of magnesium (which had been activated with methyl iodide) in diethyl ether was added to start the reaction. After the reaction mixture had been heated under reflux for a further 3 hours, it was cooled to 0°C. A solution of 2.62 g (9.7 mmoles) of (R)-(2,4-difluorophenyl) 1-(2-tetrahydropyranyloxy)ethyl ketone (prepared as described in Example 174) in 15 ml of tetrahydrofuran was then added dropwise over a period of 10 minutes, whilst stirring at 0°C, to the reaction mixture. The mixture was then warmed to room temperature, stirred for 15 minutes, and then again cooled to 0°C. A saturated aqueous solution of ammonium chloride was added to the mixture, which was then extracted with ethyl acetate. The extract was washed with a saturated aqueous solution of sodium chloride, and the solvent was distilled off to give 4.02 g of the title compound as an oil.

Infrared Absorption Spectrum ($CHCl_3$) $\nu_{max}$ $cm^{-1}$:
3425.

EXAMPLE 176

(2R, 3R)-2-(2,4-Difluorophenyl)-3-(2-tetrahydropyranyloxy)-1,2-butanediol

0.7 g (8.33 mmoles) of sodium bicarbonate and 7 ml of 35% v/v aqueous hydrogen peroxide were added to a solution of 4.54 g (11.28 mmoles) of (2S, 3R)-2-(2,4-difluorophenyl)-1-(dimethylisopropoxysilyl)-3-(2-tetrahydropyranyloxy)-2-butanol (prepared as described in Example 175) in 50 ml of a 1 : 1 by volume mixture of tetrahydrofuran and methanol, and then the mixture was stirred at 70°C for 1.5 hours. After the reaction mixture had been cooled, it was diluted with 100 ml of ethyl acetate and washed with a saturated aqueous solution of sodium chloride. The solvent was then distilled off under reduced pressure, to give 4.5 g of the title compound.

Infrared Absorption Spectrum ($CHCl_3$) $\nu_{max}$ $cm^{-1}$:
3550.

Nuclear Magnetic Resonance Spectrum ($CDCl_3$) δ ppm:
0.92 (1.5H, doublet, J = 6.5 Hz);
1.03 (1.5H, doublet, J = 6.5 Hz);
1.3 - 2.0 (6H, multiplet);
3.2 - 4.8 (8H, multiplet);
6.5 - 7.1 (2H, multiplet);
7.78 (1H, triplet of doublets, J = 9 & 7 Hz).

EXAMPLE 177

(2R, 3R)-2-(2,4-Difluorophenyl)-1,2,3-butanetriol

120 mg (0.63 mmole) of p-toluenesulphonic acid were added to a solution of 2.85 g (9.43 mmoles) of (2R, 3R)-2-(2,4-difluorophenyl)-3-(2-tetrahydropyranyloxy)-1,2-butanediol (prepared as described in Example 176) in 40 ml of methanol, and then the mixture was stirred at room temperature for 30 minutes. At the end of this time, 0.097 ml (0.70 mmole) of triethylamine was added and the stirring was continued for a further 5 minutes. The solvent was then distilled off under reduced pressure, to give a crystalline product, which was recrystallized from a mixture of acetone and cyclohexane, to give 1.58 g (yield 77%) of the title compound, melting at 81 - 82°C.

Specific Rotation $[\alpha]_D^{25}$ : +11.4° (c = 1.11, $CHCl_3$).

Infrared Absorption Spectrum (KBr) $\nu_{max}$ $cm^{-1}$:
3430 (broad), 3370 (broad).

Nuclear Magnetic Resonance Spectrum ($CD_3COCD_3$) $\delta$ ppm:
0.87 (3H, doublet, J = 6.5 Hz);
3.5 - 4.7 (6H, multiplet);
6.7 - 7.2 (2H, singlet);
7.81 (1H, triplet of doublets, J = 9 & 7 Hz).

EXAMPLE 178

(2R, 3R)-2-(2,4-Difluorophenyl)-1,3-bis(methanesulphonyloxy)-2-butanol

1.51 ml (19.5 mmoles) of methanesulphonyl chloride were added, whilst stirring at 0°C, to a solution of 1.54 g (7.06 mmoles) of (2R, 3R)-2-(2,4-difluorophenyl)-1,2,3-butanetriol (prepared as described in Example 177) in 6 ml of pyridine, and then the mixture was stirred at the same temperature for 30 minutes. At the end of this time, the pyridine was distilled off under reduced pressure to leave an oil, which was diluted with 50 ml of ethyl acetate and washed, in turn, with a dilute aqueous solution of sodium bicarbonate and with a saturated aqueous solution of sodium chloride. The solvent was then distilled off under reduced pressure, to give 2.64 g (yield 96%) of the title compound as an oil.

Nuclear Magnetic Resonance Spectrum ($CDCl_3$) $\delta$ ppm:
1.23 (3H, doublet, J = 6.5 Hz);
2.95 (3H, singlet);
3.10 (3H, singlet);
3.82 (1H, singlet);
4.70 (2H, singlet);
5.32 (1H, quartet, J = 6.5 Hz)
6.7 - 7.2 (2H, multiplet);
7.6 - 8.0 (1H, multiplet).

EXAMPLE 179

(2R, 3S)-2-(2,4-Difluorophenyl)-3-methyl-2-[(1H-1,2,4-triazol-1-yl)methyl]oxirane

1.08 g (24.7 mmoles) of sodium hydride (as a 55% w/w suspension in mineral oil) was washed with dry hexane and suspended in 30 ml of dimethylformamide. 1.95 g (28.2 mmoles) of triazole were then slowly added to the suspension, whilst stirring at 0°C. When the hydrogen gas ceased to evolve, a solution of 2.62 g (7.05 mmoles) of (2R, 3R)-2-(2,4-difluorophenyl)-1,3-bis(methanesulphonyloxy)-2-butanol (prepared as described in Example 178) in 5 ml of dimethylformamide were added. The mixture was then stirred at 65 - 70°C for 2 hours, after which it was cooled. The solvent was then distilled off under reduced pressure, and water was added to the reaction mixture, which was then extracted twice, each time with 50 ml of benzene. The organic phase was washed with a saturated aqueous solution of sodium chloride, and the solvent was distilled off under reduced pressure, to leave an oil. This oil was purified by column chromatography through 30 g of silica gel, eluted with a 2 : 1 by volume mixture of benzene and hexane, to give crystals. These crystals were recrystallized from a mixture of benzene and hexane to give 1.40 g (yield 79%) of the title compound, melting at 88 - 89.5°C.

Specific Rotation $[\alpha]_D^{25}$ : -7.55° (c = 1.06, $CHCl_3$).

Nuclear Magnetic Resonance Spectrum ($CDC\ell_3$) δ ppm:
1.64 (3H, doublet, J = 5.5 Hz);
3.16 (1H, quartet, J = 5.5 Hz);
4.40 (1H, doublet, J = 15 Hz);
4.88 (1H, doublet, J = 15 Hz);
6.5 - 7.2 (3H, multiplet);
7.79 (1H, singlet);
7.98 (1H, singlet).

EXAMPLE 180

(3S, 4R)-3-(2,4-Difluorophenyl)-4-(2-tetrahydropyranyloxy)-1-penten-3-ol

95 mg (3.91 mmoles) of magnesium and 0.44 ml (6.22 mmoles) of vinyl bromide were added to 6 ml of anhydrous tetrahydrofuran, and the mixture was stirred at 45 - 50 °C for 5 minutes under an atmosphere of nitrogen. When the reaction started, the mixture was cooled to room temperature and was then stirred for a further 20 minutes. At the end of this time, the reaction mixture was cooled with dry ice in acetone. A solution of 420 mg (1.55 mmoles) of (R)-(2,4-difluorophenyl) 1-(2-tetrahydropyranyloxy)ethyl ketone (prepared as described in Example 174) in 4 ml of anhydrous tetrahydrofuran was then added dropwise to the reaction mixture over a period of 10 minutes, whilst stirring. When the addition was complete, the reaction mixture was warmed to -30 °C, after which stirring was continued for a further 20 minutes. At the end of this time, a saturated aqueous solution of ammonium chloride was added to the reaction mixture, which was then extracted with ethyl acetate. The organic phase was washed with a saturated aqueous solution of sodium chloride, and the solvent was then distilled off under reduced pressure, to give 464 mg (yield 100%) of the title compound as an oil.

Nuclear Magnetic Resonance Spectrum ($CDC\ell_3$) δ ppm:
0.92 (1.5H, doublet, J = 6.5 Hz);
1.01 (1.5H, doublet, J = 6.5 Hz);
1.2 - 2.0 (6H, multiplet);
3.19 (1H, doublet, J = 4 Hz);
3.3 - 4.1 (3H, multiplet);
4.1 - 4.6 (1H, multiplet);
4.6 - 4.9 (1H, multiplet);
5.0 - 5.6 (2H, multiplet);
6.2 - 7.0 (3H, multiplet);
7.6 - 0.0 (1H, multiplet).

EXAMPLE 181

(2R, 3S)-3-(2,4-Difluorophenyl)-4-penten-2,3-diol

30 mg (0.16 mmole) of p-toluenesulphonic acid were added to a solution of 464 mg (1.56 mmoles) of (3S, 4R)-3-(2,4-difluorophenyl)-4-(2-tetrahydropyranyloxy)-1-penten-3-ol (prepared as described in Example 180) in 5 ml of methanol, and then the mixture was strirred at room temperature for 20 minutes. At the end of this time, the reaction mixture was diluted with 50 ml of ethyl acetate, and it was then washed, in turn, with a dilute aqueous solution of sodium bicarbonate and with a saturated aqueous solution of sodium chloride. The solvent was then distilled off under reduced pressure, to leave an oil, which was purified by column chromatography through 10 g of silica gel, eluted with a 10 : 1 by volume mixture of benzene and ethyl acetate, to give 280 mg (yield 84%) of the title compound as an oil.

Specific Rotation $[\alpha]_D^{25}$ : -49.3° (c = 1.22, $CHC\ell_3$).

Nuclear Magnetic Resonance Spectrum ($CDC\ell_3$ + $D_2O$) δ ppm:
0.98 (3H, doublet, J = 6.5 Hz);
4.38 (1H, quartet of doublets, J = 6.5 & 3 Hz);
5.23 (1H, broad doublet, J = 11 Hz);
5.44 (1H, broad doublet, J = 18 Hz);
6.50 (1H, doublet of doublets of doublets, J = 18, 11 & 3 Hz);
6.5 - 7.0 (2H, multiplet);
7.5 - 8.0 (1H, multiplet).

EXAMPLE 182

(3S, 4R)-3-(2,4-Difluorophenyl)-4-(methanesulphonyloxy)penten-3-ol

0.18 ml (2.32 mmoles) of methanesulphonyl chloride was added, whilst stirring at 0°C, to a solution of 280 mg (1.31 mmoles) of (2R, 3S)-3-(2,4-difluorophenyl)-4-penten-2,3-diol (prepared as described in Example 181) in 4 ml of pyridine. After 15 minutes, the pyridine was distilled off in vacuo. The residue was diluted with 50 ml of ethyl acetate and then washed, in turn, with a dilute aqueous solution of sodium bicarbonate and with a saturated aqueous solution of sodium chloride. The solvent was then distilled off under reduced pressure, to give 382 mg of the title compound as an oil.

Infrared Absorption Spectrum ($CHC\ell_3$) $\nu_{max}$ $cm^{-1}$:
3600, 1350, 1180.

Nuclear Magnetic Resonance Spectrum ($CDC\ell_3$) $\delta$ ppm:
1.25 (3H, doublet, J = 6.5 Hz);
3.03 (3H, singlet);
4.30 (1H, quartet, J = 6.5 Hz);
5.2 - 5.7 (2H, multiplet);
6.54 (1H, doublet of doublets of doublets, J = 17, 11 & 3 Hz);
6.6 - 7.1 (2H, multiplet);
7.4 - 8.0 (1H, multiplet).

EXAMPLE 183

(2S, 3S)-2-(2,4-Difluorophenyl)-3-methyl-2-vinyloxirane

32 mg (0.98 mmole) of sodium hydride (as a 55% w/w suspension in mineral oil) were washed with dry hexane and suspended in 4 ml of dimethylformamide. A solution of 191 mg (0.65 mmole) of (3S, 4R)-3-(2,4-difluorophenyl)-4-(methanesulphonyloxy)-1-penten-3-ol (prepared as described in Example 182) in 2 ml of anhydrous tetrahydrofuran was then added dropwise, whilst stirring at 0°C, to the previously prepared suspension over a period of 5 minutes. The mixture was then warmed to room temperature and stirred overnight. At the end of this time, the reaction mixture was mixed with ice-water and extracted with hexane. The solvent was distilled under reduced pressure from the extract, to leave an oil, which was purified by column chromatography through 10 g of silica gel, eluted with a 4 : 1 by volume mixture of benzene and hexane, to give 86 mg (yield 70%) of the title compound as an oil.

Specific Rotation $[\alpha]_D^{25}$ : -61.1° (c = 0.71, $CHC\ell_3$).

Nuclear Magnetic Resonance Spectrum ($CDC\ell_3$) $\delta$ ppm:
1.40 (3H, doublet, J = 5.5 Hz);
3.17 (1H, quartet, J = 5.5 Hz);
5.15 (1H, doublet of triplets, J = 17 & 1.5 Hz);
5.30 (1H, doublet of triplets, J = 11 & 1.5 Hz);
6.03 (1H, doublet of doublets of doublets, J = 17, 11 & 1.5 Hz);
6.6 - 7.6 (3H, multiplet).

EXAMPLE 184

(2S, 3S)-2-(2,4-Difluorophenyl)-2,3-epoxybutanal

164 mg (0.76 mmole) of sodium metaperiodate and 1.5 mg of osmium tetraoxide were added to a solution of 50 mg (0.255 mmole) of (2S, 3S)-2-(2,4-difluorophenyl)-3-methyl-2-vinyloxirane (prepared as described in Example 183) in 3 ml of a 5 : 2 by volume mixture of methanol and water, and then the whole mixture was stirred at room temperature overnight. At the end of this time, the reaction mixture was diluted with ethyl acetate and washed with a saturated aqueous solution of sodium chloride. The solvent was then distilled off to give 50 mg of the title compound as an oil.

Nuclear Magnetic Resonance Spectrum ($CDC\ell_3$) $\delta$ ppm:
1.60 (3H, doublet, J = 5.5 Hz);
3.42 (1H, quartet, J = 5.5 Hz);
6.7 - 7.0 (2H, multiplet),
7.1 - 7.5 (1H, multiplet);

9.65 (1H, doublet, J = 2 Hz).

EXAMPLE 185

(2R, 3S)-2-(2,4-Difluorophenyl)-2,3-epoxy-1-butanol

10.5 mg (0.28 mmole) of sodium borohydride were added, whilst stirring and ice-cooling, to a solution of 50 mg (0.28 mmole) of (2S, 3S)-2-(2,4-difluorophenyl)-2,3-epoxybutanal (prepared as described in Example 184) in 1.6 ml of methanol, and then the mixture was stirred at the same temperature for 1 hour. The reaction mixture was then diluted with ethyl acetate and washed with a saturated aqueous solution of sodium chloride. The solvent was then distilled off under reduced pressure, to leave an oil, which was purified by column chromatography through 10 g of silica gel, eluted with a 4 : 1 by volume mixture of benzene and hexane, to give 38 mg (yield 75%) of the title compound as an oil.

Specific Rotation $[\alpha]_D^{25}$ : +18.2° (c = 1.14, $CHCl_3$).

Infrared Absorption Spectrum ($CHCl_3$) $\nu_{max}$ $cm^{-1}$:
3500, 1100.

Nuclear Magnetic Resonance Spectrum ($CDCl_3$) δ ppm:
1.48 (3H, doublet, J = 5.5 Hz);
2.02 (1H, singlet);
3.10 (1H, quartet, J = 5.5 Hz);
3.83 (1H, doublet, J = 12 Hz);
4.17 (1H, doublet of doublets, J = 12 & 0.5 Hz);
6.6 - 7.01(2H, multiplet);
7.2 - 7.6 (1H, multiplet);

EXAMPLE 186

(2R, 3S)-2-(2,4-Difluorophenyl)-3-methyl-2-(methanesulphonyloxymethyl)oxirane

42 mg (0.42 mmole) of triethylamine and 48 mg (0.42 mmole) of methanesulphonyl chloride were added, whilst stirring at 0°C, to a solution of 76 mg (0.38 mmole) of (2R, 3S)-2-(2,4-difluorophenyl)-2,3-epoxy-1-butanol (prepared as described in Example 185) in 1.5 ml of methylene chloride, and then the mixture was stirred for 30 minutes. At the end of this time, a dilute aqueous solution of sodium bicarbonate was added to the reaction mixture, which was then extracted with ethyl acetate. The extract was dried and the solvent was distilled off under reduced pressure, to give 103 mg (yield 98%) of the title compound as an oil.

Nuclear Magnetic Resonance Spectrum ($CDCl_3$) δ ppm:
1.50 (3H, doublet, J = 5.5 Hz);
2.91 (3H, singlet);
3.12 (1H, quartet, J = 5.5 Hz);
4.41 (1H, doublet, J = 11.5 Hz);
4.80 (1H, doublet, J = 11.5 Hz);
6.6 - 7.1 (2H, multiplet);
7.2 - 7.7 (1H, multiplet).

EXAMPLE 187

(2R, 3S)-2-(2,4-Difluorophenyl)-3-methyl-2-[(1H-1,2,4-triazol-1-yl)methyl]oxirane

47 mg (1.08 mmoles) of sodium hydride (as a 55% w/w suspension in mineral oil) were washed with dry hexane and suspended in 2 ml of dimethylformamide. 87 mg (1.26 mmoles) of triazole were then slowly added to the suspension, whilst stirring at 0°C. When the hydrogen gas ceased to evolve, a solution of 100 mg (0.36 mmoles) of (2R, 3S)-2-(2,4-difluorophenyl)-3-methyl-2-(methanesulphonyloxymethyl)oxirane (prepared as described in Example 186) in 2 ml of dimethylformamide was added. The mixture was then stirred at 85°C for 35 minutes, after which it was cooled. The solvent was then distilled off under reduced pressure, to give 69 mg of a crude product, which was recrystallized from a mixture of benzene and hexane to give the title compound melting at 88 - 89.5°C.

The specific rotation and the nuclear magnetic resonance spectrum of the compound thus prepared coincided with those of the compound prepared as described in Example 179.

EXAMPLE 188

(R)-(4-Chlorophenyl) 1-chloroethyl ketone

Following a procedure similar to that described in Example 94, the title compound was prepared as an oil, boiling point 115 - 120 °C at 4 mmHg (533 Pascals).

Specific Rotation $[\alpha]_D^{25}$ : -25.6 ° (c = 2.20, $CHCl_3$).

Infrared Absorption Spectrum ($CHCl_3$) $\nu_{max}$ $cm^{-1}$:
1695, 1595.

Nuclear Magnetic Resonance Spectrum ($CDCl_3$) $\delta$ ppm:
1.72 (3H, doublet, J = 6.5 Hz);
5.15 (1H, quartet, J = 6.5 Hz);
7.45 (2H, doublet);
7.98 (2H, doublet).

EXAMPLE 189

(R)-3-Chloro-2-(4-chlorophenyl)-1-butene

Following a procedure similar to that described in Example 95, the title compound was prepared as an oil.

Specific Rotation $[\alpha]_D^{25}$ : +21.2 ° (c = 2.00, $CHCl_3$).

Nuclear Magnetic Resonance Spectrum ($CDCl_3$) $\delta$ ppm:
1.66 (3H, doublet, J = 6.5 Hz);
4.94 (1H, quartet, J = 6.5 Hz);
5.34 (1H, singlet);
5.49 (1H, broad singlet);
7.34 (4H, singlet).

EXAMPLE 190

(2R, 3R)-3-Chloro-2-(4-chlorophenyl)-1,2-butanediol

Following a procedure similar to that described in Example 96, the title compound was prepared as an oil.

Specific Rotation $[\alpha]_D^{25}$ : +16.0 ° (c = 0.94, $CHCl_3$).

Infrared Absorption Spectrum ($CHCl_3$) $\nu_{max}$ $cm^{-1}$:
3570, 3400 (broad).

Nuclear Magnetic Resonance Spectrum ($CDCl_3$) $\delta$ ppm:
1.25 (3H, doublet, J = 6.5 Hz);
2.15 (1H, broad);
3.00 (1H, broad);
3.80 (1H, doublet, J = 12 Hz);
4.00 (1H, doublet, J = 12 Hz);
4.40 (1H, quartet, J = 6.5 Hz);
7.38 (4H, singlet).

EXAMPLE 191

(2R, 3R)-3-Chloro-2-(4-chlorophenyl)-1-(methanesulphonyloxy)-2-butanol

Following a procedure similar to that described in Example 97, the title compound was prepared as an oil.

Specific Rotation $[\alpha]_D^{25}$ : +13.4 ° (c = 1.08, $CHCl_3$).

Infrared Absorption Spectrum ($CHCl_3$) $\nu_{max}$ $cm^{-1}$:

3560.

Nuclear Magnetic Resonance Spectrum ($CDCl_3$) δ ppm:
1.27 (3H, doublet, J = 6.5 Hz);
2.88 (3H, singlet);
4.42 (1H, quartet, J = 6.5 Hz);
4.52 (2H, singlet);
7.38 (4H, singlet).

EXAMPLE 192

(2R, 3S)-2-(4-Chlorophenyl)-3-methyl-2-[(1H-1,2,4-triazol-1-yl)methyl]oxirane

Following a procedure similar to that described in Example 98, the title compound was prepared as an oil.

Specific Rotation $[\alpha]_D^{25}$ : -7.1° (c = 1.16, $CHCl_3$).

Nuclear Magnetic Resonance Spectrum ($CDCl_3$) δ ppm:
1.60 (3H, doublet, J = 5.5 Hz);
3.11 (1H, quartet, J = 5.5 Hz);
4.39 (1H, doublet, J = 15 Hz);
4.82 (1H, doublet, J = 15 Hz);
6.95 - 7.35 (4H, multiplet);
7.86 (2H, singlet);
7.95 (4H, singlet).

EXAMPLE 193

(2R, 3R)-3-Amino-2-(4-chlorophenyl)-1-(1H-1,2,4-triazol-1-yl)-2-butanol

Following a procedure similar to that described in Example 100, the title compound was prepared as an oil.

Nuclear Magnetic Resonance Spectrum ($CDCl_3$) δ ppm:
0.86 (3H, doublet, J = 6.5 Hz);
2.6 (3H, broad);
3.31 (1H, quartet, J = 6.5 Hz);
4.49 (2H, singlet);
7.25 (4H, singlet);
7.88 (2H, singlet).

EXAMPLE 194

(S)-(2,4-Difluorophenyl) 1-(2-tetrahydropyranyloxy)ethyl ketone

5.87 g (69.8 mmoles) of 2,3-dihydropyran and 945 mg of pyridinium p-toluenesulphonate were added, whilst stirring and ice-cooling, to a solution of 10.0 g (53.7 mmoles) of (S)-(2,4-difluorophenyl) 1-(hydroxy)-ethyl ketone (prepared as described in Example 171) in 100 ml of methylene chloride. The resulting mixture was warmed to room temperature and allowed to stand at that temperature for 3.5 hours. At the end of this time, the mixture was diluted with ethyl acetate and washed, in turn, with an aqueous solution of sodium bicarbonate and with a saturated aqueous solution of sodium chloride. After the mixture had been dried, the solvent was distilled off under reduced pressure, to leave an oil, which was purified by column chromatography through silica gel, eluted with a 4 : 1 by volume mixture of benzene and hexane, to give 13.0 g of the title compound as an oil.

Specific Rotation $[\alpha]_D^{25}$ : -56.3° (c = 1.11, $CHCl_3$).

The infrared absorption spectrum and the nuclear magnetic resonance spectrum of the compound thus prepared coincided with those of the compound prepared as described in Example 174.

EXAMPLE 195

(S)-3-(2,4-Difluorophenyl)-3-buten-2-ol

2.01 g (82.6 mmoles) of magnesium and 1.00 g (8.13 moles) of chloromethyltrimethylsilane were added to 20 ml of diethyl ether, and the mixture was heated under reflux under an atmosphere of nitrogen. A suspension of a small amount of magnesium (which had been activated with methyl iodide) in diethyl ether was added to start the reaction, and then a solution of 9.02 g (74.8 mmoles) of chloromethyltrimethylsilane in 130 ml of diethyl ether was added dropwise over a period of 30 minutes. The mixture was then heated under reflux for a further 30 minutes, after which it was ice-cooled. A solution of 11.2 g (41.4 mmoles) of (S)-(2,4-difluorophenyl) 1-(2-tetrahydropyranyloxy)ethyl ketone (prepared as described in Example 194) in 50 ml of diethyl ether was added dropwise over a period of 10 minutes. The mixture was then stirred for 1 hour whilst ice-cooling. At the end of this time, an aqueous solution of ammonium chloride was added to the mixture, which was then extracted with benzene. The extract was washed with a saturated aqueous solution of sodium chloride, and the solvent was distilled off under reduced pressure, to leave 15.0 g of an oil. The whole of the oil was dissolved in 100 ml of methanol, and 0.50 g of p-toluenesulphonic acid was added to the resulting solution. After 12 hours, benzene was added to the mixture, which was then washed, in turn, with a dilute aqueous solution of sodium bicarbonate and with a saturated aqueous solution of sodium chloride. The solvent was then distilled off to leave an oil, which was distilled in vacuo to give 7.0 g of the title compound, boiling at 49 - 51 °C at 1.5 mmHg (200 Pascals).

Specific Rotation $[\alpha]_D^{25}$ : +49° (c = 1.22, $CHCl_3$).

Infrared Absorption Spectrum ($CHCl_3$) $\nu_{max}$ $cm^{-1}$:
3600, 3430 (broad).

Nuclear Magnetic Resonance Spectrum ($CDCl_3$) δ ppm:
1.28 (3H, doublet, J = 6.5 Hz);
2.1 (1H, broad);
4.69 (1H, broad quartet, J = 6.5 Hz);
5.17 (1H, broad singlet);
5.54 (1H, triplet, J = 1.5 Hz);
6.6 - 7.6 (3H, multiplet).

EXAMPLE 196

(2S, 3R)-3-(2,4-Difluorophenyl)-3,4-epoxy-2-butanol

9.8 g (76 mmoles, 70% purity) of t-butyl hydroperoxide and 140 mg of vanadium(IV)oxy acetylacetate were added to a solution of 7.00 g (38 mmoles) of (S)-3-(2,4-difluorophenyl)-3-buten-2-ol (prepared as described in Example 195) in 35 ml of benzene, and then the mixture was stirred at room temperature overnight. At the end of this time, the mixture was washed with a saturated aqueous solution of sodium chloride and dried. The solvent was then distilled off under reduced pressure, to leave an oil, which was purified by column chromatography through silica gel, eluted with a 3 : 1 by volume mixture of hexane and ethyl acetate, to give 6.58 g of the title compound as an oil.

Specific Rotation $[\alpha]_D^{25}$ : -26.4° (c = 1.25, $CHCl_3$).

Infrared Absorption Spectrum ($CHCl_3$) $\nu_{max}$ $cm^{-1}$:
3560, 3500 (broad).

Nuclear Magnetic Resonance Spectrum ($CDCl_3$) δ ppm:
1.19 (3H, doublet of doublets, J = 6.5 & 1.5 Hz);
2.4 (1H, broad);
2.89 (1H, doublet, J = 5 Hz);
3.26 (1H, doublet, J = 5 Hz);
4.10 (1H, quartet, J = 6.5 Hz);
6.6 - 7.1 (2H, multiplet);
7.2 - 7.6 (1H, multiplet).

EXAMPLE 197

(2R, 3R)-(2,4-Difluorophenyl)-3,4-epoxy-2-butyl benzoate

144 mg (55 mmoles) of triphenylphosphine, 67 mg (55 mmoles) of benzoic acid and 96 mg (55 mmoles) of diethyl azodicarboxylate were added, in that order, whilst stirring and ice-cooling, to a solution of 55 mg (27.5 mmoles) of (2S, 3R)-(2,4-difluorophenyl)-3,4-epoxy-2-butanol (prepared as described in Example 196) in 1 ml of tetrahydrofuran. The mixture was then warmed to room temperature, stirred for 1 hour, and then mixed with water and benzene. The organic phase was separated and the solvent was distilled off to leave an oil, which was purified by preparative thin layer chromatography (developing solvent: a 7 : 1 by volume mixture of hexane and ethyl acetate) to give 71 mg of the title compound as an oil.

Specific Rotation $[\alpha]_D^{25}$ : -52.6° (c = 1.33, $CHCl_3$).

Infrared Absorption Spectrum ($CHCl_3$) $\nu_{max}$ $cm^{-1}$:
1716.

Nuclear Magnetic Resonance Spectrum ($CDCl_3$) δ ppm:
1.35 (3H, doublet of doublets, J = 6.5 & 1.5 Hz);
2.88 (1H, doublet, J = 4.5 Hz);
3.22 (1H, doublet, J = 4.5 Hz);
5.44 (1H, quartet, J = 6.5 Hz);
6.6 - 7.1 (2H, multiplet);
7.2 - 7.8 (4H, multiplet);
7.9 - 8.2 (2H, multiplet).

EXAMPLE 198

(2R, 3R)-3-(2,4-Difluorophenyl)-3,4-epoxy-2-butanol

71 mg of (2R, 3R)-(2,4-difluorophenyl)-3,4-epoxy-2-butyl benzoate (prepared as described in Example 197) were added to a solution of 1 mg of sodium in 1.5 ml of methanol, and then the mixture was allowed to stand at room temperature overnight. At the end of this time, water was added, and the mixture was then extracted with ethyl acetate. The extract was dried and the solvent was distilled off to leave an oil. The oil was purified by preparative thin layer chromatography (developing solvent: a 4 : 1 by volume mixture of hexane and ethyl acetate) to give 40 mg of the title compound as an oil.

Specific Rotation $[\alpha]_D^{25}$ : -54.9° (c = 1.45, $CHCl_3$).

Infrared Absorption Spectrum ($CHCl_3$) $\nu_{max}$ $cm^{-1}$:
3590, 3470 (broad).

Nuclear Magnetic Resonance Spectrum ($CDCl_3$) δ ppm:
1.15 (3H, doublet of doublets, J = 6.5 & 1.5 Hz);
2.2 (1H, broad);
2.79 (1H, doublet, J = 5 Hz);
3.28 (1H, doublet, J = 5 Hz);
4.15 (1H, broad);
6.6 - 7.1 (2H, multiplet);
7.2 - 7.7 (1H, multiplet).

EXAMPLE 199

(2R, 3R)-3-(2,4-Difluorophenyl)-3,4-epoxy-2-butyl methanesulphonate

30 mg (0.30 mmole) of triethylamine and 30 mg (0.26 mmole) of methanesulphonyl chloride were added, whilst stirring at -15°C, to a solution of 40 mg (0.20 mmole) of (2R, 3R)-3-(2,4-difluorophenyl)-3,4-epoxy-2-butanol (prepared as described in Example 198) in 1 ml of methylene chloride. After 10 minutes, water was added to the mixture, which was then extracted with ethyl acetate. The extract was dried, and the solvent was distilled off under reduced pressure, to give 53 mg of the title compound as an oil.

Nuclear Magnetic Resonance Spectrum ($CDCl_3$) δ ppm:
1.41 (3H, doublet of doublets, J = 6.5 & 1.5 Hz);
3.01 (1H, doublet, J = 5 Hz);
3.08 (3H, singlet);

3.20 (1H, doublet, J = 5 Hz);
4.75 (1H, quartet, J = 6.5 Hz);
6.6 - 7.1 (2H, multiplet);
7.2 - 7.7 (1H, multiplet).

EXAMPLE 200

(2R, 3S)-2-(2,4-Difluorophenyl)-3-methyl-2-[(1H-1,2,4-triazol-1-yl)methyl]oxirane

25 mg (0.57 mmole) of sodium hydride (as a 55% w/w suspension in mineral oil) were washed with dry hexane and suspended in 1 ml of dimethylformamide. 45 mg (0.65 mmole) of triazole were then slowly added to the suspension, whilst stirring at 0°C. When the hydrogen gas ceased to evolve, a solution of 53 mg (0.19 mmole) of (2R, 3R)-3-(2,4-difluorophenyl)-3,4-epoxy-2-butyl methanesulphonate (prepared as described in Example 199) in 0.5 ml of dimethylformamide was added, and the mixture was stirred at 60°C for 3 hours. After the reaction mixture had been cooled, it was diluted with ethyl acetate and washed with a saturated aqueous solution of sodium chloride. The solvent was then distilled off under reduced pressure, to leave an oil, which was purified by preparative thin layer chromatography on silica gel (developing solvent: a 3 : 2 by volume mixture of ethyl acetate and hexane), to give 37 mg of the title compound. The title compound was further purified by recrystallization from a mixture of benzene and hexane to give the title compound as crystals melting at 88 - 89.5°C.

The specific rotation and the nuclear magnetic resonance spectrum of this compound coincided with those of the compound prepared as described in Example 179.

EXAMPLE 201

3-(4-Chlorobenzoylamino)-2-(2,4-dichlorophenyl)-5-methyl-1-(1H-1,2,4-triazol-1-yl)-2-hexanol

Following a procedure similar to that described in Example 1, the title compound (stereochemistry unknown), melting at 185 - 190°C, was prepared.

EXAMPLE 202

(2R*, 3R*)-2-(2,4-Difluorophenyl)-3-tetrahydropyranyloxy)-1-(1H-1,2,4-triazol-1-yl)-2-pentanol

Following a procedure similar to that described in the Abstract Papers of the 8th Symposium on Medicinal Chemistry, Osaka (1986), page 9], but using α-bromobutyryl bromide, the title compound was obtained as an oil.

$n_D^{23}$ = 1.5035.

Mass Spectrum (m/z): 368, 294, 266, 224.

Nuclear Magnetic Resonance Spectrum ($CDCl_3$) δ ppm:
0.81 (3H, triplet, J = 7.2 Hz);
1.10 - 2.00 (8H, multiplet);
3.15 - 3.78 (1H, multiplet);
3.79 - 4.28 (3H, multiplet);
4.32 - 4.70 (2H, multiplet);
4.78 (1H, singlet);
6.43 - 6.92 (2H, multiplet);
7.15 - 7.47 (1H, multiplet);
7.60 (0.5H, singlet);
7.65 (0.5H, singlet);
7.83 (0.5H, singlet);
7.92 (0.5H, singlet).

EXAMPLES 203 TO 206

The following compounds were also prepared by essentially the same procedures as in the foregoing Examples. The compounds are characterised by the biological properties shown in the subsequent Experiments. The compounds of Table 10 have the formula (I-1). In Table 10, as well as following Tables,

the abbreviations used are as defined above.

Table 10

| Ex. No. | $X_m$ | $R^1$ | $R^2$ | $Y_n$ | Ar | Stereochem. |
|---|---|---|---|---|---|---|
| 203 | - | Me | 6-Cℓ-3-Pyr | -NHCO- | 2,4-diFPh | (2R*, 3R*) |
| 204 | - | Et | Me | $-OSO_2-$ | 2,4-diFPh | (2R*, 3R*) |
| 205 | - | Me | 4-CℓPh | -NHCO- | 4-CℓPh | (2R*, 3R*) |
| 206 | - | Me | 4-CℓPh | -NHCO- | 4-CℓPh | (2R*, 3S*) |

In the following Preparations are described the preparation of a number of pharmaceutical formulations in accordance with the invention.

PREPARATION 1

Capsules

A well blended mixture of 100 mg of powdery active compound, 150 mg of lactose, 50 mg of cellulose and 6 mg of magnesium stearate is encapsulated in a hard gelatin capsule which is washed and dried. The active compounds referred to in this Preparation are those identified by the compound Nos. shown below.

PREPARATION 2

Tablets

A mixture of 100 mg of the active compound, 0.2 mg of collodial silica, 5 mg of magnesium stearate, 275 mg of microcrystalline cellulose, 11 mg of starch and 98.9 mg of lactose is compounded and tableted.

PREPARATION 3

Injectable solutions

1.5 parts by weight of the active compound are mixed with 10 parts by volume of propylene glycol. The mixture is made up with sterilized water and then sterilized.

PREPARATION 4

Creams

5 g parts of a base cream is prepared from 40% white petrolatum, 10% lanolin, 5% Span 20, 0.3% Tween 20 and 41.7% water, with which 100 mg of a powdery active compound are admixed.

PREPARATION 5

Coating liquids

1 part by weight of the active compound is mixed with 99 parts by weight of polyethylene glycol 300.

PREPARATION 6

Ointments

2 parts of the active compound, 40 parts of polyethylene glycol 400 and 58 parts by weight of polyethylene glycol 1500 are mixed and solubilized, with heating, and then cooled.

The active compounds referred to in the above Preparations are the following Compound Nos.:
1-20, 1-24, 1-26, 1-32, 1-68, 1-89, 1-117, 1-124 and 1-127, as identified in foregoing Table 1.

The compounds of the present invention have valuable anti-fungal activity, which may be used both for the treatment and protection of plants and for the treatment and protection of animals, including human beings, as is illustrated by the following Experiments:

EXPERIMENT 1

Curative activity against rice blast

Rice seedlings (variety Sachikaze) at the 4 - 5 leaf stage were inoculated with the fungus, Pyricularia oryzae, by spraying them with a spore suspension of the fungus and maintaining the seedlings in a moist chamber at 20 - 22 °C. After 24 hours, the rice seedlings were sprayed with an aqueous suspension of the test compound at a concentration of 10 ppm (30 ml/3 pots) and were then kept in the moist chamber for a further 6 days. The disease controlling index was given on the basis of the number of lesions formed on the upper two leaves. The results are shown in Table 11.

Table 11

Curative Activity against Rice Blast

The compounds tested have the formula (I-1), in which Ar represents the group shown at the beginning of each part of the Table and $R^1$, $R^2$, X and Y are as defined within each corresponding part of the Table. In this Table, as with Tables 12 to 19, the disease controlling index is as defined hereafter following Table 19.

Table 11A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 10 | - | Me | 4-CℓPh | -NHCO- | (2R, 3R) (oxalate) | 4 |
| 12 | - | Me | 4-TfmPh | -NHCO- | (2R*, 3R*) | 5 |
| 13 | - | Me | 4-TfmPh | -NHCO- | (2R, 3R) | 5 |
| 2 | - | Me | - | $-N_3$ | (2R*, 3R*) | 4 |
| 90 | $-CH_2-$ | Me | - | $-N_3$ | (2R*, 3S*) | 5 |
| 154 | $-CH_2-$ | Me | - | $-N_3$ | (2R*, 3R*) | 4 |
| Compound X | | | | | | 0 |

Table 11B

Ar=2,4-diFPh

| Ex. No. | $X_m$ | $R^1$ | $R^2$ | $Y_n$ | Stereo-chem. | Index |
|---|---|---|---|---|---|---|
| 16 | - | Me | 4-CℓPh | -NHCO- | (2R*, 3R*) | 5 |
| 155 | - | Me | 2,4-diFPh | -NHCO- | (2R*, 3R*) | 5 |
| 107 | - | Me | 2,5-diFPh | -NHCO- | (2R*, 3R*) | 4 |
| 17 | - | Me | 4-TfmPh | -NHCO- | (2R*, 3R*) | 5 |
| 65 | - | Me | 2-F-4-TfmPh | -NHCO- | (2R*, 3R*) | 5 |
| 159 | - | Me | 4-TfmOPh | -NHCO- | (2R*, 3R*) | 5 |
| 203 | - | Me | 6-Cℓ-3-Pyr | -NHCO- | (2R*, 3R*) | 4 |
| 117 | - | Et | 4-CℓPh | -NHCO- | (2R*, 3R*) | 5 |
| 111 | - | Me | 4-tBuPh | -NHCO- | (2R*, 3R*) | 5 |
| 124 | - | Me | 4-MeOPh | -NHCO- | (2R*, 3R*) | 5 |
| 125 | - | Me | 4-MePh | -NHCO- | (2R*, 3R*) | 5 |
| 130 | - | Me | 4-BzOPh | -NHCO- | (2R*, 3R*) | 4 |
| 135 | - | Me | 4-CℓPhtm | - | (2R*, 3R*) (nitrate) | 4 |
| 142 | - | Me | - | $-N_3$ | (2R*, 3R*) | 5 |
| 99 | - | Me | - | $-N_3$ | (2R, 3R) | 5 |
| 143 | - | Me | - | $-N_3$ | (2R*, 3S*) | 4 |
| 149 | - | Et | - | $-N_3$ | (2R*, 3R*) | 5 |
| 150 | - | Et | - | $-N_3$ | (2R*, 3S*) | 5 |

Table 11B (cont)

| Ex. No. | $X_m$ | $R^1$ | $R^2$ | $Y_n$ | Stereo-chem. | Index |
|---|---|---|---|---|---|---|
| 83 (C-4 isomer 1) | -CH(Me)- | Me | - | $-N_3$ | (2R*, 3S*) | 5 |
| 83 (oxalate, C-4 isomer 2) | -CH(Me)- | Me | - | $-N_3$ | (2R*, 3S*) | 5 |
| 153 | $-CH_2-$ | Me | - | $-N_3$ | (2R*, 3S*) | 5 |
| 81 (oxalate) | $-(CH_2)_2-$ | Me | - | $-N_3$ | (2R*, 3S*) | 5 |
| 202 | - | Et | 2-Thp | -O- | (2R*, 3R*) | 4 |

Table 11C

| Ar = 2-F-4-CℓPh | | | | | | |
|---|---|---|---|---|---|---|
| Ex. No. | $X_m$ | $R^1$ | $R^2$ | $Y_n$ | Stereo-chem. | Index |
| 118 | - | Me | 4-TfmPh | -NHCO- | (2R*, 3R*) | 5 |
| 163 | - | Me | 4-CℓPh | -NHCO- | (2R*, 3R*) | 5 |
| 119 | - | Me | 2-F-4-TfmPh | -NHCO- | (2R*, 3R*) | 5 |
| 145 | - | Me | - | $-N_3$ | (2R*, 3R*) | 5 |

EXPERIMENT 2

Preventive activity against sheath blight of rice plants

Rice seedlings (variety Nihonbare) at the 4 - 5 leaf stage were sprayed with an aqueous suspension of the test compound at a concentration of 100 ppm (30 ml/3 pots). The seedlings were then kept for 24 hours at room temperature, after which they were inoculated with the fungus, Rhizoctonia solani, by placing 4 - 5 oat grains on which the fungus had previously been cultured around the base of each seedling. The seedlings were then kept in a moist chamber for 5 days at 25 - 27 °C. The disease controlling index was given on the basis of the height of the lesions formed on the rice seedlings. The results are shown in Table 12.

Table 12

Preventive Activity against Sheath Blight of Rice Plants

Except in Table 12G, the compounds tested have the formula (I-1), in which Ar represents the group shown at the beginning of each part of the Table and $R^1$, $R^2$, X and Y are as defined in the Table.

Table 12A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 5 | - | Me | 4-CℓPh | -NHCO- | (2R*, 3R*) (oxalate) | 5 |
| 10 | - | Me | 4-CℓPh | -NHCO- | (2R, 3R) (oxalate) | 5 |
| 11 | - | Me | 2,4-diCℓPh | -NHCO- | (2R*, 3R*) | 4 |
| 120 | - | Me | 3,4-diCℓPh | -NHCO- | (2R*, 3R*) | 4 |
| 4 | - | Me | 4-FPh | -NHCO- | (2R*, 3R*) | 4 |
| 12 | - | Me | 4-TfmPh | -NHCO- | (2R*, 3R*) | 5 |
| 13 | - | Me | 4-TfmPh | -NHCO- | (2R, 3R) | 4 |
| 121 | - | Me | tBu | -NHCO- | (2R*, 3R*) (oxalate) | 5 |
| 90 | $-CH_2-$ | Me | - | $-N_3$ | (2R*, 3S*) | 5 |

Table 12B

Ar=2,4-diFPh

| Ex. No. | $X_m$ | $R^1$ | $R^2$ | $Y_n$ | Stereo-chem. | Index |
|---|---|---|---|---|---|---|
| 16 | - | Me | 4-CℓPh | -NHCO- | (2R*, 3R*) | 5 |
| 105 | - | Me | 3-FPh | -NHCO- | (2R*, 3R*) | 4 |
| 155 | - | Me | 2,4-diFPh | -NHCO- | (2R*, 3R*) | 5 |
| 107 | - | Me | 2,5-diFPh | -NHCO- | (2R*, 3R*) | 4 |
| 108 | - | Me | 3,5-diFPh | -NHCO- | (2R*, 3R*) | 4 |
| 17 | - | Me | 4-TfmPh | -NHCO- | (2R*, 3R*) | 4 |
| 65 | - | Me | 2-F-4-TfmPh | -NHCO- | (2R*, 3R*) | 5 |
| 67 | - | Me | 4-CNPh | -NHCO- | (2R*, 3R*) | 4 |
| 159 | - | Me | 4-TfmOPh | -NHCO- | (2R*, 3R*) | 5 |
| 111 | - | Me | tBu | -NHCO- | (2R*, 3R*) | 5 |
| 203 | - | Me | 6-Cℓ-3-Pyr | -NHCO- | (2R*, 3R*) | 5 |
| 117 | - | Et | 4-CℓPh | -NHCO- | (2R*, 3R*) | 5 |
| 125 | - | Me | 4-MePh | -NHCO- | (2R*, 3R*) | 5 |
| 130 | - | Me | 4-BzOPh | -NHCO- | (2R*, 3R*) | 4 |
| 168 | - | Me | 3-TfmPh | -N(Me)CO- | (2R*, 3R*) | 5 |
| 59 | - | Me | 4-TfmPh | -N(Me)CO- | (2R*, 3R*) | 5 |
| 60 | - | Me | 2-F-4-TfmPh | -N(Me)CO- | (2R*, 3R*) | 5 |
| 61 | - | Me | 4-CℓPh | -N(Me)COCH=CH- | (2R*, 3R*) | 5 |
| 142 | - | Me | - | $-N_3$ | (2R*, 3R*) | 5 |
| 144 | - | Me | - | $-N_3$ | (2R, 3R) | 5 |
| 149 | - | Et | - | $-N_3$ | (2R*, 3R*) | 5 |
| 150 | - | Et | - | $-N_3$ | (2R*, 3S*) | 5 |

Table 12B (cont)

| Ex. No. | $X_m$ | $R^1$ | $R^2$ | $Y_n$ | Stereo-chem. | Index |
|---|---|---|---|---|---|---|
| 83 (C-4 isomer 1) | -CH(Me)- | Me | - | $-N_3$ | (2R*, 3S*) | 5 |
| 83 (oxalate, C-4 isomer 2) | -CH(Me)- | Me | - | $-N_3$ | (2R*, 3S*) | 5 |
| 153 | $-CH_2-$ | Me | - | $-N_3$ | (2R*, 3S*) | 5 |
| 81 | $-(CH_2)_2-$ | Me | - | $-N_3$ | (2R*, 3S*) (oxalate) | 5 |
| 204 | - | Et | Me | $-OSO_2-$ | (2R*, 3R*) | 5 |
| 202 | - | Et | 2-Thp | -O- | (2R*, 3R*) | 4 |

Table 12C

| Ar = 2-F-4-CℓPh | | | | | | |
|---|---|---|---|---|---|---|
| Ex. No. | $X_m$ | $R^1$ | $R^2$ | $Y_n$ | Stereochem. | Index |
| 118 | - | Me | 4-TfmPh | -NHCO- | (2R*, 3R*) | 5 |
| 163 | - | Me | 4-CℓPh | -NHCO- | (2R*, 3R*) | 5 |
| 119 | - | Me | 2-F-4-TfmPh | -NHCO- | (2R*, 3R*) | 5 |

Table 12D

| Ar = 4-CℓPh | | | | | | |
|---|---|---|---|---|---|---|
| Ex. No. | $X_m$ | $R^1$ | $R^2$ | $Y_n$ | Stereochem. | Index |
| 205 | - | Me | 4-CℓPh | -NHCO- | (2R*, 3R*) | 5 |
| 70 | - | Me | 2-F-4-TfmPh | -NHCO- | (2R*, 3R*) | 5 |
| 141 | - | Me | - | $-N_3$ | (2R*, 3S*) | 5 |
| (oxalate) | | | | | | |

Table 12E

| Ar = 4-FPh | | | | | | |
|---|---|---|---|---|---|---|
| Ex. No. | $X_m$ | $R^1$ | $R^2$ | $Y_n$ | Stereochem. | Index |
| 122 | - | Me | 4-CℓPh | -NHCO- | (2R*, 3R*) | 5 |
| 147 | - | Me | - | $-N_3$ | (2R*, 2R*) | 5 |

Table 12F

| Ar = 4-BrPh | | | | | | |
|---|---|---|---|---|---|---|
| Ex. No. | $X_m$ | $R^1$ | $R^2$ | $Y_n$ | Stereochem. | Index |
| 148 | - | Me | - | $N_3$ | (2R*, 3R*) | 5 |

Table 12G

| The compound tested has the formula (I-2), in which Ar represents the 4-CℓPh group and $R^1$, $R^2$, p and q are as defined in the Table. | | | | | | |
|---|---|---|---|---|---|---|
| Ex. No. | $R^1$ | $R^2$ | p | q | Stereochem. | Index |
| 76 | Me | 2-F-4-TfmPh | 1 | 0 | (4R*, 5R*) | 4 |
| (oxalate) | | | | | | |

EXPERIMENT 3

Curative activity against sheath blight of rice plants

Rice seedlings (variety Nihonbare) at the 4 - 5 leaf stage were inoculated with the fungus, Rhizoctonia solani, by placing 4 - 5 oat grains on which the fungus had previously been cultured around the base of each rice seedling and keeping them in a moist chamber at 25 - 27°C. After 24 hours, the rice seedlings were sprayed with an aqueous suspension of the test compound at a concentration of 10 ppm (30 ml/3 pots) and they then continued to be kept in the moist chamber for a further 5 days. The disease controlling index was given on the basis of the height of the lesions formed on the rice seedlings. The results are shown in Table 13.

Table 13

Curative Activity against Sheath Blight of Rice Plants

The compounds tested have the formula (I-1), in which Ar represents the group shown at the beginning of each part of the Table and $R^1$, $R^2$, X and Y are as defined in the Table.

Table 13A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 10 | - | Me | 4-CℓPh | -NHCO- | (2R, 3R) (oxalate) | 5 |
| 12 | - | Me | 4-TfmPh | -NHCO- | (2R*, 3R*) | 5 |
| 2 | - | Me | - | $-N_3$ | (2R*, 3R*) | 5 |
| 154 | $-CH_2-$ | Me | - | $N_3$ | (2R*, 3R*) | 5 |
| Compound X | | | | | | 0 |

Table 13B

Ar=2,4-diFPh

| Ex. No. | $X_m$ | $R^1$ | $R^2$ | $Y_n$ | Stereo-chem. | Index |
|---|---|---|---|---|---|---|
| 16 | - | Me | 4-CℓPh | -NHCO- | (2R*, 3R*) | 5 |
| 155 | - | Me | 2,4-diFPh | -NHCO- | (2R*, 3R*) | 4 |
| 107 | - | Me | 2,5-diFPh | -NHCO- | (2R*, 3R*) | 4 |
| 17 | - | Me | 4-TfmPh | -NHCO- | (2R*, 3R*) | 4 |
| 65 | - | Me | 2-F-4-TfmPh | -NHCO- | (2R*, 3R*) | 5 |
| 67 | - | Me | 4-CNPh | -NHCO- | (2R*, 3R*) | 4 |
| 159 | - | Me | 4-TfmOPh | -NHCO- | (2R*, 3R*) | 5 |
| 117 | - | Et | 4-CℓPh | -NHCO- | (2R*, 3R*) | 5 |
| 130 | - | Me | 4-BzOPh | -NHCO- | (2R*, 3R*) | 5 |
| 168 | - | Me | 3-TfmPh | -N(Me)CO- | (2R*, 3R*) | 4 |
| 59 | - | Me | 4-TfmPh | -N(Me)CO- | (2R*, 3R*) | 5 |
| 61 | - | Me | 4-CℓPh | -N(Me)COCH=CH- | (2R*, 3R*) | 5 |
| 142 | - | Me | - | $-N_3$ | (2R*, 3R*) | 5 |
| 99 | - | Me | - | $-N_3$ | (2R, 3R) | 5 |
| 143 | - | Me | - | $-N_3$ | (2R*, 3S*) | 4 |
| 149 | - | Et | - | $-N_3$ | (2R*, 3R*) | 5 |
| 150 | - | Et | - | $-N_3$ | (2R*, 3S*) | 5 |
| 83 | -CH(Me)- | Me | - | $-N_3$ | (2R*, 3S*) | 5 |
| (C-4 isomer 1) | | | | | | |
| | -CH(Me)- | Me | - | $-N_3$ | (2R*, 3S*) | 5 |
| (oxalate, C-4 isomer 2) | | | | | | |
| 153 | $-CH_2-$ | Me | - | $-N_3$ | (2R*, 3S*) | 5 |

Table 13B (cont)

| Ex. No. | $X_m$ | $R^1$ | $R^2$ | $Y_n$ | Stereo- chem. | Index |
|---|---|---|---|---|---|---|
| 81 | $-(CH_2)_2-$ | Me | - | $-N_3$ | (2R*, 3S*) (oxalate) | 5 |
| 204 | - | Et | Me | $-OSO_2-$ | (2R*, 3R*) | 5 |
| 202 | - | Et | 2-Thp | -O- | (2R*, 3R*) | 4 |

Table 13C

| Ar = 2-F-4-CℓPh | | | | | | |
|---|---|---|---|---|---|---|
| Ex. No. | $X_m$ | $R^1$ | $R^2$ | $Y_n$ | Stereochem. | Index |
| 118 | - | Me | 4-TfmPh | -NHCO- | (2R*, 3R*) | 4 |
| 119 | - | Me | 2-F-4-TfmPh | -NHCO- | (2R*, 3R*) | 4 |
| 145 | - | Me | - | $-N_3$ | (2R*, 3R*) | 5 |

Table 13D

| Ar = 4-CℓPh | | | | | | |
|---|---|---|---|---|---|---|
| Ex. No. | $X_m$ | $R^1$ | $R^2$ | $Y_n$ | Stereochem. | Index |
| 205 | - | Me | 4-CℓPh | -NHCO- | (2R*, 3R*) | 4 |

Table 13E

| Ar = 4-FPh | | | | | | |
|---|---|---|---|---|---|---|
| Ex. No. | $X_m$ | $R^1$ | $R^2$ | $Y_n$ | Stereochem. | Index |
| 122 | - | Me | 4-CℓPh | -NHCO- | (2R*, 3R*) | 4 |

Table 13F

| Ar = 4-BrPh | | | | | | |
|---|---|---|---|---|---|---|
| Ex. No. | $X_m$ | $R^1$ | $R^2$ | $Y_n$ | Stereochem. | Index |
| 148 | - | Me | - | $-N_3$ | (2R*, 3R*) | 4 |

EXPERIMENT 4

Preventive activity against sheath blight of rice plants by submerged application

Rice seedlings (variety Nihonbare) at the 4 - 5 leaf stage grown in pots were flooded to a depth of 1 cm with water. The test compound was then applied to the water in the pots in an amount corresponding to 100 g per 10 ares. After keeping the seedlings in a greenhouse for 7 days, the water was drained from the pots and the seedlings were inoculated with the fungus, Rhizoctonia solani, by placing 4 - 5 oat grains on which the fungus had previously been cultured around the base of each seedling. The seedlings were then kept in a moist chamber for 5 days at 25 - 27 °C. The disease controlling index was given on the basis of the height of the lesions formed on the rice seedlings. The results are shown in Table 14.

Table 14

Preventive Activity against Sheath Blight of Rice Plants By Submerged Application

Except for Table 14F, the compounds tested have the formula (I-1), in which Ar represents the group shown at the beginning of each part of the Table and $R^1$, $R^2$, X and Y are as defined in the Table.

Table 14A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 5 | - | Me | 4-CℓPh | -NHCO- | (2R*, 3R*) (oxalate) | 4 |
| 2 | - | Me | - | $-N_3$ | (2R*, 3R*) | 5 |
| 90 | $-CH_2-$ | Me | - | $-N_3$ | (2R*, 3S*) | 5 |
| Compound X | | | | | | 0 |

Table 14B

| Ar = 2,4-diFPh | | | | | | |
|---|---|---|---|---|---|---|
| Ex. No. | $X_m$ | $R^1$ | $R^2$ | $Y_n$ | Stereochem. | Index |
| 117 | - | Et | 4-CℓPh | -NHCO- | (2R*, 3R*) | 4 |
| 59 | - | Me | 4-TfmPh | -N(Me)CO- | (2R*, 3R*) | 4 |
| 142 | - | Me | - | $-N_3$ | (2R*, 3R*) | 5 |
| 99 | - | Me | - | $-N_3$ | (2R, 3R) | 4 |
| 143 | - | Me | - | $-N_3$ | (2R*, 3S*) | 4 |
| 149 | - | Et | - | $-N_3$ | (2R*, 3R*) | 5 |
| 150 | - | Et | - | $-N_3$ | (2R*, 3S*) | 5 |
| 83 | -CH(Me)- | Me | - | $-N_3$ | (2R*, 3S*) | 5 |
| (C-4 isomer 1) | | | | | | |
| 83 | -CH(Me)- | Me | - | $-N_3$ | (2R*, 3S*) | 4 |
| (oxalate, C-4 isomer 2) | | | | | | |
| 153 | $-CH_2-$ | Me | - | $-N_3$ | (2R*, 3S*) | 4 |
| 81 | $-(CH_2)_2-$ | Me | - | $-N_3$ | (2R*, 3S*) (oxalate) | 4 |

Table 14C

| Ar = 2-F-4-CℓPh | | | | | | |
|---|---|---|---|---|---|---|
| Ex. No. | $X_m$ | $R^1$ | $R^2$ | $Y_n$ | Stereochem. | Index |
| 145 | - | Me | - | $-N_3$ | (2R*, 3R*) | 5 |

Table 14D

| Ar = 4-CℓPh | | | | | | |
|---|---|---|---|---|---|---|
| Ex. No. | $X_m$ | $R^1$ | $R^2$ | $Y_n$ | Stereochem. | Index |
| 141 | - | Me | - | $-N_3$ | (2R*, 3S*) | 5 |
| (oxalate) | | | | | | |

Table 14E

| Ar = 4-FPh | | | | | | |
|---|---|---|---|---|---|---|
| Ex. No. | $X_m$ | $R^1$ | $R^2$ | $Y_n$ | Stereochem. | Index |
| 147 | - | Me | - | $-N_3$ | (2R*, 3R*) | 5 |

Table 14F

| The compound tested has the formula (I-2), in which Ar represents the 4-CℓPh group and $R^1$, $R^2$, p and q are as defined in the Table. | | | | | | |
|---|---|---|---|---|---|---|
| Ex. No. | $R^1$ | $R^2$ | p | q | Stereochem. | Index |
| 76 | Me | 2-P-4-TfmPh | 1 | 0 | (4R*, 5R*) (oxalate) | 4 |

## EXPERIMENT 5

### Activity against bakanae disease of rice plants by seed soaking

Ten grams of rice seeds (variety Tanginbozu), infected with Gibberella Fujikuroi, were immersed in 20 ml of an aqueous suspension of the test compound for 3 days. At the end of this time, the treated seeds were densely sown in soil in pots, and the pots were placed in a greenhouse at 20 - 30 °C for 3 weeks. The disease controlling index was given on the basis of the number of diseased seedlings. The results are shown in Table 15.

Table 15

Activity against Bakanae Disease of Rice Plants By Seed Soaking

The compounds tested have the formula (I-1), in which Ar represents the group shown at the beginning of each part of the Table and $R^1$, $R^2$, X and Y are as defined in the Table.

Table 15A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 10 | - | Me | 4-CℓPh | -NHCO- | (2R, 3R) (oxalate) | 5 |
| 11 | - | Me | 2,4-diCℓPh | -NHCO- | (2R*, 3R*) | 4 |
| 22 | - | Me | 2,4-diFPh | -NHCO- | (2R*, 3R*) | 4 |
| 12 | - | Me | 4-TfmPh | -NHCO- | (2R*, 3R*) | 4 |
| 13 | - | Me | 4-TfmPh | -NHCO- | (2R, 3R) | 5 |
| 121 | - | Me | tBu | -NHCO- | (2R*, 3R*) (oxalate) | 4 |
| 142 | - | Me | - | $-N_3$ | (2R*, 3R*) | 5 |
| 90 | $-CH_2-$ | Me | - | $-N_3$ | (2R*, 3S*) | 5 |
| 154 | $-CH_2-$ | Me | - | $-N_3$ | (2R*, 3R*) | 4 |

Table 15B

| Ar = 2,4-diFPh | | | | | | |
|---|---|---|---|---|---|---|
| Ex. No. | $X_m$ | $R^1$ | $R^2$ | $Y_n$ | Stereochem. | Index |
| 16 | - | Me | 4-CℓPh | -NHCO- | (2R*, 3R*) | 4 |
| 15 | - | Me | 4-FPh | -NHCO- | (2R*, 3R*) | 4 |
| 17 | - | Me | 4-TfmPh | -NHCO- | (2R*, 3R*) | 4 |
| 65 | - | Me | 2-F-4-TfmPh | -NHCO- | (2R*, 3R*) | 4 |
| 111 | - | Me | tBu | -NHCO- | (2R*, 3R*) | 4 |
| 112 | - | Me | $-CHC\ell_2$ | -NHCO- | (2R*, 3R*) (oxalate) | 4 |
| 114 | - | Me | 2-Thi | -NHCO- | (2R*, 3R*) | 4 |
| 203 | - | Me | 6-Cℓ-3-Pyr | -NHCO- | (2R*, 3R*) | 4 |
| 168 | - | Me | 3-TfmPh | -N(Me)CO- | (2R*, 3R*) | 5 |
| 59 | - | Me | 4-TfmPh | -N(Me)CO- | (2R*, 3R*) | 4 |
| 60 | - | Me | 2-F-4-TfmPh | -N(Me)CO- | (2R*, 3R*) | 4 |
| 135 | - | Me | 4-Phtm | - | (2R*, 3R*) (oxalate) | 4 |
| 139 | - | Me | 4-CℓPh | -OCO- | (2R*, 3R*) | 4 |
| 142 | - | Me | - | $-N_3$ | (2R*, 3R*) | 5 |
| 99 | - | Me | - | $-N_3$ | (2R, 3R) | 5 |
| 143 | - | Me | - | $-N_3$ | (2R*, 3S*) | 5 |
| 149 | - | Et | - | $-N_3$ | (2R*, 3R*) | 5 |
| 150 | - | Et | - | $-N_3$ | (2R*, 3S*) | 5 |
| 83 | -CH(Me)- | Me | - | $-N_3$ | (2R*, 3S*) | 5 |
| (C-4 isomer 1) | | | | | | |
| 83 | -CH(Me)- | Me | - | $-N_3$ | (2R*, 3S*) | 5 |
| (oxalate, C-4 isomer 2) | | | | | | |
| 153 | $-CH_2-$ | Me | - | $-N_3$ | (2R*, 3S*) | 5 |
| 81 | $-(CH_2)_2-$ | Me | - | $-N_3$ | (2R*, 3S*) (oxalate) | 5 |

Table 15C

| Ar = 4-CℓPh | | | | | | |
|---|---|---|---|---|---|---|
| Ex. No. | $X_m$ | $R^1$ | $R^2$ | $Y_n$ | Stereochem. | Index |
| 63 | - | Me | - | $-N_3$ | (2R*, 3R*) (oxalate) | 5 |
| 141 | - | Me | - | $-N_3$ | (2R*, 3S*) (oxalate) | 5 |

Table 15D

| Ar = 4-BrPh | | | | | | |
|---|---|---|---|---|---|---|
| Ex. No. | $X_m$ | $R^1$ | $R^2$ | $Y_n$ | Stereochem. | Index |
| 148 | - | Me | - | $-N_3$ | (2R*, 3R*) | 5 |

EXPERIMENT 6

Curative activity against leaf rust of wheat

Wheat seedlings (variety Norin No 61) at the first leaf stage were inoculated with the fungus, Puccinia recondita, by spraying them with a spore suspension of the fungus. They were then kept in a moist chamber for 24 hours at 20 - 22°C, after which they were moved to a greenhouse at 15 - 20°C. After 2 days, the seedlings were sprayed with an aqueous suspension of the test compound at a concentration of 3 ppm (30 ml/3 pots). The seedlings were then continuously kept in the greenhouse for 10 days. The disease controlling index was given on the basis of diseased area on the first leaf. The results are shown in Table 16.

Table 16

Curative Activity against Leaf Rust of Wheat

Except for Table 16H, the compounds tested have the formula (I-1), in which Ar represents the group shown at the beginning of each part of the Table and $R^1$, $R^2$, X and Y are as defined in the Table.

Table 16A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 5 | - | Me | 4-CℓPh | -NHCO- | (2R*, 3R*) | 5 |
| 5 | - | Me | 2,4-diFPh | -NHCO- | (2R*, 3R*) (oxalate) | 5 |
| 4 | - | Me | 4-FPh | -NHCO- | (2R*, 3R*) | 5 |
| 22 | - | Me | 2,4-diFPh | -NHCO- | (2R*, 3R*) | 5 |
| 12 | - | Me | 4-TfmPh | -NHCO- | (2R*, 3R*) | 5 |
| 13 | - | Me | 4-TfmPh | -NHCO- | (2R, 3R) | 5 |
| 121 | - | Me | tBu | -NHCO- | (2R*, 3R*) (oxalate) | 5 |
| 2 | - | Me | - | $-N_3$ | (2R*, 3R*) | 5 |

Table 16B

Ar = 2,4-diFPh

| Ex. No. | $X_m$ | $R^1$ | $R^2$ | $Y_n$ | Stereo-chem. | Index |
|---|---|---|---|---|---|---|
| 16 | - | Me | 4-CℓPh | -NHCO- | (2R*, 3R*) | 5 |
| 105 | - | Me | 3-FPh | -NHCO- | (2R*, 3R*) | 5 |
| 15 | - | Me | 4-FPh | -NHCO- | (2R*, 3R*) | 5 |
| 106 | - | Me | 3,4-diFPh | -NHCO- | (2R*, 3R*) | 5 |
| 107 | - | Me | 2,5-diFPh | -NHCO- | (2R*, 3R*) | 5 |
| 108 | - | Me | 3,5-diFPh | -NHCO- | (2R*, 3R*) | 5 |
| 109 | - | Me | 2,6-diFPh | -NHCO- | (2R*, 3R*) | 5 |
| 110 | - | Me | 2-TfmPh | -NHCO- | (2R*, 3R*) | 5 |
| 66 | - | Me | 3-TfmPh | -NHCO- | (2R*, 3R*) | 5 |
| 17 | - | Me | 4-TfmPh | -NHCO- | (2R*, 3R*) | 5 |
| 65 | - | Me | 2-F-4-TfmPh | -NHCO- | (2R*, 3R*) | 5 |
| 67 | - | Me | 4-CNPh | -NHCO- | (2R*, 3R*) | 4 |
| 159 | - | Me | 4-TfmOPh | -NHCO- | (2R*, 3R*) | 5 |
| 111 | - | Me | tBu | -NHCO- | (2R*, 3R*) | 5 |
| 112 | - | Me | $-CHCℓ_2$ | -NHCO- | (2R*, 3R*) (oxalate) | 5 |
| 68 | - | Me | 5-Cℓ-Thi | -NHCO- | (2R*, 3R*) | 5 |
| 203 | - | Me | 6-Cℓ-3-Pyr | -NHCO- | (2R*, 3R*) | 5 |
| 116 | - | Me | 2-Pyr | -NHCO- | (2R*, 3R*) | 5 |
| 117 | - | Et | 4-CℓPh | -NHCO- | (2R*, 3R*) | 5 |
| 123 | - | Me | 4-tBuPh | -NHCO- | (2R*, 3R*) | 4 |
| 124 | - | Me | 4-MeOPh | -NHCO- | (2R*, 3R*) | 5 |
| 125 | - | Me | 4-MePh | -NHCO- | (2R*, 3R*) | 5 |
| 127 | - | Me | 2-Np | -NHCO- | (2R*, 3R*) | 5 |
| 130 | - | Me | 4-BzOPh | -NHCO- | (2R*, 3R*) | 4 |
| 60 | - | Me | 2-F-4-TfmPh | -N(Me)CO- | (2R*, 3R*) | 5 |
| 61 | - | Me | 4-CℓPh | -N(Me)CO-CH=CH- | (2R*, 3R*) | 5 |

Table 16B (cont)

| Ex. No. | $X_m$ | $R^1$ | $R^2$ | $Y_n$ | Stereo- chem. | Index |
|---|---|---|---|---|---|---|
| 135 | - | Me | 4-Cl-Phtm | - | (2R*, 3R*) (nitrate) | 5 |
| 139 | - | Me | 4-ClPh | -OCO- | (2R*, 3R*) | 5 |
| 140 | - | Me | 4-TfmPh | -OCO- | (2R*, 3R*) | 5 |
| 142 | - | Me | - | $-N_3$ | (2R*, 3R*) | 5 |
| 149 | - | Et | - | $-N_3$ | (2R*, 3R*) | 5 |
| 150 | - | Et | - | $-N_3$ | (2R*, 3S*) | 5 |
| 83 (C-4 isomer 1) | -CH(Me)- | Me | - | $-N_3$ | (2R*, 3R*) | 5 |
| 83 (oxalate, C-4 isomer 2) | -CH(Me)- | Me | - | $-N_3$ | (2R*, 3S*) | 5 |
| 153 | $-CH_2-$ | Me | - | $-N_3$ | (2R*, 3S*) | 5 |
| 81 | $-(CH_2)_2-$ | Me | - | $-N_3$ | (2R*, 3S*) (oxalate) | 5 |
| 202 | - | Et | 2-Thp | -O- | (2R*, 3R*) | 5 |

Table 16C

| Ar = 2-F-4-ClPh | | | | | | |
|---|---|---|---|---|---|---|
| Ex. No. | $X_m$ | $R^1$ | $R^2$ | $Y_n$ | Stereochem. | Index |
| 118 | - | Me | 4-TfmPh | -NHCO- | (2R*, 3R*) | 5 |
| 163 | - | Me | 4-ClPh | -NHCO- | (2R*, 3R*) | 5 |
| 119 | - | Me | 2-F-4-TfmPh | -NHCO- | (2R*, 3R*) | 5 |
| 142 | - | Me | - | $-N_3$ | (2R*, 3R*) | 5 |
| 151 | - | Et | - | $-N_3$ | (2R*, 3R*) | 5 |

## Table 16D

Ar = 2-Cℓ-4-FPh

| Ex. No. | $X_m$ | $R^1$ | $R^2$ | $Y_n$ | Stereo-chem. | Index |
|---|---|---|---|---|---|---|
| 146 | - | Me | | $-N_3$ | (2R*, 3R*) | 5 |

Table 16E

| Ar = 4-CℓPh | | | | | | |
|---|---|---|---|---|---|---|
| Ex. No. | $X_m$ | $R^1$ | $R^2$ | $Y_n$ | Stereochem. | Index |
| 70 | - | Me | 2-F-4-TfmPh | -NHCO- | (2R*, 3R*) | 5 |
| 63 | - | Me | - | $-N_3$ | (2R*, 3R*) (oxalate) | 5 |
| 141 | - | Me | - | $-N_3$ | (2R*, 3S*) (oxalate) | 4 |

Table 16F

| Ar = 4-FPh | | | | | | |
|---|---|---|---|---|---|---|
| Ex. No. | $X_m$ | $R^1$ | $R^2$ | $Y_n$ | Stereochem. | Index |
| 147 | - | Me | - | $-N_3$ | (2R*, 3R*) | 4 |

Table 16G

| Ar = 4-BrPh | | | | | | |
|---|---|---|---|---|---|---|
| Ex. No. | $X_m$ | $R^1$ | $R^2$ | $Y_n$ | Stereochem. | Index |
| 148 | - | Me | - | $-N_3$ | (2R*, 3R*) | 4 |

Table 16H

| The compounds tested had the formula (I-2), in which Ar, $R^1$, $R^2$, p and q are as defined in the Table. | | | | | | | |
|---|---|---|---|---|---|---|---|
| Ex. No. | Ar | $R^1$ | $R^2$ | p | q | Stereochem. | Index |
| 78 | 2,4-diFPh | Me | 2-F-4-TfmPh | 1 | 0 | (4R*, 5R*) (oxalate) | 4 |
| 76 | 4-CℓPh | Me | 2-F-4-TfmPh | 1 | 0 | (4R*, 5R*) (oxalate) | 5 |

EXPERIMENT 7

Curative activity against powdery mildew of barley

Barley seedlings (variety Sekishinriki) at the first leaf stage were inoculated with conidia of *Erysiphe graminis* f.sp. *hordei* and kept in a greenhouse at 15 - 20 °C. After one day, the seedlings were sprayed with an aqueous suspension of the test compound at a concentration of 3 ppm (30 ml/3 pots), and they continued to be kept in the greenhouse at that temperature for a further 10 days. The disease controlling index was given on the basis of the diseased area on the first leaf. The results are shown in Table 17.

Table 17

Curative Activity against Powdery Mildew of Barley

Except for Table 17H, the compounds tested have the formula (I-1), in which Ar represents the group shown at the beginning of each part of the Table and $R^1$, $R^2$, X and Y are as defined in the Table.

Table 17A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 5 | - | Me | 4-CℓPh | -NHCO- | (2R*, 3R*) | 5 |
| 5 | - | Me | 4-CℓPh | -NHCO- | (2R*, 3R*) (oxalate) | 5 |
| 120 | - | Me | 3,4-diCℓPh | -NHCO- | (2R*, 3R*) | 4 |
| 4 | - | Me | 4-FPh | -NHCO- | (2R*, 3R*) | 5 |
| 22 | - | Me | 2,4-diFPh | -NHCO- | (2R*, 3R*) | 5 |
| 12 | - | Me | 4-TfmPh | -NHCO- | (2R*, 3R*) | 5 |
| 13 | - | Me | 4-TfmPh | -NHCO- | (2R, 3R) | 5 |

Table 17B

Ar=2,4-diFPh

| Ex. No. | $X_m$ | $R^1$ | $R^2$ | $Y_n$ | Stereo-chem. | Index |
|---|---|---|---|---|---|---|
| 16 | - | Me | 4-CℓPh | -NHCO- | (2R*, 3R*) | 5 |
| 105 | - | Me | 3-FPh | -NHCO- | (2R*, 3R*) | 5 |
| 15 | - | Me | 4-FPh | -NHCO- | (2R*, 3R*) | 5 |
| 106 | - | Me | 3,4-diFPh | -NHCO- | (2R*, 3R*) | 5 |
| 108 | - | Me | 3,5-diFPh | -NHCO- | (2R*, 3R*) | 5 |
| 109 | - | Me | 2-6-diFPh | -NHCO- | (2R*, 3R*) | 5 |
| 110 | - | Me | 2-TfmPh | -NHCO- | (2R*, 3R*) | 5 |
| 66 | - | Me | 3-TfmPh | -NHCO- | (2R*, 3R*) | 5 |
| 17 | - | Me | 4-TfmPh | -NHCO- | (2R*, 3R*) | 5 |
| 65 | - | Me | 2-F-4-TfmPh | -NHCO- | (2R*, 3R*) | 5 |
| 67 | - | Me | 4-CNPh | -NHCO- | (2R*, 3R*) | 4 |
| 159 | - | Me | 4-TfmOPh | -NHCO- | (2R*, 3R*) | 5 |
| 111 | - | Me | 4-tBu | -NHCO- | (2R*, 3R*) | 5 |
| 112 | - | Me | $-CHCℓ_2$ | -NHCO- | (2R*, 3R*) | 5 |
| 68 | - | Me | 5-Cℓ-2-Thi | -NHCO- | (2R*, 3R*) | 5 |
| 203 | - | Me | 6-Cℓ-3-Pyr | -NHCO- | (2R*, 3R*) | 5 |
| 116 | - | Me | 2-Pyr | -NHCO- | (2R*, 3R*) | 5 |
| 117 | - | Et | 4-CℓPh | -NHCO- | (2R*, 3R*) | 5 |
| 123 | - | Me | 4-tBuPh | -NHCO- | (2R*, 3R*) | 4 |
| 124 | - | Me | 4-MeOPh | -NHCO- | (2R*, 3R*) | 5 |
| 125 | - | Me | 4-MePh | -NHCO- | (2R*, 3R*) | 5 |
| 127 | - | Me | 2-Np | -NHCO- | (2R*, 3R*) | 5 |
| 130 | - | Me | 4-BzOPh | -NHCO- | (2R*, 3R*) | 5 |
| 135 | - | Me | 4-Cℓ-Phtm | - | (2R*, 3R*) (nitrate) | 5 |
| 139 | - | Me | 4-CℓPh | -OCO- | (2R*, 3R*) | 5 |

Table 17B (cont)

| Ex. No. | $X_m$ | $R^1$ | $R^2$ | $Y_n$ | Stereo-chem. | Index |
|---|---|---|---|---|---|---|
| 142 | - | Me | - | $-N_3$ | (2R*, 3R*) | 5 |
| 149 | - | Et | - | $-N_3$ | (2R*, 3R*) | 5 |
| 150 | - | Et | - | $-N_3$ | (2R*, 3S*) | 5 |
| 83 (C-4 isomer 1) | -CH(Me)- | Me | - | $-N_3$ | (2R*, 3S*) | 5 |
| 83 (oxalate, C-4 isomer 2) | -CH(Me)- | Me | - | $-N_3$ | (2R*, 3S*) | 5 |
| 202 | - | Et | 2-Thp | -O- | (2R*, 3R*) | 5 |

Table 17C

| Ar = 2-F-4-CℓPh | | | | | | |
|---|---|---|---|---|---|---|
| Ex. No. | $X_m$ | $R^1$ | $R^2$ | $Y_n$ | Stereochem. | Index |
| 118 | - | Me | 4-TfmPh | -NHCO- | (2R*, 3R*) | 5 |
| 163 | - | Me | 4-CℓPh | -NHCO- | (2R*, 3R*) | 5 |
| 119 | - | Me | 2-F-4-TfmPh | -NHCO- | (2R*, 3R*) | 5 |
| 145 | - | Me | - | $-N_3$ | (2R*, 3R*) | 5 |
| 151 | - | Et | - | $-N_3$ | (2R*, 3R*) | 5 |

Table 17D

| Ar = 2-Cℓ-4-FPh | | | | | | |
|---|---|---|---|---|---|---|
| Ex. No. | $X_m$ | $R^1$ | $R^2$ | $Y_n$ | Stereochem. | Index |
| 152 | - | Me | - | $-N_3$ | (2R*, 3R*) | 5 |

Table 17E

| Ar = 4-CℓPh | | | | | | |
|---|---|---|---|---|---|---|
| Ex. No. | $X_m$ | $R^1$ | $R^2$ | $Y_n$ | Stereochem. | Index |
| 70 | - | Me | 2-F-4-TfmPh | -NHCO- | (2R*, 3R*) | 5 |
| 141 | - | Me | - | $-N_3$ | (2R*, 3S*) | 5 |

Table 17F

| Ar = 4-FPh | | | | | | |
|---|---|---|---|---|---|---|
| Ex. No. | $X_m$ | $R^1$ | $R^2$ | $Y_n$ | Stereochem. | Index |
| 122 | - | Me | 4-CℓPh | -NHCO- | (2R*, 3R*) | 5 |
| 147 | - | Me | - | $-N_3$ | (2R*, 3R*) | 5 |

Table 17G

| Ar = 4-BrPh | | | | | | |
|---|---|---|---|---|---|---|
| Ex. No. | $X_m$ | $R^1$ | $R^2$ | $Y_n$ | Stereochem. | Index |
| 148 | - | Me | - | $-N_3$ | (2R*, 3R*) | 5 |

Table 17H

| The compounds tested had the formula (I-2), in which Ar, $R^1$, $R^2$, p and q are as defined in the Table. | | | | | | | |
|---|---|---|---|---|---|---|---|
| Ex. No. | Ar | $R^1$ | $R^2$ | p | q | Stereochem. | Index |
| 78 | 2,4-diFPh | Me | 2-F-4-TfmPh | 1 | 0 | (4R*, 5R*) (oxalate) | 5 |
| 76 | 4-CℓPh | Me | 2-F-4-TfmPh | 1 | 0 | (4R*, 5R*) (oxalate) | 5 |
| 41 | 2,4-diFPh | Me | 4-TfmPh | 1 | 1 | (4R*, 5R*) | 4 |
| 39 | 2,4-diFPh | Me | 4-CℓPh | 1 | 1 | (4R*, 5R*) | 4 |

EXPERIMENT 8

Preventive activity against powdery mildew of cucumber

Cucumber seedlings (variety Sagamihanpaku) at the 3 - 4 leaf stage were sprayed with an aqueous suspension of the test compound at a concentration of 300 ppm (30 ml/3 pots). The seedlings were then kept for 24 hours at room temperature, after which they were inoculated with conidida of Sphaerotheca fuliginea. After keeping the seedlings in a greenhouse for 7 days, the disease controlling index was evaluated on the basis of the diseased area on the third and fourth leaves. The results are shown in Table 18.

Table 18

Preventive Activity against Powdery Mildew of Cucumber

Except for Table 18G, the compounds tested have the formula (I-1), in which Ar represents the group shown at the beginning of each part of the Table and $R^1$, $R^2$, X and Y are as defined in the Table.

Table 18A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 5 | - | Me | 4-CℓPh | -NHCO- | (2R*, 3R*) | 5 |
| 5 | - | Me | 4-CℓPh | -NHCO- | (2R*, 3R*) (oxalate) | 5 |
| 164 | - | Me | 4-CℓPh | -NHCO- | (2R*, 3S*) (oxalate) | 5 |
| 10 | - | Me | 4-CℓPh | -NHCO- | (2R, 3R) (oxalate) | 5 |
| 11 | - | Me | 2,4-diCℓPh | -NHCO- | (2R*, 3R*) | 5 |
| 120 | - | Me | 3,4-diCℓPh | -NHCO- | (2R*, 3R*) | 5 |
| 22 | - | Me | 2,4-diFPh | -NHCO- | (2R*, 3R*) | 5 |
| 12 | - | Me | 4-TfmPh | -NHCO- | (2R*, 3R*) | 5 |
| 13 | - | Me | 4-TfmPh | -NHCO- | (2R, 3R) | 5 |
| 121 | - | Me | tBu | -NHCO- | (2R*, 3R*) (oxalate) | 5 |
| 46 | - | Me | 4-TfmPh | -SCO- | (2R*, 3R*) | 5 |
| 2 | - | Me | - | $N_3$ | (2R*, 3R*) | 5 |
| 154 | $-CH_2-$ | Me | - | $N_3$ | (2R*, 3R*) | 5 |

Table 18B

Ar=2,4-diFPh

| Ex. No. | $X_m$ | $R^1$ | $R^2$ | $Y_n$ | Stereo-chem. | Index |
|---|---|---|---|---|---|---|
| 16 | - | Me | 4-CℓPh | -NHCO- | (2R*, 3R*) | 5 |
| 105 | - | Me | 3-FPh | -NHCO- | (2R*, 3R*) | 5 |
| 15 | - | Me | 4-FPh | -NHCO- | (2R*, 3R*) | 5 |
| 155 | - | Me | 2,4-diFPh | -NHCO- | (2R*, 3R*) | 5 |
| 106 | - | Me | 3,4-diFPh | -NHCO- | (2R*, 3R*) | 5 |
| 107 | - | Me | 2,5-diFPh | -NHCO- | (2R*, 3R*) | 5 |
| 108 | - | Me | 3,5-diFPh | -NHCO- | (2R*, 3R*) | 5 |
| 109 | - | Me | 2,6-diFPh | -NHCO- | (2R*, 3R*) | 5 |
| 110 | - | Me | 2-TfmPh | -NHCO- | (2R*, 3R*) | 5 |
| 66 | - | Me | 3-TfmPh | -NHCO- | (2R*, 3R*) | 5 |
| 17 | - | Me | 4-TfmPh | -NHCO- | (2R*, 3R*) | 4 |
| 65 | - | Me | 2-F-4-TfmPh | -NHCO- | (2R*, 3R*) | 5 |
| 67 | - | Me | 4-CNPh | -NHCO- | (2R*, 3R*) | 5 |
| 159 | - | Me | 4-TfmOPh | -NHCO- | (2R*, 3R*) | 5 |
| 160 | - | Me | 4-TfmOPh | -NHCO- | (2R*, 3S*) | 5 |
| 111 | - | Me | tBu | -NHCO- | (2R*, 3R*) | 4 |
| 112 | - | Me | $-CHCℓ_2$ | -NHCO- | (2R*, 3R*) (oxalate) | 5 |
| 113 | - | Me | 2-Fur | -NHCO- | (2R*, 3R*) (oxalate) | 5 |
| 114 | - | Me | 2-Thi | -NHCO- | (2R*, 3R*) | 5 |
| 68 | - | Me | 5-Cℓ-2-Thi | -NHCO- | (2R*, 3R*) | 5 |
| 203 | - | Me | 6-Cℓ-3-Pyr | -NHCO- | (2R*, 3R*) | 5 |
| 115 | - | Me | 4-Pyr | -NHCO- | (2R*, 3R*) | 5 |
| 116 | - | Me | 2-Pyr | -NHCO- | (2R*, 3R*) | 5 |
| 117 | - | Et | 4-CℓPh | -NHCO- | (2R*, 3R*) | 5 |

Table 18B (cont)

| Ex. No. | $X_m$ | $R^1$ | $R^2$ | $Y_n$ | Stereo-chem. | Index |
|---|---|---|---|---|---|---|
| 123 | - | Me | 4-tBuPh | -NHCO- | (2R*, 3R*) | 5 |
| 124 | - | Me | 4-MeOPh | -NHCO- | (2R*, 3R*) | 5 |
| 125 | - | Me | 4-MePh | -NHCO- | (2R*, 3R*) | 5 |
| 126 | - | Me | 4-N(Me)$_2$Ph | -NHCO- | (2R*, 3R*) | 5 |
| 128 | - | Et | 2,5-diMe-3-Fur | -NHCO- | (2R*, 3R*) | 5 |
| 129 | - | Et | 1,3,5-triMe-4-Pyaz | -NHCO- | (2R*, 3R*) | 5 |
| 168 | - | Me | 3-TfmPh | -N(Me)CO- | (2R*, 3R*) | 5 |
| 59 | - | Me | 4-TfmPh | -N(Me)CO- | (2R*, 3R*) | 5 |
| 60 | - | Me | 2-F-4-TfmPh | -N(Me)CO- | (2R*, 3R*) | 5 |
| 61 | - | Me | 4-CℓPh | -N(Me)CO-CH=CH- | (2R*, 3R*) | 5 |
| 135 | - | Me | 4-Cℓ-Phtm | - | (2R*, 3R*) (nitrate) | 5 |
| 139 | - | Me | 4-CℓPh | -OCO- | (2R*, 3R*) | 5 |
| 140 | - | Me | 4-TfmPh | -OCO- | (2R*, 3R*) | 5 |
| 51 | - | Me | 4-TfmPh | -SCO- | (2R*, 3R*) | 5 |
| 142 | - | Me | - | $-N_3$ | (2R*, 3R*) | 5 |
| 99 | - | Me | - | $-N_3$ | (2R, 3R) | 5 |
| 143 | - | Me | - | $-N_3$ | (2R*, 3S*) | 5 |
| 144 | - | Me | - | $-N_3$ | (2S, 3R) | 5 |
| 149 | - | Et | - | $-N_3$ | (2R*, 3R*) | 5 |
| 150 | - | Et | - | $-N_3$ | (2R*, 3S*) | 5 |
| 83 (C-4 isomer 1) | -CH(Me)- | Me | - | $-N_3$ | (2R*, 3S*) | 5 |
| 83 (oxalate, C-4 isomer 2) | -CH(Me)- | Me | - | $-N_3$ | (2R*, 3S*) | 5 |
| 153 | $-CH_2-$ | Me | - | $-N_3$ | (2R*, 3S*) | 5 |

Table 18B (cont)

| Ex. No. | $X_m$ | $R^1$ | $R^2$ | $Y_n$ | Stereo-chem. | Index |
|---|---|---|---|---|---|---|
| 81 | $-(CH_2)_2-$ | Me | - | $-N_3$ | (2R*, 3S*) (oxalate) | 5 |
| 204 | - | Et | Me | $-OSO_2-$ | (2R*, 3R*) | 5 |
| 202 | - | Et | 2-Thp | -O- | (2R*, 3R*) | 5 |

Table 18C

| Ar = 2-F-4-CℓPh | | | | | | |
|---|---|---|---|---|---|---|
| Ex. No. | $X_m$ | $R^1$ | $R^2$ | $Y_n$ | Stereochem. | Index |
| 118 | - | Me | 4-TfmPh | -NHCO- | (2R*, 3R*) | 5 |
| 163 | - | Me | 4-CℓPh | -NHCO- | (2R*, 3R*) | 5 |
| 119 | - | Me | 2-F-4-TfmPh | -NHCO- | (2R*, 3R*) | 5 |

Table 18D

| Ar = 4-CℓPh | | | | | | |
|---|---|---|---|---|---|---|
| Ex. No. | $X_m$ | $R^1$ | $R^2$ | $Y_n$ | Stereochem. | Index |
| 205 | - | Me | 4-CℓPh | -NHCO- | (2R*, 3R*) | 5 |
| 206 | - | Me | 4-CℓPh | -NHCO- | (2R*, 3S*) | 5 |
| 70 | - | Me | 2-F-4-TfmPh | -NHCO- | (2R*, 3R*) | 5 |
| 142 | - | Me | - | $-N_3$ | (2R*, 3R*) (oxalate) | 5 |
| 143 | - | Me | - | $-N_3$ | (2R*, 3S*) (oxalate) | 5 |

Table 18E

| Ar = 4-FPh | | | | | | |
|---|---|---|---|---|---|---|
| Ex. No. | $X_m$ | $R^1$ | $R^2$ | $Y_n$ | Stereochem. | Index |
| 122 | - | Me | 4-CℓPh | -NHCO- | (2R*, 3R*) | 5 |
| 147 | - | Me | - | $-N_3$ | (2R*, 3R*) | 5 |

Table 18F

| Ar = 4-BrPh | | | | | | |
|---|---|---|---|---|---|---|
| Ex. No. | $X_m$ | $R^1$ | $R^2$ | $Y_n$ | Stereochem. | Index |
| 148 | - | Me | - | $-N_3$ | (2R*, 3R*) | 5 |

Table 18G

| The compounds tested had the formula (I-2), in which Ar, $R^1$, $R^2$, p and q are as defined in the Table. | | | | | | | |
|---|---|---|---|---|---|---|---|
| Ex. No. | Ar | $R^1$ | $R^2$ | p | q | Stereochem. | Index |
| 39 | 2,4-diFPh | Me | 4-CℓPh | 1 | 0 | (4R*, 5R*) (oxalate) | 5 |
| 78 | 2,4-diFPh | Me | 4-F-4-TfmPh | 1 | 0 | (4R*, 5R*) (oxalate) | 5 |
| 76 | 4-CℓPh | Me | 2-F-4-TfmPh | 1 | 0 | (4R*, 5R*) (oxalate) | 5 |
| 72 | 2,4-diFPh | Me | 3-TfmPh | 1 | 1 | (4R*, 5R*) | 5 |
| 71 | 2,4-diFPh | Me | 4-CℓPh | 1 | 1 | (4R*, 5R*) (oxalate) | 5 |
| 75 | 4-CℓPh | Me | 4-Tfmph | 1 | 1 | (4R*, 5R*) | 5 |

EXPERIMENT 9

Prevention activity against apple scab

Apple seedlings at the 3 - 4 leaf stage were sprayed with an aqueous suspension of the test compound at a concentration of 300 ppm (30 ml/3 pots). The seedlings were then kept for 24 hours at room temperature, after which they were inoculated with the fungus, Venturia inaequalis, by spraying a spore suspension of the fungus over the seedlings. After inoculation, the seedlings were kept in a moist chamber for 3 days at 20 - 22 °C and then moved to a greenhouse at 20 - 22 °C for 10 days. The disease controlling index was given on the basis of the diseased area on the third and fourth leaves. The results are shown in Table 19.

Table 19

Preventive Activity against Apple Scab

Except for Table 19F, the compounds tested have the formula (I-1), in which Ar represents the group shown at the beginning of each part of the Table and $R^1$, $R^2$, X and Y are as defined in the Table.

Table 19A

| Ar = 2,4-diCℓPh | | | | | | |
|---|---|---|---|---|---|---|
| Ex. No. | $X_m$ | $R^1$ | $R^2$ | $Y_n$ | Stereochem. | Index |
| 5 | - | Me | 4-CℓPh | -NHCO- | (2R*, 3R*) (oxalate) | 5 |
| 10 | - | Me | 4-CℓPh | -NHCO- | (2R, 3R) (oxalate) | 5 |
| 11 | - | Me | 2,4-diCℓPh | -NHCO- | (2R*, 3R*) | 5 |
| 4 | - | Me | 4-FPh | -NHCO- | (2R*, 3R*) | 5 |
| 12 | - | Me | 4-TfmPh | -NHCO- | (2R*, 3R*) | 5 |
| 121 | - | Me | tBu | -NHCO- | (2R*, 3R*) (oxalate) | 5 |
| 46 | - | Me | 4-TfmPh | -SCO- | (2R*, 3R*) | 5 |
| 2 | - | Me | - | $-N_3$ | (2R*, 3R*) | 5 |
| 154 | $-CH_2-$ | Me | - | $-N_3$ | (2R*, 3R*) | 5 |

Table 19B

Ar=2,4-diFPh

| Ex. No. | $X_m$ | $R^1$ | $R^2$ | $Y_n$ | Stereo-chem. | Index |
|---|---|---|---|---|---|---|
| 16 | - | Me | 4-CℓPh | -NHCO- | (2R*, 3R*) | 5 |
| 105 | - | Me | 3-FPh | -NHCO- | (2R*, 3R*) | 5 |
| 155 | - | Me | 2,4-diFPh | -NHCO- | (2R*, 3R*) | 5 |

Table 19B (cont)

| Ex. No. | $X_m$ | $R^1$ | $R^2$ | $Y_n$ | Stereo-chem. | Index |
|---|---|---|---|---|---|---|
| 106 | - | Me | 3,4-diFPh | -NHCO- | (2R*, 3R*) | 5 |
| 107 | - | Me | 2,5-diFPh | -NHCO- | (2R*, 3R*) | 5 |
| 108 | - | Me | 3,5-diFPh | -NHCO- | (2R*, 3R*) | 5 |
| 109 | - | Me | 2,6-diFPh | -NHCO- | (2R*, 3R*) | 5 |
| 159 | - | Me | 4-TfmOPh | -NHCO- | (2R*, 3R*) | 5 |
| 160 | - | Me | 4-TfmOPh | -NHCO- | (2R*, 3S*) | 5 |
| 111 | - | Me | tBu | -NHCO- | (2R*, 3R*) | 4 |
| 114 | - | Me | 2-Thi | -NHCO- | (2R*, 3R*) | 5 |
| 68 | - | Me | 5-Cl-2-Thi | -NHCO- | (2R*, 3R*) | 5 |
| 203 | - | Me | 6-Cl-3-Pyr | -NHCO- | (2R*, 3R*) | 5 |
| 116 | - | Me | 2-Pyr | -NHCO- | (2R*, 3R*) | 5 |
| 117 | - | Et | 4-ClPh | -NHCO- | (2R*, 3R*) | 5 |
| 123 | - | Me | 4-tBuPh | -NHCO- | (2R*, 3R*) | 5 |
| 124 | - | Me | 4-MeOPh | -NHCO- | (2R*, 3R*) | 5 |
| 125 | - | Me | 4-MePh | -NHCO- | (2R*, 3R*) | 5 |
| 168 | - | Me | 3-TfmPh | -N(Me)CO- | (2R*, 3R*) | 5 |
| 59 | - | Me | 4-TfmPh | -N(Me)CO- | (2R*, 3R*) | 5 |
| 61 | - | Me | 4-ClPh | -N(Me)CO-CH=CH- | (2R*, 3R*) | 5 |
| 142 | - | Me | - | $-N_3$ | (2R*, 3R*) | 5 |
| 99 | - | Me | - | $-N_3$ | (2R, 3R) | 5 |
| 149 | - | Et | - | $-N_3$ | (2R*, 3R*) | 5 |
| 150 | - | Et | - | $-N_3$ | (2R*, 3S*) | 5 |
| 83 (C-4 isomer 1) | -CH(Me)- | Me | - | $-N_3$ | (2R*, 3S*) | 5 |
| 83 (oxalate, C-4 isomer 2) | -CH(Me)- | Me | - | $-N_3$ | (2R*, 3S*) | 5 |
| 153 | $-CH_2-$ | Me | - | $-N_3$ | (2R*, 3S*) | 5 |

Table 19B (cont)

| Ex. No. | $X_m$ | $R^1$ | $R^2$ | $Y_n$ | Stereo-chem. | Index |
|---|---|---|---|---|---|---|
| 81 | $-(CH_2)_2-$ | Me | - | $-N_3$ | (2R*, 3S*) (oxalate) | 5 |
| 204 | - | Et | Me | $-OSO_2$ | (2R*, 3R*) | 5 |
| 202 | - | Et | 2-Thp | -O- | (2R*, 3R*) | 5 |

Table 19C

| Ar = 2-F-4-CℓPh | | | | | | |
|---|---|---|---|---|---|---|
| Ex. No. | $X_m$ | $R^1$ | $R^2$ | $Y_n$ | Stereochem. | Index |
| 118 | - | Me | 4-TfmPh | -NHCO- | (2R*, 3R*) | 5 |
| 163 | - | Me | 4-CℓPh | -NHCO- | (2R*, 3R*) | 5 |
| 119 | - | Me | 2-F-4-TfmPh | -NHCO- | (2R*, 3R*) | 5 |

Table 19D

| Ar = 4-CℓPh | | | | | | |
|---|---|---|---|---|---|---|
| Ex. No. | $X_m$ | $R^1$ | $R^2$ | $Y_n$ | Stereochem. | Index |
| 205 | -Me | 4-CℓPh | | -NHCO- | (2R*, 3R*) | 5 |
| 63 | - | Me | - | $-N_3$ | (2R*, 3R*) (oxalate) | 5 |
| 141 | - | Me | - | $-N_3$ | (2R*, 3S*) (oxalate) | 5 |

Table 19E

| Ar = 4-FPh | | | | | | |
|---|---|---|---|---|---|---|
| Ex. No. | $X_m$ | $R^1$ | $R^2$ | $Y_n$ | Stereochem. | Index |
| 122 | - | Me | 4-CℓPh | -NHCO- | (2R*, 3R*) | 5 |
| 147 | - | Me | - | $-N_3$ | (2R*, 3R*) | 5 |

Table 19E

| Ar = 4-BrPh | | | | | | |
|---|---|---|---|---|---|---|
| Ex. No. | $X_m$ | $R^1$ | $R^2$ | $Y_n$ | Stereochem. | Index |
| 148 | - | Me | - | $-N_3$ | (2R*, 3R*) | 5 |

Table 19F

| The compounds tested had the formula (I-2), in which Ar, $R^1$, $R^2$, p and q are as defined in the Table. | | | | | | | |
|---|---|---|---|---|---|---|---|
| Ex. No. | Ar | $R^1$ | $R^2$ | p | q | Stereochem. | Index |
| 76 | 4-CℓPh | Me | 2-F-4-TfmPh | 1 | 0 | (2R*, 3R*) (oxalate) | 5 |
| 72 | 2,4-diFPh | Me | 3-TfmPh | 1 | 1 | (2R*, 3R*) | 5 |
| 73 | 4-CℓPh | Me | 4-TfmPh | 1 | 1 | (2R*, 3R*) | 5 |

The disease controlling index shown in the foregoing Tables 11 to 19 is indicated by the following codes:

5--no disease (complete control)
4--disease rate is less than 10% of the untreated plant
3--disease rate is 10% - 30% of the untreated plant
2--disease rate is 30% - 50% of the untreated plant
1--disease rate is 50% - 70% of the untreated plant
0--disease rate is almost same as the untreated plant.

The compounds of the present invention are also useful for the therapeutic treatment of fungal infections in humans and other animals, as is illustrated by the following Experiments:

EXPERIMENT 10

Groups of mice (each group contained 10 mice) were inoculated with 7 to 9 x $10^6$ spores per mouse of Candida albicans. 1, 4, and 24 hours after the inoculation, each mouse was orally administered 20 mg/kg of the test compound each time. The anti-fungal activity was assessed by the survival rate 9 days after infection. The survival rate was recorded as 0% 2 days after infection in an untreated (control) group. The results are reported in the following Table 20:

The experiment was also carried out employing the known compound, ketoconazole, and a similar compound disclosed in Japanese Patent Publication Kokai No. Sho 59-62574, 3-(4-chlorobenzoylamino)-2-(2,4-dichlorophenyl)-1-(1H-1,2,4-triaiol-1-yl)-2-propanol, referred to in this Table as "Compound X".

Table 20

| Compound of Example No. | Survival Rate (%) |
|---|---|
| 4 | 90 |
| 5 | 100 |
| 10 | 100 |
| 12 | 100 |
| 13 | 100 |
| 17 | 100 |
| 20 | 100 |
| 21 | 90 |
| 22 | 90 |
| 35 | 90 |
| 37 | 100 |
| 39 | 90 |
| 41 | 100 |
| 42 | 90 |
| 46 | 90 |
| 50 | 100 |
| 51 | 100 |
| 52 | 100 |
| 53 | 100 |
| 54 | 100 |
| 55 | 100 |
| 56 | 80 |
| 57 | 100 |
| 59 | 100 |
| 60 | 100 |
| 61 | 100 |
| 65 | 100 |
| 66 | 100 |

Table 20 (cont)

| Compound of Example No. | Survival Rate (%) |
|---|---|
| 67 | 100 |
| 69 | 100 |
| 70 | 100 |
| 71 | 100 |
| 72 | 100 |
| 76 | 100 |
| 78 | 100 |
| 104 | 100 |
| 156 | 100 |
| ketoconazole | 50 |
| compound X | 50 |

EXPERIMENT 11

Following essentially the same procedure as that described in Experiment 10, mice were infected with Candida albicans, and treated orally with various compounds of the invention, as shown in the following Table 21. Fifteen days after infection (or fourteen days in the case of the compounds of Example Nos. 5 and 10), $ED_{50}$ values were determined and are reported in the following Table 21.

Table 21

| Compound of Example No. | $ED_{50}$ (mg/kg) |
|---|---|
| 5 | 7.07 |
| 10 | <2.5 |
| 17 | 0.86 |
| 46 | 0.73 |
| 54 | 1.22 |
| 59 | 0.66 |
| 65 | <0.625 |
| 67 | 1.25 |
| 103 | 0.48 |
| ketoconazole | 31.4 |

As can be seen from the above results, the compounds of the present invention have anti-fungal activities far better than the activities of the commercially available ketoconazole or the most potent anti-fungal acitivity of the control compound described in Japanese Patent Publication Kokai No. Sho 59-62574.

## Claims

**Claims for the following Contracting States : AT, BE, CH, DE, FR, GB, IT, LI, LU, NL, SE**

**1.** Compounds of formula (I):

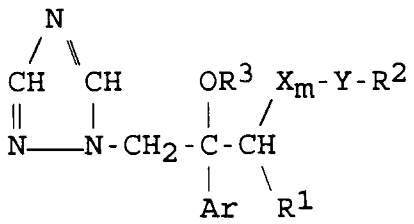

(I)

in which:

Ar represents a phenyl group or a phenyl group having one or two halogen and/or trifluoromethyl substituents;

$R^1$ represents a $C_1$ - $C_6$ alkyl group or, where $R^3$ and $-X_m-Y-R^2$ together represent said group of formula (II), $R^1$ represents a hydrogen atom or a $C_1$ - $C_6$ alkyl group;

X represents a $C_1$ - $C_6$ alkylene group, a $C_2$ - $C_6$ aliphatic hydrocarbon group having one or two carbon-carbon double bonds, a $C_2$ - $C_6$ aliphatic hydrocarbon group having one or two carbon-carbon triple bonds, a $C_3$ - $C_6$ cycloalkylene group, a $C_3$ - $C_6$ cycloalkylene group having one or two carbon-carbon double bonds, a ($C_1$ - $C_3$ alkylene)-($C_3$ - $C_6$ cycloalkylene) group or a ($C_3$ - $C_6$ cycloalkylene)-($C_1$ - $C_3$ alkylene) group;

m is 0 or 1;

$-Y-R^2$ represents the azido group, the phthalimido group, the 1-oxo-2,3-dihydro-2-isoindolyl group, or the phthalimido group or 1-oxo-2,3-dihydro-2-isoindolyl group having at least one of substituents (a), defined below;

or

Y represents a group of formula $-N(R^5)CO-$, $-N(R^5)CO-CH=CH-$, -O-CO-, -O-CO-CH=CH-, -S-CO- or -S-CO-CH=CH-, in which:

$R^5$ represents a hydrogen atom or a $C_1$ - $C_4$ alkyl group;

$R^2$ represents a $C_1$ - $C_6$ alkyl group, a $C_1$ - $C_6$ haloalkyl group, a phenyl group, a phenyl group having at least one of substituents (a), defined below, a naphthyl group or a heterocyclic group having 5 or 6 ring atoms of which from 1 to 3 are nitrogen and/or oxygen and/or sulphur hetero-atoms, said heterocyclic group being unsubstituted or having from 1 to 3 of substituents (b), defined below; and

$R^3$ represents a hydrogen atom;

or

$R^3$ and $-X_m-Y-R^2$ together represent a group of formula (II):

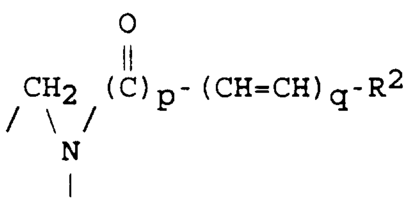

(II)

in which:

$R^2$ is as defined above;

p is 0 or 1; and

q is 0 or 1;

substituents (a):

$C_1$ - $C_4$ alkyl groups, $C_1$ - $C_4$ haloalkyl groups, $C_1$ - $C_4$ alkoxy groups, $C_1$ - $C_4$ haloalkoxy groups, halogen atoms, nitro groups, cyano groups, hydroxy groups, benzyloxy groups, hydroxymethyl groups, groups of formula $-CH_2-OCO-R^7$, groups of formula $-CO-R^6$, groups of formula $-COOR^7$, groups of formula $-SO_rR^7$, amino groups, mono- and di- alkylamino groups in which each alkyl group is $C_1$ - $C_4$ and carboxylic acylamino groups;

substituents (b):
$C_1$ - $C_4$ alkyl groups, $C_1$ - $C_4$ haloalkyl groups, halogen atoms, cyano groups and nitro groups;
$R^6$ represents a $C_1$ - $C_6$ alkyl group or a $C_1$ - $C_6$ haloalkyl group;
$R^7$ represents a $C_1$ - $C_6$ alkyl group;
r is 0, 1 or 2; and
acid addition salts thereof; provided that when m is O and Y is a group of formula -N($R^5$)CO-, $R^5$ must be hydrogen.

**2.** Compounds according to Claim 1, in which Ar represents a phenyl group having one or two halogen substituents.

**3.** Compounds according to Claim 1, in which Ar represents a phenyl group having one or two fluorine and/or chlorine substituents.

**4.** Compounds according to Claim 1, in which Ar represents a 4-chlorophenyl group, a 4-fluorophenyl group, a 2,4-dichlorophenyl group, a 2,4-difluorophenyl group, a 2-chloro-4-fluorophenyl group or a 4-chloro-2-fluorophenyl group.

**5.** Compounds according to any one of Claims 1 to 4, in which $R^1$ represents a $C_1$ - $C_3$ alkyl group.

**6.** Compounds according to any one of Claims 1 to 4, in which $R^1$ represents a methyl or ethyl group.

**7.** Compounds according to any one of Claims 1 to 6, in which X represents a $C_1$ or $C_2$ alkylene group.

**8.** Compounds according to any one of Claims 1 to 6, in which m is 0.

**9.** Compounds according to any one of Claims 1 to 8, in which -Y-$R^2$ represents the azido group.

**10.** Compounds according to any one of Claims 1 to 8, in which Y represents a group of formula -N($R^5$)CO-, -N($R^5$)CO-CH=CH-, -S-CO- or -S-CO-CH=CH-, in which:
$R^5$ represents a hydrogen atom or a $C_1$ - $C_4$ alkyl group.

**11.** Compounds according to any one of Claims 1 to 8 and 10, in which $R^2$ represents a phenyl group having one or two substituents selected from halogen atoms and trifluoromethyl, trifluoromethoxy and cyano groups.

**12.** Compounds according to Claim 1, in which:
Ar represents a phenyl group having one or two halogen substituents;
$R^1$ represents a $C_1$ - $C_3$ alkyl group;
m is 0 or X represents a $C_1$ or $C_2$ alkylene group;
Y represents a group of formula -N($R^5$)CO-, -N($R^5$)CO-CH=CH-, -S-CO- or -S-CO-CH=CH-, in which:
$R^5$ represents a hydrogen atom or a $C_1$ - $C_4$ alkyl group; and
$R^2$ represents a phenyl group having one or two substituents selected from halogen atoms and trifluoromethyl, trifluoromethoxy and cyano groups;
or
-Y-$R^2$ represents an azido group.

**13.** Compounds according to Claim 1, in which:
Ar represents a phenyl group having one or two fluorine and/or chlorine substituents;
$R^1$ represents a methyl or ethyl group;
m is 0 or X represents a $C_1$ or $C_2$ alkylene group;
Y represents a group of formula -N($R^5$)CO-, -N($R^5$)CO-CH=CH-, -S-CO- or -S-CO-CH=CH-, in which:
$R^5$ represents a hydrogen atom or a $C_1$ - $C_4$ alkyl group; and
$R^2$ represents a phenyl group having one or two substituents selected from halogen atoms and trifluoromethyl, trifluoromethoxy and cyano groups;
or
-Y-$R^2$ represents an azido group.

**14.** Compounds according to Claim 1, in which:
$R^1$ represents a methyl or ethyl group;
m is 0 or X represents a $C_1$ or $C_2$ alkylene group;
Y represents a group of formula -N($R^5$)CO-, -N($R^5$)CO-CH=CH-, -S-CO- or -S-CO-CH=CH-, in which:
$R^5$ represents a hydrogen atom or a $C_1$ - $C_4$ alkyl group;
$R^2$ represents a phenyl group having one or two substituents selected from halogen atoms and trifluoromethyl, trifluoromethoxy and cyano groups;
or
-Y-$R^2$ represents an azido group; and
Ar represents a 4-chlorophenyl group, a 4-fluorophenyl group, a 2,4-dichlorophenyl group, a 2,4-difluorophenyl group, a 2-chloro-4-fluorophenyl group or a 4-chloro-2-fluorophenyl group.

**15.** 2-(2,4-Dichlorophenyl)-1-(1H-1,2,4-triazol-1-yl)-3-[4-(trifluoromethyl)benzoylamino]-2-butanol and acid addition salts thereof.

**16.** 2-(2,4-Difluorophenyl)-3-[2-fluoro-4-(trifluoromethyl)benzoylamino]-1-(1H-1,2,4-triazol-1-yl)-2-butanol and acid addition salts thereof.

**17.** 3-(4-Chlorobenzoylamino)-2-(2,4-difluorophenyl)-1-(1H-1,2,4-triazol-1-yl)-2-pentanol and acid addition salts thereof.

**18.** 3-Azido-2-(2,4-difluorophenyl)-1-(1H-1,2,4-triazol-1-yl)-2-butanol and acid addition salts thereof.

**19.** 3-Azido-2-(4-chloro-2-fluorophenyl)-1-(1H-1,2,4-triazol-1-yl)-2-butanol and acid addition salts thereof.

**20.** 3-Azido-2-(2,4-difluorophenyl)-1-(1H-1,2,4-triazol-1-yl)-2-pentanol and acid addition salts thereof.

**21.** 3-Azido-2-(2-chloro-4-fluorophenyl)-1-(1H-1,2,4-triazol-1-yl)-2-pentanol and acid addition salts thereof.

**22.** 4-Azido-2-(2,4-difluorophenyl)-3-methyl-1-(1H-1,2,4-triazol-1-yl)-2-pentanol and acid addition salts thereof.

**23.** 4-Azido-2-(2,4-difluorophenyl)-3-methyl-1-(1H-1,2,4-triazol-1-yl)-2-butanol and acid addition salts thereof.

**24.** 4-Azido-2-(2,4-dichlorophenyl)-3-methyl-1-(1H-1,2,4-triazol-1-yl)-2-butanol and acid addition salts thereof.

**25.** 3-Azido-2-(2,4-dichlorophenyl)-1-(1H-1,2,4-triazol-1-yl)-2-pentanol and acid addition salts thereof.

**26.** 2-(4-Chlorophenyl)-1-(1H-1,2,4-triazol-1-yl)-3-[4-(trifluoromethyl)benzoylamino]-2-butanol and acid addition salts thereof.

**27.** 2-(2,4-Difluorophenyl)-1-(1H-1,2,4-triazol-1-yl)-3-[4-(trifluoromethyl)benzoylamino]-2-butanol and acid addition salts thereof.

**28.** 3-(4-Cyanobenzoylamino)-2-(2,4-difluorophenyl)-1-(1H-1,2,4-triazol-1-yl)-2-butanol and acid addition salts thereof.

**29.** 2-(4-Chlorophenyl)-3-[2-fluoro-4-(trifluoromethyl)benzoylamino]-1-(1H-1,2,4-triazol-1-yl)-2-butanol and acid addition salts thereof.

**30.** 2-(2,4-Dichlorophenyl)-1-(1H-1,2,4-triazol-1-yl)-3-[4-(trifluoromethyl)benzoylthio]-2-butanol and acid addition salts thereof.

**31.** 3-(4-Chlorocinnamoylamino)-2-(2,4-difluorophenyl)-1-(1H-1,2,4-triazol-1-yl)-2-butanol and acid addition salts thereof.

**32.** 2-(2,4-Difluorophenyl)-1-(1H-1,2,4-triazol-1-yl)-3-{N-methyl-N-[4-(trifluoromethyl)benzoyl]amino}-2-butanol and acid addition salts thereof.

**33.** 2-(2,4-Difluorophenyl)-3-{N-[2-fluoro-4-(trifluoromethyl)benzoyl]-N-methylamino}-1-(1H-1,2,4-triazol-1-yl)-2-butanol and acid addition salts thereof.

**34.** Compounds of formula (IX)

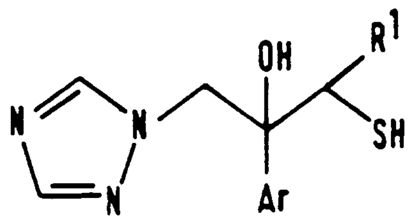

(IX)

in which:

Ar represents a phenyl group or a phenyl group having one or two halogen and/or trifluoromethyl substituents; and

$R^1$ represents a $C_1$ - $C_6$ alkyl group;

and acid addition salts thereof.

**35.** An agrochemical composition comprising an agriculturally acceptable carrier or diluent in admixture with at least one fungicide, in which the fungicide is at least one compound of formula (I):

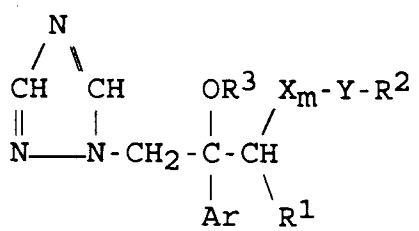

(I)

in which:

Ar represents a phenyl group or a phenyl group having one or two halogen atoms and/or trifluoromethyl substituents;

$R^1$ represents a $C_1$ - $C_6$ alkyl group or, where $R^3$ and $-X_m$-Y-$R^2$ together represent said group of formula (II), $R^1$ represents a hydrogen atom or a $C_1$ - $C_6$ alkyl group;

X represents a $C_1$ - $C_6$ alkylene group, a $C_2$ - $C_6$ aliphatic hydrocarbon group having one or two carbon-carbon double bonds, a $C_2$ - $C_6$ aliphatic hydrocarbon group having one or two carbon-carbon triple bonds, a $C_3$ - $C_6$ cycloalkylene group, a $C_3$ - $C_6$ cycloalkylene group having one or two carbon-carbon double bonds, a ($C_1$ - $C_3$ alkylene)-($C_3$ - $C_6$ cycloalkylene) group or a ($C_3$ - $C_6$ cycloalkylene)-($C_1$ - $C_3$ alkylene) group;

m is 0 or 1;

-Y-$R^2$ represents the azido group, the phthalimido group, the 1-oxo-2,3-dihydro-2-isoindolyl group, the phthalimido group or 1-oxo-2,3-dihydro-2-isoindolyl group having at least one of substituents (a), defined below, a protected hydroxy group or a group of formula $-OSO_2R^4$, in which:

$R^4$ represents a $C_1$ - $C_4$ alkyl group, a $C_1$ - $C_4$ haloalkyl group, a phenyl group or a phenyl group having one substituent selected from nitro groups, halogen atoms and $C_1$ - $C_4$ alkyl groups;

or

Y represents a group of formula $-N(R^5)CO-$, $-N(R^5)CO-CH=CH-$, -O-CO-, -O-CO-CH=CH-, -S-CO- or -S-CO-CH=CH-, in which:

$R^5$ represents a hydrogen atom or a $C_1$ - $C_4$ alkyl group;

n is 0 or 1;

$R^2$ represents a $C_1$ - $C_6$ alkyl group, a $C_1$ - $C_6$ haloalkyl group, a phenyl group, a phenyl group having at least one of substituents (a), defined below, a naphthyl group or a heterocyclic group having 5 or 6 ring atoms of which from 1 to 3 are nitrogen and/or oxygen and/or sulphur hetero-atoms, said

heterocyclic group being unsubstituted or having from 1 to 3 of substituents (b), defined below; and
$R^3$ represents a hydrogen atom;

or

$R^3$ and $-X_m-Y_n-R^2$ together represent a group of formula (II):

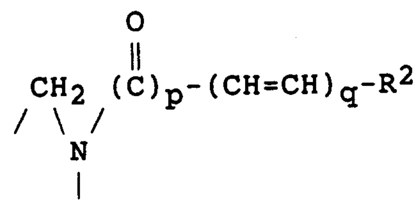

(II)

in which:

$R^2$ is as defined above;

p is 0 or 1; and

q is 0 or 1;

substituents (a):

$C_1$ - $C_4$ alkyl groups, $C_1$ - $C_4$ haloalkyl groups, $C_1$ - $C_4$ alkoxy groups, $C_1$ - $C_4$ haloalkoxy groups, halogen atoms, nitro groups, cyano groups, hydroxy groups, benzyloxy groups, hydroxymethyl groups, groups of formula $-CH_2-OCO-R^7$, groups of formula $-CO-R^6$, groups of formula $-COOR^7$, groups of formula $-SO_rR^7$, amino groups, mono- and di- alkylamino groups in which each alkyl group is $C_1$ - $C_4$ and carboxylic acylamino groups;

substituents (b):

$C_1$ - $C_4$ alkyl groups, $C_1$ - $C_4$ haloalkyl groups, halogen atoms, cyano groups and nitro groups;

$R^6$ represents a $C_1$ - $C_6$ alkyl group or a $C_1$ - $C_6$ haloalkyl group;

$R^7$ represents a $C_1$ - $C_6$ alkyl group;

r is 0, 1 or 2;

or an acid addition salt thereof.

**36.** A composition according to Claim 35, in which said fungicide is a compound according to any one of Claims 1 to 33.

**37.** A composition according to Claim 35, in which said fungicide is at least one of:
2-(2,4-dichlorophenyl)-1-(1H-1,2,4-triazol-1-yl)-3-[4-(trifluoromethyl)benzoylamino]-2-butanol;
2-(2,4-difluorophenyl)-3-[2-fluoro-4-(trifluoromethyl)benzoylamino]-1-(1H-1,2,4-triazol-1-yl)-2-butanol;
3-(4-chlorobenzoylamino)-2-(2,4-difluorophenyl)-1-(1H-1,2,4-triazol-1-yl)-2-pentanol;
3-azido-2-(2,4-difluorophenyl)-1-(1H-1,2,4-triazol-1-yl)-2-butanol;
3-azido-2-(4-chloro-2-fluorophenyl)-1-(1H-1,2,4-triazol-1-yl)-2-butanol;
3-azido-2-(2,4-difluorophenyl)-1-(1H-1,2,4-triazol-1-yl)-2-pentanol;
3-azido-2-(2-chloro-4-fluorophenyl)-1-(1H-1,2,4-triazol-1-yl)-2-pentanol;
4-azido-2-(2,4-difluorophenyl)-3-methyl-1-(1H-1,2,4-triazol-1-yl)-2-pentanol;
4-azido-2-(2,4-difluorophenyl)-3-methyl-1-(1H-1,2,4-triazol-1-yl)-2-butanol;
4-azido-2-(2,4-dichlorophenyl)-3-methyl-1-(1H-1,2,4-triazol-1-yl)-2-butanol; and
3-azido-2-(2,4-dichlorophenyl)-1-(1H-1,2,4-triazol-1-yl)-2-pentanol;
or an acid addition salt thereof.

**38.** A fungicidal pharmaceutical composition comprising a pharmaceutically acceptable carrier or diluent in admixture with at least one fungicide, in which the fungicide is at least one compound of formula (I), as defined in Claim 35.

**39.** A composition according to Claim 38, in which said fungicide is a compound according to any one of Claims 1 to 33.

**40.** A composition according to Claim 38, in which said fungicide is at least one of:
2-(4-chlorophenyl)-1-(1H-1,2,4-triazol-1-yl)-3-[4-(trifluoromethyl)benzoylamino]-2-butanol;
2-(2,4-difluorophenyl)-1-(1H-1,2,4-triazol-1-yl)-3-[4-(trifluoromethyl)benzoylamino]-2-butanol;
2-(2,4-difluorophenyl)-3-[2-fluoro-4-(trifluoromethyl)benzoylamino]-1-(1H-1,2,4-triazol-1-yl)-2-butanol;

3-(4-cyanobenzoylamino)-2-(2,4-difluorophenyl)-1-(1H-1,2,4-triazol-1-yl)-2-butanol;
2-(4-chlorophenyl)-3-[2-fluoro-4-(trifluoromethyl)benzoylamino]-1-(1H-1,2,4-triazol-1-yl)-2-butanol;
2-(2,4-dichlorophenyl)-1-(1H-1,2,4-triazol-1-yl)-3-[4-(trifluoromethyl)benzoylthio]-2-butanol;
3-(4-chlorocinnamoylamino)-2-(2,4-difluorophenyl)-1-(1H-1,2,4-triazol-1-yl)-2-butanol;
2-(2,4-difluorophenyl)-1-(1H-1,2,4-triazol-1-yl)-3-{N-methyl-N-[4-(trifluoromethyl)benzoyl]amino}-2-butanol; and
2-(2,4-difluorophenyl)-3-{N-[2-fluoro-4-(trifluoromethyl)benzoyl]-N-methylamino}-1-(1H-1,2,4-triazol-1-yl)-2-butanol;
or a pharmaceutically acceptable acid addition salt thereof.

**41.** A process for preparing a compound according to any one of Claims 1 to 33, which process comprises:
(a) reacting a compound of formula (IIIA):

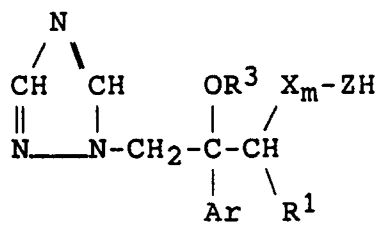

(IIIA)

[in which:
$R^1$, $R^3$, X, m and Ar are as defined in Claim 1; and
Z represents an oxygen or sulphur atom or a group of formula -NH- or $-N(CHR^8R^9)-$, in which $R^8$ and $R^9$ are the same or different and each represents a hydrogen atom or a methyl or ethyl group]
with a compound of formula (V):

$R^{2'}$-COOH (V)

(in which $R^{2'}$ represents any of the groups defined in Claim 1 for $R^2$ or represents a group of formula $R^2$-CH=CH-, and $R^2$ is as defined in Claim 1) or with a reactive derivative thereof, to give a compound of formula (I'):

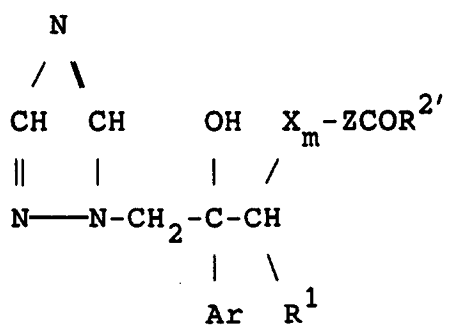

(I')

(in which $R^1$, $R^{2'}$, Ar, X, Z and m are as defined above);
or
(b) reacting a compound of formula (X'):

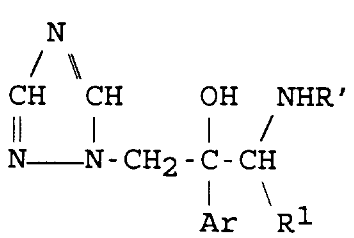

(X')

(in which: $R^1$ and Ar are as defined above, and R' represents a hydrogen atom or $R^2$) with formaldehyde, to give a compound of formula (X''):

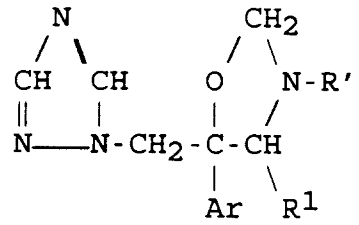

(X'')

(in which $R^1$, Ar and R' are as defined above)
and, where R' represents a hydrogen atom, reacting said compound of formula (X'') with a compound of formula (XVII):

$R^2$-(CH = CH)$_q$-COOH (XVII)

(in which $R^2$ is as defined above, and q is 0 or 1) or with a reactive derivative thereof, to give a compound of formula (Ig):

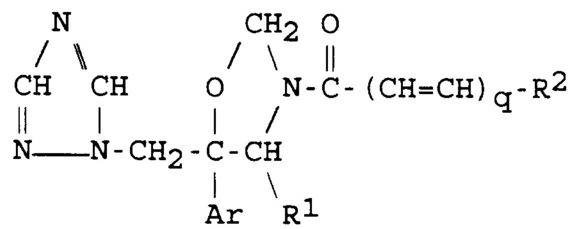

(Ig)

(in which $R^1$, $R^2$, Ar and q are as defined above);
or
(c) reacting said compound of formula (IIIA) in which Z represents an oxygen atom with a compound of formula (V'):

$R^4$-$SO_2$-OH (V')

(in which $R^4$ is as defined above) or with a reactive derivative thereof, to give a compound of formula (IIIB):

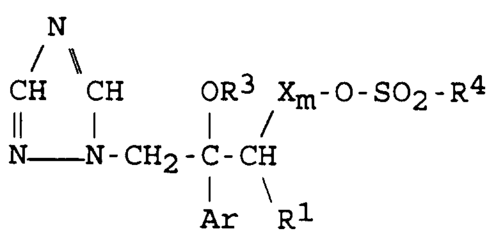

(IIIB)

(in which $R^1$, $R^3$, $R^4$, X, m and Ar are as defined above);
or
(d) reacting said compound of formula (IIIA) in which Z represents a group of formula -NH- with phthalic acid, a substituted phthalic acid in which the substituent is at least one of substituents (a), or a reactive derivative of said phthalic acid or substituted phthalic acid, to give a compound of formula (I) in which -Y-$R^2$ represents the phthalimido group or a substituted phthalimido group;
or
(e) reacting a compound of formula (IIIC):

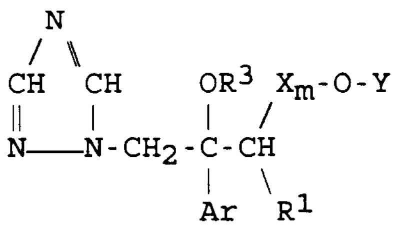

(IIIC)

(in which $R^1$, $R^3$, X, m and Ar are as defined above, and Y represents a hydrogen atom or a hydroxy-protecting group) or a corresponding oxirane compound in which m is 0, Y represents a hydrogen atom and the two hydroxy groups are condensed to form an oxirane group with an alkali metal azide to give a compound of formula (IIID):

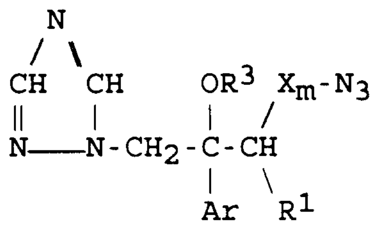

(IIID)

(in which $R^1$, $R^3$, X, m and Ar are as defined above).

**42.** The use for the manufacture of a medicament for the treatment or prophylaxis of fungal infections of a compound of formula (I), as defined in claim 35, or of a pharmaceutically acceptable acid addition salt thereof.

**43.** The use according to Claim 42 of a compound according to any one of Claims 1 to 33.

**44.** The use according to Claim 42 of any one of the compounds referred to in Claim 40.

**45.** The use for the manufacture of a fungicide for agrochemical use of a compound of formula (I), as defined in Claim 35, or of an acid addition salt thereof.

**46.** The use according to Claim 45 of a compound according to any one of Claims 1 to 33.

**47.** The use according to Claim 45 of any one of the compounds referred to in Claim 37.

**48.** A process for protecting plants, seeds, and portions of plants or seeds from fungal infection, which comprises applying to said plants, seeds, portions of plants or seeds or a locus including the same at least one compound of formula (I) or an acid addition salt thereof, as defined in Claim 35.

**Claims for the following Contracting State : ES**

**1.** A process for preparing a compound of formula (I):

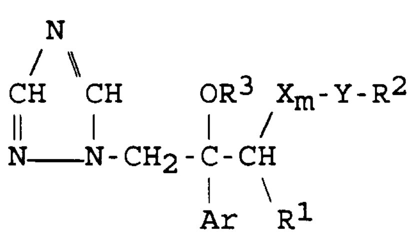

(I)

[in which:

Ar represents a phenyl group or a phenyl group having one or two halogen and/or trifluoromethyl substituents;

$R^1$ represents a $C_1$ - $C_6$ alkyl group or, where $R^3$ and $-X_m-Y-R^2$ together represent said group of formula (II), $R^1$ represents a hydrogen atom or a $C_1$ - $C_6$ alkyl group;

X represents a $C_1$ - $C_6$ alkylene group, a $C_2$ - $C_6$ aliphatic hydrocarbon group having one or two carbon-carbon double bonds, a $C_2$ - $C_6$ aliphatic hydrocarbon group having one or two carbon-carbon triple bonds, a $C_3$ - $C_6$ cycloalkylene group, a $C_3$ - $C_6$ cycloalkylene group having one or two carbon-carbon double bonds, a ($C_1$ - $C_3$ alkylene)-($C_3$ - $C_6$ cycloalkylene) group or a ($C_3$ - $C_6$ cycloalkylene)-($C_1$ - $C_3$ alkylene) group;

m is 0 or 1;

$-Y-R^2$ represents the azido group, the phthalimido group, the 1-oxo-2,3-dihydro-2-isoindolyl group, or the phthalimido group or 1-oxo-2,3-dihydro-2-isoindolyl group having at least one of substituents (a), defined below;

or

Y represents a group of formula $-N(R^5)CO-$, $-N(R^5)CO-CH=CH-$, -O-CO-, -O-CO-CH=CH-, -S-CO- or -S-CO-CH=CH-, in which:

$R^5$ represents a hydrogen atom or a $C_1$ - $C_4$ alkyl group;

$R^2$ represents a $C_1$ - $C_6$ alkyl group, a $C_1$ - $C_6$ haloalkyl group, a phenyl group, a phenyl group having at least one of substituents (a), defined below, a naphthyl group or a heterocyclic group having 5 or 6 ring atoms of which from 1 to 3 are nitrogen and/or oxygen and/or sulphur hetero-atoms, said heterocyclic group being unsubstituted or having from 1 to 3 of substituents (b), defined below; and

$R^3$ represents a hydrogen atom;

or

$R^3$ and $-X_m-Y-R^2$ together represent a group of formula (II):

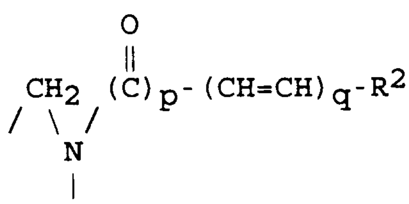

(II)

in which:

$R^2$ is as defined above;

p is 0 or 1; and

q is 0 or 1;

substituents (a):

$C_1$ - $C_4$ alkyl groups, $C_1$ - $C_4$ haloalkyl groups, $C_1$ - $C_4$ alkoxy groups, $C_1$ - $C_4$ haloalkoxy groups, halogen atoms, nitro groups, cyano groups, hydroxy groups, benzyloxy groups, hydroxymethyl groups, groups of formula $-CH_2-OCO-R^7$, groups of formula $-CO-R^6$, groups of formula $-COOR^7$, groups of formula $-SO_rR^7$, amino groups, mono- and di- alkylamino groups in which each alkyl group is $C_1$ - $C_4$ and carboxylic acylamino groups;

substituents (b):

$C_1$ - $C_4$ alkyl groups, $C_1$ - $C_4$ haloalkyl groups, halogen atoms, cyano groups and nitro groups;

$R^6$ represents a $C_1$ - $C_6$ alkyl group or a $C_1$ - $C_6$ haloalkyl group;

$R^7$ represents a $C_1$ - $C_6$ alkyl group;

r is 0, 1 or 2]; or

an acid addition salt thereof; provided that when m is O and Y is a group of formula $-N(R^5)CO-$, $R^5$ must be hydrogen. which process comprises:

(a) reacting a compound of formula (IIIA):

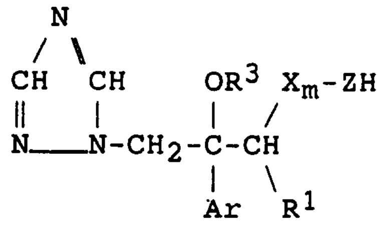

(IIIA)

[in which:
$R^1$, $R^3$, X, m and Ar are as defined above; and
Z represents an oxygen or sulphur atom or a group of formula -NH- or -N($CHR^8R^9$)-, in which $R^8$ and $R^9$ are the same or different and each represents a hydrogen atom or a methyl or ethyl group] with a compound of formula (V):

$R^{2'}$-COOH (V)

(in which $R^{2'}$ represents any of the groups defined above for $R^2$ or represents a group of formula $R^2$-CH=CH-, and $R^2$ is as defined above) or with a reactive derivative thereof, to give a compound of formula (I'):

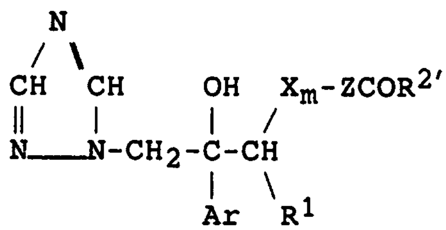

(I')

(in which $R^1$, $R^{2'}$, Ar, X, Z and m are as defined above);
(b) reacting a compound of formula (X'):

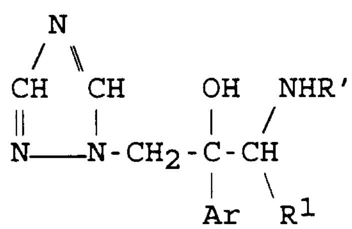

(X')

(in which: $R^1$ and Ar are as defined above, and R' represents a hydrogen atom or $R^2$) with formaldehyde, to give a compound of formula (X''):

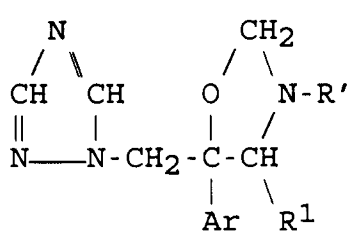

(X'')

(in which $R^1$, Ar and R' are as defined above)
and, where R' represents a hydrogen atom, reacting said compound of formula (X'') with a compound of formula (XVII):

$R^2$-(CH = CH)$_q$-COOH (XVII)

(in which $R^2$ is as defined above, and q is 0 or 1) or with a reactive derivative thereof, to give a compound of formula (Ig):

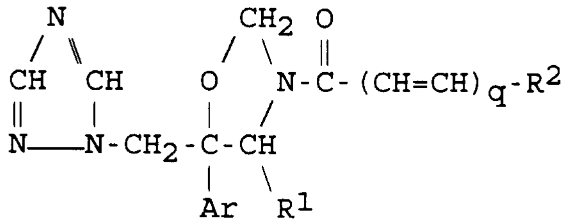

(Ig)

(in which $R^1$, $R^2$, Ar and q are as defined above);

or

(c) reacting said compound of formula (IIIA) in which Z represents an oxygen atom with a compound of formula (V'):

$R^4$-$SO_2$-OH (V')

(in which $R^4$ is as defined above) or with a reactive derivative thereof, to give a compound of formula (IIIB):

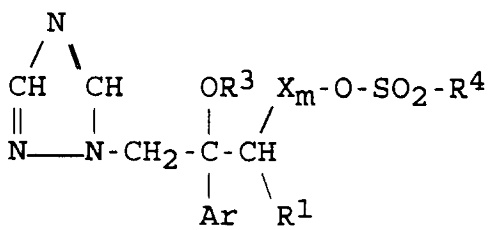

(IIIB)

(in which $R^1$, $R^3$, $R^4$, X, m and Ar are as defined above);

or

(d) reacting said compound of formula (IIIA) in which Z represents a group of formula -NH- with phthalic acid, a substituted phthalic acid in which the substituent is at least one of substituents (a), or a reactive derivative of said phthalic acid or substituted phthalic acid, to give a compound of formula (I) in which -Y-$R^2$ represents the phthalimido group or a substituted phthalimido group;

or

(e) reacting a compound of formula (IIIC):

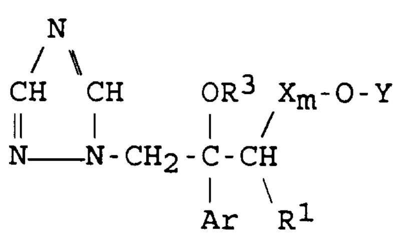

(IIIC)

(in which $R^1$, $R^3$, X, m and Ar are as defined above, and Y represents a hydrogen atom or a hydroxy-protecting group) or a corresponding oxirane compound in which m is 0, Y represents a hydrogen atom and the two hydroxy groups are condensed to form an oxirane group with an alkali metal azide to give a compound of formula (IIID):

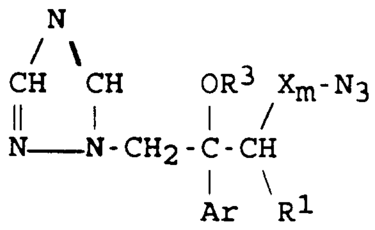

(IIID)

(in which $R^1$, $R^3$, X, m and Ar are as defined above).

**2.** A process according to Claim 1, in which Ar in said compound of formula (IIIA) or (X') represents a phenyl group having one or two halogen substituents.

**3.** A process according to Claim 1, in which Ar in said compound of formula (IIIA) or (X') represents a phenyl group having one or two fluorine and/or chlorine substituents.

**4.** A process according to Claim 1, in which Ar in said compound of formula (IIIA) or (X') represents a 4-chlorophenyl group, a 4-fluorophenyl group, a 2,4-dichlorophenyl group, a 2,4-difluorophenyl group, a 2-chloro-4-fluorophenyl group or a 4-chloro-2-fluorophenyl group.

**5.** A process according to any one of Claims 1 to 4, in which $R^1$ in said compound of formula (IIIA) or (X') represents a $C_1$ - $C_3$ alkyl group.

**6.** A process according to any one of Claims 1 to 4, in which $R^1$ in said compound of formula (IIIA) or (X') represents a methyl or ethyl group.

**7.** A process according to any one of Claims 1 to 6, in which X in said compound of formula (IIIA) represents a $C_1$ or $C_2$ alkylene group.

**8.** A process according to any one of Claims 1 to 6, in which m in said compound of formula (IIIA) is 0.

**9.** A process according to any one of Claims 1 to 8, in which the reagents and reaction conditions are so chosen as to prepare a compound of formula (I) in which $-Y-R^2$ represents the azido group.

**10.** A process according to any one of Claims 1 to 8, in which the reagents and reaction conditions are so chosen as to prepare a compound of formula (I) in which Y represents a group of formula $-N(R^5)CO-$, $-N(R^5)CO-CH=CH-$, -S-CO- or -S-CO-CH=CH-, in which:
$R^5$ represents a hydrogen atom or a $C_1$ - $C_4$ alkyl group.

**11.** A process according to any one of Claims 1 to 8 and 10, in which the reagents and reaction conditions are so chosen as to prepare a compound of formula (I) in which $R^2$ represents a phenyl group having one or two substituents selected from halogen atoms and trifluoromethyl, trifluoromethoxy and cyano groups.

**12.** A process according to Claim 1, in which the reagents and reaction conditions are so chosen as to prepare a compound of formula (I) in which:
Ar represents a phenyl group having one or two halogen substituents;
$R^1$ represents a $C_1$ - $C_3$ alkyl group;
m is 0 or X represents a $C_1$ or $C_2$ alkylene group;
Y represents a group of formula $-N(R^5)CO-$, $-N(R^5)CO-CH=CH-$, -S-CO- or -S-CO-CH=CH-, in which:
$R^5$ represents a hydrogen atom or a $C_1$ - $C_4$ alkyl group; and
$R^2$ represents a phenyl group having one or two substituents selected from halogen atoms and trifluoromethyl, trifluoromethoxy and cyano groups;
or
$-Y-R^2$ represents an azido group.

**13.** A process according to Claim 1, in which the reagents and reaction conditions are so chosen as to prepare a compound of formula (I) in which:
Ar represents a phenyl group having one or two fluorine and/or chlorine substituents;
$R^1$ represents a methyl or ethyl group;
m is 0 or X represents a $C_1$ or $C_2$ alkylene group;
Y represents a group of formula -N($R^5$)CO-, -N($R^5$)CO-CH = CH-, -S-CO- or -S-CO-CH = CH-, in which:
$R^5$ represents a hydrogen atom or a $C_1$ - $C_4$ alkyl group; and
$R^2$ represents a phenyl group having one or two substituents selected from halogen atoms and trifluoromethyl, trifluoromethoxy and cyano groups;
or
-Y-$R^2$ represents an azido group.

**14.** A process according to Claim 1, in which the reagents and reaction conditions are so chosen as to prepare a compound of formula (I) in which:
$R^1$ represents a methyl or ethyl group;
m is 0 or X represents a $C_1$ or $C_2$ alkylene group;
Y represents a group of formula -N($R^5$)CO-, -N($R^5$)CO-CH = CH-, -S-CO- or -S-CO-CH = CH-, in which:
$R^5$ represents a hydrogen atom or a $C_1$ - $C_4$ alkyl group;
$R^2$ represents a phenyl group having one or two substituents selected from halogen atoms and trifluoromethyl, trifluoromethoxy and cyano groups;
or
-Y-$R^2$ represents an azido group; and
Ar represents a 4-chlorophenyl group, a 4-fluorophenyl group, a 2,4-dichlorophenyl group, a 2,4-difluorophenyl group, a 2-chloro-4-fluorophenyl group or a 4-chloro-2-fluorophenyl group.

**15.** A process according to Claim 1, in which the reagents and reaction conditions are so chosen as to prepare:
2-(2,4-Dichlorophenyl)-1-(1H-1,2,4-triazol-1-yl)-3-[4-(trifluoromethyl)benzoylamino]-2-butanol;
2-(2,4-Difluorophenyl)-3-[2-fluoro-4-(trifluoromethyl)benzoylamino]-1-(1H-1,2,4-triazol-1-yl)-2-butanol;
3-(4-Chlorobenzoylamino)-2-(2,4-difluorophenyl)-1-(1H-1,2,4-triazol-1-yl)-2-pentanol;
3-Azido-2-(2,4-difluorophenyl)-1-(1H-1,2,4-triazol-1-yl)-2-butanol;
3-Azido-2-(4-chloro-2-fluorophenyl)-1-(1H-1,2,4-triazol-1-yl)-2-butanol;
3-Azido-2-(2,4-difluorophenyl)-1-(1H-1,2,4-triazol-1-yl)-2-pentanol;
3-Azido-2-(2-chloro-4-fluorophenyl)-1-(1H-1,2,4-triazol-1-yl)-2-pentanol;
4-Azido-2-(2,4-difluorophenyl)-3-methyl-1-(1H-1,2,4-triazol-1-yl)-2-pentanol;
4-Azido-2-(2,4-difluorophenyl)-3-methyl-1-(1H-1,2,4-triazol-1-yl)-2-butanol;
4-Azido-2-(2,4-dichlorophenyl)-3-methyl-1-(1H-1,2,4-triazol-1-yl)-2-butanol;
3-Azido-2-(4-chlorophenyl)-1-(1H-1,2,4-triazol-1-yl)-2-pentanol;
2-(4-Chlorophenyl)-1-(1H-1,2,4-triazol-1-yl)-3-[4-(trifluoromethyl)benzoylamino]-2-butanol;
2-(2,4-Difluorophenyl)-1-(1H-1,2,4-triazol-1-yl)-3-[4-(trifluoromethyl)benzoylamino]-2-butanol;
3-(4-Cyanobenzoylamino)-2-(2,4-difluorophenyl)-1-(1H-1,2,4-triazol-1-yl)-2-butanol;
2-(4-Chlorophenyl)-3-[2-fluoro-4-(trifluoromethyl)benzoylamino]-1-(1H-1,2,4-triazol-1-yl)-2-butanol;
2-(2,4-Dichlorophenyl)-1-(1H-1,2,4-triazol-1-yl)-3-[4-(trifluoromethyl)benzoylthio]-2-butanol;
3-(4-Chlorocinnamoylamino)-2-(2,4-difluorophenyl)-1-(1H-1,2,4-triazol-1-yl)-2-butanol;
2-(2,4-Difluorophenyl)-1-(1H-1,2,4-triazol-1-yl)-3-{N-methyl-N-[4-(trifluoromethyl)benzoyl]amino}-2-butanol;
or
2-(2,4-Difluorophonyl)-3-{N-[2-fluoro-4-(trifluoromethyl)benzoyl]-N-methylamino}-1-(1H-1,2,4-triazol-1-y1)-2-butanol;
or an acid addition salt thereof.

**16.** A process for preparing a compound of formula (IX)

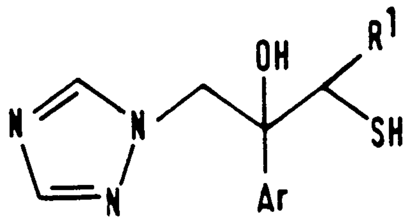

(IX)

in which:

Ar represents a phenyl group or a phenyl group having one or two halogen and/or trifluoromethyl substituents:

$R^1$ represents a $C_1$ - $C_6$ alkyl group; and acid addition salts thereof, which comprises contacting a compound of formula (VIII)

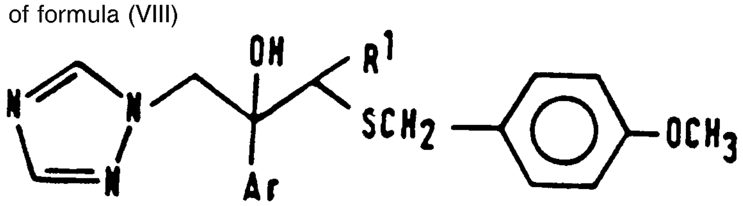

(VIII)

in which Ar and $R^1$ are as defined above, with a strong acid.

**17.** A process for the manufacture of an agrochemical composition comprising mixing an agriculturally acceptable carrier or diluent with at least one fungicide, in which the fungicide is at least one compound of formula (I):

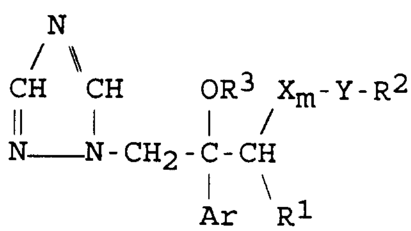

(I)

in which:

Ar represents a phenyl group or a phenyl group having one or two halogen atoms and/or trifluoromethyl substituents;

$R^1$ represents a $C_1$ - $C_6$ alkyl group or, where $R^3$ and $-X_m$-Y-$R^2$ together represent said group of formula (II), $R^1$ represents a hydrogen atom or a $C_1$ - $C_6$ alkyl group;

X represents a $C_1$ - $C_6$ alkylene group, a $C_2$ - $C_6$ aliphatic hydrocarbon group having one or two carbon-carbon double bonds, a $C_2$ - $C_6$ aliphatic hydrocarbon group having one or two carbon-carbon triple bonds, a $C_3$ - $C_6$ cycloalkylene group, a $C_3$ - $C_6$ cycloalkylene group having one or two carbon-carbon double bonds, a ($C_1$ - $C_3$ alkylene)-($C_3$ - $C_6$ cycloalkylene) group or a ($C_3$ - $C_6$ cycloalkylene)-($C_1$ - $C_3$ alkylene) group;

m is 0 or 1;

-Y-$R^2$ represents the azido group, the phthalimido group, the 1-oxo-2,3-dihydro-2-isoindolyl group, the phthalimido group or 1-oxo-2,3-dihydro-2-isoindolyl group having at least one of substituents (a), defined below, a protected hydroxy group or a group of formula $-OSO_2R^4$, in which:

$R^4$ represents a $C_1$ - $C_4$ alkyl group, a $C_1$ - $C_4$ haloalkyl group, a phenyl group or a phenyl group having one substituent selected from nitro groups, halogen atoms and $C_1$ - $C_4$ alkyl groups;

or

Y represents a group of formula $-N(R^5)CO-$, $-N(R^5)CO-CH=CH-$, -O-CO-, -O-CO-CH=CH-, -S-CO- or -S-CO-CH=CH-, in which:

$R^5$ represents a hydrogen atom or a $C_1$ - $C_4$ alkyl group;

$R^2$ represents a $C_1$ - $C_6$ alkyl group, a $C_1$ - $C_6$ haloalkyl group, a phenyl group, a phenyl group having at least one of substituents (a), defined below, a naphthyl group or a heterocyclic group having 5 or 6

ring atoms of which from 1 to 3 are nitrogen and/or oxygen and/or sulphur hetero-atoms, said heterocyclic group being unsubstituted or having from 1 to 3 of substituents (b), defined below; and
$R^3$ represents a hydrogen atom;
or
$R^3$ and $-X_m-Y-R^2$ together represent a group of formula (II):

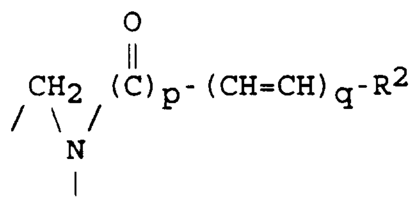

(II)

in which:
$R^2$ is as defined above;
p is 0 or 1; and
q is 0 or 1;
substituents (a):
$C_1$ - $C_4$ alkyl groups, $C_1$ - $C_4$ haloalkyl groups, $C_1$ - $C_4$ alkoxy groups, $C_1$ - $C_4$ haloalkoxy groups, halogen atoms, nitro groups, cyano groups, hydroxy groups, benzyloxy groups, hydroxymethyl groups, groups of formula $-CH_2-OCO-R^7$, groups of formula $-CO-R^6$, groups of formula $-COOR^7$, groups of formula $-SO_rR^7$, amino groups, mono- and di- alkylamino groups in which each alkyl group is $C_1$ - $C_4$ and carboxylic acylamino groups;
substituents (b):
$C_1$ - $C_4$ alkyl groups, $C_1$ - $C_4$ haloalkyl groups, halogen atoms, cyano groups and nitro groups;
$R^6$ represents a $C_1$ - $C_6$ alkyl group or a $C_1$ - $C_6$ haloalkyl group;
$R^7$ represents a $C_1$ - $C_6$ alkyl group;
r is 0, 1 or 2;
or an acid addition salt thereof.

**18.** A process according to Claim 17, in which said fungicide is a compound according to any one of Claims 1 to 15.

**19.** A process according to Claim 17, in which said fungicide is at least one of:
2-(2,4-dichlorophenyl)-1-(1H-1,2,4-triazol-1-yl)-3-[4-(trifluoromethyl)benzoylamino]-2-butanol;
2-(2,4-difluorophenyl)-3-[2-fluoro-4-(trifluoromethyl)benzoylamino]-1-(1H-1,2,4-triazol-1-yl)-2-butanol;
3-(4-chlorobenzoylamino)-2-(2,4-difluorophenyl)-1-(1H-1,2,4-triazol-1-yl)-2-pentanol;
3-azido-2-(2,4-difluorophenyl)-1-(1H-1,2,4-triazol-1-yl)-2-butanol;
3-azido-2-(4-chloro-2-fluorophenyl)-1-(1H-1,2,4-triazol-1-yl)-2-butanol;
3-azido-2-(2,4-difluorophenyl)-1-(1H-1,2,4-triazol-1-yl)-2-pentanol;
3-azido-2-(2-chloro-4-fluorophenyl)-1-(1H-1,2,4-triazol-1-yl)-2-pentanol;
4-azido-2-(2,4-difluorophenyl)-3-methyl-1-(1H-1,2,4-triazol-1-yl)-2-pentanol;
4-azido-2-(2,4-difluorophenyl)-3-methyl-1-(1H-1,2,4-triazol-1-yl)-2-butanol;
4-azido-2-(2,4-dichlorophenyl)-3-methyl-1-(1H-1,2,4-triazol-1-yl)-2-butanol; and
3-azido-2-(4-chlorophenyl)-1-(1H-1,2,4-triazol-1-yl)-2-pentanol;
or an acid addition salt thereof.

**20.** A process for the manufacture of a fungicidal pharmaceutical composition comprising mixing a pharmaceutically acceptable carrier or diluent with at least one fungicide, in which the fungicide is at least one compound of formula (I), as defined in Claim 17.

**21.** A process according to Claim 20, in which said fungicide is a compound according to any one of Claims 1 to 15.

**22.** A process according to Claim 21, in which said fungicide is at least one of:
2-(4-chlorophenyl)-1-(1H-1,2,4-triazol-1-yl)-3-[4-(trifluoromethyl)benzoylamino]-2-butanol;
2-(2,4-difluorophenyl)-1-(1H-1,2,4-triazol-1-yl)-3-[4-(trifluoromethyl)benzoylamino]-2-butanol;

2-(2,4-difluorophenyl)-3-[2-fluoro-4-(trifluoromethyl)benzoylamino]-1-(1H-1,2,4-triazol-1-yl)-2-butanol;
3-(4-cyanobenzoylamino)-2-(2,4-difluorophenyl)-1-(1H-1,2,4-triazol-1-yl)-2-butanol;
2-(4-chlorophenyl)-3-[2-fluoro-4-(trifluoromethyl)benzoylamino]-1-(1H-1,2,4-triazol-1-yl)-2-butanol;
2-(2,4-dichlorophenyl)-1-(1H-1,2,4-triazol-1-yl)-3-[4-(trifluoromethyl)benzoylthio]-2-butanol;
3-(4-chlorocinnamoylamino)-2-(2,4-difluorophenyl)-1-(1H-1,2,4-triazol-1-yl)-2-butanol;
2-(2,4-difluorophenyl)-1-(1H-1,2,4-triazol-1-yl)-3-{N-methyl-N-[4-(trifluoromethyl)benzoyl]amino}-2-butanol; and
2-(2,4-difluorophenyl)-3-{N-[2-fluoro-4-(trifluoromethyl)benzoyl]-N-methylamino}-1-(1H-1,2,4-triazol-1-yl)-2-butanol;
or a pharmaceutically acceptable acid addition salt thereof.

**23.** A process for protecting plants, seeds, and portions of plants or seeds from fungal infection, which comprises applying to said plants, seeds, portions of plants or seeds or a locus including the same at least one compound of formula (I) or an acid addition salt thereof, as defined in Claim 17.

**24.** The use for the manufacture of a medicament for the treatment or prophylaxis of fungal infections of a compound of formula (I), as defined in Claim 17, or of a pharmaceutically acceptable acid addition salt thereof.

**25.** The use according to Claim 24 of a compound of formula (I), or an acid addition salt thereof, as defined in any one of Claims 1 to 15.

**26.** The use according to Claim 24 of any one of the compounds referred to in Claim 22.

**27.** The use for the manufacture of a fungicide for agrochemical use of a compound of formula (I), as defined in Claim 17, or of an acid addition salt thereof.

**28.** The use according to Claim 27 of a compound of formula (I), or an acid addition salt thereof, as defined in any one of Claims 1 to 15.

**29.** The use according to Claim 27 of any one of the compounds referred to in Claim 19.

**Claims for the following Contracting State : GR**

**1.** Compounds for agrochemical use having the formula (I):

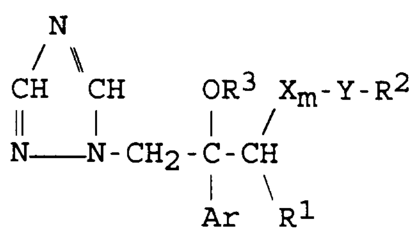

(I)

in which:
Ar represents a phenyl group or a phenyl group having one or two halogen and/or trifluoromethyl substituents;
$R^1$ represents a $C_1$ - $C_6$ alkyl group or, where $R^3$ and $-X_m$-Y-$R^2$ together represent said group of formula (II), $R^1$ represents a hydrogen atom or a $C_1$ - $C_6$ alkyl group;
X represents a $C_1$ - $C_6$ alkylene group, a $C_2$ - $C_6$ aliphatic hydrocarbon group having one or two carbon-carbon double bonds, a $C_2$ - $C_6$ aliphatic hydrocarbon group having one or two carbon-carbon triple bonds, a $C_3$ - $C_6$ cycloalkylene group, a $C_3$ - $C_6$ cycloalkylene group having one or two carbon-carbon double bonds, a ($C_1$ - $C_3$ alkylene)-($C_3$ - $C_6$ cycloalkylene) group or a ($C_3$ - $C_6$ cycloalkylene)-($C_1$ - $C_3$ alkylene) group;
m is 0 or 1;
-Y-$R^2$ represents the azido group, the phthalimido group, the 1-oxo-2,3-dihydro-2-isoindolyl group, or the phthalimido group or 1-oxo-2,3-dihydro-2-isoindolyl group having at least one of substituents (a),

defined below;

or

Y represents a group of formula $-N(R^5)CO-$, $-N(R^5)CO-CH=CH-$, -O-CO-, -O-CO-CH=CH-, -S-CO- or -S-CO-CH=CH-, in which:

$R^5$ represents a hydrogen atom or a $C_1$ - $C_4$ alkyl group;

$R^2$ represents a $C_1$ - $C_6$ alkyl group, a $C_1$ - $C_6$ haloalkyl group, a phenyl group, a phenyl group having at least one of substituents (a), defined below, a naphthyl group or a heterocyclic group having 5 or 6 ring atoms of which from 1 to 3 are nitrogen and/or oxygen and/or sulphur hetero-atoms, said heterocyclic group being unsubstituted or having from 1 to 3 of substituents (b), defined below; and

$R^3$ represents a hydrogen atom;

or

$R^3$ and $-X_m-Y-R^2$ together represent a group of formula (II):

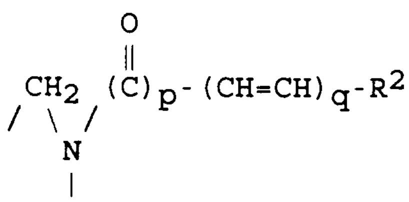

(II)

in which:

$R^2$ is as defined above;

p is 0 or 1; and

q is 0 or 1;

substituents (a):

$C_1$ - $C_4$ alkyl groups, $C_1$ - $C_4$ haloalkyl groups, $C_1$ - $C_4$ alkoxy groups, $C_1$ - $C_4$ haloalkoxy groups, halogen atoms, nitro groups, cyano groups, hydroxy groups, benzyloxy groups, hydroxymethyl groups, groups of formula $-CH_2-OCO-R^7$, groups of formula $-CO-R^6$, groups of formula $-COOR^7$, groups of formula $-SO_rR^7$, amino groups, mono- and di- alkylamino groups in which each alkyl group is $C_1$ - $C_4$ and carboxylic acylamino groups;

substituents (b):

$C_1$ - $C_4$ alkyl groups, $C_1$ - $C_4$ haloalkyl groups, halogen atoms, cyano groups and nitro groups;

$R^6$ represents a $C_1$ - $C_6$ alkyl group or a $C_1$ - $C_6$ haloalkyl group;

$R^7$ represents a $C_1$ - $C_6$ alkyl group;

r is 0, 1 or 2; and

acid addition salts thereof; provided that, when m is O and Y is a group of formula $-N(R^5)CO-$, $R^5$ must be hydrogen.

2. Compounds according to Claim 1, in which Ar represents a phenyl group having one or two halogen substituents.

3. Compounds according to Claim 1, in which Ar represents a phenyl group having one or two fluorine and/or chlorine substituents.

4. Compounds according to Claim 1, in which Ar represents a 4-chlorophenyl group, a 4-fluorophenyl group, a 2,4-dichlorophenyl group, a 2,4-difluorophenyl group, a 2-chloro-4-fluorophenyl group or a 4-chloro-2-fluorophenyl group.

5. Compounds according to any one of Claims 1 to 4, in which $R^1$ represents a $C_1$ - $C_3$ alkyl group.

6. Compounds according to any one of Claims 1 to 4, in which $R^1$ represents a methyl or ethyl group.

7. Compounds according to any one of Claims 1 to 6, in which X represents a $C_1$ or $C_2$ alkylene group.

8. Compounds according to any one of Claims 1 to 6, in which m is 0.

**9.** Compounds according to any one of Claims 1 to 8, in which -Y-$R^2$ represents the azido group.

**10.** Compounds according to any one of Claims 1 to 8, in which Y represents a group of formula -N($R^5$)CO-, -N($R^5$)CO-CH=CH-, -S-CO- or -S-CO-CH=CH-, in which:
$R^5$ represents a hydrogen atom or a $C_1$ - $C_4$ alkyl group.

**11.** Compounds according to any one of Claims 1 to 8 and 10, in which $R^2$ represents a phenyl group having one or two substituents selected from halogen atoms and trifluoromethyl, trifluoromethoxy and cyano groups.

**12.** Compounds according to Claim 1, in which:
Ar represents a phenyl group having one or two halogen substituents;
$R^1$ represents a $C_1$ - $C_3$ alkyl group;
m is 0 or X represents a $C_1$ or $C_2$ alkylene group;
Y represents a group of formula -N($R^5$)CO-, -N($R^5$)CO-CH=CH-, -S-CO- or -S-CO-CH=CH-, in which:
$R^5$ represents a hydrogen atom or a $C_1$ - $C_4$ alkyl group; and
$R^2$ represents a phenyl group having one or two substituents selected from halogen atoms and trifluoromethyl, trifluoromethoxy and cyano groups;
or
-Y-$R^2$ represents an azido group.

**13.** Compounds according to Claim 1, in which:
Ar represents a phenyl group having one or two fluorine and/or chlorine substituents;
$R^1$ represents a methyl or ethyl group;
m is 0 or X represents a $C_1$ or $C_2$ alkylene group;
Y represents a group of formula -N($R^5$)CO-, -N($R^5$)CO-CH=CH-, -S-CO- or -S-CO-CH=CH-, in which:
$R^5$ represents a hydrogen atom or a $C_1$ - $C_4$ alkyl group; and
$R^2$ represents a phenyl group having one or two substituents selected from halogen atoms and trifluoromethyl, trifluoromethoxy and cyano groups;
or
-Y-$R^2$ represents an azido group.

**14.** Compounds according to Claim 1, in which:
$R^1$ represents a methyl or ethyl group;
m is 0 or X represents a $C_1$ or $C_2$ alkylene group;
Y represents a group of formula -N($R^5$)CO-, -N($R^5$)CO-CH=CH-, -S-CO- or -S-CO-CH=CH-, in which:
$R^5$ represents a hydrogen atom or a $C_1$ - $C_4$ alkyl group;
$R^2$ represents a phenyl group having one or two substituents selected from halogen atoms and trifluoromethyl, trifluoromethoxy and cyano groups;
or
-Y-$R^2$ represents an azido group; and
Ar represents a 4-chlorophenyl group, a 4-fluorophenyl group, a 2,4-dichlorophenyl group, a 2,4-difluorophenyl group, a 2-chloro-4-fluorophenyl group or a 4-chloro-2-fluorophenyl group.

**15.** Compounds according to Claim 1, selected from:
2-(2,4-Dichlorophenyl)-1-(1H-1,2,4-triazol-1-yl)-3-[4-(trifluoromethyl)benzoylamino]-2-butanol;
2-(2,4-Difluorophenyl)-3-[2-fluoro-4-(trifluoromethyl)benzoylamino]-1-(1H-1,2,4-triazol-1-yl)-2-butanol;
3-(4-Chlorobenzoylamino)-2-(2,4-difluorophenyl)-1-(1H-1,2,4-triazol-1-yl)-2-pentanol;
3-Azido-2-(2,4-difluorophonyl)-1-(1H-1,2,4-triazol-1-yl)-2-butanol;
3-Azido-2-(4-chloro-2-fluorophenyl)-1-(1H-1,2,4-triazol-1-yl)-2-butanol;
3-Azido-2-(2,4-difluorophenyl)-1-(1H-1,2,4-triazol-1-yl)-2-pentanol;
3-Azido-2-(2-chloro-4-fluorophenyl)-1-(1H-1,2,4-triazol-1-yl)-2-pentanol;
4-Azido-2-(2,4-difluorophenyl)-3-methyl-1-(1H-1,2,4-triazol-1-yl)-2-pentanol;
4-Azido-2-(2,4-difluorophenyl)-3-methyl-1-(1H-1,2,4-triazol-1-yl)-2-butanol;
4-Azido-2-(2,4-dichlorophenyl)-3-methyl-1-(1H-1,2,4-triazol-1-yl)-2-butanol;
3-Azido-2-(4-chlorophenyl)-1-(1H-1,2,4-triazol-1-yl)-2-pentanol;
2-(4-Chlorophenyl)-1-(1H-1,2,4-triazol-1-yl)-3-[4-(trifluoromethyl)benzoylamino]-2-butanol;
2-(2,4-Difluorophenyl)-1-(1H-1,2,4-triazol-1-yl)-3-[4-(trifluoromethyl)benzoylamino]-2-butanol;

3-(4-Cyanobenzoylamino)-2-(2,4-difluorophenyl)-1-(1H-1,2,4-triazol-1-yl)-2-butanol;
2-(4-Chlorophenyl)-3-[2-fluoro-4-(trifluoromethyl)benzoylamino]-1-(1H-1,2,4-triazol-1-yl)-2-butanol;
2-(2,4-Dichlorophenyl)-1-(1H-1,2,4-triazol-1-yl)-3-[4-(trifluoromethyl)benzoylthio]-2-butanol;
3-(4-Chlorocinnamoylamino)-2-(2,4-difluorophenyl)-1-(1H-1,2,4-triazol-1-yl)-2-butanol;
2-(2,4-Difluorophenyl)-1-(1H-1,2,4-triazol-1-yl)-3-{N-methyl-N-[4-(trifluoromethyl)benzoyl]amino}-2-butanol;
and
2-(2,4-Difluorophenyl)-3-{N-[2-fluoro-4-(trifluoromethyl)benzoyl]-N-methylamino}-1-(1H-1,2,4-triazol-1-yl)-2-butanol;
and acid addition salts thereof.

**16.** Compounds for agrochemical use having the formula (IX):

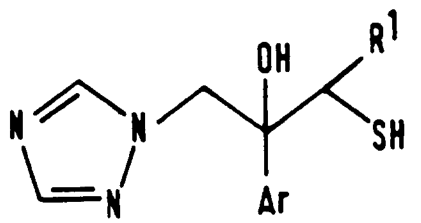

(IX)

in which:

Ar represents a phenyl group or a phenyl group having one or two halogen and/or trifluoromethyl substituents;

$R^1$ represents a $C_1$ - $C_6$ alkyl group: and acid addition salts thereof.

**17.** An agrochemical composition comprising an agriculturally acceptable carrier or diluent in admixture with at least one fungicide, in which the fungicide is at least one compound of formula (I):

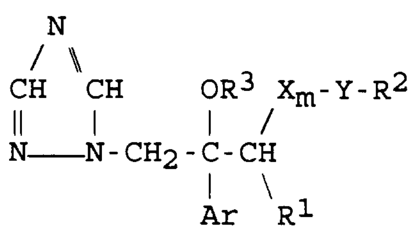

(I)

in which:

Ar represents a phenyl group or a phenyl group having one or two halogen atoms and/or trifluoromethyl substituents;

$R^1$ represents a $C_1$ - $C_6$ alkyl group or, where $R^3$ and $-X_m-Y-R^2$ together represent said group of formula (II), $R^1$ represents a hydrogen atom or a $C_1$ - $C_6$ alkyl group;

X represents a $C_1$ - $C_6$ alkylene group, a $C_2$ - $C_6$ aliphatic hydrocarbon group having one or two carbon-carbon double bonds, a $C_2$ - $C_6$ aliphatic hydrocarbon group having one or two carbon-carbon triple bonds, a $C_3$ - $C_6$ cycloalkylene group, a $C_3$ - $C_6$ cycloalkylene group having one or two carbon-carbon double bonds, a ($C_1$ - $C_3$ alkylene)-($C_3$ - $C_6$ cycloalkylene) group or a ($C_3$ - $C_6$ cycloalkylene)-($C_1$ - $C_3$ alkylene) group;

m is 0 or 1;

$-Y-R^2$ represents the azido group, the phthalimido group, the 1-oxo-2,3-dihydro-2-isoindolyl group, the phthalimido group or 1-oxo-2,3-dihydro-2-isoindolyl group having at least one of substituents (a), defined below, a protected hydroxy group or a group of formula $-OSO_2R^4$, in which:

$R^4$ represents a $C_1$ - $C_4$ alkyl group, a $C_1$ - $C_4$ haloalkyl group, a phenyl group or a phenyl group having one substituent selected from nitro groups, halogen atoms and $C_1$ - $C_4$ alkyl groups;

or

Y represents a group of formula $-N(R^5)CO-$, $-N(R^5)CO-CH=CH-$, $-O-CO-$, $-O-CO-CH=CH-$, $-S-CO-$ or $-S-CO-CH=CH-$, in which:

$R^5$ represents a hydrogen atom or a $C_1$ - $C_4$ alkyl group;

$R^2$ represents a $C_1$ - $C_6$ alkyl group, a $C_1$ - $C_6$ haloalkyl group, a phenyl group, a phenyl group having at least one of substituents (a), defined below, a naphthyl group or a heterocyclic group having 5 or 6 ring atoms of which from 1 to 3 are nitrogen and/or oxygen and/or sulphur hetero-atoms, said heterocyclic group being unsubstituted or having from 1 to 3 of substituents (b), defined below; and

$R^3$ represents a hydrogen atom;

or

$R^3$ and $-X_m-Y-R^2$ together represent a group of formula (II):

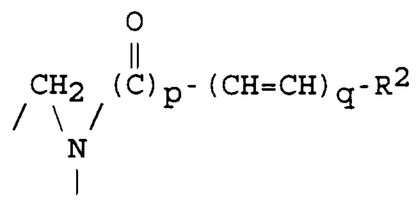

(II)

in which:

$R^2$ is as defined above;

p is 0 or 1; and

q is 0 or 1;

substituents (a):

$C_1$ - $C_4$ alkyl groups, $C_1$ - $C_4$ haloalkyl groups, $C_1$ - $C_4$ alkoxy groups, $C_1$ - $C_4$ haloalkoxy groups, halogen atoms, nitro groups, cyano groups, hydroxy groups, benzyloxy groups, hydroxymethyl groups, groups of formula $-CH_2-OCO-R^7$, groups of formula $-CO-R^6$, groups of formula $-COOR^7$, groups of formula $-SO_rR^7$, amino groups, mono- and di- alkylamino groups in which each alkyl group is $C_1$ - $C_4$ and carboxylic acylamino groups;

substituents (b):

$C_1$ - $C_4$ alkyl groups, $C_1$ - $C_4$ haloalkyl groups, halogen atoms, cyano groups and nitro groups;

$R^6$ represents a $C_1$ - $C_6$ alkyl group or a $C_1$ - $C_6$ haloalkyl group;

$R^7$ represents a $C_1$ - $C_6$ alkyl group;

r is 0, 1 or 2;

or an acid addition salt thereof.

**18.** A composition according to Claim 17, in which said fungicide is a compound according to any one of Claims 1 to 15.

**19.** A composition according to Claim 18, in which said fungicide is at least one of:
2-(2,4-dichlorophenyl)-1-(1H-1,2,4-triazol-1-yl)-3-[4-(trifluoromethyl)benzoylamino]-2-butanol;
2-(2,4-difluorophenyl)-3-[2-fluoro-4-(trifluoromethyl)benzoylamino]-1-(1H-1,2,4-triazol-1-yl)-2-butanol;
3-(4-chlorobenzoylamino)-2-(2,4-difluorophenyl)-1-(1H-1,2,4-triazol-1-yl)-2-pentanol;
3-azido-2-(2,4-difluorophenyl)-1-(1H-1,2,4-triazol-1-yl)-2-butanol;
3-azido-2-(4-chloro-2-fluorophenyl)-1-(1H-1,2,4-triazol-1-yl)-2-butanol;
3-azido-2-(2,4-difluorophenyl)-1-(1H-1,2,4-triazol-1-yl)-2-pentanol;
3-azido-2-(2-chloro-4-fluorophenyl)-1-(1H-1,2,4-triazol-1-yl)-2-pentanol;
4-azido-2-(2,4-difluorophenyl)-3-methyl-1-(1H-1,2,4-triazol-1-yl)-2-pentanol;
4-azido-2-(2,4-difluorophenyl)-3-methyl-1-(1H-1,2,4-triazol-1-yl)-2-butanol;
4-azido-2-(2,4-dichlorophenyl)-3-methyl-1-(1H-1,2,4-triazol-1-yl)-2-butanol; and
3-azido-2-(2,4-dichlorophenyl)-1-(1H-1,2,4-triazol-1-yl)-2-pentanol;
or an acid addition salt thereof.

**20.** A fungicidal pharmaceutical composition comprising a pharmaceutically acceptable carrier or diluent in admixture with at least one fungicide, in which the fungicide is at least one compound of formula (I), as defined in Claim 17.

**21.** A composition according to Claim 20, in which said fungicide is a compound as defined in any one of Claims 1 to 15.

**22.** A composition according to Claim 20, in which said fungicide is at least one of:
2-(4-chlorophenyl)-1-(1H-1,2,4-triazol-1-yl)-3-[4-(trifluoromethyl)benzoylamino]-2-butanol;
2-(2,4-difluorophenyl)-1-(1H-1,2,4-triazol-1-yl)-3-[4-(trifluoromethyl)benzoylamino]-2-butanol;
2-(2,4-difluorophenyl)-3-[2-fluoro-4-(trifluoromethyl)benzoylamino]-1-(1H-1,2,4-triazol-1-yl)-2-butanol;
3-(4-cyanobenzoylamino)-2-(2,4-difluorophenyl)-1-(1H-1,2,4-triazol-1-yl)-2-butanol;
2-(4-chlorophenyl)-3-[2-fluoro-4-(trifluoromethyl)benzoylamino]-1-(1H-1,2,4-triazol-1-yl)-2-butanol;
2-(2,4-dichlorophenyl)-1-(1H-1,2,4-triazol-1-yl)-3-[4-(trifluoromethyl)benzoylthio]-2-butanol;
3-(4-chlorocinnamoylamino)-2-(2,4-difluorophenyl)-1-(1H-1,2,4-triazol-1-yl)-2-butanol;
2-(2,4-difluorophenyl)-1-(1H-1,2,4-triazol-1-yl)-3-{N-methyl-N-[4-(trifluoromethyl)benzoyl]amino}-2-butanol; and
2-(2,4-difluorophenyl)-3-{N-[2-fluoro-4-(trifluoromethyl)benzoyl]-N-methylamino}-1-(1H-1,2,4-triazol-1-yl)-2-butanol;
or a pharmaceutically acceptable acid addition salt thereof.

**23.** A process for preparing a compound of formula (I), or an acid addition salt thereof, as defined in any one of Claims 1 to 15, which process comprises:
(a) reacting a compound of formula (IIIA):

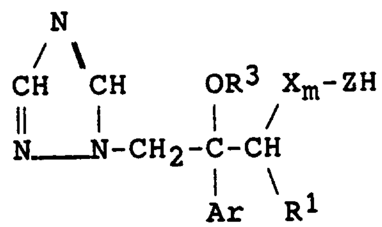

(IIIA)

[in which:
$R^1$, $R^3$, X, m and Ar are as defined in Claim 1; and
Z represents an oxygen or sulphur atom or a group of formula -NH- or -N($CHR^8R^9$)-, in which $R^8$ and $R^9$ are the same or different and each represents a hydrogen atom or a methyl or ethyl group]
with a compound of formula (V):

$R^{2'}$-COOH (V)

(in which $R^{2'}$ represents any of the groups defined in Claim 1 for $R^2$ or represents a group of formula $R^2$-CH=CH-, and $R^2$ is as defined in Claim 1) or with a reactive derivative thereof, to give a compound of formula (I'):

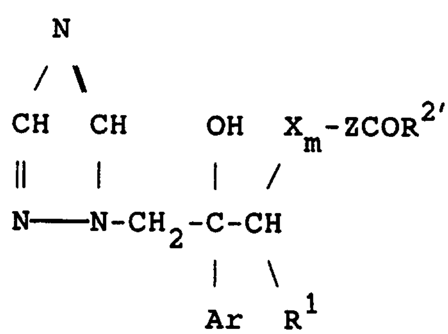

(I')

(in which $R^1$, $R^{2'}$, Ar, X, Z and m are as defined above);
or
(b) reacting a compound of formula (X'):

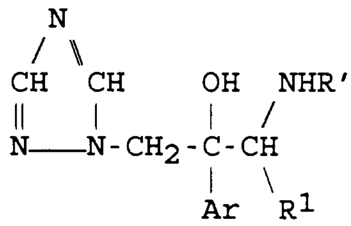

(X')

(in which: $R^1$ and Ar are as defined above, and R' represents a hydrogen atom or $R^2$) with formaldehyde, to give a compound of formula (X"):

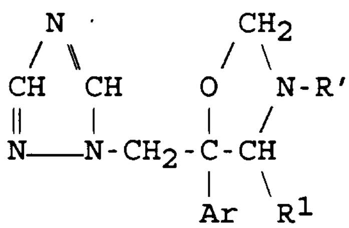

(X")

(in which $R^1$, Ar and R' are as defined above)
and, where R' represents a hydrogen atom, reacting said compound of formula (X") with a compound of formula (XVII):

$R^2$-(CH = CH)$_q$-COOH (XVII)

(in which $R^2$ is as defined above, and q is 0 or 1) or with a reactive derivative thereof, to give a compound of formula (Ig):

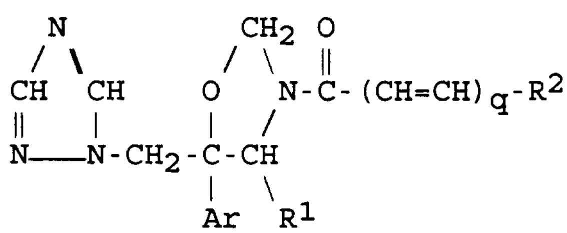

(Ig)

(in which $R^1$, $R^2$, Ar and q are as defined above);
or
(c) reacting said compound of formula (IIIA) in which Z represents an oxygen atom with a compound of formula (V'):

$R^4$-$SO_2$-OH (V')

(in which $R^4$ is as defined above) or with a reactive derivative thereof, to give a compound of formula (IIIB):

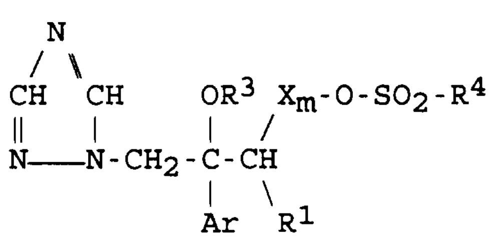

(IIIB)

(in which $R^1$, $R^3$, $R^4$, X, m and Ar are as defined above);

or

(d) reacting said compound of formula (IIIA) in which Z represents a group of formula -NH- with phthalic acid, a substituted phthalic acid in which the substituent is at least one of substituents (a), or a reactive derivative of said phthalic acid or substituted phthalic acid, to give a compound of formula (I) in which $-Y-R^2$ represents the phthalimido group or a substituted phthalimido group;

or

(e) reacting a compound of formula (IIIC):

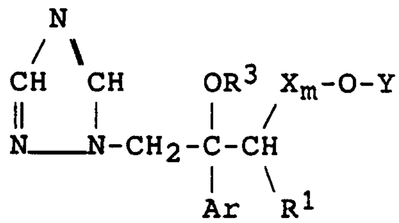

(IIIC)

(in which $R^1$, $R^3$, X, m and Ar are as defined above, and Y represents a hydrogen atom or a hydroxy-protecting group) or a corresponding oxirane compound in which m is 0, Y represents a hydrogen atom and the two hydroxy groups are condensed to form an oxirane group with an alkali metal azide to give a compound of formula (IIID):

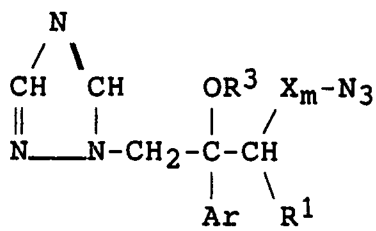

(IIID)

(in which $R^1$, $R^3$, X, m and Ar are as defined above).

**24.** The use for the manufacture of a medicament for the treatment or prophylaxis of fungal infections of a compound of formula (I), as defined in Claim 17, or of a pharmaceutically acceptable acid addition salt thereof.

**25.** The use according to Claim 24 of a compound of formula (I), or an acid addition salt thereof, as defined in any one of Claims 1 to 15.

**26.** The use according to Claim 24 of any one of the compounds referred to in Claim 22.

**27.** The use for the manufacture of a fungicide for agrochemical use of a compound of formula (I), as defined in Claim 17, or of an acid addition salt thereof.

**28.** The use according to Claim 27 of a compound of formula (I), or an acid addition salt thereof, as defined in any one of Claims 1 to 15.

**29.** The use according to Claim 27 of any one of the compounds referred to in Claim 19.

**30.** A process for protecting plants, seeds, and portions of plants or seeds from fungal infection, which comprises applying to said plants, seeds, portions of plants or seeds or a locus including the same at least one compound of formula (I) or an acid addition salt thereof, as defined in Claim 17.

**Patentansprüche**

**Patentansprüche für folgende Vertragsstaaten : AT, BE, CH, DE, FR, GB, IT, LI, LU, NL, SE**

**1.** Verbindungen der Formel (I):

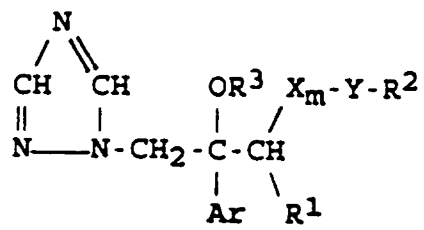

(I)

worin:

Ar eine Phenylgruppe oder eine Phenylgruppe mit einem oder zwei Halogen- und/oder Trifluormethyl-substituenten darstellt;

$R^1$ eine $C_1$ - $C_6$-Alkylgruppe oder, wenn $R^3$ und -$X_m$-Y-$R^2$ zusammen die Gruppe der Formel (II) darstellen, $R^1$ ein Wasserstoffatom oder eine $C_1$ - $C_6$-Alkylgruppe darstellt;

X eine $C_1$ - $C_6$-Alkylengruppe, eine $C_2$ - $C_6$-aliphatische Kohlenwasserstoff-Gruppe mit einer oder zwei Kohlenstoff-Kohlenstoff-Doppelbindungen, eine $C_2$ - $C_6$-aliphatische Kohlenwasserstoff-Gruppe mit einer oder zwei Kohlenstoff-Kohlenstoff-Dreifachbindungen, eine $C_3$ - $C_6$-Cycloalkylengruppe, eine $C_3$ - $C_6$-Cycloalkylengruppe mit einer oder zwei Kohlenstoff-Kohlenstoff-Doppelbindungen, eine ($C_1$ - $C_3$-Alkylen)-($C_3$ - $C_6$-Cycloalkylen)-Gruppe oder eine ($C_3$ - $C_6$-Cycloalkylen)-($C_1$ - $C_3$-Alkylen)-Gruppe darstellt;

m 0 oder 1 ist;

-Y-$R^2$ die Azidogruppe, die Phthalimid-Gruppe, die 1-Oxo-2,3-dihydro-2-isoindolyl-Gruppe oder die Phthalimid- oder 1-Oxo-2,3-dihydro-2-isoindolyl-Gruppe mit mindestens einem der nachstehend definierten Substituenten (a) darstellt;

oder

Y eine Gruppe der Formel -N($R^5$)-CO-, -N($R^5$)CO-CH = CH-, -O-CO-, -O-CO-CH = CH-, -S-CO- oder -S-CO-CH = CH- darstellt, wobei:

$R^5$ ein Wasserstoffatom oder eine $C_1$ - $C_4$-Alkylgruppe darstellt;

$R^2$ eine $C_1$ - $C_6$-Alkylgruppe, eine $C_1$ - $C_6$-Halogenalkylgruppe, eine Phenylgruppe, eine Phenylgruppe mit mindestens einem der nachstehend definierten Substituenten (a), eine Naphthylgruppe oder eine heterocyclische Gruppe mit 5 oder 6 Ringatomen darstellt, von denen 1 bis 3 Stickstoff- und/oder Sauerstoff- und/oder Schwefel-Heteroatome sind, wobei die heterocyclische Gruppe unsubstituiert ist oder 1 bis 3 der nachstehend definierten Substituenten (b) aufweist; und

$R^3$ ein Wasserstoffatom darstellt;

oder

$R^3$ und -$X_m$-Y-$R^2$ zusammengenommen eine Gruppe der Formel (II) darstellen:

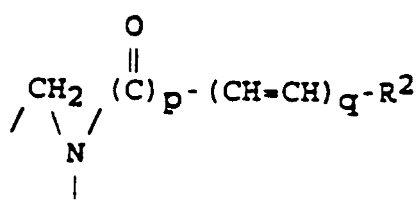

(II)

worin:

$R^2$ wie oben definiert ist;

p 0 oder 1 ist; und

q 0 oder 1 ist;

Substituenten (a):

$C_1$ - $C_4$-Alkylgruppen, $C_1$ - $C_4$-Halogenalkylgruppen, $C_1$ - $C_4$-Alkoxygruppen, $C_1$ - $C_4$-Halogenalkoxygruppen, Halogenatome, Nitrogruppen, Cyangruppen, Hydroxylgruppen, Benzyloxygruppen, Hydroxymethylgruppen, Gruppen der Formel -$CH_2$-OCO-$R^7$, Gruppen der Formel -CO-$R^6$, Gruppen der Formel -COO$R^7$, Gruppen der Formel -$SO_r R^7$, Aminogruppen, Mono- und Dialkylaminogruppen, worin jede Alkylgruppe $C_1$ - $C_4$ ist und Acylaminogruppen von Carbonsäuren;

Substituenten (b):

$C_1$ - $C_4$-Alkylgruppen, $C_1$ - $C_4$-Halogenalkylgruppen, Halogenatome, Cyangruppen und Nitrogruppen,

$R^6$ eine $C_1$ - $C_6$-Alkylgruppe oder eine $C_1$ - $C_6$-Halogenalkylgruppe darstellt,
$R^7$ eine $C_1$ - $C_6$-Alkylgruppe darstellt,
r 0, 1 oder 2 ist,
und deren Säureadditionssalze,
vorausgesetzt, daß, falls m 0 ist und Y eine Gruppe der Formel -N($R^5$)CO- ist, $R^5$ Wasserstoff sein muß.

**2.** Verbindungen gemäß Anspruch 1, worin Ar eine Phenylgruppe mit einem oder zwei Halogensubstituenten darstellt.

**3.** Verbindungen gemäß Anspruch 1, worin Ar eine Phenylgruppe mit einem oder zwei Fluor- und/oder Chlorsubstituenten darstellt.

**4.** Verbindungen gemäß Anspruch 1, worin Ar eine 4-Chlorphenylgruppe, eine 4-Fluorphenylgruppe, eine 2,4-Dichlorphenylgruppe, eine 2,4-Difluorphenylgruppe, eine 2-Chlor-4-fluorphenylgruppe oder eine 4-Chlor-2-fluorphenylgruppe darstellt.

**5.** Verbindungen gemäß einem der Ansprüche 1 bis 4, worin $R^1$ eine $C_1$ - $C_3$-Alkylgruppe darstellt.

**6.** Verbindungen gemäß einem der Ansprüche 1 bis 4, worin $R^1$ eine Methyl- oder Ethylgruppe darstellt.

**7.** Verbindungen gemäß einem der Ansprüche 1 bis 6, worin X eine $C_1$- oder $C_2$-Alkylengruppe darstellt.

**8.** Verbindungen gemäß einem der Ansprüche 1 bis 6, worin m 0 ist.

**9.** Verbindungen gemäß einem der Ansprüche 1 bis 8, worin -Y-$R^2$ die Azidogruppe darstellt.

**10.** Verbindungen gemäß einem der Ansprüche 1 bis 8, worin Y eine Gruppe der Formel -N($R^5$)CO-, -N($R^5$)CO-CH=CH-, -S-CO- oder -S-CO-CH=CH- darstellt, wobei:
$R^5$ ein Wasserstoffatom oder eine $C_1$ - $C_4$-Alkylgruppe darstellt.

**11.** Verbindungen gemäß einem der Ansprüche 1 bis 8 und 10, worin $R^2$ eine Phenylgruppe mit einem oder zwei Substituenten, ausgewählt unter Halogenatomen und Trifluormethyl-, Trifluormethoxy- und Cyangruppen, darstellt.

**12.** Verbindungen gemäß Anspruch 1, worin:
Ar eine Phenylgruppe mit einem oder zwei Halogensubstituenten darstellt;
$R^1$ eine $C_1$ - $C_3$-Alkylgruppe darstellt;
m 0 ist oder X eine $C_1$- oder $C_2$-Alkylengruppe darstellt;
Y eine Gruppe der Formel -N($R^5$)CO-, -N($R^5$)CO-CH=CH-, -S-CO- oder -S-CO-CH=CH- darstellt, wobei:
$R^5$ ein Wasserstoffatom oder eine $C_1$ - $C_4$-Alkylgruppe darstellt; und
$R^2$ eine Phenylgruppe mit einem oder zwei Substituenten, ausgewählt unter Halogenatomen und Trifluormethyl-, Trifluormethoxy- und Cyangruppen, darstellt;
oder
-Y-$R^2$ eine Azidogruppe darstellt.

**13.** Verbindungen gemäß Anspruch 1, worin:
Ar eine Phenylgruppe mit einem oder zwei Fluor- und/oder Chlorsubstituenten darstellt;
$R^1$ eine Methyl- oder Ethylgruppe darstellt;
m 0 ist oder X eine $C_1$- oder $C_2$-Alkylengruppe darstellt;
Y eine Gruppe der Formel -N($R^5$)CO-, -N($R^5$)CO-CH=CH-, -S-CO- oder -S-CO-CH=CH- darstellt, wobei
$R^5$ ein Wasserstoffatom oder eine $C_1$ - $C_4$-Alkylgruppe darstellt, und
$R^2$ eine Phenylgruppe mit einem oder zwei Substituenten, ausgewählt unter Halogenatomen und Trifluormethyl-, Trifluormethoxy- und Cyangruppen, darstellt;
oder
-Y-$R^2$ eine Azidogruppe darstellt.

**14.** Verbindungen gemäß Anspruch 1, worin:
$R^1$ eine Methyl- oder Ethylgruppe darstellt;
m 0 ist oder X eine $C_1$- oder $C_2$-Alkylengruppe darstellt;
Y eine Gruppe der Formel $-N(R^5)CO-$, $-N(R^5)CO-CH=CH-$, -S-CO- oder -S-CO-CH=CH- darstellt, wobei
$R^5$ ein Wasserstoffatom oder eine $C_1$ - $C_4$-Alkylgruppe darstellt;
$R^2$ eine Phenylgruppe mit einem oder zwei Substituenten, ausgewählt unter Halogenatomen und Trifluormethyl-, Trifluormethoxy- und Cyangruppen, darstellt;
oder
$-Y-R^2$ eine Azidogruppe darstellt; und
Ar eine 4-Chlorphenylgruppe, eine 4-Fluorphenylgruppe, eine 2,4-Dichlorphenylgruppe, eine 2,4-Difluorphenylgruppe, eine 2-Chlor-4-fluorphenylgruppe oder eine 4-Chlor-2-fluorphenylgruppe darstellt.

**15.** 2-(2,4-Dichlorphenyl)-1-(1H-1,2,4-triazol-1-yl)-3-[4-(trifluormethyl)benzoylamino]-2-butanol und dessen Säureadditionssalze.

**16.** 2-(2,4-Difluorphenyl)-3-[2-fluor-4-(trifluormethyl)benzoylamino]-1-(1H-1,2,4-triazol-1-yl)-2-butanol und dessen Säureadditionssalze.

**17.** 3-(4-Chlorbenzoylamino)-2-(2,4-difluorphenyl)-1-(1H-1,2,4-triazol-1-yl)-2-pentanol und dessen Säureadditionssalze.

**18.** 3-Azido-2-(2,4-difluorphenyl)-1-(1H-1,2,4-triazol-1-yl)-2-butanol und dessen Säureadditionssalze.

**19.** 3-Azido-2-(4-chlor-2-fluorphenyl)-1-(1H-1,2,4-triazol-1-yl)-2-butanol und dessen Säureadditionssalze.

**20.** 3-Azido-2-(2,4-difluorphenyl)-1-(1H-1,2,4-triazol-1-yl)-2-pentanol und dessen Säureadditionssalze.

**21.** 3-Azido-2-(2-chlor-4-fluorphenyl)-1-(1H-1,2,4-triazol-1-yl)-2-pentanol und dessen Säureadditionssalze.

**22.** 4-Azido-2-(2,4-difluorphenyl)-3-methyl-1-(1H-1,2,4-triazol-1-yl)-2-pentanol und dessen Säureadditionssalze.

**23.** 4-Azido-2-(2,4-difluorphenyl)-3-methyl-1-(1H-1,2,4-triazol-1-yl)-2-butanol und dessen Säureadditionssalze.

**24.** 4-Azido-2-(2,4-dichlorphenyl)-3-methyl-1-(1H-1,2,4-triazol-1-yl)-2-butanol und dessen Säureadditionssalze.

**25.** 3-Azido-2-(2,4-dichlorphenyl)-1-(1H-1,2,4-triazol-1-yl)-2-pentanol und dessen Säureadditionssalze.

**26.** 2-(4-Chlorphenyl)-1-(1H-1,2,4-triazol-1-yl)-3-[4-(trifluormethyl)benzoylamino]-2-butanol und dessen Säureadditionssalze.

**27.** 2-(2,4-Difluorphenyl)-1-(1H-1,2,4-triazol-1-yl)-3-[4-(trifluormethyl)benzoylamino]-2-butanol und dessen Säureadditionssalze.

**28.** 3-(4-Cyanobenzoylamino)-2-(2,4-difluorphenyl)-1-(1H-1,2,4-triazol-1-yl)-2-butanol und dessen Säureadditionssalze.

**29.** 2-(4-Chlorphenyl)-3-[2-fluor-4-(trifluormethyl)benzoylamino]-1-[1H-1,2,4-triazol-1-yl)-2-butanol und dessen Säureadditionssalze.

**30.** 2-(2,4-Dichlorphenyl)-1-(1H-1,2,4-triazol-1-yl)-3-[4-(trifluormethyl)benzoylthio]-2-butanol und dessen Säureadditionssalze.

**31.** 3-(4-Chlorcinnamoylamino)-2-(2,4-difluorphenyl)-1-[1H-1,2,4-triazol-1-yl)-2-butanol und dessen Säureadditionssalze.

**32.** 2-(2,4-Difluorphenyl)-1-(1H-1,2,4-triazol-1-yl)-3-(N-methyl-N-[4-(trifluormethyl)benzoyl]amino}-2-butanol und dessen Säureadditionssalze.

**33.** 2-(2,4-Difluorphenyl)-3-[N-[2-fluor-4-(trifluormethyl)benzoyl]-N-methylamino}-1-(1H-1,2,4-triazol-1-yl)-2-butanol und dessen Säureadditionssalze.

**34.** Verbindungen der Formel (IX)

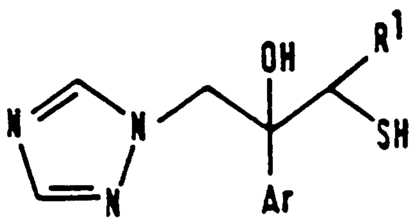

(IX)

worin:
Ar eine Phenylgruppe oder eine Phenylgruppe mit einem oder zwei Halogen- und/oder Trifluormethyl-substituenten darstellt,
$R^1$ eine $C_1$ - $C_6$-Alkylgruppe darstellt;
und deren Säureadditionssalze.

**35.** Agrochemikalien-Zusammensetzung, die ein für landwirtschaftliche Zwecke verträgliches Trägermaterial oder Verdünnungsmittel im Gemisch mit mindestens einem Fungizid enthält, worin das Fungizid mindestens eine Verbindung der Formel (I) ist:

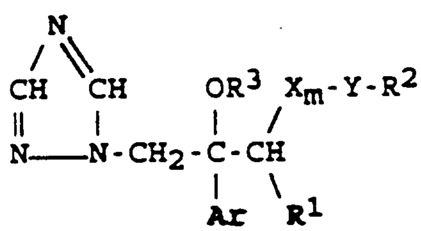

(I)

worin:
Ar eine Phenylgruppe oder eine Phenylgruppe mit einem oder zwei Halogenatomen und/oder Trifluormethyl-Substituenten darstellt;
$R^1$ eine $C_1$ - $C_6$-Alkylgruppe oder, wenn $R^3$ und $-X_m$-Y-$R^2$ zusammen die Gruppe der Formel (II) darstellen, $R^1$ ein Wasserstoffatom oder eine $C_1$ - $C_6$-Alkylgruppe darstellt;
X eine $C_1$ - $C_6$-Alkylengruppe, eine $C_2$ - $C_6$-aliphatische Kohlenwasserstoff-Gruppe mit einer oder zwei Kohlenstoff-Kohlenstoff-Doppelbindungen, eine $C_2$ - $C_6$-aliphatische Kohlenwasserstoff-Gruppe mit einer oder zwei Kohlenstoff-Kohlenstoff-Dreifachbindungen, eine $C_3$ - $C_6$-Cycloalkylengruppe, eine $C_3$ - $C_6$-Cycloalkylengruppe mit einer oder zwei Kohlenstoff-Kohlenstoff-Doppelbindungen, eine ($C_1$ - $C_3$-Alkylen)-($C_3$ - $C_6$-cycloalkylen)-Gruppe oder eine ($C_3$ - $C_6$-Cycloalkylen)-($C_1$ - $C_3$-alkylen)-Gruppe darstellt;
m 0 oder 1 ist;
-Y-$R^2$ die Azidogruppe, die Phthalimid-Gruppe, die 1-Oxo-2,3-dihydro-2-isoindolyl-Gruppe, die Phthalimid- oder 1-Oxo-2,3-di-2-isoindolyl-Gruppe mit mindestens einem der nachstehend definierten Substituenten (a), eine geschützte Hydroxylgruppe oder eine Gruppe der Formel $-OSO_2R^4$ darstellt, in der:

$R^4$ eine $C_1$ - $C_4$-Alkylgruppe, eine $C_1$ - $C_4$-Halogenalkylgruppe, eine Phenylgruppe oder eine Phenylgruppe mit einem Substituenten, ausgewählt unter Nitrogruppen, Halogenatomen und $C_1$ - $C_4$-Alkylgruppen, darstellt;

oder

Y eine Gruppe der Formel $-N(R^5)CO-$, $-N(R^5)CO-CH=CH-$, -O-CO-, -O-CO-CH=CH-, -S-CO- oder -S-CO-CH=CH- darstellt, worin:

$R^5$ ein Wasserstoffatom oder eine $C_1$ - $C_4$-Alkylgruppe darstellt;

n 0 oder 1 ist;
$R^2$ eine $C_1$ - $C_6$-Alkylgruppe, eine $C_1$ - $C_6$-Halogenalkylgruppe, eine Phenylgruppe, eine Phenylgruppe mit mindestens einem der nachstehend definierten Substituenten (a), eine Naphthylgruppe oder eine heterocyclische Gruppe mit 5 oder 6 Ringatomen darstellt, von denen 1 bis 3 Stickstoff- und/oder Sauerstoff- und/oder Schwefel-Heteroatome sind, wobei die heterocyclische Gruppe unsubstituiert ist oder 1 bis 3 der nachstehend definierten Substituenten (b) aufweist; und
$R^3$ ein Wasserstoffatom darstellt;
oder
$R^3$ und $-X_m-Y_n-R^2$ zusammen eine Gruppe der Formel (II) darstellen:

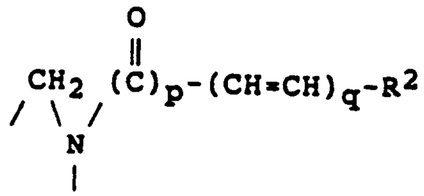

(II)

worin:
$R^2$ wie oben definiert ist;
p 0 oder 1 ist und
q 0 oder 1 ist;
<u>Substituenten (a):</u>
$C_1$ - $C_4$-Alkylgruppen, $C_1$ - $C_4$-Halogenalkylgruppen, $C_1$ - $C_4$-Alkoxygruppen, $C_1$ - $C_4$-Halogenalkoxygruppen, Halogenatome, Nitrogruppen, Cyangruppen, Hydroxylgruppen, Benzyloxygruppen, Hydroxymethylgruppen, Gruppen der Formel $-CH_2-OCO-R^7$, Gruppen der Formel $-CO-R^6$, Gruppen der Formel $-COOR^7$, Gruppen der Formel $-SO_rR^7$, Aminogruppen, Mono- und Dialkylaminogruppen, worin jede Alkylgruppe $C_1$ - $C_4$ umfaßt und Acylaminogruppen von Carbonsäuren;
<u>Substituenten (b):</u>
$C_1$ - $C_4$-Alkylgruppen, $C_1$ - $C_4$-Halogenalkylgruppen, Halogenatome, Cyangruppen und Nitrogruppen;
$R^6$ eine $C_1$ - $C_6$-Alkylgruppe oder eine $C_1$ - $C_6$-Halogenalkylgruppe darstellt,
$R^7$ eine $C_1$ - $C_6$-Alkylgruppe darstellt,
r 0, 1 oder 2 ist,
oder deren Säureadditionssalze.

**36.** Zusammensetzung gemäß Anspruch 35, worin das Fungizid eine Verbindung gemäß einem der Ansprüche 1 bis 33 ist.

**37.** Zusammensetzung gemäß Anspruch 35, worin das Fungizid mindestens eine der folgenden Verbindungen ist:
2-(2,4-Dichlorphenyl)-1-(1H-1,2,4-triazol-1-yl)-3-[4-(trifluormethyl)benzoylamino]-2-butanol;
2-(2,4-Difluorphenyl)-3-[2-(fluor-4-(trifluormethyl)-benzoylamino]-1-(1H-1,2,4-triazol-1-yl)-2-butanol;
3-(4-Chlorbenzoylamino)-2-(2,4-difluorphenyl)-1-(1H-1,2,4-triazol-1-yl)-2-pentanol;
3-Azido-2-(2,4-difluorphenyl)-1-(1H-1,2,4-triazol-1-yl)-2-butanol;
3-Azido-2-(4-Chlor-2-fluorphenyl)-1-(1H-1,2,4-triazol-1-yl)-2-butanol;
3-Azido-2-(2,4-difluorphenyl)-1-(1H-1,2,4-triazol-1-yl)-2-pentanol;
3-Azido-2-(2-Chlor-4-fluorphenyl)-1-(1H-1,2,4-triazol-1-yl)-2-pentanol;
4-Azido-2-(2,4-difluorphenyl)-3-methyl-1-(1H-1,2,4-triazol-1-yl)-2-pentanol;
4-Azido-2-(2,4-difluorphenyl)-3-methyl-1-(1H-1,2,4-triazol-1-yl)-2-butanol;
4-Azido-2-(2,4-dichlorphenyl)-3-methyl-1-(1H-1,2,4-triazol-1-yl)-2-butanol; und
3-Azido-2-(2,4-dichlorphenyl)-1-(1H-1,2,4-triazol-1-yl)-2-pentanol;
oder deren Säureadditionssalze.

**38.** Fungizide Arzneimittelzusammensetzung, welche ein pharmazeutisch verträgliches Trägermaterial oder Verdünnungsmittel im Gemisch mit mindestens einem Fungizid enthält, worin das Fungizid mindestens eine in Anspruch 35 definierte Verbindung der Formel (I) ist.

**39.** Zusammensetzung gemäß Anspruch 38, worin das Fungizid eine Verbindung gemäß einem der Ansprüche 1 bis 33 ist.

**40.** Zusammensetzung gemäß Anspruch 38, worin das Fungizid mindestens eine der folgenden Verbindungen ist:
2-(4-Chlorphenyl)-1-(1H-1,2,4-triazol-1-yl)-3-[4-(trifluormethyl)benzoylamino]-2-butanol;
2-(2,4-Difluorphenyl)-1-(1H-1,2,4-triazol-1-yl)-3-[4-(trifluormethyl)benzoylamino]-2-butanol;
2-(2,4-Difluorphenyl)-3-[2-fluor-4-(trifluormethyl)benzoylamino]-1-(1H-1,2,4-triazol-1-yl)-2-butanol;
3-(4-Cyanobenzoylamino)-2-(2,4-difluorphenyl)-1-(1H-1,2,4-triazol-1-yl)-2-butanol;
2-(4-Chlorphenyl)-3-[2-fluor-4-(trifluormethyl)benzoylamino]-1-(1H-1,2,4-triazol-l-yl)-2-butanol;
2-(2,4-Dichlorphenyl)-1-(1H-1,2,4-triazol-1-yl)-3-[4-(trifluormethyl)benzoylthio]-2-butanol;
3-(4-Chlorcinnamoylamino)-2-(2,4-difluorphenyl)-1-(1H-1,2,4-triazol-1-yl)-2-butanol;
2-(2,4-Difluorphenyl)-1-(1H-1,2,4-triazol-1-yl)-3-{N-methyl-N-[4-(trifluormethyl)benzoyl]amino}-2-butanol; und
2-(2,4-Difluorphenyl)-3-{N-[2-fluor-4-(trifluormethyl)benzoyl]-N-methylamino)-1-(1H-1,2,4-triazol-1-yl)-2-butanol;
oder ein pharmazeutisch verträgliches Säureadditionssalz derselben.

**41.** Verfahren zur Herstellung einer Verbindung gemäß einem der Ansprüche 1 bis 33, welches umfaßt:
(a) Umsetzung einer Verbindung der Formel (IIIA):

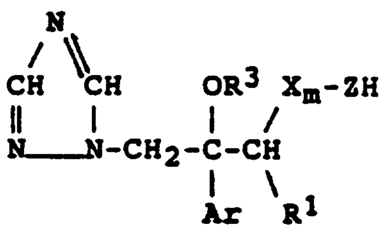

(IIIA)

[worin:
$R^1$, $R^3$, X, m und Ar wie in Anspruch 1 definiert sind; und Z ein Sauerstoff- oder Schwefelatom oder eine Gruppe der Formel -NH- oder -N($CHR^8R^9$)- bedeutet, worin $R^8$ und $R^9$ gleich oder verschieden sind und jeweils ein Wasserstoffatom oder eine Methyl- oder Ethylgruppe darstellen,]
mit einer Verbindung der Formel (V):

$R^{2'}$-COOH (V)

(worin $R^{2'}$irgendeine der in Anspruch 1 für $R^2$ definierten Gruppen darstellt, oder eine Gruppe der Formel $R^2$-CH=CH-darstellt, wobei $R^2$ wie in Anspruch 1 definiert ist), oder mit einem reaktivem Derivat derselben,
wobei eine Verbindung der Formel (I') gebildet wird:

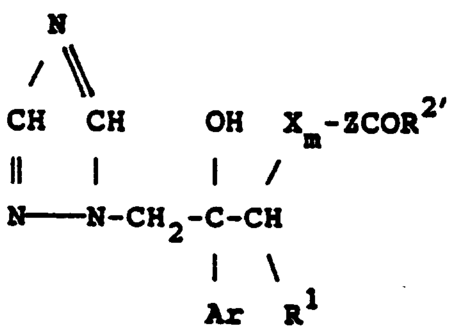

(I')

(worin $R^1$, $R^{2'}$, Ar, X, Z und m wie oben definiert sind);
oder
(b) Umsetzung einer Verbindung der Formel (X'):

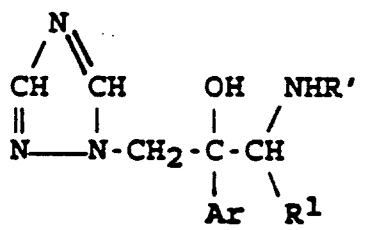

(X')

(worin: $R^1$ und Ar wie oben definiert sind und R' ein Wasserstoffatom oder $R^2$ darstellt) mit Formaldehyd, wobei eine Verbindung der Formel (X'') gebildet wird:

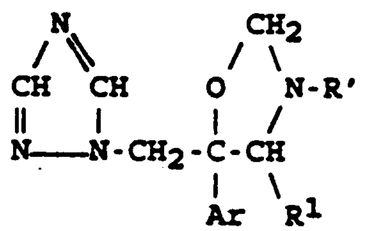

(X'')

(worin R1, Ar und R' wie oben definiert sind)
und, wenn R' ein Wasserstoffatom darstellt,
Umsetzung der Verbindung der Formel (X'') mit einer Verbindung der Formel (XVII):

$R^2$-(CH = CH)$_q$-COOH (XVII)

(worin $R^2$ wie oben definiert ist und q 0 oder 1 ist)
oder
mit einem reaktiven Derivat derselben, wobei eine Verbindung der Formel (Ig) gebildet wird:

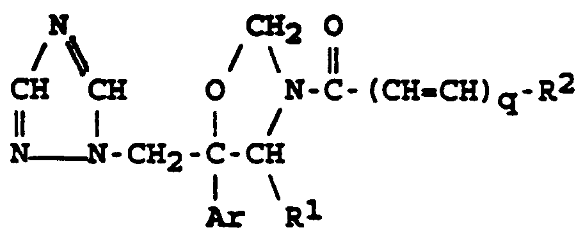

(Ig)

(worin $R^1$, $R^2$, Ar und q wie oben definiert sind);
oder
(c) Umsetzung der Verbindung der Formel (IIIA), worin Z ein Sauerstoffatom darstellt, mit einer Verbindung der Formel (V'):

$R^4$-$SO_2$-OH (V')

(worin $R^4$ wie oben definiert ist), oder mit einem reaktiven Derivat derselben, wobei eine Verbindung der Formel (IIIB) gebildet wird:

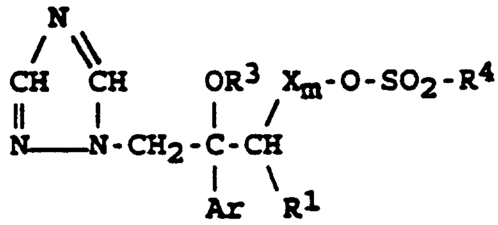

(IIIB)

(worin $R^1$, $R^3$, $R^4$, X, m und Ar wie oben definiert sind);

oder

(d) Umsetzung der Verbindung der Formel (IIIA), worin Z eine Gruppe der Formel -NH- darstellt, mit Phthalsäure, einer substituierten Phthalsäure, in der der Substituent mindestens einer der Substituenten (a) ist, oder einem reaktiven Derivat der Phthalsäure oder der substituierten Phthalsäure, wobei eine Verbindung der Formel (I) gebildet wird,

worin $-Y-R^2$ die Phthalimid-Gruppe oder eine substituierte Phthalimid-Gruppe darstellt;

oder

(e) Umsetzung einer Verbindung der Formel (IIIC):

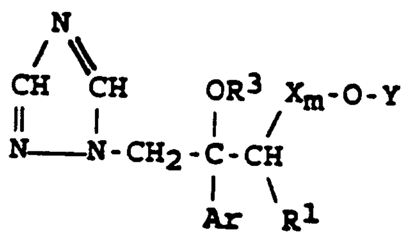

(IIIC)

(worin $R^1$, $R^3$, X, m und Ar wie oben definiert sind und Y ein Wasserstoffatom oder eine Hydroxyl-Schutzgruppe darstellt)

oder einer entsprechenden Oxiranverbindung, worin m 0 ist, Y ein Wasserstoffatom darstellt und die zwei Hydroxylgruppen unter Bildung einer Oxirangruppe kondensiert sind,

mit einem Alkalimetallazid, wobei eine Verbindung der Formel (IIID) gebildet wird:

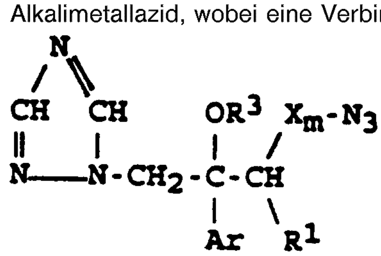

(IIID)

(worin $R^1$, $R^3$, X, m und Ar wie oben definiert sind.

**42.** Verwendung einer Verbindung der Formel (I), gemäß der Definition in Anspruch 35, oder eines pharmazeutisch verträglichen Säureadditionssalzes derselben, zur Herstellung eines Arzneimittels zur Behandlung oder zur Prophylaxe von Pilzinfektionen.

**43.** Verwendung gemäß Anspruch 42 einer Verbindung gemäß einem der Ansprüche 1 bis 33.

**44.** Verwendung gemäß Anspruch 42 einer der Verbindungen, auf die in Anspruch 40 Bezug genommen wird.

**45.** Verwendung einer Verbindung der Formel (I), wie in Anspruch 35 definiert, oder deren Säureadditionssalze zur Herstellung eines Fungizids zur Verwendung als Agrochemikalie.

**46.** Verwendung gemäß Anspruch 45 einer Verbindung gemäß einem der Ansprüche 1 bis 33.

**47.** Verwendung gemäß Anspruch 45 einer der Verbindungen, auf die in Anspruch 37 Bezug genommen wird.

**48.** Verfahren zum Schutz von Pflanzen, Samen sowie Teilen von Pflanzen oder Samen vor einer Pilzinfektion, welches das Auftragen mindestens einer Verbindung der Formel (I) oder deren Säureadditionssalze, wie in Anspruch 35 definiert, auf die Pflanzen, Samen, Teile von Pflanzen oder Samen, oder auf einen Ort, welcher diese einschließt, umfaßt.

**Patentansprüche für folgenden Vertragsstaat : ES**

**1.** Verfahren zur Herstellung einer Verbindung der Formel (I):

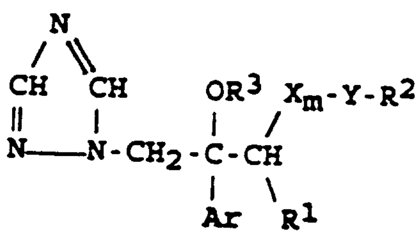

(I)

worin:
Ar eine Phenylgruppe oder eine Phenylgruppe mit einem oder zwei Halogen- und/oder Trifluormethylsubstituenten darstellt;
$R^1$ eine $C_1$ - $C_6$-Alkylgruppe oder, wenn $R^3$ und $-X_m-Y-R^2$ zusammen die Gruppe der Formel (II) darstellen, $R^1$ ein Wasserstoffatom oder eine $C_1$ - $C_6$-Alkylgruppe darstellt;
X eine $C_1$ - $C_6$-Alkylengruppe, eine $C_2$ - $C_6$-aliphatische Kohlenwasserstoff-Gruppe mit einer oder zwei Kohlenstoff-Kohlenstoff-Doppelbindungen, eine $C_2$ - $C_6$-aliphatische Kohlenwasserstoff-Gruppe mit einer oder zwei Kohlenstoff-Kohlenstoff-Dreifachbindungen, eine $C_3$ - $C_6$-Cycloalkylengruppe, eine $C_3$ - $C_6$-Cycloalkylengruppe mit einer oder zwei Kohlenstoff-Kohlenstoff-Doppelbindungen, eine ($C_1$ - $C_3$-Alkylen)-($C_3$ - $C_6$-Cycloalkylen)-Gruppe oder eine ($C_3$ - $C_6$-Cycloalkylen)-($C_1$ - $C_3$-Alkylen)-Gruppe darstellt;
m 0 oder 1 ist;
$-Y-R^2$ die Azidogruppe, die Phthalimid-Gruppe, die 1-Oxo-2,3-dihydro-2-isoindolyl-Gruppe oder die Phthalimid- oder 1-Oxo-2,3-dihydro-2-isoindolyl-Gruppe mit mindestens einem der nachstehend definierten Substituenten (a) darstellt;
oder
Y eine Gruppe der Formel $-N(R^5)-CO-$, $-N(R^5)CO-CH=CH-$, -O-CO-, -O-CO-CH=CH-, -S-CO- oder -S-CO-CH=CH- darstellt, wobei:
$R^5$ ein Wasserstoffatom oder eine $C_1$ - $C_4$-Alkylgruppe darstellt;
$R^2$ eine $C_1$ - $C_6$-Alkylgruppe, eine $C_1$ - $C_6$-Halogenalkylgruppe, eine Phenylgruppe, eine Phenylgruppe mit mindestens einem der nachstehend definierten Substituenten (a), eine Naphthylgruppe oder eine heterocyclische Gruppe mit 5 oder 6 Ringatomen darstellt, von denen 1 bis 3 Stickstoff- und/oder Sauerstoff- und/oder Schwefel-Heteroatome sind, wobei die heterocyclische Gruppe unsubstituiert ist oder 1 bis 3 der nachstehend definierten Substituenten (b) aufweist; und
$R^3$ ein Wasserstoffatom darstellt;
oder
$R^3$ und $-X_m-Y-R^2$ zusammengenommen eine Gruppe der Formel (II) darstellen:

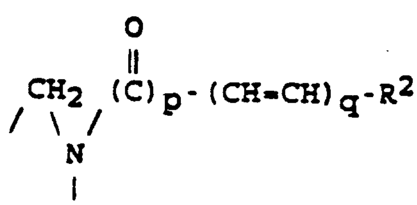

(II)

worin:

$R^2$ wie oben definiert ist;
p 0 oder 1 ist; und
q 0 oder 1 ist;
Substituenten (a):
$C_1$ - $C_4$-Alkylgruppen, $C_1$ - $C_4$-Halogenalkylgruppen, $C_1$ - $C_4$-Alkoxygruppen, $C_1$ - $C_4$-Halogenalkoxygruppen, Halogenatome, Nitrogruppen, Cyangruppen, Hydroxylgruppen, Benzyloxygruppen, Hydroxymethylgruppen, Gruppen der Formel $-CH_2-OCO-R^7$, Gruppen der Formel $-CO-R^6$, Gruppen der Formel $-COOR^7$, Gruppen der Formel $-SO_rR^7$, Aminogruppen, Mono- und Dialkylaminogruppen, worin jede Alkylgruppe $C_1$ - $C_4$ ist und Acylaminogruppen von Carbonsäuren;
Substituenten (b):
$C_1$ - $C_4$-Alkylgruppen, $C_1$ - $C_4$-Halogenalkylgruppen, Halogenatome, Cyangruppen und Nitrogruppen;
$R^6$ eine $C_1$ - $C_6$-Alkylgruppe oder eine $C_1$ - $C_6$-Halogenalkylgruppe darstellt,
$R^7$ eine $C_1$ - $C_6$-Alkylgruppe darstellt,
r 0, 1 oder 2 ist,
oder deren Säureadditionssalze,
vorausgesetzt, daß, falls m 0 ist und Y eine Gruppe der Formel $-N(R^5)CO-$ ist, $R^5$ Wasserstoff sein muß,
wobei das Verfahren umfaßt:

(a) Umsetzung einer Verbindung der Formel (IIIA):

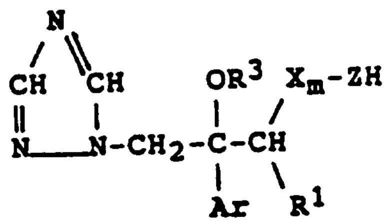

(IIIA)

[worin:
$R^1$, $R^3$, X, m und Ar wie in Anspruch 1 definiert sind; und Z ein Sauerstoff- oder Schwefelatom oder eine Gruppe der Formel -NH- oder $-N(CHR^8R^9)-$ bedeutet, worin $R^8$ und $R^9$ gleich oder verschieden sind und jeweils ein Wasserstoffatom oder eine Methyl- oder Ethylgruppe darstellen,]
mit einer Verbindung der Formel (V):

$R^{2'}-COOH$ (V)

(worin $R^{2'}$ irgendeine der in Anspruch 1 für $R^2$ definierten Gruppen darstellt, oder eine Gruppe der Formel $R^2-CH=CH-$darstellt, wobei $R^2$ wie in Anspruch 1 definiert ist), oder mit einem reaktivem Derivat derselben,
wobei eine Verbindung der Formel (I') gebildet wird:

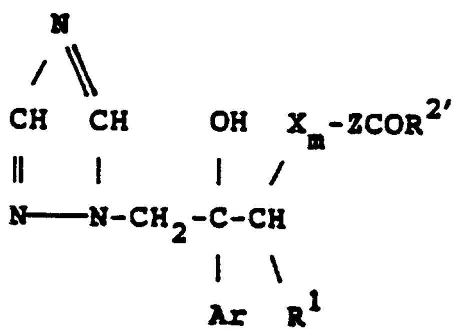

(I')

(worin $R^1$, $R^{2'}$, Ar, X, Z und m wie oben definiert sind);
oder
(b) Umsetzung einer Verbindung der Formel (X'):

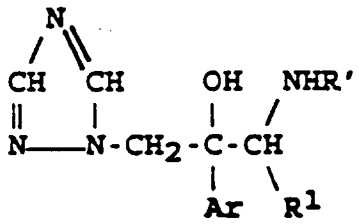

(X')

(worin: $R^1$ und Ar wie oben definiert sind und R' ein Wasserstoffatom oder $R^2$ darstellt) mit Formaldehyd, wobei eine Verbindung der Formel (X'') gebildet wird:

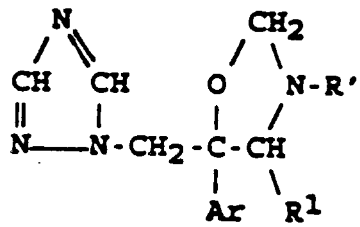

(X'')

(worin R1, Ar und R' wie oben definiert sind)
und, wenn R' ein Wasserstoffatom darstellt,
Umsetzung der Verbindung der Formel (X'') mit einer Verbindung der Formel (XVII):

$R^2$-(CH = CH)$_q$-COOH (XVII)

(worin $R^2$ wie oben definiert ist und q 0 oder 1 ist)
oder
mit einem reaktiven Derivat derselben, wobei eine Verbindung der Formel (Ig) gebildet wird:

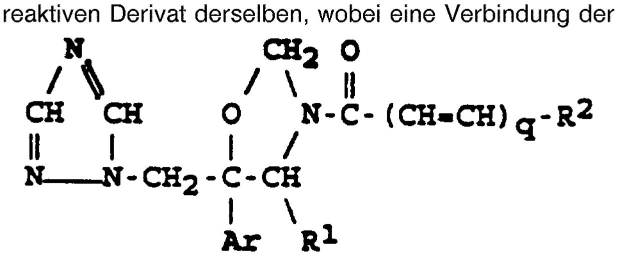

(Ig)

(worin $R^1$, $R^2$, Ar und q wie oben definiert sind);
oder
(c) Umsetzung der Verbindung der Formel (IIIA), worin Z ein Sauerstoffatom darstellt, mit einer Verbindung der Formel (V'):

$R^4$-$SO_2$-OH (V')

(worin $R^4$ wie oben definiert ist), oder mit einem reaktiven Derivat derselben, wobei eine Verbindung der Formel (IIIB) gebildet wird:

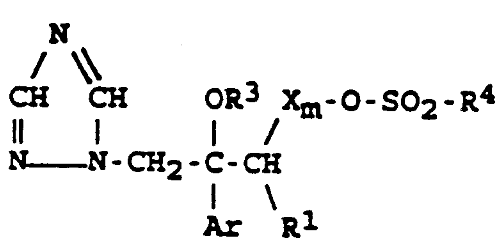

(IIIB)

(worin $R^1$, $R^3$, $R^4$, X, m und Ar wie oben definiert sind);
oder
(d) Umsetzung der Verbindung der Formel (IIIA), worin Z eine Gruppe der Formel -NH- darstellt, mit Phthalsäure, einer substituierten Phthalsäure, in der der Substituent mindestens einer der Substituenten (a) ist, oder einem reaktiven Derivat der Phthalsäure oder der substituierten Phthalsäure, wobei eine Verbindung der Formel (I) gebildet wird, worin -Y-$R^2$ die Phthalimid-Gruppe oder eine substituierte Phthalimid-Gruppe darstellt;
oder
(e) Umsetzung einer Verbindung der Formel (IIIC):

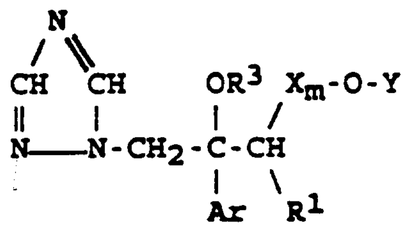

(IIIC)

(worin $R^1$, $R^3$, X, m und Ar wie oben definiert sind und Y ein Wasserstoffatom oder eine Hydroxyl-Schutzgruppe darstellt)
oder einer entsprechenden Oxiranverbindung, worin m 0 ist, Y ein Wasserstoffatom darstellt und die zwei Hydroxylgruppen unter Bildung einer Oxirangruppe kondensiert sind,
mit einem Alkalimetallazid, um eine Verbindung der Formel (IIID) zu ergeben:

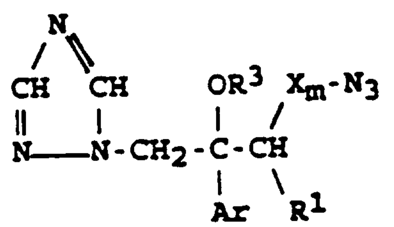

(IIID)

(worin $R^1$, $R^3$, X, m und Ar wie oben definiert sind).

**2.** Verfahren gemäß Anspruch 1, worin Ar in der Verbindung der Formel (IIIA) oder (X') eine Phenylgruppe mit einem oder zwei Halogensubstituenten darstellt.

**3.** Verfahren gemäß Anspruch 1, worin Ar in der Verbindung der Formel (IIIA) oder (X') eine Phenylgruppe mit einem oder zwei Fluor- und/oder Chlorsubstituenten darstellt.

**4.** Verfahren gemäß Anspruch 1, worin Ar in der Verbindung der Formel (IIIA) oder (X') eine 4-Chlorphenylgruppe, eine 4-Fluorphenylgruppe, eine 2,4-Dichlorphenylgruppe, eine 2,4-Difluorphenylgruppe, eine 2-Chlor-4-fluorphenylgruppe oder eine 4-Chlor-2-fluorphenylgruppe darstellt.

**5.** Verfahren gemäß einem der Ansprüche 1 bis 4, worin $R^1$ in der Verbindung der Formel (IIIA) oder (X') eine $C_1$ - $C_3$-Alkylgruppe darstellt.

**6.** Verfahren gemäß einem der Ansprüche 1 bis 4, worin $R^1$ in der Verbindung der Formel (IIIA) oder (X') eine Methyl- oder Ethylgruppe darstellt.

**7.** Verfahren gemäß einem der Ansprüche 1 bis 6, worin X in der Verbindung der Formel (IIIA) eine $C_1$- oder $C_2$-Alkylengruppe darstellt.

**8.** Verfahren gemäß einem der Ansprüche 1 bis 6, worin m in der Verbindung der Formel (IIIA) 0 ist.

**9.** Verfahren gemäß einem der Ansprüche 1 bis 8, worin die Reagenzien und Reaktionsbedingungen so gewählt sind, daß eine Verbindung der Formel (I) hergestellt wird, in der -Y-$R^2$ die Azidogruppe darstellt.

**10.** Verfahren gemäß einem der Ansprüche 1 bis 8, worin die Reagenzien und Reaktionsbedingungen so gewählt sind, daß eine Verbindung der Formel (I) hergestellt wird, in der Y eine Gruppe der Formel -N-($R^5$)CO-, -N($R^5$)CO-CH = CH-, -S-CO- oder -S-CO-CH = CH- darstellt, wobei
$R^5$ ein Wasserstoffatom oder eine $C_1$ - $C_4$-Alkylgruppe darstellt.

**11.** Verfahren gemäß einem der Ansprüche 1 bis 8 und 10, worin die Reagenzien und Reaktionsbedingungen so gewählt sind, daß eine Verbindung der Formel (I) hergestellt wird, in der $R^2$ eine Phenylgruppe mit einem oder zwei Substituenten, ausgewählt unter Halogenatomen und Trifluormethyl-, Trifluormethoxy- und Cyangruppen, darstellt.

**12.** Verfahren gemäß Anspruch 1, worin die Reagenzien und Reaktionsbedingungen so gewählt sind, daß eine Verbindung der Formel (I) hergestellt wird, in der:
Ar eine Phenylgruppe mit einem oder zwei Halogensubstituenten darstellt;
$R^1$ eine $C_1$ - $C_3$-Alkylgruppe darstellt;
m 0 ist oder X eine $C_1$- oder $C_2$-Alkylengruppe darstellt;
Y eine Gruppe der Formel -N($R^5$)CO-, -N($R^5$)CO-CH = CH-, -S-CO- oder -S-CO-CH = CH- darstellt, wobei
$R^5$ ein Wasserstoffatom oder eine $C_1$ - $C_4$-Alkylgruppe darstellt; und
$R^2$ eine Phenylgruppe mit einem oder zwei Substituenten, ausgewählt unter Halogenatomen und Trifluormethyl-, Trifluormethoxy- und Cyangruppen, darstellt;
oder
-Y-$R^2$ eine Azidogruppe darstellt.

**13.** Verfahren gemäß Anspruch 1, worin die Reagenzien und Reaktionsbedingungen so gewählt sind, daß eine Verbindung der Formel (I) hergestellt wird, in der:
Ar eine Phenylgruppe mit einem oder zwei Fluor- und/oder Chlorsubstituenten darstellt;
$R^1$ eine Methyl- oder Ethylgruppe darstellt;
m 0 ist oder X eine $C_1$- oder $C_2$-Alkylengruppe darstellt;
Y eine Gruppe der Formel -N($R^5$)CO-, -N($R^5$)CO-CH = CH-, -S-CO- oder -S-CO-CH = CH- darstellt, wobei
$R^5$ ein Wasserstoffatom oder eine $C_1$ - $C_4$-Alkylgruppe darstellt, und
$R^2$ eine Phenylgruppe mit einem oder zwei Substituenten, ausgewählt unter Halogenatomen und Trifluormethyl-, Trifluormethoxy- und Cyangruppen, darstellt;
oder
-Y-$R^2$ eine Azidogruppe darstellt.

**14.** Verfahren gemäß Anspruch 1, worin die Reagenzien und Reaktionsbedingungen so gewählt sind, daß eine Verbindung der Formel (I) hergestellt wird, in der:
$R^1$ eine Methyl- oder Ethylgruppe darstellt;
m 0 ist oder X eine $C_1$- oder $C_2$-Alkylengruppe darstellt;
Y eine Gruppe der Formel -N($R^5$)CO-, -N($R^5$)CO-CH = CH-, -S-CO- oder -S-CO-CH = CH- darstellt, wobei
$R^5$ ein Wasserstoffatom oder eine $C_1$ - $C_4$-Alkylgruppe darstellt;
$R^2$ eine Phenylgruppe mit einem oder zwei Substituenten, ausgewählt unter Halogenatomen und Trifluormethyl-, Trifluormethoxy- und Cyangruppen, darstellt;
oder
-Y-$R^2$ eine Azidogruppe darstellt; und
Ar eine 4-Chlorphenylgruppe, eine 4-Fluorphenylgruppe, eine 2,4-Dichlorphenylgruppe, eine 2,4-Difluorphenylgruppe, eine 2-Chlor-4-fluorphenylgruppe oder eine 4-Chlor-2-fluorphenylgruppe darstellt.

**15.** Verfahren gemäß Anspruch 1, worin die Reagenzien und Reaktionsbedingungen so gewählt sind, daß folgende Verbindungen oder deren Säureadditionssalze hergestellt werden:
2-(2,4-Dichlorphenyl)-1-(1H-1,2,4-triazol-1-yl)-3-[4-(trifluormethyl)benzoylamino]-2-butanol;
2-(2,4-Difluorphenyl)-3-[2-fluor-4-(trifluormethyl)benzoylamino]-1-(1H-1,2,4-triazol-1-yl)-2-butanol;

3-(4-Chlorbenzoylamino)-2-(2,4-difluorphenyl)-1-(1H-1,2,4-triazol-1-yl)-2-pentanol;
3-Azido-2-(2,4-difluorphenyl)-1-(1H-1,2,4-triazol-1-yl)-2-butanol;
3-Azido-2-(4-chlor-2-fluorphenyl)-1-(1H-1,2,4-triazol-1-yl)-2-butanol;
3-Azido-2-(2,4-difluorphenyl)-1-(1H-1,2,4-triazol-1-yl)-2-pentanol;
3-Azido-2-(2-chlor-4-fluorphenyl)-1-(1H-1,2,4-triazol-1-yl)-2-pentanol;
4-Azido-2-(2,4-difluorphenyl)-3-methyl-1-(1H-1,2,4-triazol-1-yl)-2-pentanol;
4-Azido-2-(2,4-difluorphenyl)-3-methyl-1-(1H-1,2,4-triazol-1-yl)-2-butanol;
4-Azido-2-(2,4-dichlorphenyl)-3-methyl-1-(1H-1,2,4-triazol-1-yl)-2-butanol;
3-Azido-2-(2,4-dichlorphenyl)-1-(1H-1,2,4-triazol-1-yl)-2-pentanol;
2-(4-Chlorphenyl)-1-(1H-1,2,4-triazol-1-yl)-3-[4-(trifluormethyl)benzoylamino]-2-butanol;
2-(2,4-Difluorphenyl)-1-(1H-1,2,4-triazol-1-yl)-3-[4-(trifluormethyl)benzoylamino]-2-butanol;
3-(4-Cyanobenzoylamino)-2-(2,4-difluorphenyl)-1-(1H-1,2,4-triazol-1-yl)-2-butanol;
2-(4-Chlorphenyl)-3-[2-fluor-4-(trifluormethyl)benzoylamino]-1-[1H-1,2,4-triazol-1-yl)-2-butanol;
2-(2,4-Dichlorphenyl)-1-(1H-1,2,4-triazol-1-yl)-3-[4-(trifluormethyl)benzoylthio]-2-butanol;
3-(4-Chlorcinnamoylamino)-2-(2,4-difluorphenyl)-1-[1H-1,2,4-triazol-1-yl)-2-butanol;
2-(2,4-Difluorphenyl)-1-(1H-1,2,4-triazol-1-yl)-3-(N-methyl-N-[4-(trifluormethyl)benzoyl]amino)-2-butanol;
oder
2-(2,4-Difluorphenyl)-3-(N-[2-fluor-4-(trifluormethyl)benzoyl]-N-methylamino}-1-(1H-1,2,4-triazol-1-yl)-2-butanol.

**16.** Verfahren zur Herstellung einer Verbindung der Formel (IX) oder deren Säureadditionssalze

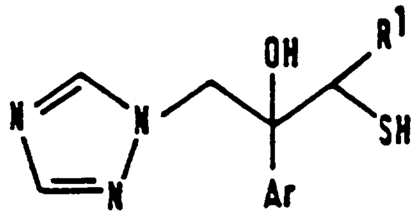

(IX)

worin:
Ar eine Phenylgruppe oder eine Phenylgruppe mit einem oder zwei Halogen- und/oder Trifluormethylsubstituenten darstellt,
$R^1$ eine $C_1$ - $C_6$-Alkylgruppe darstellt;
wobei das Verfahren das Inkontaktbringen einer Verbindung der Formel (VIII)

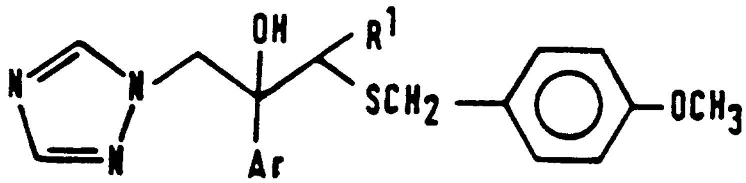

(VIII)

worin Ar und $R^1$ wie oben definiert sind,
mit einer starken Säure umfaßt.

**17.** Verfahren zur Herstellung einer Agrochemikalien-Zusammensetzung, welches umfaßt:
Vermischen eines für landwirtschaftliche Zwecke verträglichen Trägermaterials oder Verdünnungsmittels mit mindestens einem Fungizid, worin das Fungizid mindestens eine Verbindung der Formel (I) ist:

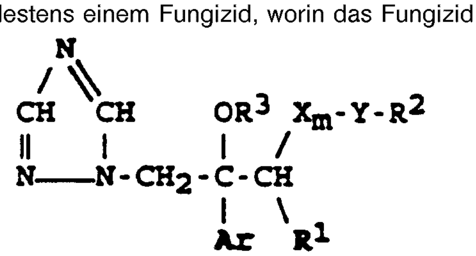

(I)

worin:
Ar eine Phenylgruppe oder eine Phenylgruppe mit einem oder zwei Halogenatomen und/oder Trifluormethyl-Substituenten darstellt;
$R^1$ eine $C_1$ - $C_6$-Alkylgruppe oder, wenn $R^3$ und $-X_m-Y-R^2$ zusammen die Gruppe der Formel (II) darstellen, $R^1$ ein Wasserstoffatom oder eine $C_1$ - $C_6$-Alkylgruppe darstellt;
X eine $C_1$ - $C_6$-Alkylengruppe, eine $C_2$ - $C_6$-aliphatische Kohlenwasserstoff-Gruppe mit einer oder zwei Kohlenstoff-Kohlenstoff-Doppelbindungen, eine $C_2$ - $C_6$-aliphatische Kohlenwasserstoff-Gruppe mit einer oder zwei Kohlenstoff-Kohlenstoff-Dreifachbindungen, eine $C_3$ - $C_6$-Cycloalkylengruppe, eine $C_3$ - $C_6$-Cycloalkylengruppe mit einer oder zwei Kohlenstoff-Kohlenstoff-Doppelbindungen, eine ($C_1$ - $C_3$-Alkylen)-($C_3$ - $C_6$-cycloalkylen)-Gruppe oder eine ($C_3$ - $C_6$-Cycloalkylen)-($C_1$ - $C_3$-alkylen)-Gruppe darstellt;
m 0 oder 1 ist;
$-Y-R^2$ die Azidogruppe, die Phthalimid-Gruppe, die 1-Oxo-2,3-dihydro-2-isoindolyl-Gruppe, die Phthalimid- oder 1-Oxo-2,3-dihydro-2-isoindolyl-Gruppe mit mindestens einem der nachstehend definierten Substituenten (a), eine geschützte Hydroxylgruppe oder eine Gruppe der Formel $-OSO_2R^4$ darstellt, worin:

$R^4$ eine $C_1$ - $C_4$-Alkylgruppe, eine $C_1$ - $C_4$-Halogenalkylgruppe, eine Phenylgruppe oder eine Phenylgruppe mit einem Substituenten, ausgewählt unter Nitrogruppen, Halogenatomen und $C_1$ - $C_4$-Alkylgruppen, darstellt;

oder

Y eine Gruppe der Formel $-N(R^5)CO-$, $-N(R^5)CO-CH=CH-$, -O-CO-, -O-CO-CH = CH-, -S-CO- oder -S-CO-CH = CH- darstellt, worin:

$R^5$ ein Wasserstoffatom oder eine $C_1$ - $C_4$-Alkylgruppe darstellt;

$R^2$ eine $C_1$ - $C_6$-Alkylgruppe, eine $C_1$ - $C_6$-Halogenalkylgruppe, eine Phenylgruppe, eine Phenylgruppe mit mindestens einem der nachstehend definierten Substituenten (a), eine Naphthylgruppe oder eine heterocyclische Gruppe mit 5 oder 6 Ringatomen darstellt, von denen 1 bis 3 Stickstoff- und/oder Sauerstoff- und/oder Schwefel-Heteroatome sind, wobei die heterocyclische Gruppe unsubstituiert ist oder 1 bis 3 der nachstehend definierten Substituenten (b) aufweist; und
$R^3$ ein Wasserstoffatom darstellt;

oder

$R^3$ und $-X_m-Y_n-R^2$ zusammen eine Gruppe der Formel (II) darstellen:

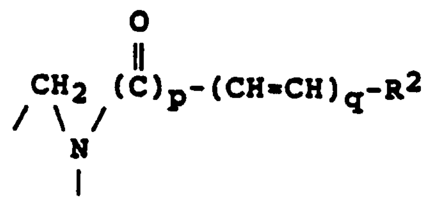

(II)

worin:

$R^2$ wie oben definiert ist;
p 0 oder 1 ist und
q 0 oder 1 ist;

Substituenten (a):
$C_1$ - $C_4$-Alkylgruppen, $C_1$ - $C_4$-Halogenalkylgruppen, $C_1$ - $C_4$-Alkoxygruppen, $C_1$ - $C_4$-Halogenalkoxygruppen, Halogenatome, Nitrogruppen, Cyangruppen, Hydroxylgruppen, Benzyloxygruppen, Hydroxymethylgruppen, Gruppen der Formel $-CH_2-OCO-R^7$, Gruppen der Formel $-CO-R^6$, Gruppen der Formel $-COOR^7$, Gruppen der Formel $-SO_rR^7$, Aminogruppen, Mono- und Dialkylaminogruppen, worin jede Alkylgruppe $C_1$ - $C_4$ umfaßt und Acylaminogruppen von Carbonsäuren;
Substituenten (b):
$C_1$ - $C_4$-Alkylgruppen, $C_1$ - $C_4$-Halogenalkylgruppen, Halogenatome, Cyangruppen und Nitrogruppen;
$R^6$ eine $C_1$ - $C_6$-Alkylgruppe oder eine $C_1$ - $C_6$-Halogenalkylgruppe darstellt,
$R^7$ eine $C_1$ - $C_6$-Alkylgruppe darstellt,
r 0, 1 oder 2 ist,
oder deren Säureadditionssalze.

**18.** Verfahren gemäß Anspruch 17, worin das Fungizid eine Verbindung gemäß einem der Ansprüche 1 bis 15 ist.

**19.** Verfahren gemäß Anspruch 17, worin das Fungizid mindestens eine der folgenden Verbindungen ist:
2-(2,4-Dichlorphenyl)-1-(1H-1,2,4-triazol-1-yl)-3-[4-(trifluormethyl)benzoylamino]-2-butanol;
2-(2,4-Difluorphenyl)-3-[2-(fluor-4-(trifluormethyl)-benzoylamino]-1-(1H-1,2,4-triazol-1-yl)-2-butanol;
3-(4-Chlorbenzoylamino)-2-(2,4-difluorphenyl)-1-(1H-1,2,4-triazol-1-yl)-2-pentanol;
3-Azido-2-(2,4-difluorphenyl)-1-(1H-1,2,4-triazol-1-yl)-2-butanol;
3-Azido-2-(4-Chlor-2-fluorphenyl)-1-(1H-1,2,4-triazol-1-yl)-2-butanol;
3-Azido-2-(2,4-difluorphenyl)-1-(1H-1,2,4-triazol-1-yl)-2-pentanol;
3-Azido-2-(2-Chlor-4-fluorphenyl)-1-(1H-1,2,4-triazol-1-yl)-2-pentanol;
4-Azido-2-(2,4-difluorphenyl)-3-methyl-1-(1H-1,2,4-triazol-1-yl)-2-pentanol;
4-Azido-2-(2,4-difluorphenyl)-3-methyl-1-(1H-1,2,4-triazol-1-yl)-2-butanol;
4-Azido-2-(2,4-dichlorphenyl)-3-methyl-1-(1H-1,2,4-triazol-1-yl)-2-butanol; und
3-Azido-2-(4-chlorphenyl)-1-(1H-1,2,4-triazol-1-yl)-2-pentanol;
oder deren Säureadditionssalze.

**20.** Verfahren zur Herstellung einer fungiziden Arzneimittelzusammensetzung, welches das Mischen eines pharmazeutisch verträglichen Trägermaterials oder Verdünnungsmittels mit mindestens einem Fungizid umfaßt, worin das Fungizid mindestens eine in Anspruch 17 definierte Verbindung der Formel (I) ist.

**21.** Verfahren gemäß Anspruch 20, worin das Fungizid eine Verbindung gemäß einem der Ansprüche 1 bis 15 ist.

**22.** Verfahren gemäß Anspruch 21, worin das Fungizid mindestens eine der folgenden Verbindungen ist:
2-(4-Chlorphenyl)-1-(1H-1,2,4-triazol-1-yl)-3-[4-(trifluormethyl)benzoylamino]-2-butanol;
2-(2,4-Difluorphenyl)-1-(1H-1,2,4-triazol-1-yl)-3-[4-(trifluormethyl)benzoylamino]-2-butanol;
2-(2,4-Difluorphenyl)-3-[2-fluor-4-(trifluormethyl)benzoylamino]-1-(1H-1,2,4-triazol-1-yl)-2-butanol;
3-(4-Cyanobenzoylamino)-2-(2,4-difluorphenyl)-1-(1H-1,2,4-triazol-1-yl)-2-butanol;
2-(4-Chlorphenyl)-3-[2-fluor-4-(trifluormethyl)benzoylamino]-1-(1H-1,2,4-triazol-1-yl)-2-butanol;
2-(2,4-Dichlorphenyl)-1-(1H-1,2,4-triazol-1-yl)-3-[4-(trifluormethyl)benzoylthio]-2-butanol;
3-(4-Chlorcinnamoylamino)-2-(2,4-difluorphenyl)-1-(1H-1,2,4-triazol-1-yl)-2-butanol;
2-(2,4-Difluorphenyl)-1-(1H-1,2,4-triazol-1-yl)-3-{N-methyl-N-[4-(trifluormethyl)benzoyl]amino}-2-butanol; und
2-(2,4-Difluorphenyl)-3-{N-[2-fluor-4-(trifluormethyl)benzoyl]-N-methylamino}-1-(1H-1,2,4-triazol-1-yl)-2-butanol;
oder ein pharmazeutisch verträgliches Säureadditionssalz derselben.

**23.** Verfahren zum Schutz von Pflanzen, Samen sowie Teilen von Pflanzen oder Samen vor einer Pilzinfektion, welches das Auftragen mindestens einer Verbindung der Formel (I) oder deren Säureadditionssalze, wie in Anspruch 17 definiert, auf die Pflanzen, Samen, Teile von Pflanzen oder Samen, oder auf einen Ort, welcher diese einschließt, umfaßt.

**24.** Verwendung einer Verbindung der Formel (I), gemäß der Definition in Anspruch 17, oder eines pharmazeutisch verträglichen Säureadditionssalzes derselben, zur Herstellung eines Arzneimittels zur Behandlung oder zur Prophylaxe von Pilzinfektionen.

**25.** Verwendung gemäß Anspruch 24 einer Verbindung der Formel (I), gemäß der Definition in einem der Ansprüche 1 bis 15, oder eines pharmazeutisch verträglichen Säureadtionssalzes derselben.

**26.** Verwendung gemäß Anspruch 24 einer der Verbindungen, auf die in Anspruch 22 Bezug genommen wird.

**27.** Verwendung einer Verbindung der Formel (I), wie in Anspruch 17 definiert, oder deren Säureadditionssalze, zur Herstellung eines Fungizids zur Verwendung als Agrochemikalie.

**28.** Verwendung gemäß Anspruch 27 einer Verbindung der Formel (I) oder deren Säureadditionssalze, wie in einem der Ansprüche 1 bis 15 definiert.

**29.** Verwendung gemäß Anspruch 27 einer der Verbindungen, auf die in Anspruch 19 Bezug genommen wird.

**Patentansprüche für folgenden Vertragsstaat : GR**

**1.** Verbindungen zur Verwendung als Agrochemikalie der Formel (I):

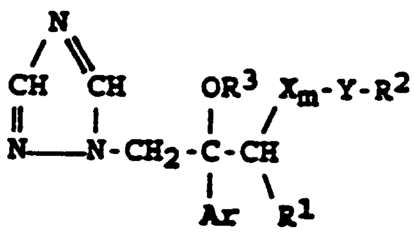

(I)

worin:

Ar eine Phenylgruppe oder eine Phenylgruppe mit einem oder zwei Halogen- und/oder Trifluormethylsubstituenten darstellt;

$R^1$ eine $C_1$ - $C_6$-Alkylgruppe oder, wenn $R^3$ und $-X_m$-Y-$R^2$ zusammen die Gruppe der Formel (II) darstellen, $R^1$ ein Wasserstoffatom oder eine $C_1$ - $C_6$-Alkylgruppe darstellt;

X eine $C_1$ - $C_6$-Alkylengruppe, eine $C_2$ - $C_6$-aliphatische Kohlenwasserstoff-Gruppe mit einer oder zwei Kohlenstoff-Kohlenstoff-Doppelbindungen, eine $C_2$ - $C_6$-aliphatische Kohlenwasserstoff-Gruppe mit einer oder zwei Kohlenstoff-Kohlenstoff-Dreifachbindungen, eine $C_3$ - $C_6$-Cycloalkylengruppe, eine $C_3$ - $C_6$-Cycloalkylengruppe mit einer oder zwei Kohlenstoff-Kohlenstoff-Doppelbindungen, eine ($C_1$ - $C_3$-Alkylen)-($C_3$ - $C_6$-Cycloalkylen)-Gruppe oder eine ($C_3$ - $C_6$-Cycloalkylen)-($C_1$ - $C_3$-Alkylen)-Gruppe darstellt;

m 0 oder 1 ist;

-Y-$R^2$ die Azidogruppe, die Phthalimid-Gruppe, die 1-Oxo-2,3-dihydro-2-isoindolyl-Gruppe oder die Phthalimid- oder 1-Oxo-2,3-dihydro-2-isoindolyl-Gruppe mit mindestens einem der nachstehend definierten Substituenten (a) darstellt;

oder

Y eine Gruppe der Formel -N($R^5$)-CO-, -N($R^5$)CO-CH = CH-, -O-CO-, -O-CO-CH = CH-, -S-CO- oder -S-CO-CH = CH- darstellt, wobei:

$R^5$ ein Wasserstoffatom oder eine $C_1$ - $C_4$-Alkylgruppe darstellt;

$R^2$ eine $C_1$ - $C_6$-Alkylgruppe, eine $C_1$ - $C_6$-Halogenalkylgruppe, eine Phenylgruppe, eine Phenylgruppe mit mindestens einem der nachstehend definierten Substituenten (a), eine Naphthylgruppe oder eine heterocyclische Gruppe mit 5 oder 6 Ringatomen darstellt, von denen 1 bis 3 Stickstoff- und/oder Sauerstoff- und/oder Schwefel-Heteroatome sind, wobei die heterocyclische Gruppe unsubstituiert ist oder 1 bis 3 der nachstehend definierten Substituenten (b) aufweist; und

$R^3$ ein Wasserstoffatom darstellt;

oder

$R^3$ und $-X_m$-Y-$R^2$ zusammengenommen eine Gruppe der Formel (II) darstellen:

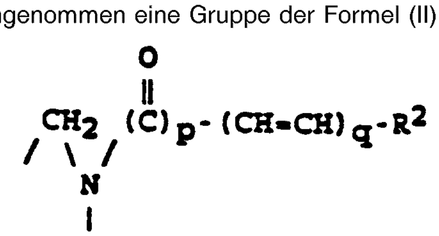

(II)

worin:

$R^2$ wie oben definiert ist;

p 0 oder 1 ist; und

q 0 oder 1 ist;

Substituenten (a):

$C_1$ - $C_4$-Alkylgruppen, $C_1$ - $C_4$-Halogenalkylgruppen, $C_1$ - $C_4$-Alkoxygruppen, $C_1$ - $C_4$-Halogenalkoxy-

gruppen, Halogenatome, Nitrogruppen, Cyangruppen, Hydroxylgruppen, Benzyloxygruppen, Hydroxymethylgruppen, Gruppen der Formel $-CH_2-OCO-R^7$, Gruppen der Formel $-CO-R^6$, Gruppen der Formel $-COOR^7$, Gruppen der Formel $-SO_rR^7$, Aminogruppen, Mono- und Dialkylaminogruppen, worin jede Alkylgruppe $C_1$ - $C_4$ ist und Acylaminogruppen von Carbonsäuren;
Substituenten (b):
$C_1$ - $C_4$-Alkylgruppen, $C_1$ - $C_4$-Halogenalkylgruppen, Halogenatome, Cyangruppen und Nitrogruppen,
$R^6$ eine $C_1$ - $C_6$-Alkylgruppe oder eine $C_1$ - $C_6$-Halogenalkylgruppe darstellt,
$R^7$ eine $C_1$ - $C_6$-Alkylgruppe darstellt,
r 0, 1 oder 2 ist;
und deren Säureadditionssalze,
vorausgesetzt, daß, falls m 0 ist und Y eine Gruppe der Formel $-N(R^5)CO-$ ist, $R^5$ Wasserstoff sein muß.

**2.** Verbindungen gemäß Anspruch 1, worin Ar eine Phenylgruppe mit einem oder zwei Halogensubstituenten darstellt.

**3.** Verbindungen gemäß Anspruch 1, worin Ar eine Phenylgruppe mit einem oder zwei Fluor- und/oder Chlorsubstituenten darstellt.

**4.** Verbindungen gemäß Anspruch 1, worin Ar eine 4-Chlorphenylgruppe, eine 4-Fluorphenylgruppe, eine 2,4-Dichlorphenylgruppe, eine 2,4-Difluorphenylgruppe, eine 2-Chlor-4-fluorphenylgruppe oder eine 4-Chlor-2-fluorphenylgruppe darstellt.

**5.** Verbindungen gemäß einem der Ansprüche 1 bis 4, worin $R^1$ eine $C_1$ - $C_3$-Alkylgruppe darstellt.

**6.** Verbindungen gemäß einem der Ansprüche 1 bis 4, worin $R^1$ eine Methyl- oder Ethylgruppe darstellt.

**7.** Verbindungen gemäß einem der Ansprüche 1 bis 6, worin X eine $C_1$- oder $C_2$-Alkylengruppe darstellt.

**8.** Verbindungen gemäß einem der Ansprüche 1 bis 6, worin m 0 ist.

**9.** Verbindungen gemäß einem der Ansprüche 1 bis 8, worin $-Y-R^2$ die Azidogruppe darstellt.

**10.** Verbindungen gemäß einem der Ansprüche 1 bis 8, worin Y eine Gruppe der Formel $-N(R^5)CO-$, $-N(R^5)CO-CH=CH-$, -S-CO- oder -S-CO-CH=CH- darstellt, wobei:
$R^5$ ein Wasserstoffatom oder eine $C_1$ - $C_4$-Alkylgruppe darstellt.

**11.** Verbindungen gemäß einem der Ansprüche 1 bis 8 und 10, worin $R^2$ eine Phenylgruppe mit einem oder zwei Substituenten, ausgewählt unter Halogenatomen und Trifluormethyl-, Trifluormethoxy- und Cyangruppen, darstellt.

**12.** Verbindungen gemäß Anspruch 1, worin:
Ar eine Phenylgruppe mit einem oder zwei Halogensubstituenten darstellt;
$R^1$ eine $C_1$ - $C_3$-Alkylgruppe darstellt;
m 0 ist oder X eine $C_1$- oder $C_2$-Alkylengruppe darstellt;
Y eine Gruppe der Formel $-N(R^5)CO-$, $-N(R^5)CO-CH=CH-$, -S-CO- oder -S-CO-CH=CH- darstellt, wobei:
$R^5$ ein Wasserstoffatom oder eine $C_1$ - $C_4$-Alkylgruppe darstellt; und
$R^2$ eine Phenylgruppe mit einem oder zwei Substituenten, ausgewählt unter Halogenatomen und Trifluormethyl-, Trifluormethoxy- und Cyangruppen, darstellt;
oder
$-Y-R^2$ eine Azidogruppe darstellt.

**13.** Verbindungen gemäß Anspruch 1, worin:
Ar eine Phenylgruppe mit einem oder zwei Fluor- und/oder Chlorsubstituenten darstellt;
$R^1$ eine Methyl- oder Ethylgruppe darstellt;
m 0 ist oder X eine $C_1$- oder $C_2$-Alkylengruppe darstellt;
Y eine Gruppe der Formel $-N(R^5)CO-$, $-N(R^5)CO-CH=CH-$, -S-CO- oder -S-CO-CH=CH- darstellt,

wobei

$R^5$ ein Wasserstoffatom oder eine $C_1$ - $C_4$-Alkylgruppe darstellt, und

$R^2$ eine Phenylgruppe mit einem oder zwei Substituenten, ausgewählt unter Halogenatomen und Trifluormethyl-, Trifluormethoxy- und Cyangruppen, darstellt;

oder

-Y-$R^2$ eine Azidogruppe darstellt.

**14.** Verbindungen gemäß Anspruch 1, worin:
$R^1$ eine Methyl- oder Ethylgruppe darstellt;
m 0 ist oder X eine $C_1$- oder $C_2$-Alkylengruppe darstellt;
Y eine Gruppe der Formel -N($R^5$)CO-, -N($R^5$)CO-CH=CH-, -S-CO- oder -S-CO-CH=CH- darstellt, wobei

$R^5$ ein Wasserstoffatom oder eine $C_1$ - $C_4$-Alkylgruppe darstellt;

$R^2$ eine Phenylgruppe mit einem oder zwei Substituenten, ausgewählt unter Halogenatomen und Trifluormethyl-, Trifluormethoxy- und Cyangruppen, darstellt;

oder

-Y-$R^2$ eine Azidogruppe darstellt; und

Ar eine 4-Chlorphenylgruppe, eine 4-Fluorphenylgruppe, eine 2,4-Dichlorphenylgruppe, eine 2,4-Difluorphenylgruppe, eine 2-Chlor-4-fluorphenylgruppe oder eine 4-Chlor-2-fluorphenylgruppe darstellt.

**15.** Verbindungen gemäß Anspruch 1, ausgewählt unter folgenden Verbindungen:
2-(2,4-Dichlorphenyl)-1-(1H-1,2,4-triazol-1-yl)-3-[4-(trifluormethyl)benzoylamino]-2-butanol;
2-(2,4-Difluorphenyl)-3-[2-fluor-4-(trifluormethyl)benzoylamino]-1-(1H-1,2,4-triazol-1-yl)-2-butanol;
3-(4-Chlorbenzoylamino)-2-(2,4-difluorphenyl)-1-(1H-1,2,4-triazol-1-yl)-2-pentanol;
3-Azido-2-(2,4-difluorphenyl)-1-(1H-1,2,4-triazol-1-yl)-2-butanol;
3-Azido-2-(4-chlor-2-fluorphenyl)-1-(1H-1,2,4-triazol-1-yl)-2-butanol;
3-Azido-2-(2,4-difluorphenyl)-1-(1H-1,2,4-triazol-1-yl)-2-pentanol;
3-Azido-2-(2-chlor-4-fluorphenyl)-1-(1H-1,2,4-triazol-1-yl)-2-pentanol;
4-Azido-2-(2,4-difluorphenyl)-3-methyl-1-(1H-1,2,4-triazol-1-yl)-2-pentanol;
4-Azido-2-(2,4-difluorphenyl)-3-methyl-1-(1H-1,2,4-triazol-1-yl)-2-butanol;
4-Azido-2-(2,4-dichlorphenyl)-3-methyl-1-(1H-1,2,4-triazol-1-yl)-2-butanol;
3-Azido-2-(4-chlorphenyl)-1-(1H-1,2,4-triazol-1-yl)-2-pentanol;
2-(4-Chlorphenyl)-1-(1H-1,2,4-triazol-1-yl)-3-[4-(trifluormethyl)benzoylamino]-2-butanol;
2-(2,4-Difluorphenyl)-1-(1H-1,2,4-triazol-1-yl)-3-[4-(trifluormethyl)benzoylamino]-2-butanol;
3-(4-Cyanobenzoylamino)-2-(2,4-difluorphenyl)-1-(1H-1,2,4-triazol-1-yl)-2-butanol;
2-(4-Chlorphenyl)-3-[2-fluor-4-(trifluormethyl)benzoylamino]-1-[1H-1,2,4-triazol-1-yl)-2-butanol;
2-(2,4-Dichlorphenyl)-1-(1H-1,2,4-triazol-1-yl)-3-[4-(trifluormethyl)benzoylthio]-2-butanol;
3-(4-Chlorcinnamoylamino)-2-(2,4-difluorphenyl)-1-[1H-1,2,4-triazol-1-yl)-2-butanol;
2-(2,4-Difluorphenyl)-1-(1H-1,2,4-triazol-1-yl)-3-{N-methyl-N-[4-(trifluormethyl)benzoyl]amino}-2-butanol;
und
2-(2,4-Difluorphenyl)-3-{N-[2-fluor-4-(trifluormethyl)benzoyl]-N-methylamino}-1-(1H-1,2,4-triazol-1-yl)-2-butanol;
und deren Säureadditionssalze.

**16.** Verbindungen zur Verwendung als Agrochemikalie der Formel (IX)

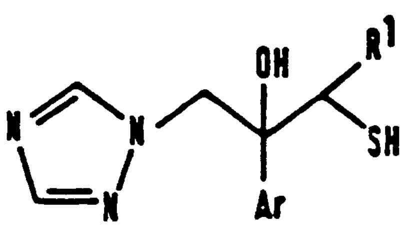

(IX)

worin:

Ar eine Phenylgruppe oder eine Phenylgruppe mit einem oder zwei Halogen- und/oder Trifluormethylsubstituenten darstellt,

$R^1$ eine $C_1$ - $C_6$-Alkylgruppe darstellt;
und deren Säureadditionssalze.

17. Agrochemikalien-Zusammensetzung, die ein für landwirtschaftliche Zwecke verträgliches Trägermaterial oder Verdünnungsmittel im Gemisch mit mindestens einem Fungizid enthält, worin das Fungizid mindestens eine Verbindung der Formel (I) ist:

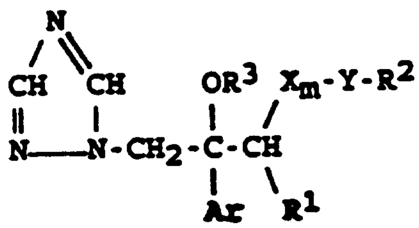

(I)

worin:
Ar eine Phenylgruppe oder eine Phenylgruppe mit einem oder zwei Halogenatomen und/oder Trifluormethyl-Substituenten darstellt;
$R^1$ eine $C_1$ - $C_6$-Alkylgruppe oder, wenn $R^3$ und $-X_m$-Y-$R^2$ zusammen die Gruppe der Formel (II) darstellen, $R^1$ ein Wasserstoffatom oder eine $C_1$ - $C_6$-Alkylgruppe darstellt;
X eine $C_1$ - $C_6$-Alkylengruppe, eine $C_2$ - $C_6$-aliphatische Kohlenwasserstoff-Gruppe mit einer oder zwei Kohlenstoff-Kohlenstoff-Doppelbindungen, eine $C_2$ - $C_6$-aliphatische Kohlenwasserstoff-Gruppe mit einer oder zwei Kohlenstoff-Kohlenstoff-Dreifachbindungen, eine $C_3$ - $C_6$-Cycloalkylengruppe, eine $C_3$ - $C_6$-Cycloalkylengruppe mit einer oder zwei Kohlenstoff-Kohlenstoff-Doppelbindungen, eine ($C_1$ - $C_3$-Alkylen)-($C_3$ - $C_6$-cycloalkylen)-Gruppe oder eine ($C_3$ - $C_6$-Cycloalkylen)-($C_1$ - $C_3$-alkylen)-Gruppe darstellt;
m 0 oder 1 ist;
-Y-$R^2$ die Azidogruppe, die Phthalimid-Gruppe, die 1-Oxo-2,3-dihydro-2-isoindolyl-Gruppe, die Phthalimid- oder 1-Oxo-2,3-di-2-isoindolyl-Gruppe mit mindestens einem der nachstehend definierten Substituenten (a), eine geschützte Hydroxylgruppe odereine Gruppe der Formel $-OSO_2R^4$ darstellt, in der:

$R^4$ eine $C_1$ - $C_4$-Alkylgruppe, eine $C_1$ - $C_4$-Halogenalkylgruppe, eine Phenylgruppe oder eine Phenylgruppe mit einem Substituenten, ausgewählt unter Nitrogruppen, Halogenatomen und $C_1$ - $C_4$-Alkylgruppen, darstellt;

oder

Y eine Gruppe der Formel -N($R^5$)CO-, -N($R^5$)CO-CH=CH-, -O-CO-, -O-CO-CH=CH-, -S-CO- oder -S-CO-CH=CH- darstellt, worin:

$R^5$ ein Wasserstoffatom oder eine $C_1$ - $C_4$-Alkylgruppe darstellt;

$R^2$ eine $C_1$ - $C_6$-Alkylgruppe, eine $C_1$ - $C_6$-Halogenalkylgruppe, eine Phenylgruppe, eine Phenylgruppe mit mindestens einem der nachstehend definierten Substituenten (a), eine Naphthylgruppe oder eine heterocyclische Gruppe mit 5 oder 6 Ringatomen darstellt, von denen 1 bis 3 Stickstoff- und/oder Sauerstoff- und/oder Schwefel-Heteroatome sind, wobei die heterocyclische Gruppe unsubstituiert ist oder 1 bis 3 der nachstehend definierten Substituenten (b) aufweist; und
$R^3$ ein Wasserstoffatom darstellt;

oder

$R^3$ und $-X_m-Y_n-R^2$ zusammen eine Gruppe der Formel (II) darstellen:

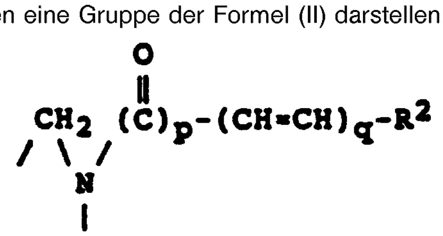

(II)

worin:

$R^2$ wie oben definiert ist;
p 0 oder 1 ist und

q 0 oder 1 ist;
Substituenten (a):
$C_1$ - $C_4$-Alkylgruppen, $C_1$ - $C_4$-Halogenalkylgruppen, $C_1$ - $C_4$-Alkoxygruppen, $C_1$ - $C_4$-Halogenalkoxygruppen, Halogenatome, Nitrogruppen, Cyangruppen, Hydroxylgruppen, Benzyloxygruppen, Hydroxymethylgruppen, Gruppen der Formel $-CH_2-OCO-R^7$, Gruppen der Formel $-CO-R^6$, Gruppen der Formel $-COOR^7$, Gruppen der Formel $-SO_rR^7$, Aminogruppen, Mono- und Dialkylaminogruppen, worin jede Alkylgruppe $C_1$ - $C_4$ umfaßt und Acylaminogruppen von Carbonsäuren;
Substituenten (b):
$C_1$ - $C_4$-Alkylgruppen, $C_1$ - $C_4$-Halogenalkylgruppen, Halogenatome, Cyangruppen und Nitrogruppen,
$R^6$ eine $C_1$ - $C_6$-Alkylgruppe oder eine $C_1$ - $C_6$-Halogenalkylgruppe darstellt,
$R^7$ eine $C_1$ - $C_6$-Alkylgruppe darstellt,
r 0, 1 oder 2 ist,
oder deren Säureadditionssalze.

**18.** Zusammensetzung gemäß Anspruch 17, worin das Fungizid eine Verbindung gemäß einem der Ansprüche 1 bis 15 ist.

**19.** Zusammensetzung gemäß Anspruch 18, worin das Fungizid mindestens eine der folgenden Verbindungen ist:
2-(2,4-Dichlorphenyl)-1-(1H-1,2,4-triazol-1-yl)-3-[4-(trifluormethyl)benzoylamino]-2-butanol;
2-(2,4-Difluorphenyl)-3-[2-(fluor-4-(trifluormethyl)-benzoylamino]-1-(1H-1,2,4-triazol-1-yl)-2-butanol;
3-(4-Chlorbenzoylamino)-2-(2,4-difluorphenyl)-1-(1H-1,2,4-triazol-1-yl)-2-pentanol;
3-Azido-2-(2,4-difluorphenyl)-1-(1H-1,2,4-triazol-1-yl)-2-butanol;
3-Azido-2-(4-Chlor-2-fluorphenyl)-1-(1H-1,2,4-triazol-1-yl)-2-butanol;
3-Azido-2-(2,4-difluorphenyl)-1-(1H-1,2,4-triazol-1-yl)-2-pentanol;
3-Azido-2-(2-Chlor-4-fluorphenyl)-1-(1H-1,2,4-triazol-1-yl)-2-pentanol;
4-Azido-2-(2,4-difluorphenyl)-3-methyl-1-(1H-1,2,4-triazol-1-yl)-2-pentanol;
4-Azido-2-(2,4-difluorphenyl)-3-methyl-1-(1H-1,2,4-triazol-1-yl)-2-butanol;
4-Azido-2-(2,4-dichlorphenyl)-3-methyl-1-(1H-1,2,4-triazol-1-yl)-2-butanol; und
3-Azido-2-(2,4-dichlorphenyl)-1-(1H-1,2,4-triazol-1-yl)-2-pentanol;
oder deren Säureadditionssalze.

**20.** Fungizide Arzneimittelzusammensetzung, welche ein pharmazeutisch verträgliches Trägermaterial oder Verdünnungsmittel im Gemisch mit mindestens einem Fungizid enthält, worin das Fungizid mindestens eine in Anspruch 17 definierte Verbindung der Formel (I) ist.

**21.** Zusammensetzung gemäß Anspruch 20, worin das Fungizid eine Verbindung gemäß einem der Ansprüche 1 bis 15 ist.

**22.** Zusammensetzung gemäß Anspruch 20, worin das Fungizid mindestens eine der folgenden Verbindungen ist:
2-(4-Chlorphenyl)-1-(1H-1,2,4-triazol-1-yl)-3-[4-(trifluormethyl)benzoylamino]-2-butanol;
2-(2,4-Difluorphenyl)-1-(1H-1,2,4-triazol-1-yl)-3-[4-(trifluormethyl)benzoylamino]-2-butanol;
2-(2,4-Difluorphenyl)-3-[2-fluor-4-(trifluormethyl)benzoylamino]-1-(1H-1,2,4-triazol-1-yl)-2-butanol;
3-(4-Cyanobenzoylamino)-2-(2,4-difluorphenyl)-1-(1H-1,2,4-triazol-1-yl)-2-butanol;
2-(4-Chlorphenyl)-3-[2-fluor-4-(trifluormethyl)benzoylamino]-1-(1H-1,2,4-triazol-1-yl)-2-butanol;
2-(2,4-Dichlorphenyl)-1-(1H-1,2,4-triazol-1-yl)-3-[4-(trifluormethyl)benzoylthio]-2-butanol;
3-(4-Chlorcinnamoylamino)-2-(2,4-difluorphenyl)-1-(1H-1,2,4-triazol-1-yl)-2-butanol;
2-(2,4-Difluorphenyl)-1-(1H-1,2,4-triazol-1-yl)-3-{N-methyl-N-[4-(trifluormethyl)benzoyl]amino}-2-butanol; und
2-(2,4-Difluorphenyl)-3-{N-[2-fluor-4-(trifluormethyl)benzoyl]-N-methylamino}-1-(1H-1,2,4-triazol-1-yl)-2-butanol;
oder ein pharmazeutisch verträgliches Säureadditionssalz derselben.

**23.** Verfahren zur Herstellung einer Verbindung der Formel (I), oder deren Säureadditionssalze, wie in einem der Ansprüche 1 bis 15 definiert, welches umfaßt:
(a) Umsetzung einer Verbindung der Formel (IIIA):

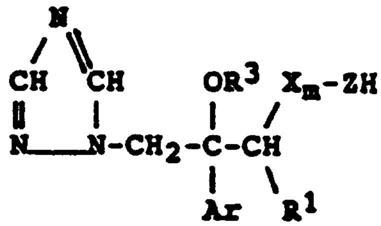

(IIIA)

[worin:
$R^1$, $R^3$, X, m und Ar wie in Anspruch 1 definiert sind; und Z ein Sauerstoff- oder Schwefelatom oder eine Gruppe der Formel -NH- oder -N($CHR^8R^9$)- bedeutet, worin $R^8$ und $R^9$ gleich oder verschieden sind und jeweils ein Wasserstoffatom oder eine Methyl- oder Ethylgruppe darstellen,]
mit einer Verbindung der Formel (V):

$R^{2'}$-COOH (V)

(worin $R^{2'}$irgendeine der in Anspruch 1 für $R^2$ definierten Gruppen darstellt, oder eine Gruppe der Formel $R^2$-CH=CH-darstellt, wobei $R^2$ wie in Anspruch 1 definiert ist), oder mit einem reaktivem Derivat derselben,
wobei eine Verbindung der Formel (I') gebildet wird:

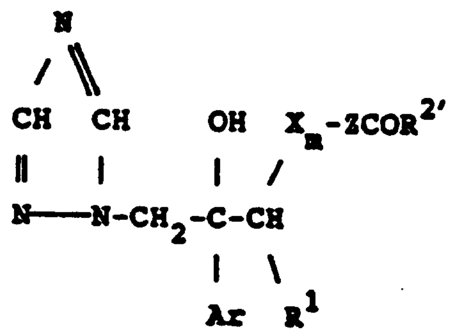

(I')

(worin $R^1$, $R^{2'}$, Ar, X, Z und m wie oben definiert sind);
oder
(b) Umsetzung einer Verbindung der Formel (X'):

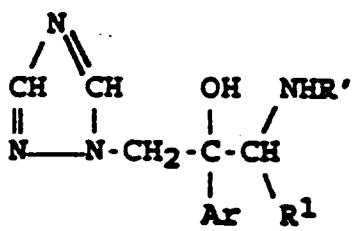

(X')

(worin: $R^1$ und Ar wie oben definiert sind und R' ein Wasserstoffatom oder $R^2$ darstellt)
mit Formaldehyd, wobei eine Verbindung der Formel (X'') gebildet wird:

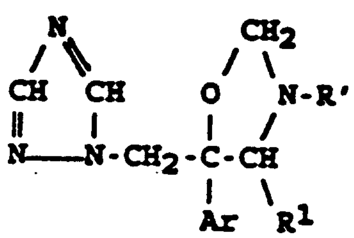

(X'')

(worin R1, Ar und R' wie oben definiert sind)
und, wenn R' ein Wasserstoffatom darstellt,
Umsetzung der Verbindung der Formel (X'') mit einer Verbindung der Formel (XVII):

$R^2$-(CH = CH)$_q$-COOH (XVII)

(worin $R^2$ wie oben definiert ist und q 0 oder 1 ist)
oder
mit einem reaktiven Derivat derselben, wobei eine Verbindung der Formel (Ig) gebildet wird:

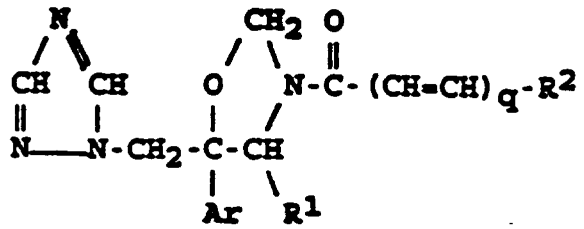

(Ig)

(worin $R^1$, $R^2$, Ar und q wie oben definiert sind);
oder
(c) Umsetzung der Verbindung der Formel (IIIA), worin Z ein Sauerstoffatom darstellt, mit einer Verbindung der Formel (V'):

$R^4$-$SO_2$-OH (V')

(worin $R^4$ wie oben definiert ist), oder mit einem reaktiven Derivat derselben, wobei eine Verbindung der Formel (IIIB) gebildet wird:

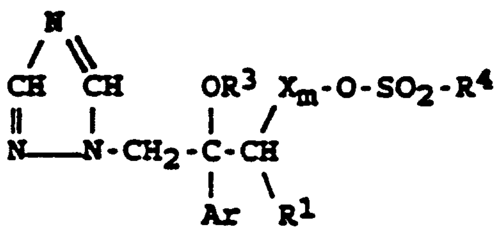

(IIIB)

(worin $R^1$, $R^3$, $R^4$, X, m und Ar wie oben definiert sind);
oder
(d) Umsetzung der Verbindung der Formel (IIIA), worin Z eine Gruppe der Formel -NH- darstellt, mit Phthalsäure, einer substituierten Phthalsäure, in der der Substituent mindestens einer der Substituenten (a) ist, oder einem reaktiven Derivat der Phthalsäure oder der substituierten Phthalsäure, wobei eine Verbindung der Formel (I) gebildet wird, worin -Y-$R^2$ die Phthalimid-Gruppe oder eine substituierte Phthalimid-Gruppe darstellt;
oder
(e) Umsetzung einer Verbindung der Formel (IIIC):

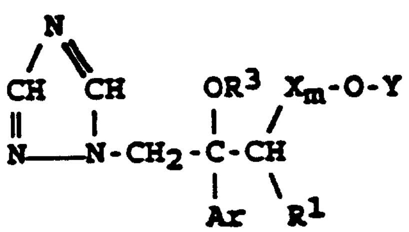

(IIIC)

(worin $R^1$, $R^3$, X, m und Ar wie oben definiert sind und Y ein Wasserstoffatom oder eine Hydroxyl-Schutzgruppe darstellt)
oder einer entsprechenden Oxiranverbindung, worin m 0 ist, Y ein Wasserstoffatom darstellt und die zwei Hydroxylgruppen unter Bildung einer Oxirangruppe kondensiert sind,
mit einem Alkalimetallazid, um eine Verbindung der Formel (IIID) zu ergeben:

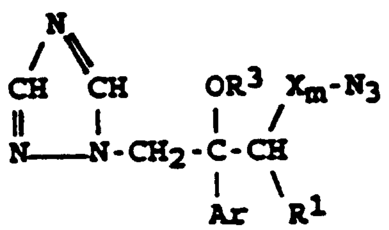

(IIID)

(worin $R^1$, $R^3$, X, m und Ar wie oben definiert sind.

**24.** Verwendung einer Verbindung der Formel (I), wie in Anspruch 17 definiert, oder eines pharmazeutisch verträglichen Säureadditonssalzes derselben, zur Herstellung eines Arzneimittels zur Behandlung oder zur Prophylaxe von Pilzinfektionen.

**25.** Verwendung gemäß Anspruch 24 einer Verbindung der Formel (I) oder deren Säureadditionssalze, wie in einem der Ansprüche 1 bis 15 definiert.

**26.** Verwendung gemäß Anspruch 24 einer der Verbindungen, auf die in Anspruch 22 Bezug genommen wird.

**27.** Verwendung einer Verbindung der Formel (I), wie in Anspruch 17 definiert, oder deren Säureadditionssalze zur Herstellung eines Fungizids zur Verwendung als Agrochemikalie.

**28.** Verwendung gemäß Anspruch 27 einer Verbindung der Formel (I) oder deren Säureadditonssalze, wie in einem der Ansprüche 1 bis 15 definiert.

**29.** Verwendung gemäß Anspruch 27 einer der Verbindungen, auf die in Anspruch 19 Bezug genommen wird.

**30.** Verfahren zum Schutz von Pflanzen, Samen sowie Teilen von Pflanzen oder Samen vor einer Pilzinfektion, welches das Auftragen mindestens einer Verbindung der Formel (I) oder deren Säureadditionssalze, wie in Anspruch 17 definiert, auf die Pflanzen, Samen, Teile von Pflanzen oder Samen, oder auf einen Ort, welcher diese einschließt, umfaßt.

**Revendications**
**Revendications pour les Etats contractants suivants : AT, BE, CH, DE, FR, GB, IT, LI, LU, NL, SE**

**1.** Composés de formule (I) :

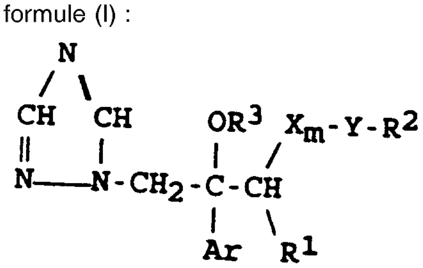

(I)

où :
Ar représente un groupe phényle ou un groupe phényle portant un ou deux substituants halogéno et/ou trifluorométhyle ;
$R^1$ représente un groupe alkyle en $C_1$-$C_6$ ou, lorsque $R^3$ et $-X_m$-Y-$R^2$ représentent ensemble ledit

groupe de formule (II), $R^1$ représente un atome d'hydrogène ou un groupe alkyle en $C_1$-$C_6$ ;

X représente un groupe alkylène en $C_1$-$C_6$, un groupe hydrocarboné aliphatique en $C_2$-$C_6$ ayant une ou deux doubles liaisons carbone-carbone, un groupe hydrocarboné aliphatique en $C_2$-$C_6$ ayant une ou deux triples liaisons carbone-carbone, un groupe cycloalkylène en $C_3$-$C_6$, un groupe cycloalkylène en $C_3$-$C_6$ ayant une ou deux doubles liaisons carbone-carbone, un groupe (alkylène en $C_1$-$C_3$)-(cycloalkylène en $C_3$-$C_6$) ou un groupe (cycloalkylène en $C_3$-$C_6$)-(alkylène en $C_1$-$C_3$) ;

m est 0 ou 1 ;

-Y-$R^2$ représente le groupe azido, le groupe phtalimido, le groupe 1-oxo-2,3-dihydro-2-isoindolyle ou le groupe phtalimido ou le groupe 1-oxo-2,3-dihydro-2-isoindolyle portant au moins l'un des substituants (a) définis ci-dessous ;

ou bien

Y représente un groupe de formule -N($R^5$)CO-, -N($R^5$)CO-CH=CH-, -O-CO-, -O-CO-CH=CH-, -S-CO- ou -S-CO-CH=CH-, où

$R^5$ représente un atome d'hydrogène ou un groupe alkyle en $C_1$-$C_4$ ;

$R^2$ représente un groupe alkyle en $C_1$-$C_6$, un groupe halogénalkyle en $C_1$-$C_6$, un groupe phényle, un groupe phényle portant au moins l'un des substituants (a) définis ci-dessous, un groupe naphtyle ou un groupe hétérocyclique ayant 5 ou 6 atomes cycliques dont 1 à 3 sont des hétéroatomes d'azote et/ou d'oxygène et/ou de soufre, ledit groupe hétérocyclique n'étant pas substitué ou portant 1 à 3 des substituants (b) définis ci-dessous ; et

$R^3$ représente un atome d'hydrogène ; ou bien

$R^3$ et -$X_m$-Y-$R^2$ représentent ensemble un groupe de formule (II) :

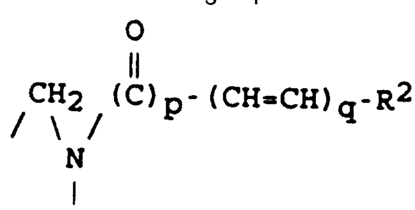

(II)

où :

$R^2$ est tel que défini ci-dessus ;

p est 0 ou 1 ; et

q est 0 ou 1 ;

substituants (a) :

groupes alkyle en $C_1$-$C_4$, groupes halogénalkyle en $C_1$-$C_4$, groupes alcoxy en $C_1$-$C_4$, groupes halogénalcoxy en $C_1$-$C_4$, atomes d'halogènes, groupes nitro, groupes cyano, groupes hydroxyle, groupes benzyloxy, groupes hydroxyméthyle, groupes de formule -$CH_2$-OCO-$R^7$, groupes de formule -CO-$R^6$, groupes de formule -COO$R^7$, groupes de formule -$SO_rR^7$, groupes amino, groupes mono- et dialkylamino dans lesquels chaque groupe alkyle est en $C_1$-$C_4$ et groupes acylamino carboxyliques ;

substituants (b) :

groupes alkyle en $C_1$-$C_4$ ; groupes halogénalkyle en $C_1$-$C_4$, atomes d'halogènes, groupes cyano et groupes nitro ;

$R^6$ représente un groupe alkyle en $C_1$-$C_6$ ou un groupe halogénalkyle en $C_1$-$C_6$ ;

$R^7$ représente un groupe alkyle en $C_1$-$C_6$ ;

r est 0, 1 ou 2 ; et

leurs sels d'addition d'acide ; à condition que si m est 0 et Y est un groupe de formule -N($R^5$)CO-, alors $R^5$ soit l'hydrogène.

**2.** Composés selon la revendication 1, dans lesquels Ar représente un groupe phényle portant un ou deux substituants halogéno.

**3.** Composés selon la revendication 1, dans lesquels Ar représente un groupe phényle portant un ou deux substituants fluoro et/ou chloro.

**4.** Composés selon la revendication 1, dans lesquels Ar représente un groupe 4-chlorophényle, un groupe 4-fluorophényle, un groupe 2,4-dichlorophényle, un groupe 2,4-difluorophényle, un groupe 2-chloro-4-fluorophényle ou un groupe 4-chloro-2-fluorophényle.

5. Composés selon l'une quelconque des revendications 1 à 4, dans lesquels $R^1$ représente un groupe alkyle en $C_1$-$C_3$.

6. Composés selon l'une quelconque des revendications 1 à 4, dans lesquels $R^1$ représente un groupe méthyle ou éthyle.

7. Composés selon l'une quelconque des revendications 1 à 6, dans lesquels X représente un groupe alkylène en $C_1$ ou $C_2$.

8. Composés selon l'une quelconque des revendications 1 à 6, dans lesquels m est 0.

9. Composés selon l'une quelconque des revendications 1 à 8, dans lesquels -Y-$R^2$ représente le groupe azido.

10. Composés selon l'une quelconque des revendications 1 à 8, dans lesquels Y représente un groupe de formule -N($R^5$)CO-, -N($R^5$)CO-CH=CH-, -S-CO- ou -S-CO-CH=CH-, où :
$R^5$ représente un atome d'hydrogène ou un groupe alkyle en $C_1$-$C_4$.

11. Composés selon l'une quelconque des revendications 1 à 8 et 10, dans lesquels $R^2$ représente un groupe phényle portant un ou deux substituants choisis parmi les atomes d'halogènes et les groupes trifluorométhyle, trifluorométhoxy et cyano.

12. Composés selon la revendication 1, dans lesquels :
Ar représente un groupe phényle portant un ou deux substituants halogéno ;
$R^1$ représente un groupe alkyle en $C_1$-$C_3$ ;
m est 0 ou bien X représente un groupe alkylène en $C_1$ ou $C_2$ ;
Y représente un groupe de formule -N($R^5$)CO-, -N($R^5$)CO-CH=CH-, -S-CO- ou -S-CO-CH=CH-, où :
$R^5$ représente un atome d'hydrogène ou un groupe alkyle en $C_1$-$C_4$ ; et
$R^2$ représente un groupe phényle portant un ou deux substituants choisis parmi les atomes d'halogènes et les groupes trifluorométhyle, trifluorométhoxy et cyano ; ou bien
-Y-$R^2$ représente un groupe azido.

13. Composés selon la revendication 1, dans lesquels :
Ar représente un groupe phényle portant un ou deux substituants fluoro et/ou chloro ;
$R^1$ représente un groupe méthyle ou éthyle ;
m est 0 ou bien X représente un groupe alkylène en $C_1$ ou $C_2$ ;
Y représente un groupe de formule -N($R^5$)CO-, -N($R^5$)CO-CH=CH-, -S-CO- ou -S-CO-CH=CH-, où :
$R^5$ représente un atome d'hydrogène ou un groupe alkyle en $C_1$-$C_4$ ; et
$R^2$ représente un groupe phényle portant un ou deux substituants choisis parmi les atomes d'halogènes et les groupes trifluorométhyle, trifluorométhoxy et cyano ; ou bien
-Y-$R^2$ représente un groupe azido.

14. Composés selon la revendication 1, dans lesquels :
$R^1$ représente un groupe méthyle ou éthyle ;
m est 0 ou bien X représente un groupe alkylène en $C_1$ ou $C_2$ ;
Y représente un groupe de formule -N($R^5$)CO-, -N($R^5$)CO-CH=CH-, -S-CO- ou -S-CO-CH=CH-, où :
$R^5$ représente un atome d'hydrogène ou un groupe alkyle en $C_1$-$C_4$ ;
$R^2$ représente un groupe phényle portant un ou deux substituants choisis parmi les atomes d'halogènes et les groupes trifluorométhyle, trifluorométhoxy et cyano ; ou bien
-Y-$R^2$ représente un groupe azido ; et
Ar représente un groupe 4-chlorophényle, un groupe 4-fluorophényle, un groupe 2,4-dichlorophényle, un groupe 2,4-difluorophényle, un groupe 2-chloro-4-fluorophényle ou un groupe 4-chloro-2-fluorophényle.

**15.** 2-(2,4-dichlorophényl)-1-(1H-1,2,4-triazole-1-yl)-3-[4-(trifluorométhyl)benzoylamino]-2-butanol et ses sels d'addition d'acide.

**16.** 2-(2,4-difluorophényl)-3-[2-fluoro-4-(trifluorométhyl)benzoylamino]-1-(1H-1,2,4-triazole-1-yl)-2-butanol et ses sels d'addition d'acide.

**17.** 3-(4-chlorobenzoylamino)-2-(2,4-difluorophényl)-1-(1H-1,2,4-triazole-1-yl)-2-pentanol et ses sels d'addition d'acide.

**18.** 3-azido-2-(2,4-difluorophényl)-1-(1H-1,2,4-triazole-1-yl)-2-butanol et ses sels d'addition d'acide.

**19.** 3-azido-2-(4-chloro-2-fluorophényl)-1-(1H-1,2,4-triazole-1-yl)-2-butanol et ses sels d'addition d'acide.

**20.** 3-azido-2-(2,4-difluorophényl)-1-(1H-1,2,4-triazole-1-yl)-2-pentanol et ses sels d'addition d'acide.

**21.** 3-azido-2-(2-chloro-4-fluorophényl)-1-(1H-1,2,4-triazole-1-yl)-2-pentanol et ses sels d'addition d'acide.

**22.** 4-azido-2-(2,4-difluorophényl)-3-méthyl-1-(1H-1,2,4-triazole-1-yl)-2-pentanol et ses sels d'addition d'acide.

**23.** 4-azido-2-(2,4-difluorophényl)-3-méthyl-1-(1H-1,2,4-triazole-1-yl)-2-butanol et ses sels d'addition d'acide.

**24.** 4-azido-2-(2,4-dichlorophényl)-3-méthyl-1-(1H-1,2,4-triazole-1-yl)-2-butanol et ses sels d'addition d'acide.

**25.** 3-azido-2-(2,4-dichlorophényl)-1-(1H-1,2,4-triazole-1-yl)-2-pentanol et ses sels d'addition d'acide.

**26.** 2-(4-chlorophényl)-1-(1H-1,2,4-triazole-1-yl)-3-[4-(trifluorométhyl)benzoylamino]-2-butanol et ses sels d'addition d'acide.

**27.** 2-(2,4-difluorophényl)-1-(1H-1,2,4-triazole-1-yl)-3-[4-(trifluorométhyl)benzoylamino]-2-butanol et ses sels d'addition d'acide.

**28.** 3-(4-cyanobenzoylamino)-2-(2,4-difluorophényl)-1-(1H-1,2,4-triazole-1-yl)-2-butanol et ses sels d'addition d'acide.

**29.** 2-(4-chlorophényl)-3-[2-fluoro-4-(trifluorométhyl)-benzoylamino]-1-(1H-1,2,4-triazole-1-yl)-2-butanol et ses sels d'addition d'acide.

**30.** 2-(2,4-dichlorophényl)-1-(1H-1,2,4-triazole-1-yl)-3-[4-(trifluorométhyl)benzoylthio]-2-butanol et ses sels d'addition d'acide.

**31.** 3-(4-chlorocinnamoylamino)-2-(2,4-difluorophényl)-1-(1H-1,2,4-triazole-1-yl)-2-butanol et ses sels d'addition d'acide.

**32.** 2-(2,4-difluorophényl)-1-(1H-1,2,4-triazole-1-yl)-3-{N-méthyl-N-[4-(trifluorométhyl)benzoyl]amino}-2-butanol et ses sels d'addition d'acide.

**33.** 2-(2,4-difluorophényl)-3-{N-[2-fluoro-4-(trifluorométhyl)benzoyl]-N-méthylamino}-1-(1H-1,2,4-triazole-1-yl)-2-butanol et ses sels d'addition d'acide.

**34.** Composés de formule (IX)

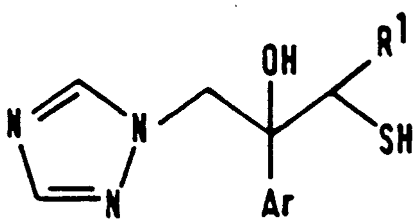

(IX)

où :

Ar représente un groupe phényle ou un groupe phényle portant un ou deux substituants halogéno et/ou trifluorométhyle ;

$R^1$ représente un groupe alkyle en $C_1$-$C_6$ ; et leurs sels d'addition d'acide.

**35.** Composition agrochimique comprenant un support ou diluant acceptable en agriculture en mélange avec au moins un fongicide, dans laquelle le fongicide est au moins un composé de formule (I) :

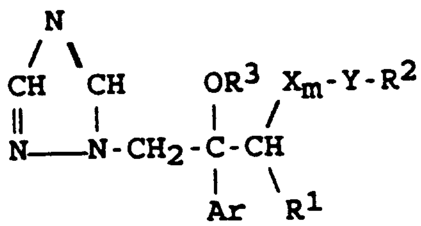

(I)

où :

Ar représente un groupe phényle ou un groupe phényle portant un ou deux atomes d'halogènes et/ou substituants trifluorométhyle ;

$R^1$ représente un groupe alkyle en $C_1$-$C_6$ ou, lorsque $R^3$ et $-X_m-Y-R^2$ représentent ensemble ledit groupe de formule (II), $R^1$ représente un atome d'hydrogène ou un groupe alkyle en $C_1$-$C_6$ ;

X représente un groupe alkylène en $C_1$-$C_6$, un groupe hydrocarboné aliphatique en $C_2$-$C_6$ ayant une ou deux doubles liaisons carbone-carbone, un groupe hydrocarboné aliphatique en $C_2$-$C_6$ ayant une ou deux triples liaisons carbone-carbone, un groupe cycloalkylène en $C_3$-$C_6$, un groupe cycloalkylène en $C_3$-$C_6$ ayant une ou deux doubles liaisons carbone-carbone, un groupe (alkylène en $C_1$-$C_3$)-(cycloalkylène en $C_3$-$C_6$) ou un groupe (cycloalkylène en $C_3$-$C_6$)-(alkylène en $C_1$-$C_3$) ;

m est 0 ou 1 ;

-Y-$R^2$ représente le groupe azido, le groupe phtalimido, le groupe 1-oxo-2,3-dihydro-2-isoindolyle, le groupe phtalimido ou le groupe 1-oxo-2,3-dihydro-2-isoindolyle portant au moins l'un des substituants (a) définis ci-dessous, un groupe hydroxyle protégé ou un groupe de formule $-OSO_2R^4$ où :

$R^4$ représente un groupe alkyle en $C_1$-$C_4$, un groupe halogénalkyle en $C_1$-$C_4$, un groupe phényle ou un groupe phényle portant un substituant choisi parmi les groupes nitro, les atomes d'halogènes et les groupes alkyle en $C_1$-$C_4$ ;

ou bien

Y représente un groupe de formule $-N(R^5)CO-$, $-N(R^5)CO-CH=CH-$, -O-CO-, -O-CO-CH=CH-,-S-CO- ou -S-CO-CH=CH-, où :

$R^5$ représente un atome d'hydrogène ou un groupe alkyle en $C_1$-$C_4$ ;

n est 0 ou 1 ;

$R^2$ représente un groupe alkyle en $C_1$-$C_6$, un groupe halogénalkyle en $C_1$-$C_6$, un groupe phényle, un groupe phényle portant au moins l'un des substituants (a) définis ci-dessous, un groupe naphtyle ou un groupe hétérocyclique ayant 5 ou 6 atomes cycliques dont 1 à 3 sont des hétéroatomes d'azote et/ou d'oxygène et/ou de soufre, ledit groupe hétérocyclique n'étant pas substitué ou portant 1 à 3 des substituants (b) définis ci-dessous ; et

$R^3$ représente un atome d'hydrogène ;

ou bien

$R^3$ et $-X_m-Y_n-R^2$ représentent ensemble un groupe de formule (II) :

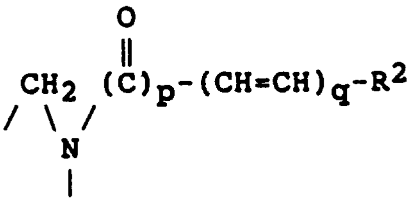

(II)

où :

$R^2$ est tel que défini ci-dessus ;

p est 0 ou 1 ; et

q est 0 ou 1 ;

substituants (a) :

groupes alkyle en $C_1$-$C_4$, groupes halogénalkyle en $C_1$-$C_4$, groupes alcoxy en $C_1$-$C_4$, groupes halogénalcoxy en $C_1$-$C_4$, atomes d'halogènes, groupes nitro, groupes cyano, groupes hydroxyle, groupes benzyloxy, groupes hydroxyméthyle, groupes de formule $-CH_2-OCO-R^7$, groupes de formule $-CO-R^6$, groupes de formule $-COOR^7$, groupes de formule $-SO_rR^7$, groupes amino, groupes mono- et dialkylamino dans lesquels chaque groupe alkyle est en $C_1$-$C_4$ et groupes acylamino carboxyliques ;

substituants (b) :

groupes alkyle en $C_1$-$C_4$ ; groupes halogénalkyle en $C_1$-$C_4$, atomes d'halogènes, groupes cyano et groupes nitro ;

$R^6$ représente un groupe alkyle en $C_1$-$C_6$ ou un groupe halogénalkyle en $C_1$-$C_6$ ;

$R^7$ représente un groupe alkyle en $C_1$-$C_6$ ;

r est 0, 1 ou 2 ;

ou un sel d'addition d'acide de ce composé.

**36.** Composition selon la revendication 35, dans laquelle ledit fongicide est un composé selon l'une quelconque des revendications 1 à 33.

**37.** Composition selon la revendication 35, dans laquelle ledit fongicide est au moins l'un des composés suivants :

2-(2,4-dichlorophényl)-1-(1H-1,2,4-triazole-1-yl)-3-[4-(trifluorométhyl)benzoylamino]-2-butanol ;

2-(2,4-difluorophényl)-3-[2-fluoro-4-(trifluorométhyl)benzoylamino]-1-(1H-1,2,4-triazole-1-yl)-2-butanol ;

3-(4-chlorobenzoylamino)-2-(2,4-difluorophényl)-1-(1H-1,2,4-triazole-1-yl)-2-pentanol ;

3-azido-2-(2,4-difluorophényl)-1-(1H-1,2,4-triazole-1-yl)-2-butanol ;

3-azido-2-(4-chloro-2-fluorophényl)-1-(1H-1,2,4-triazole-1-yl)-2-butanol ;

3-azido-2-(2,4-difluorophényl)-1-(1H-1,2,4-triazole-1-yl)-2-pentanol ;

3-azido-2-(2-chloro-4-fluorophényl)-1-(1H-1,2,4-triazole-1-yl)-2-pentanol ;

4-azido-2-(2,4-difluorophényl)-3-méthyl-1-(1H-1,2,4-triazole-1-yl)-2-pentanol ;

4-azido-2-(2,4-difluorophényl)-3-méthyl-1-(1H-1,2,4-triazole-1-yl)-2-butanol ;

4-azido-2-(2,4-dichlorophényl)-3-méthyl-1-(1H-1,2,4-triazole-1-yl)-2-butanol ; et

3-azido-2-(2,4-dichlorophényl)-1-(1H-1,2,4-triazole-1-yl)-2-pentanol ;

ou de leurs sels d'addition d'acide.

**38.** Composition pharmaceutique fongicide comprenant un support ou diluant pharmaceutiquement acceptable en mélange avec au moins un fongicide, dans laquelle le fongicide est au moins un composé de formule (I) tel que défini dans la revendication 35.

**39.** Composition selon la revendication 38, dans laquelle ledit fongicide est un composé selon l'une quelconque des revendications 1 à 33.

**40.** Composition selon la revendication 38, dans laquelle ledit fongicide est au moins l'un des composés suivants :

2-(4-chlorophényl)-1-(1H-1,2,4-triazole-1-yl)-3-[4-(trifluorométhyl)benzoylamino]-2-butanol ;

2-(2,4-difluorophényl)-1-(1H-1,2,4-triazole-1-yl)-3-[4-(trifluorométhyl)benzoylamino]-2-butanol ;

2-(2,4-difluorophényl)-3-[2-fluoro-4-(trifluorométhyl)benzoylamino]-1-(1H-1,2,4-triazole-1-yl)-2-butanol

;

3-(4-cyanobenzoylamino)-2-(2,4-difluorophényl)-1-(1H-1,2,4-triazole-1-yl)-2-butanol ;

2-(4-chlorophényl)-3-[2-fluoro-4-(trifluorométhyl)benzoylamino]-1-(1H-1,2,4-triazole-1-yl)-2-butanol ;

2-(2,4-dichlorophényl)-1-(1H-1,2,4-triazole-1-yl)-3-[4-(trifluorométhyl)benzoylthio]-2-butanol ;

3-(4-chlorocinnamoylamino)-2-(2,4-difluorophényl)-1-(1H-1,2,4-triazole-1-yl)-2-butanol ;

2-(2,4-difluorophényl)-1-(1H-1,2,4-triazole-1-yl)-3-{N-méthyl-N-[4-(trifluorométhyl)benzoyl]amino}-2-butanol ;

et

2-(2,4-difluorophényl)-3-{N-[2-fluoro-4-(trifluorométhyl)benzoyl]-N-méthylamino}-1-(1H-1,2,4-triazole-1-yl)-2-butanol ;

ou de leurs sels d'addition d'acide pharmaceutiquement acceptables.

**41.** Procédé pour préparer un composé selon l'une quelconque des revendications 1 à 33, lequel procédé consiste à :

(a) faire réagir un composé de formule (IIIA) :

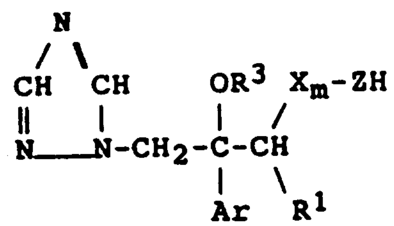

(IIIA)

[où :

$R^1$, $R^3$, X, m et Ar sont tels que définis dans la revendication 1 ; et

Z représente un atome d'oxygène ou de soufre ou un groupe de formule -NH- ou $-N(CHR^8R^9)-$ où $R^8$ et $R^9$ sont identiques ou différents et représentent chacun un atome d'hydrogène ou un groupe méthyle ou éthyle]

avec un composé de formule (V) :

$R^{2'}$-COOH (V)

(où $R^{2'}$ représente l'un quelconque des groupes définis dans la revendication 1 pour $R^2$ ou représente un groupe de formule $R^2$-CH=CH-, et $R^2$ est tel que défini dans la revendication 1) ou avec un dérivé réactif de celui-ci, pour obtenir un composé de formule (I') :

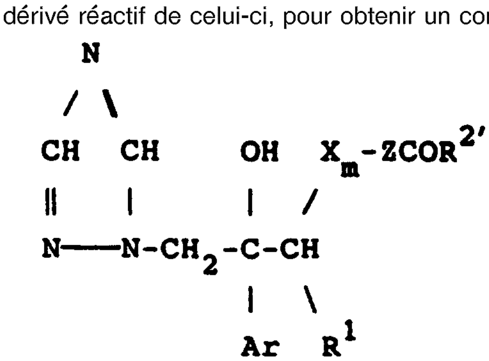

(I')

(où $R^1$, $R^{2'}$, Ar, X, Z et m sont tels que définis ci-dessus) ; ou

(b) faire réagir un composé de formule (X') :

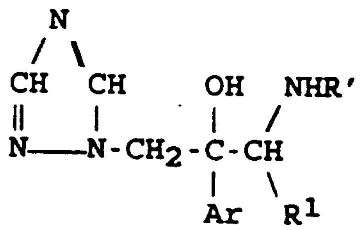

(X')

(où : $R^1$ et Ar sont tels que définis ci-dessus, et R' représente un atome d'hydrogène ou $R^2$) avec le formaldéhyde, pour obtenir un composé de formule (X") :

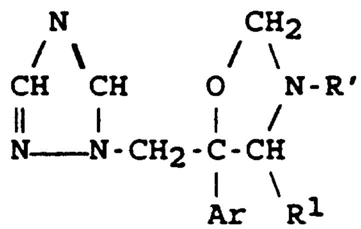

(X")

(où $R^1$, Ar et R' sont tels que définis ci-dessus)
et, lorsque R' représente un atome d'hydrogène, faire réagir ledit composé de formule (X") avec un composé de formule (XVII) :

$R^2$-(CH = CH)$_q$-COOH (XVII)

(où $R^2$ est tel que défini ci-dessus, et q est 0 ou 1) ou avec un dérivé réactif de celui-ci, pour obtenir un composé de formule (Ig) :

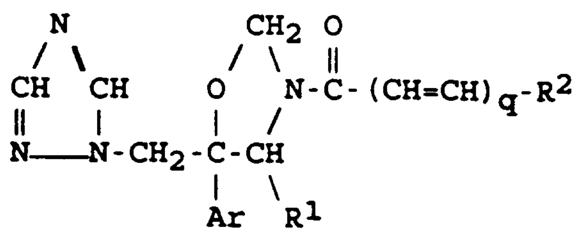

(Ig)

(où $R^1$, $R^2$, Ar et q sont tels que définis ci-dessus) ;
ou
(c) faire réagir ledit composé de formule (IIIA) dans lequel Z représente un atome d'oxygène avec un composé de formule (V') :

$R^4$-$SO_2$-OH (V')

(où $R^4$ est tel que défini ci-dessus) ou avec un dérivé réactif de celui-ci, pour obtenir un composé de formule (IIIB) :

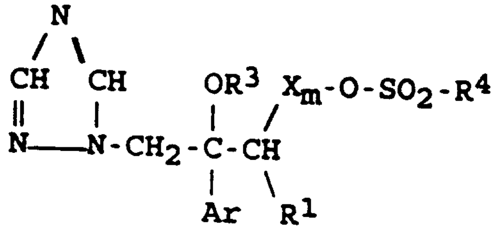

(IIIB)

(où $R^1$, $R^3$, $R^4$, X, m et Ar sont tels que définis ci-dessus) ;

ou

(d) faire réagir ledit composé de formule (IIIA) dans lequel Z représente un groupe de formule -NH- avec l'acide phtalique, un acide phtalique substitué dans lequel le substituant est au moins l'un des substituants (a), ou un dérivé réactif dudit acide phtalique ou acide phtalique substitué, pour obtenir un composé de formule (I) dans lequel -Y-$R^2$ représente le groupe phtalimido ou un groupe phtalimido substitué ;

ou

(e) faire réagir un composé de formule (IIIC) :

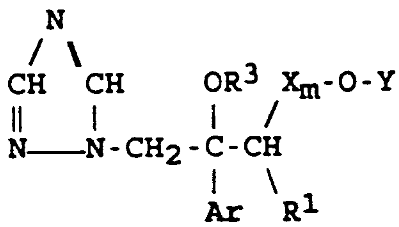

(IIIC)

(où $R^1$, $R^3$, X, m et Ar sont tels que définis ci-dessus et Y représente un atome d'hydrogène ou un groupe protecteur d'hydroxyle) ou un composé d'oxiranne correspondant dans lequel m est 0, Y représente un atome d'hydrogène et les deux groupes hydroxyle sont condensés pour former un groupe oxiranne, avec un azoture de métal alcalin pour obtenir un composé de formule (IIID) :

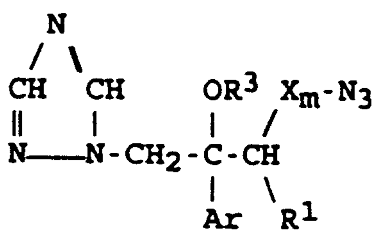

(IIID)

(où $R^1$, $R^3$, X, m et Ar sont tels que définis ci-dessus).

**42.** Utilisation pour la fabrication d'un médicament destiné au traitement ou à la prophylaxie d'infections fongiques d'un composé de formule (I), tel que défini dans la revendication 35, ou d'un sel d'addition d'acide pharmaceutiquement acceptable de ce composé.

**43.** Utilisation selon la revendication 42 d'un composé selon l'une quelconque des revendications 1 à 33.

**44.** Utilisation selon la revendication 42 de l'un quelconque des composés mentionnés dans la revendication 40.

**45.** Utilisation pour la fabrication d'un fongicide à usage agrochimique d'un composé de formule (I), tel que défini dans la revendication 35, ou d'un sel d'addition d'acide de ce composé.

**46.** Utilisation selon la revendication 45 d'un composé selon l'une quelconque des revendications 1 à 33.

**47.** Utilisation selon la revendication 45 de l'un quelconque des composés mentionnés dans la revendication 37.

**48.** Procédé pour protéger des plantes, graines et parties de plantes ou de graines contre une infection fongique, qui consiste à appliquer auxdites plantes, graines, parties de plantes ou de graines, ou à un site qui les contient, au moins un composé de formule (I) ou sel d'addition d'acide de ce composé, tel que défini dans la revendication 35.

**Revendications pour l'Etat contractant suivant : ES**

**1.** Procédé pour préparer un composé de formule (I) :

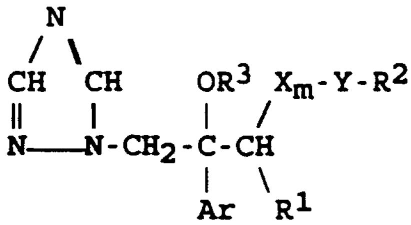

(I)

[où :

Ar représente un groupe phényle ou un groupe phényle portant un ou deux substituants halogéno et/ou trifluorométhyle ;

$R^1$ représente un groupe alkyle en $C_1$-$C_6$ ou, lorsque $R^3$ et $-X_m$-Y-$R^2$ représentent ensemble ledit groupe de formule (II), $R^1$ représente un atome d'hydrogène ou un groupe alkyle en $C_1$-$C_6$ ;

X représente un groupe alkylène en $C_1$-$C_6$, un groupe hydrocarboné aliphatique en $C_2$-$C_6$ ayant une ou deux doubles liaisons carbone-carbone, un groupe hydrocarboné aliphatique en $C_2$-$C_6$ ayant une ou deux triples liaisons carbone-carbone, un groupe cycloalkylène en $C_3$-$C_6$, un groupe cycloalkylène en $C_3$-$C_6$ ayant une ou deux doubles liaisons carbone-carbone, un groupe (alkylène en $C_1$-$C_3$)-(cycloalkylène en $C_3$-$C_6$) ou un groupe (cycloalkylène en $C_3$-$C_6$)-(alkylène en $C_1$-$C_3$) ;

m est 0 ou 1 ;

-Y-$R^2$ représente le groupe azido, le groupe phtalimido, le groupe 1-oxo-2,3-dihydro-2-isoindolyle ou le groupe phtalimido ou le groupe 1-oxo-2,3-dihydro-2-isoindolyle portant au moins l'un des substituants (a) définis ci-dessous ;

ou bien

Y représente un groupe de formule -N($R^5$)CO-, -N($R^5$)CO-CH=CH-, -O-CO-, -O-CO-CH=CH-, -S-CO- ou -S-CO-CH=CH-, où

$R^5$ représente un atome d'hydrogène ou un groupe alkyle en $C_1$-$C_4$ ;

$R^2$ représente un groupe alkyle en $C_1$-$C_6$, un groupe halogénalkyle en $C_1$-$C_6$, un groupe phényle, un groupe phényle portant au moins l'un des substituants (a) définis ci-dessous, un groupe naphtyle ou un groupe hétérocyclique ayant 5 ou 6 atomes cycliques dont 1 à 3 sont des hétéroatomes d'azote et/ou d'oxygène et/ou de soufre, ledit groupe hétérocyclique n'étant pas substitué ou portant 1 à 3 des substituants (b) définis ci-dessous ; et

$R^3$ représente un atome d'hydrogène ;

ou bien

$R^3$ et $-X_m$-Y-$R^2$ représentent ensemble un groupe de formule (II) :

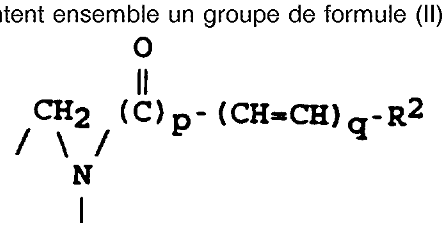

(II)

où :

$R^2$ est tel que défini ci-dessus ;

p est 0 ou 1 ; et

q est 0 ou 1 ;

substituants (a) :

groupes alkyle en $C_1$-$C_4$, groupes halogénalkyle en $C_1$-$C_4$, groupes alcoxy en $C_1$-$C_4$, groupes halogénalcoxy en $C_1$-$C_4$, atomes d'halogènes, groupes nitro, groupes cyano, groupes hydroxyle, groupes benzyloxy, groupes hydroxyméthyle, groupes de formule $-CH_2$-OCO-$R^7$, groupes de formule -CO-$R^6$, groupes de formule -COOR$^7$, groupes de formule $-SO_rR^7$, groupes amino, groupes mono- et dialkylamino dans lesquels chaque groupe alkyle est en $C_1$-$C_4$ et groupes acylamino carboxyliques ;

substituants (b) :

groupes alkyle en $C_1$-$C_4$ ; groupes halogénalkyle en $C_1$-$C_4$, atomes d'halogènes, groupes cyano et groupes nitro ;

$R^6$ représente un groupe alkyle en $C_1$-$C_6$ ou un groupe halogénalkyle en $C_1$-$C_6$ ;

$R^7$ représente un groupe alkyle en $C_1$-$C_6$ ;

r est 0, 1 ou 2] ;

ou un sel d'addition d'acide de ce composé ; à condition que si m est 0 et Y est un groupe de formule -N($R^5$)CO-, alors $R^5$ soit l'hydrogène, procédé qui consiste à :

(a) faire réagir un composé de formule (IIIA) :

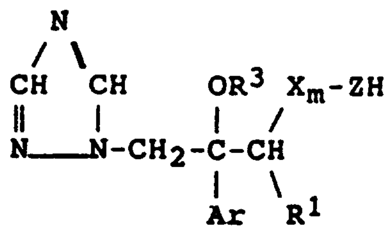

(IIIA)

[où :

$R^1$, $R^3$, X, m et Ar sont tels que définis ci-dessus ;

et

Z représente un atome d'oxygène ou de soufre ou un groupe de formule -NH- ou -N($CHR^8R^9$)- où $R^8$ et $R^9$ sont identiques ou différents et représentent chacun un atome d'hydrogène ou un groupe méthyle ou éthyle]

avec un composé de formule (V) :

$R^{2'}$-COOH (V)

(où $R^{2'}$ représente l'un quelconque des groupes définis ci-dessus pour $R^2$ ou représente un groupe de formule $R^2$-CH=CH-, et $R^2$ est tel que défini ci-dessus) ou avec un dérivé réactif de celui-ci, pour obtenir un composé de formule (I') :

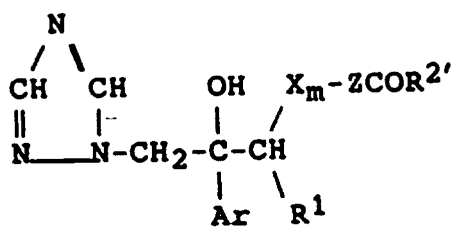

(I')

(où $R^1$, $R^{2'}$, Ar, X, Z et m sont tels que définis ci-dessus) ;

ou

(b) faire réagir un composé de formule (X') :

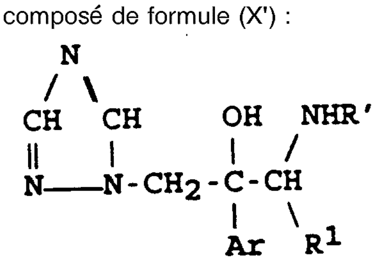

(X')

(où : $R^1$ et Ar sont tels que définis ci-dessus, et R' représente un atome d'hydrogène ou $R^2$) avec le formaldéhyde, pour obtenir un composé de formule (X'') :

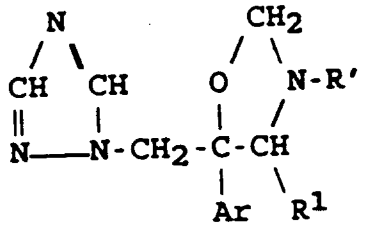

(X")

(où $R^1$, Ar et R' sont tels que définis ci-dessus)
et, lorsque R' représente un atome d'hydrogène, faire réagir ledit composé de formule (X") avec un composé de formule (XVII) :

$R^2$-(CH = CH)$_q$-COOH (XVII)

(où $R^2$ est tel que défini ci-dessus, et q est 0 ou 1) ou avec un dérivé réactif de celui-ci, pour obtenir un composé de formule (Ig) :

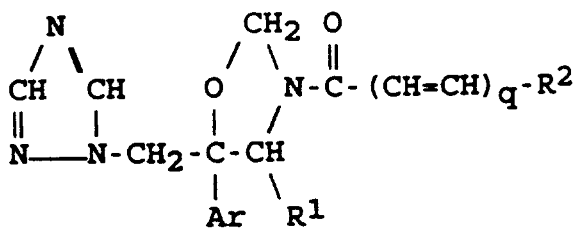

(Ig)

(où $R^1$, $R^2$, Ar et q sont tels que définis ci-dessus) ;

ou

(c) faire réagir ledit composé de formule (IIIA) dans lequel Z représente un atome d'oxygène avec un composé de formule (V') :

$R^4$-$SO_2$-OH (V')

(où $R^4$ est tel que défini ci-dessus) ou avec un dérivé réactif de celui-ci, pour obtenir un composé de formule (IIIB) :

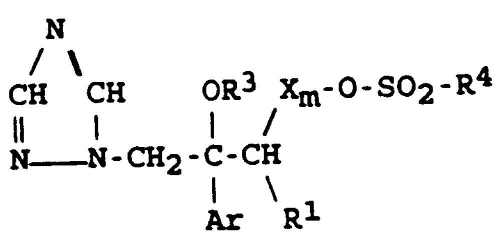

(IIIB)

(où $R^1$, $R^3$, $R^4$, X, m et Ar sont tels que définis ci-dessus) ;

ou

(d) faire réagir ledit composé de formule (IIIA) dans lequel Z représente un groupe de formule -NH- avec l'acide phtalique, un acide phtalique substitué dans lequel le substituant est au moins l'un des substituants (a), ou un dérivé réactif dudit acide phtalique ou acide phtalique substitué, pour obtenir un composé de formule (I) dans lequel -Y-$R^2$ représente le groupe phtalimido ou un groupe phtalimido substitué ;

ou

(e) faire réagir un composé de formule (IIIC) :

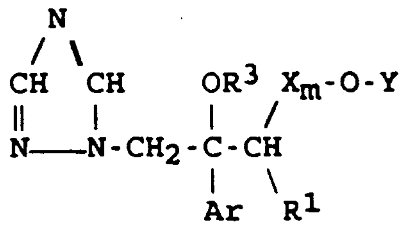

(IIIC)

(où $R^1$, $R^3$, X, m et Ar sont tels que définis ci-dessus et Y représente un atome d'hydrogène ou un groupe protecteur d'hydroxyle) ou un composé d'oxiranne correspondant dans lequel m est 0, Y représente un atome d'hydrogène et les deux groupes hydroxyle sont condensés pour former un groupe oxiranne, avec un azoture de métal alcalin pour obtenir un composé de formule (IIID) :

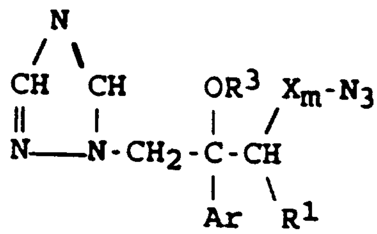

(IIID)

(où $R^1$, $R^3$, X, m et Ar sont tels que définis ci-dessus).

**2.** Procédé selon la revendication 1, dans lequel Ar, dans ledit composé de formule (IIIA) ou (X'), représente un groupe phényle portant un ou deux substituants halogéno.

**3.** Procédé selon la revendication 1, dans lequel Ar, dans ledit composé de formule (IIIA) ou (X'), représente un groupe phényle portant un ou deux substituants fluoro et/ou chloro.

**4.** Procédé selon la revendication 1, dans lequel Ar, dans ledit composé de formule (IIIA) ou (X'), représente un groupe 4-chlorophényle, un groupe 4-fluorphényle, un groupe 2,4-dichlorophényle, un groupe 2,4-difluorophényle, un groupe 2-chloro-4-fluorophényle ou un groupe 4-chloro-2-fluorophényle.

**5.** Procédé selon l'une quelconque des revendications 1 à 4, dans lequel $R^1$, dans ledit composé de formule (IIIA) ou (X'), représente un groupe alkyle en $C_1$-$C_3$.

**6.** Procédé selon l'une quelconque des revendications 1 à 4, dans lequel $R^1$, dans ledit composé de formule (IIIA) ou (X'), représente un groupe méthyle ou éthyle.

**7.** Procédé selon l'une quelconque des revendications 1 à 6, dans lequel X, dans ledit composé de formule (IIIA), représente un groupe alkylène en $C_1$ ou $C_2$.

**8.** Procédé selon l'une quelconque des revendications 1 à 6, dans lequel m, dans ledit composé de formule (IIIA), est 0.

**9.** Procédé selon l'une quelconque des revendications 1 à 8, dans lequel les réactifs et les conditions réactionnelles sont choisis de manière à préparer un composé de formule (I) dans lequel -Y-$R^2$ représente le groupe azido.

**10.** Procédé selon l'une quelconque des revendications 1 à 8, dans lequel les réactifs et les conditions réactionnelles sont choisis de manière à préparer un composé de formule (I) dans lequel Y représente un groupe de formule -N($R^5$)CO-, -N($R^5$)CO-CH = CH-, -S-CO- ou -S-CO-CH = CH-, où :

$R^5$ représente un atome d'hydrogène ou un groupe alkyle en $C_1$-$C_4$.

**11.** Procédé selon l'une quelconque des revendications 1 à 8 et 10, dans lequel les réactifs et les conditions réactionnelles sont choisis de manière à préparer un composé de formule (I) dans lequel $R^2$ représente un groupe phényle portant un ou deux substituants choisis parmi les atomes d'halogènes et les groupes trifluorométhyle, trifluorométhoxy et cyano.

**12.** Procédé selon la revendication 1, dans lequel les réactifs et les conditions réactionnelles sont choisis de manière à préparer un composé de formule (I) dans lequel :

Ar représente un groupe phényle portant un ou deux substituants halogéno ;

$R^1$ représente un groupe alkyle en $C_1$-$C_3$ ;

m est 0 ou bien X représente un groupe alkylène en $C_1$ ou $C_2$ ;

Y représente un groupe de formule -N($R^5$)CO-, -N($R^5$)CO-CH=CH-, -S-CO- ou -S-CO-CH=CH-, où :

$R^5$ représente un atome d'hydrogène ou un groupe alkyle en $C_1$-$C_4$ ; et

$R^2$ représente un groupe phényle portant un ou deux substituants choisis parmi les atomes d'halogènes et les groupes trifluorométhyle, trifluorométhoxy et cyano ;

ou bien

-Y-$R^2$ représente un groupe azido.

**13.** Procédé selon la revendication 1, dans lequel les réactifs et les conditions réactionnelles sont choisis de manière à préparer un composé de formule (I) dans lequel :

Ar représente un groupe phényle portant un ou deux substituants fluoro et/ou chloro ;

$R^1$ représente un groupe méthyle ou éthyle ;

m est 0 ou bien X représente un groupe alkylène en $C_1$ ou $C_2$ ;

Y représente un groupe de formule -N($R^5$)CO-, -N($R^5$)CO-CH=CH-, -S-CO- ou -S-CO-CH=CH-, où :

$R^5$ représente un atome d'hydrogène ou un groupe alkyle en $C_1$-$C_4$ ; et

$R^2$ représente un groupe phényle portant un ou deux substituants choisis parmi les atomes d'halogènes et les groupes trifluorométhyle, trifluorométhoxy et cyano ; ou bien

-Y-$R^2$ représente un groupe azido.

**14.** Procédé selon la revendication 1, dans lequel les réactifs et les conditions réactionnelles sont choisis de manière à préparer un composé de formule (I) dans lequel :

$R^1$ représente un groupe méthyle ou éthyle ;

m est 0 ou bien X représente un groupe alkylène en $C_1$ ou $C_2$ ;

Y représente un groupe de formule -N($R^5$)CO-, -N($R^5$)CO-CH=CH-, -S-CO- ou -S-CO-CH=CH- où :

$R^5$ représente un atome d'hydrogène ou un groupe alkyle en $C_1$-$C_4$ ;

$R^2$ représente un groupe phényle portant un ou deux substituants choisis parmi les atomes d'halogènes et les groupes trifluorométhyle, trifluorométhoxy et cyano ; ou bien

-Y-$R^2$ représente un groupe azido ; et

Ar représente un groupe 4-chlorophényle, un groupe 4-fluorophényle, un groupe 2,4-dichlorophényle, un groupe 2,4-difluorophényle, un groupe 2-chloro-4-fluorophényle ou un groupe 4-chloro-2-fluorophényle.

**15.** Procédé selon la revendication 1, dans lequel les réactifs et les conditions réactionnelles sont choisis de manière à préparer :

le 2-(2,4-dichlorophényl)-1-(1H-1,2,4-triazole-1-yl)-3-[4-(trifluorométhyl)benzoylamino]-2-butanol ;

le 2-(2,4-difluorophényl)-3-[2-fluoro-4-(trifluorométhyl)benzoylamino]-1-(1H-1,2,4-triazole-1-yl)-2-butanol ;

le 3-(4-chlorobenzoylamino)-2-(2,4-difluorophényl)-1-(1H-1,2,4-triazole-1-yl)-2-pentanol ;

le 3-azido-2-(2,4-difluorophényl)-1-(1H-1,2,4-triazole-1-yl)-2-butanol ;

le 3-azido-2-(4-chloro-2-fluorophényl)-1-(1H-1,2,4-triazole-1-yl)-2-butanol ;

le 3-azido-2-(2,4-difluorophényl)-1-(1H-1,2,4-triazole-1-yl)-2-pentanol ;

le 3-azido-2-(2-chloro-4-fluorophényl)-1-(1H-1,2,4-triazole-1-yl)-2-pentanol ;

le 4-azido-2-(2,4-difluorophényl)-3-méthyl-1-(1H-1,2,4-triazole-1-yl)-2-pentanol ;

le 4-azido-2-(2,4-difluorophényl)-3-méthyl-1-(1H-1,2,4-triazole-1-yl)-2-butanol ;

le 4-azido-2-(2,4-dichlorophényl)-3-méthyl-1-(1H-1,2,4-triazole-1-yl)-2-butanol ;

le 3-azido-2-(4-chlorophényl)-1-(1H-1,2,4-triazole-1-yl)-2-pentanol ;

le 2-(4-chlorophényl)-1-(1H-1,2,4-triazole-1-yl)-3-[4-(trifluorométhyl)benzoylamino]-2-butanol ;
le 2-(2,4-difluorophényl)-1-(1H-1,2,4-triazole-1-yl)-3-[4-(trifluorométhyl)benzoylamino]-2-butanol ;
le 3-(4-cyanobenzoylamino)-2-(2,4-difluorophényl)-1-(1H-1,2,4-triazole-1-yl)-2-butanol ;
le 2-(4-chlorophényl)-3-[2-fluoro-4-(trifluorométhyl)-benzoylamino]-1-(1H-1,2,4-triazole-1-yl)-2-butanol ;
le 2-(2,4-dichlorophényl)-1-(1H-1,2,4-triazole-1-yl)-3-[4-(trifluorométhyl)benzoylthio]-2-butanol ;
le 3-(4-chlorocinnamoylamino)-2-(2,4-difluorophényl)-1-(1H-1,2,4-triazole-1-yl)-2-butanol ;
le 2-(2,4-difluorophényl)-1-(1H-1,2,4-triazole-1-yl)-3-{N-méthyl-N-[4(trifluorométhyl)benzoyl]amino}-2-butanol ;
ou
le 2-(2,4-difluorophényl)-3-{N-[2-fluoro-4-(trifluorométhyl)benzoyl]-N-méthylamino}-1-(1H-1,2,4-triazole-1-yl)-2-butanol ;
ou un de leurs sels d'addition d'acide.

**16.** Procédé pour préparer un composé de formule (IX)

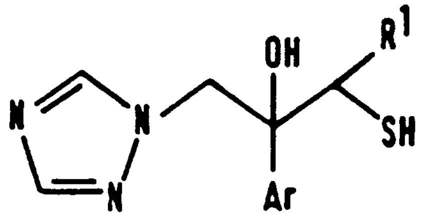

(IX)

où :

Ar représente un groupe phényle ou un groupe phényle portant un ou deux substituants halogéno et/ou trifluorométhyle ;

$R^1$ représente un groupe alkyle en $C_1$-$C_6$ ;

et ses sels d'addition d'acide, qui consiste à mettre en contact un composé de formule (VIII)

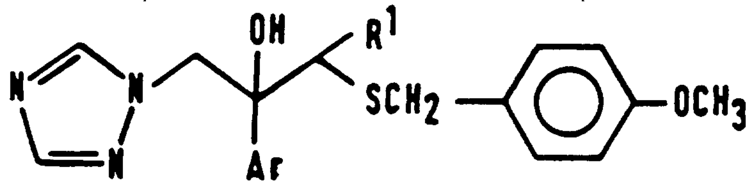

(VIII)

où Ar et $R^1$ sont tels que définis ci-dessus, avec un acide fort.

**17.** Procédé pour la fabrication d'une composition agrochimique, consistant à mélanger un support ou diluant acceptable en agriculture avec au moins un fongicide, dans lequel le fongicide est au moins un composé de formule (I) :

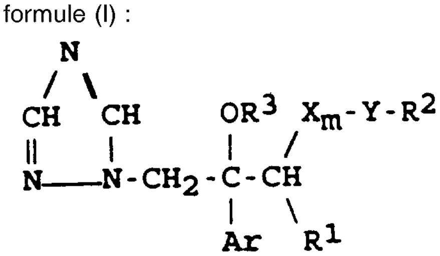

(I)

où :

Ar représente un groupe phényle ou un groupe phényle portant un ou deux atomes d'halogènes et/ou substituants trifluorométhyle ;

$R^1$ représente un groupe alkyle en $C_1$-$C_6$ ou, lorsque $R^3$ et $-X_m$-Y-$R^2$ représentent ensemble ledit

groupe de formule (II), $R^1$ représente un atome d'hydrogène ou un groupe alkyle en $C_1$-$C_6$ ;

X représente un groupe alkylène en $C_1$-$C_6$, un groupe hydrocarboné aliphatique en $C_2$-$C_6$ ayant une ou deux doubles liaisons carbone-carbone, un groupe hydrocarboné aliphatique en $C_2$-$C_6$ ayant une ou deux triples liaisons carbone-carbone, un groupe cycloalkylène en $C_3$-$C_6$, un groupe cycloalkylène en $C_3$-$C_6$ ayant une ou deux doubles liaisons carbone-carbone, un groupe (alkylène en $C_1$-$C_3$)-(cycloalkylène en $C_3$-$C_6$) ou un groupe (cycloalkylène en $C_3$-$C_6$)-(alkylène en $C_1$-$C_3$) ;

m est 0 ou 1 ;

-Y-$R^2$ représente le groupe azido, le groupe phtalimido, le groupe 1-oxo-2,3-dihydro-2-isoindolyle, le groupe phtalimido ou le groupe 1-oxo-2,3-dihydro-2-isoindolyle portant au moins l'un des substituants (a) définis ci-dessous, un groupe hydroxyle protégé ou un groupe de formule $-OSO_2R^4$ où

$R^4$ représente un groupe alkyle en $C_1$-$C_4$, un groupe halogénalkyle en $C_1$-$C_4$, un groupe phényle ou un groupe phényle portant un substituant choisi parmi les groupes nitro, les atomes d'halogènes et des groupes alkyle en $C_1$-$C_4$ ;

ou bien

Y représente un groupe de formule $-N(R^5)CO-$, $-N(R^5)CO-CH=CH-$, -O-CO-, -O-CO-CH=CH-, -S-CO- ou -S-CO-CH=CH-, où

$R^5$ représente un atome d'hydrogène ou un groupe alkyle en $C_1$-$C_4$ ;

$R^2$ représente un groupe alkyle en $C_1$-$C_6$, un groupe halogénalkyle en $C_1$-$C_6$, un groupe phényle, un groupe phényle portant au moins l'un des substituants (a) définis ci-dessous, un groupe naphtyle ou un groupe hétérocyclique ayant 5 ou 6 atomes cycliques dont 1 à 3 sont des hétéroatomes d'azote et/ou d'oxygène et/ou de soufre, ledit groupe hétérocyclique n'étant pas substitué ou portant 1 à 3 des substituants (b) définis ci-dessous ; et

$R^3$ représente un atome d'hydrogène ;

ou bien

$R^3$ et $-X_m-Y-R^2$ représentent ensemble un groupe de formule (II) :

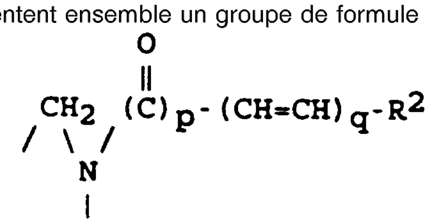

(II)

où :

$R^2$ est tel que défini ci-dessus ;

p est 0 ou 1 ; et

q est 0 ou 1 ;

substituants (a) :

groupes alkyle en $C_1$-$C_4$, groupes halogénalkyle en $C_1$-$C_4$, groupes alcoxy en $C_1$-$C_4$, groupes halogénalcoxy en $C_1$-$C_4$, atomes d'halogènes, groupes nitro, groupes cyano, groupes hydroxyle, groupes benzyloxy, groupes hydroxyméthyle, groupes de formule $-CH_2-OCO-R^7$, groupes de formule $-CO-R^6$, groupes de formule $-COOR^7$, groupes de formule $-SO_rR^7$, groupes amino, groupes mono- et dialkylamino dans lesquels chaque groupe alkyle est en $C_1$-$C_4$ et groupes acylamino carboxyliques ;

substituants (b) :

groupes alkyle en $C_1$-$C_4$ ; groupes halogénalkyle en $C_1$-$C_4$, atomes d'halogènes, groupes cyano et groupes nitro ;

$R^6$ représente un groupe alkyle en $C_1$-$C_6$ ou un groupe halogénalkyle en $C_1$-$C_6$ ;

$R^7$ représente un groupe alkyle en $C_1$-$C_6$ ;

r est 0, 1 ou 2 ;

ou un sel d'addition d'acide de ce composé.

**18.** Procédé selon la revendication 17, dans lequel ledit fongicide est un composé selon l'une quelconque des revendications 1 à 15.

**19.** Procédé selon la revendication 17, dans lequel ledit fongicide est au moins l'un des composés suivants :

2-(2,4-dichlorophényl)-1-(1H-1,2,4-triazole-1-yl)-3-[4-(trifluorométhyl)benzoylamino]-2-butanol ;

2-(2,4-difluorophényl)-3-[2-fluoro-4-(trifluorométhyl)benzoylamino]-1-(1H-1,2,4-triazole-1-yl)-2-butanol ;
3-(4-chlorobenzoylamino)-2-(2,4-difluorophényl)-1-(1H-1,2,4-triazole-1-yl)-2-pentanol ;
3-azido-2-(2,4-difluorophényl)-1-(1H-1,2,4-triazole-1-yl)-2-butanol ;
3-azido-2-(4-chloro-2-fluorophényl)-1-(1H-1,2,4-triazole-1-yl)-2-butanol ;
3-azido-2-(2,4-difluorophényl)-1-(1H-1,2,4-triazole-1-yl)-2-pentanol ;
3-azido-2-(2-chloro-4-fluorophényl)-1-(1H-1,2,4-triazole-1-yl)-2-pentanol ;
4-azido-2-(2,4-difluorophényl)-3-méthyl-1-(1H-1,2,4-triazole-1-yl)-2-pentanol ;
4-azido-2-(2,4-difluorophényl)-3-méthyl-1-(1H-1,2,4-triazole-1-yl)-2-butanol ;
4-azido-2-(2,4-dichlorophényl)-3-méthyl-1-(1H-1,2,4-triazole-1-yl)-2-butanol ; et
3-azido-2-(4-chlorophényl)-1-(1H-1,2,4-triazole-1-yl)-2-pentanol ;
ou de leurs sels d'addition d'acide.

**20.** Procédé pour la fabrication d'une composition pharmaceutique fongicide, consistant à mélanger un support ou diluant pharmaceutiquement acceptable avec au moins un fongicide, dans lequel le fongicide est au moins un composé de formule (I), tel que défini dans la revendication 17.

**21.** Procédé selon la revendication 20, dans lequel ledit fongicide est un composé selon l'une quelconque des revendications 1 à 15.

**22.** Procédé selon la revendication 21, dans lequel ledit fongicide est au moins l'un des composés suivants :

2-(4-chlorophényl)-1-(1H-1,2,4-triazole-1-yl)-3-[4-(trifluorométhyl)benzoylamino]-2-butanol ;
2-(2,4-difluorophényl)-1-(1H-1,2,4-triazole-1-yl)-3-[4-(trifluorométhyl)benzoylamino]-2-butanol ;
2-(2,4-difluorophényl)-3-[2-fluoro-4-(trifluorométhyl)benzoylamino]-1-(1H-1,2,4-triazole-1-yl)-2-butanol ;
3-(4-cyanobenzoylamino)-2-(2,4-difluorophényl)-1-(1H-1,2,4-triazole-1-yl)-2-butanol ;
2-(4-chlorophényl)-3-[2-fluoro-4-(trifluorométhyl)benzoylamino]-1-(1H-1,2,4-triazole-1-yl)-2-butanol ;
2-(2,4-dichlorophényl)-1-(1H-1,2,4-triazole-1-yl)-3-[4-(trifluorométhyl)benzoylthio]-2-butanol ;
3-(4-chlorocinnamoylamino)-2-(2,4-difluorophényl)-1-(1H-1,2,4-triazole-1-yl)-2-butanol ;
2-(2,4-difluorophényl)-1-(1H-1,2,4-triazole-1-yl)-3-{N-méthyl-N-[4-(trifluorométhyl)benzoyl]amino}-2-butanol ;
et
2-(2,4-difluorophényl)-3-{N-[2-fluoro-4-(trifluorométhyl)benzoyl]-N-méthylamino}-1-(1H-1,2,4-triazole-1-yl)-2-butanol ;
ou de leurs sels d'addition d'acide pharmaceutiquement acceptables.

**23.** Procédé pour protéger des plantes, graines et parties de plantes ou de graines contre une infection fongique, qui consiste à appliquer auxdites plantes, graines, parties de plantes ou de graines, ou à un site qui les contient, au moins un composé de formule (I) ou un sel d'addition d'acide de ce composé, tel que défini dans la revendication 17.

**24.** Utilisation pour la fabrication d'un médicament destiné au traitement ou à la prophylaxie d'infections fongiques d'un composé de formule (I) tel que défini dans la revendication 17, ou d'un sel d'addition d'acide pharmaceutiquement acceptable de ce composé.

**25.** Utilisation selon la revendication 24 d'un composé de formule (I), ou d'un sel d'addition d'acide de ce composé, tel que défini dans l'une quelconque des revendications 1 à 15.

**26.** Utilisation selon la revendication 24 de l'un quelconque des composés mentionnés dans la revendication 22.

**27.** Utilisation pour la fabrication d'un fongicide à usage agrochimique d'un composé de formule (I), tel que défini dans la revendication 17, ou d'un sel d'addition d'acide de ce composé.

**28.** Utilisation selon la revendication 27 d'un composé de formule (I) ou d'un sel d'addition d'acide de ce composé, tel que défini dans l'une quelconque des revendications 1 à 15.

**29.** Utilisation selon la revendication 27 de l'un quelconque des composés mentionnés dans la revendication 19.

**Revendications pour l'Etat contractant suivant : GR**

**1.** Composés à usage agrochimique de formule (I) :

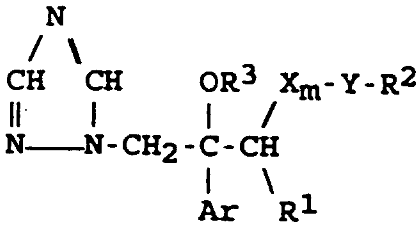

(I)

où :

Ar représente un groupe phényle ou un groupe phényle portant un ou deux substituants halogéno et/ou trifluorométhyle ;

$R^1$ représente un groupe alkyle en $C_1$-$C_6$ ou, lorsque $R^3$ et $-X_m$-Y-$R^2$ représentent ensemble ledit groupe de formule (II), $R^1$ représente un atome d'hydrogène ou un groupe alkyle en $C_1$-$C_6$ ;

X représente un groupe alkylène en $C_1$-$C_6$, un groupe hydrocarboné aliphatique en $C_2$-$C_6$ ayant une ou deux doubles liaisons carbone-carbone, un groupe hydrocarboné aliphatique en $C_2$-$C_6$ ayant une ou deux triples liaisons carbone-carbone, un groupe cycloalkylène en $C_3$-$C_6$, un groupe cycloalkylène en $C_3$-$C_6$ ayant une ou deux doubles liaisons carbone-carbone, un groupe (alkylène en $C_1$-$C_3$)-(cycloalkylène en $C_3$-$C_6$) ou un groupe (cycloalkylène en $C_3$-$C_6$)-(alkylène en $C_1$-$C_3$) ;

m est 0 ou 1 ;

-Y-$R^2$ représente le groupe azido, le groupe phtalimido, le groupe 1-oxo-2,3-dihydro-2-isoindolyle ou le groupe phtalimido ou le groupe 1-oxo-2,3-dihydro-2-isoindolyle portant au moins l'un des substituants (a) définis ci-dessous ;

ou bien

Y représente un groupe de formule -N($R^5$)CO-, -N($R^5$)CO-CH=CH-, -O-CO-, -O-CO-CH=CH-, -S-CO- ou -S-CO-CH=CH-, où

$R^5$ représente un atome d'hydrogène ou un groupe alkyle en $C_1$-$C_4$ ;

$R^2$ représente un groupe alkyle en $C_1$-$C_6$, un groupe halogénalkyle en $C_1$-$C_6$, un groupe phényle, un groupe phényle portant au moins l'un des substituants (a) définis ci-dessous, un groupe naphtyle ou un groupe hétérocyclique ayant 5 ou 6 atomes cycliques dont 1 à 3 sont des hétéroatomes d'azote et/ou d'oxygène et/ou de soufre, ledit groupe hétérocyclique n'étant pas substitué ou portant 1 à 3 des substituants (b) définis ci-dessous ; et

$R^3$ représente un atome d'hydrogène ;

ou bien

$R^3$ et $-X_m$-Y-$R^2$ représentent ensemble un groupe de formule (II) :

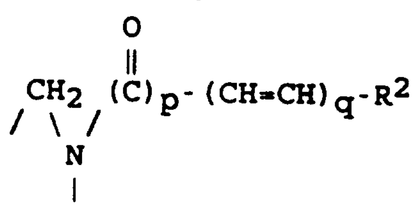

(II)

où :

$R^2$ est tel que défini ci-dessus ;

p est 0 ou 1 ; et

q est 0 ou 1 ;

substituants (a) :

groupes alkyle en $C_1$-$C_4$, groupes halogénalkyle en $C_1$-$C_4$, groupes alcoxy en $C_1$-$C_4$, groupes halogénalcoxy en $C_1$-$C_4$, atomes d'halogènes, groupes nitro, groupes cyano, groupes hydroxyle,

groupes benzyloxy, groupes hydroxyméthyle groupes de formule $-CH_2-OCO-R^7$ groupes de formule $-CO-R^6$ groupes de formule $-COOR^7$, groupes de formule $-SO_rR^7$, groupes amino, groupes mono- et dialkylamino dans lesquels chaque groupe alkyle est en $C_1-C_4$ et groupes acylamino carboxyliques ;
substituants (b) :
groupes alkyle en $C_1-C_4$ ; groupes halogénalkyle en $C_1-C_4$, atomes d'halogènes, groupes cyano et groupes nitro ;
$R^6$ représente un groupe alkyle en $C_1-C_6$ ou un groupe halogénalkyle en $C_1-C_6$ ;
$R^7$ représente un groupe alkyle en $C_1-C_6$ ;
r est 0, 1 ou 2 ; et
leurs sels d'addition d'acide ; à condition que si m est 0 et Y est un groupe de formule $-N(R^5)CO-$, alors $R^5$ soit l'hydrogène.

**2.** Composés selon la revendication 1, dans lesquels Ar représente un groupe phényle portant un ou deux substituants halogéno.

**3.** Composés selon la revendication 1, dans lesquels Ar représente un groupe phényle portant un ou deux substituants fluoro et/ou chloro.

**4.** Composés selon la revendication 1, dans lesquels Ar représente un groupe 4-chlorophényle, un groupe 4-fluorophényle, un groupe 2,4-dichlorophényle, un groupe 2,4-difluorophényle, un groupe 2-chloro-4-fluorophényle ou un groupe 4-chloro-2-fluorophényle.

**5.** Composés selon l'une quelconque des revendications 1 à 4, dans lesquels $R^1$ représente un groupe alkyle en $C_1-C_3$.

**6.** Composés selon l'une quelconque des revendications 1 à 4, dans lesquels $R^1$ représente un groupe méthyle ou éthyle.

**7.** Composés selon l'une quelconque des revendications 1 à 6, dans lesquels X représente un groupe alkylène en $C_1$ ou $C_2$.

**8.** Composés selon l'une quelconque des revendications 1 à 6, dans lesquels m est 0.

**9.** Composés selon l'une quelconque des revendications 1 à 8, dans lesquels $-Y-R^2$ représente le groupe azido.

**10.** Composés selon l'une quelconque des revendications 1 à 8, dans lesquels Y représente un groupe de formule $-N(R^5)CO-$, $-N(R^5)CO-CH=CH-$, $-S-CO-$ ou $-S-CO-CH=CH-$, où :
$R^5$ représente un atome d'hydrogène ou un groupe alkyle en $C_1-C_4$.

**11.** Composés selon l'une quelconque des revendications 1 à 8 et 10, dans lesquels $R^2$ représente un groupe phényle portant un ou deux substituants choisis parmi les atomes d'halogènes et les groupes trifluorométhyle, trifluorométhoxy et cyano.

**12.** Composés selon la revendication 1, dans lesquels :
Ar représente un groupe phényle portant un ou deux substituants halogéno ;
$R^1$ représente un groupe alkyle en $C_1-C_3$ ;
m est 0 ou bien X représente un groupe alkylène en $C_1$ ou $C_2$ ;
Y représente un groupe de formule $-N(R^5)CO-$, $-N(R^5)CO-CH=CH-$, $-S-CO-$ ou $-S-CO-CH=CH-$, où :
$R^5$ représente un atome d'hydrogène ou un groupe alkyle en $C_1-C_4$ ; et
$R^2$ représente un groupe phényle portant un ou deux substituants choisis parmi les atomes d'halogènes et les groupes trifluorométhyle, trifluorométhoxy et cyano ; ou bien
$-Y-R^2$ représente un groupe azido.

**13.** Composés selon la revendication 1, dans lesquels :
Ar représente un groupe phényle portant un ou deux substituants fluoro et/ou chloro ;
$R^1$ représente un groupe méthyle ou éthyle ;

m est 0 ou bien X représente un groupe alkylène en $C_1$ ou $C_2$ ;
Y représente un groupe de formule -N($R^5$)CO-, -N($R^5$)CO-CH=CH-, -S-CO- ou -S-CO-CH=CH-, où :
$R^5$ représente un atome d'hydrogène ou un groupe alkyle en $C_1$-$C_4$ ; et
$R^2$ représente un groupe phényle portant un ou deux substituants choisis parmi les atomes d'halogènes et les groupes trifluorométhyle, trifluorométhoxy et cyano ; ou bien
-Y-$R^2$ représente un groupe azido.

**14.** Composés selon la revendication 1, dans lesquels :
$R^1$ représente un groupe méthyle ou éthyle ;
m est 0 ou bien X représente un groupe alkylène en $C_1$ ou $C_2$ ;
Y représente un groupe de formule -N($R^5$)CO-, -N($R^5$)CO-CH=CH-, -S-CO- ou -S-CO-CH=CH-, où :
$R^5$ représente un atome d'hydrogène ou un groupe alkyle en $C_1$-$C_4$ ;
$R^2$ représente un groupe phényle portant un ou deux substituants choisis parmi les atomes d'halogènes et les groupes trifluorométhyle, trifluorométhoxy et cyano ; ou bien
-Y-$R^2$ représente un groupe azido ; et
Ar représente un groupe 4-chlorophényle, un groupe 4-fluorophényle, un groupe 2,4-dichlorophényle, un groupe 2,4-difluorophényle, un groupe 2-chloro-4-fluorophényle ou un groupe 4-chloro-2-fluorophényle.

**15.** Composés selon la revendication 1, choisis parmi :
le 2-(2,4-dichlorophényl)-1-(1H-1,2,4-triazole-1-yl)-3-[4-(trifluorométhyl)benzoylamino]-2-butanol ;
le 2-(2,4-difluorophényl)-3-[2-fluoro-4-(trifluorométhyl)benzoylamino]-1-(1H-1,2,4-triazole-1-yl)-2-butanol ;
le 3-(4-chlorobenzoylamino)-2-(2,4-difluorophényl)-(1H-1,2,4-triazole-1-yl)-2-pentanol ;
le 3-azido-2-(2,4-difluorophényl)-1-(1H-1,2,4-triazole-1-yl)-2-butanol ;
le 3-azido-2-(4-chloro-2-fluorophényl)-1-(1H-1,2,4-triazole-1-yl)-2-butanol ;
le 3-azido-2-(2,4-difluorophényl)-1-(1H-1,2,4-triazole-1-yl)-2-pentanol ;
le 3-azido-2-(2-chloro-4-fluorophényl)-1-(1H-1,2,4-triazole-1-yl)-2-pentanol ;
le 4-azido-2-(2,4-difluorophényl)-3-méthyl-1-(1H-1,2,4-triazole-1-yl)-2-pentanol ;
le 4-azido-2-(2,4-difluorophényl)-3-méthyl-1-(1H-1,2,4-triazole-1-yl)-2-butanol ;
le 4-azido-2-(2,4-dichlorophényl)-3-méthyl-1-(1H-1,2,4-triazole-1-yl)-2-butanol ;
le 3-azido-2-(4-chlorophényl)-1-(1H-1,2,4-triazole-1-yl)-2-pentanol ;
le 2-(4-chlorophényl)-1-(1H-1,2,4-triazole-1-yl)-3-[4-(trifluorométhyl)benzoylamino]-2-butanol ;
le 2-(2,4-difluorophényl)-1-(1H-1,2,4-triazole-1-yl)-3-[4-(trifluorométhyl)benzoylamino]-2-butanol ;
le 3-(4-cyanobenzoylamino)-2-(2,4-difluorophényl)-1-(1H-1,2,4-triazole-1-yl)-2-butanol ;
le 2-(4-chlorophényl)-3-[2-fluoro-4-(trifluorométhyl)-benzoylamino]-1-(1H-1,2,4-triazole-1-yl)-2-butanol ;
le 2-(2,4-dichlorophényl)-1-(1H-1,2,4-triazole-1-yl)-3-[4-(trifluorométhyl)benzoylthio]-2-butanol ;
le 3-(4-chlorocinnamoylamino)-2-(2,4-difluorophényl)-1-(1H-1,2,4-triazole-1-yl)-2-butanol ;
le 2-(2,4-difluorophényl)-1-(1H-1,2,4-triazole-1-yl)-3-{N-méthyl-N-[4-(trifluorométhyl)benzoyl]amino}-2-butanol ;
et
le 2-(2,4-difluorophényl)-3-{N-[2-fluoro-4-(trifluorométhyl)benzoyl]-N-méthylamino}-1-(1H-1,2,4-triazole-1-yl)-2-butanol ;
et leurs sels d'addition d'acide.

**16.** Composés à usage agrochimique de formule (IX) :

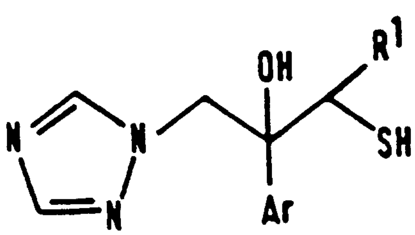

(IX)

où :

Ar représente un groupe phényle ou un groupe phényle portant un ou deux substituants halogéno et/ou trifluorométhyle ;

$R^1$ représente un groupe alkyle en $C_1$-$C_6$ ;

et leurs sels d'addition d'acide.

**17.** Composition agrochimique comprenant un support ou diluant acceptable en agriculture en mélange avec au moins un fongicide, dans laquelle le fongicide est au moins un composé de formule (I) :

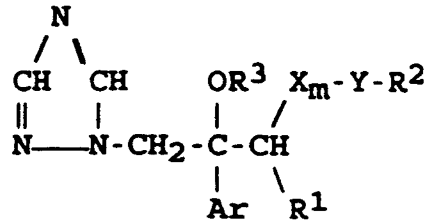

(I)

où :

Ar représente un groupe phényle ou un groupe phényle portant un ou deux atomes d'halogènes et/ou substituants trifluorométhyle ;

$R^1$ représente un groupe alkyle en $C_1$-$C_6$ ou, lorsque $R^3$ et -$X_m$-Y-$R^2$ représentent ensemble ledit groupe de formule (II), $R^1$ représente un atome d'hydrogène ou un groupe alkyle en $C_1$-$C_6$ ;

X représente un groupe alkylène en $C_1$-$C_6$, un groupe hydrocarboné aliphatique en $C_2$-$C_6$ ayant une ou deux doubles liaisons carbone-carbone, un groupe hydrocarboné aliphatique en $C_2$-$C_6$ ayant une ou deux triples liaisons carbone-carbone, un groupe cycloalkylène en $C_3$-$C_6$, un groupe cycloalkylène en $C_3$-$C_6$ ayant une ou deux doubles liaisons carbone-carbone, un groupe (alkylène en $C_1$-$C_3$)-(cycloalkylène en $C_3$-$C_6$) ou un groupe (cycloalkylène en $C_3$-$C_6$)-(alkylène en $C_1$-$C_3$) ;

m est 0 ou 1 ;

-Y-$R^2$ représente le groupe azido, le groupe phtalimido, le groupe 1-oxo-2,3-dihydro-2-isoindolyle, le groupe phtalimido ou le groupe 1-oxo-2,3-dihydro-2-isoindolyle portant au moins l'un des substituants (a) définis ci-dessous, un groupe hydroxyle protégé ou un groupe de formule -$OSO_2R^4$ où :

$R^4$ représente un groupe alkyle en $C_1$-$C_4$, un groupe halogénalkyle en $C_1$-$C_4$ un groupe phényle ou un groupe phényle portant un substituant choisi parmi les groupes nitro, les atomes d'halogènes et les groupes alkyle en $C_1$-$C_4$ ;

ou bien

Y représente un groupe de formule -N($R^5$)CO-, -N($R^5$)CO-CH=CH-, -O-CO-, -O-CO-CH=CH-,-S-CO- ou -S-CO-CH=CH-, où :

$R^5$ représente un atome d'hydrogène ou un groupe alkyle en $C_1$-$C_4$ ;

$R^2$ représente un groupe alkyle en $C_1$-$C_6$, un groupe halogénalkyle en $C_1$-$C_6$, un groupe phényle, un groupe phényle portant au moins l'un des substituants (a) définis ci-dessous, un groupe naphtyle ou un groupe hétérocyclique ayant 5 ou 6 atomes cycliques dont 1 à 3 sont des hétéroatomes d'azote et/ou d'oxygène et/ou de soufre, ledit groupe hétérocyclique n'étant pas substitué ou portant 1 à 3 des substituants (b) définis ci-dessous ; et

$R^3$ représente un atome d'hydrogène ;

ou bien

$R^3$ et -$X_m$-$Y_n$-$R^2$ représentent ensemble un groupe de formule (II) :

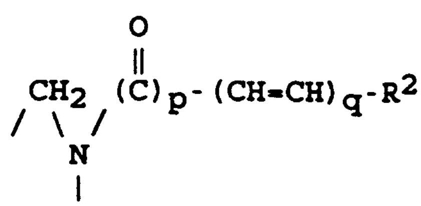

(II)

où :

$R^2$ est tel que défini ci-dessus ;

p est 0 ou 1 ; et

q est 0 ou 1 ;

substituants (a) :

groupes alkyle en $C_1$-$C_4$, groupes halogénalkyle en $C_1$-$C_4$, groupes alcoxy en $C_1$-$C_4$, groupes halogénalcoxy en $C_1$-$C_4$, atomes d'halogènes, groupes nitro, groupes cyano, groupes hydroxyle, groupes benzyloxy, groupes hydroxyméthyle, groupes de formule $-CH_2-OCO-R^7$, groupes de formule $-CO-R^6$, groupes de formule $-COOR^7$, groupes de formule $-SO_rR^7$, groupes amino, groupes mono- et dialkylamino dans lesquels chaque groupe alkyle est en $C_1$-$C_4$ et groupes acylamino carboxyliques ;

substituants (b) :

groupes alkyle en $C_1$-$C_4$ ; groupes halogénalkyle en $C_1$-$C_4$, atomes d'halogènes, groupes cyano et groupes nitro ;

$R^6$ représente un groupe alkyle en $C_1$-$C_6$ ou un groupe halogénalkyle en $C_1$-$C_6$ ;

$R^7$ représente un groupe alkyle en $C_1$-$C_6$ ;

r est 0, 1 ou 2 ;

ou un sel d'addition d'acide de ce composé.

**18.** Composition selon la revendication 17, dans laquelle ledit fongicide est un composé selon l'une quelconque des revendications 1 à 15.

**19.** Composition selon la revendication 18, dans laquelle ledit fongicide est au moins l'un des composés suivants :

2-(2,4-dichlorophényl)-1-(1H-1,2,4-triazole-1-yl)-3-[4-(trifluorométhyl)benzoylamino]-2-butanol ;

2-(2,4-difluorophényl)-3-[2-fluoro-4-(trifluorométhyl)benzoylamino]-1-(1H-1,2,4-triazole-1-yl)-2-butanol ;

3-(4-chlorobenzoylamino)-2-(2,4-difluorophényl)-1-(1H-1,2,4-triazole-1-yl)-2-pentanol ;

3-azido-2-(2,4-difluorophényl)-1-(1H-1,2,4-triazole-1-yl)-2-butanol ;

3-azido-2-(4-chloro-2-fluorophényl)-1-(1H-1,2,4-triazole-1-yl)-2-butanol ;

3-azido-2-(2,4-difluorophényl)-1-(1H-1,2,4-triazole-1-yl)-2-pentanol ;

3-azido-2-(2-chloro-4-fluorophényl)-1-(1H-1,2,4-triazole-1-yl)-2-pentanol ;

4-azido-2-(2,4-difluorophényl)-3-méthyl-1-(1H-1,2,4-triazole-1-yl)-2-pentanol ;

4-azido-2-(2,4-difluorophényl)-3-méthyl-1-(1H-1,2,4-triazole-1-yl)-2-butanol ;

4-azido-2-(2,4-dichlorophényl)-3-méthyl-1-(1H-1,2,4-triazole-1-yl)-2-butanol ; et

3-azido-2-(2,4-dichlorophényl)-1-(1H-1,2,4-triazole-1-yl)-2-pentanol ;

ou de leurs sels d'addition d'acide.

**20.** Composition pharmaceutique fongicide comprenant un support ou diluant pharmaceutiquement acceptable en mélange avec au moins un fongicide, dans laquelle le fongicide est au moins un composé de formule (I), tel que défini dans la revendication 17.

**21.** Composition selon la revendication 20, dans laquelle ledit fongicide est un composé tel que défini dans l'une quelconque des revendications 1 à 15.

**22.** Composition selon la revendication 20, dans laquelle ledit fongicide est au moins l'un des composés suivants :

2-(4-chlorophényl)-1-(1H-1,2,4-triazole-1-yl)-3-[4-(trifluorométhyl)benzoylamino]-2-butanol ;

2-(2,4-difluorophényl)-1-(1H-1,2,4-triazole-1-yl)-3-[4-(trifluorométhyl)benzoylamino]-2-butanol ;

2-(2,4-difluorophényl)-3-[2-fluoro-4-(trifluorométhyl)benzoylamino]-1-(1H-1,2,4-triazole-1-yl)-2-butanol ;

3-(4-cyanobenzoylamino)-2-(2,4-difluorophényl)-1-(1H-1,2,4-triazole-1-yl)-2-butanol ;

2-(4-chlorophényl)-3-[2-fluoro-4-(trifluorométhyl)benzoylamino]-1-(1H-1,2,4-triazole-1-yl)-2-butanol ;

2-(2,4-dichlorophényl)-1-(1H-1,2,4-triazole-1-yl)-3-[4-(trifluorométhyl)benzoylthio]-2-butanol ;

3-(4-chlorocinnamoylamino)-2-(2,4-difluorophényl)-1-(1H-1,2,4-triazole-1-yl)-2-butanol ;

2-(2,4-difluorophényl)-1-(1H-1,2,4-triazole-1-yl)-3-{N-méthyl-N-[4-(trifluorométhyl)benzoyl]amino}-2-butanol ;

et

2-(2,4-difluorophényl)-3-{N-[2-fluoro-4-(trifluorométhyl)benzoyl]-N-méthylamino}-1-(1H-1,2,4-triazole-1-yl)-2-butanol ;

ou de leurs sels d'addition d'acide pharmaceutiquement acceptables.

**23.** Procédé pour préparer un composé de formule (I), ou un sel d'addition d'acide de ce composé, tel que défini dans l'une quelconque des revendications 1 à 15, lequel procédé consiste à :

(a) faire réagir un composé de formule (IIIA) :

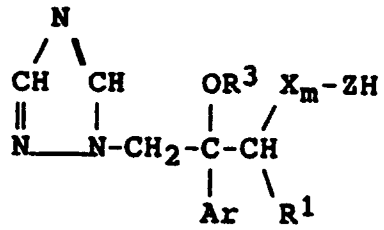

(IIIA)

[où :

$R^1$, $R^3$, X, m et Ar sont tels que définis dans la revendication 1 ; et

Z représente un atome d'oxygène ou de soufre ou un groupe de formule -NH- ou -N($CHR^8R^9$)- où $R^8$ et $R^9$ sont identiques ou différents et représentent chacun un atome d'hydrogène ou un groupe méthyle ou éthyle]

avec un composé de formule (V) :

$R^{2'}$-COOH (V)

(où $R^{2'}$ représente l'un quelconque des groupes définis dans la revendication 1 pour $R^2$ ou représente un groupe de formule $R^2$-CH=CH-, et $R^2$ est tel que défini dans la revendication 1) ou avec un dérivé réactif de celui-ci, pour obtenir un composé de formule (I') :

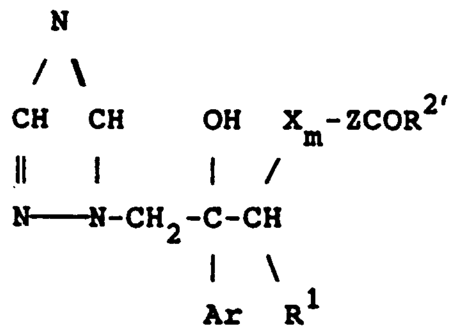

(I')

(où $R^1$, $R^{2'}$, Ar, X, Z et m sont tels que définis ci-dessus) ;

ou

(b) faire réagir un composé de formule (X') :

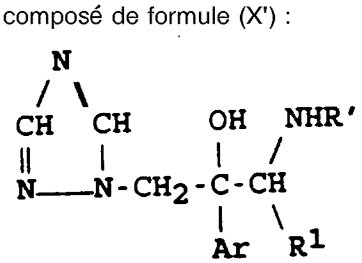

(X')

(où : $R^1$ et Ar sont tels que définis ci-dessus, et R' représente un atome d'hydrogène ou $R^2$) avec le formaldéhyde, pour obtenir un composé de formule (X''):

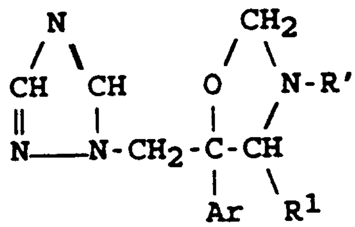

(X")

(où $R^1$, Ar et R' sont tels que définis ci-dessus)
et, lorsque R' représente un atome d'hydrogène, faire réagir ledit composé de formule (X") avec un composé de formule (XVII) :

$R^2$-(CH = CH)$_q$-COOH (XVII)

(où $R^2$ est tel que défini ci-dessus, et q est 0 ou 1) ou avec un dérivé réactif de celui-ci, pour obtenir un composé de formule (Ig) :

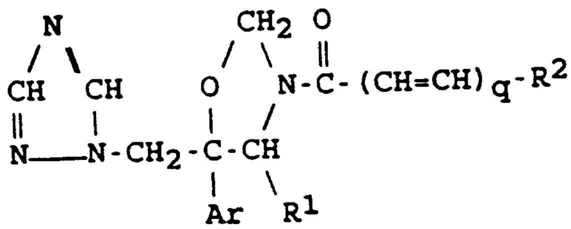

(Ig)

(où $R^1$, $R^2$, Ar et q sont tels que définis ci-dessus) ;

ou

(c) faire réagir ledit composé de formule (IIIA) dans lequel Z représente un atome d'oxygène avec un composé de formule (V') :

$R^4$-$SO_2$-OH (V')

(où $R^4$ est tel que défini ci-dessus) ou avec un dérivé réactif de celui-ci, pour obtenir un composé de formule (IIIB) :

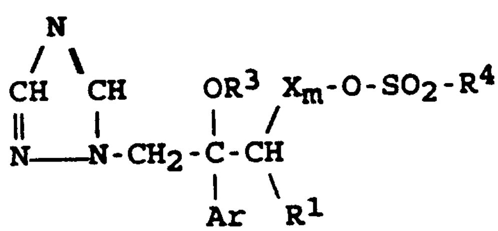

(IIIB)

(où $R^1$, $R^3$, $R^4$, X, m et Ar sont tels que définis ci-dessus) ;

ou

(d) faire réagir ledit composé de formule (IIIA) dans lequel Z représente un groupe de formule -NH- avec l'acide phtalique, un acide phtalique substitué dans lequel le substituant est au moins l'un des substituants (a), ou un dérivé réactif dudit acide phtalique ou acide phtalique substitué, pour obtenir un composé de formule (I) dans lequel -Y-$R^2$ représente le groupe phtalimido ou un groupe phtalimido substitué ;

ou

(e) faire réagir un composé de formule (IIIC) :

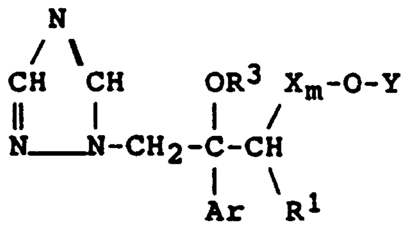

(IIIC)

(où $R^1$, $R^3$, X, m et Ar sont tels que définis ci-dessus et Y représente un atome d'hydrogène ou un groupe protecteur d'hydroxyle) ou un composé d'oxiranne correspondant dans lequel m est 0, Y représente un atome d'hydrogène et les deux groupes hydroxyle sont condensés pour former un groupe oxiranne, avec un azoture de métal alcalin pour obtenir un composé de formule (IIID) :

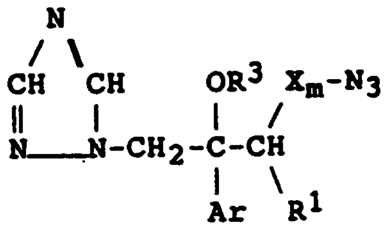

(IIID)

(où $R^1$, $R^3$, X, m et Ar sont tels que définis ci-dessus).

**24.** Utilisation pour la fabrication d'un médicament destiné au traitement ou à la prophylaxie d'infections fongiques d'un composé de formule (I), tel que défini dans la revendication 17, ou d'un sel d'addition d'acide pharmaceutiquement acceptable de ce composé.

**25.** Utilisation selon la revendication 24 d'un composé de formule (I) ou d'un sel d'addition d'acide de ce composé, tel que défini dans l'une quelconque des revendications 1 à 15.

**26.** Utilisation selon la revendication 24 de l'un quelconque des composés mentionnés dans la revendication 22.

**27.** Utilisation pour la fabrication d'un fongicide à usage agrochimique d'un composé de formule (I), tel que défini dans la revendication 17, ou d'un sel d'addition d'acide de ce composé.

**28.** Utilisation selon la revendication 27 d'un composé de formule (I), ou d'un sel d'addition d'acide de ce composé, tel que défini dans l'une quelconque des revendications 1 à 15.

**29.** Utilisation selon la revendication 27 de l'un quelconque des composés mentionnés dans la revendication 19.

**30.** Procédé pour protéger des plantes, graines et parties de plantes ou de graines contre une infection fongique, qui consiste à appliquer auxdites plantes, graines, parties de plantes ou de graines, ou à un site qui les contient, au moins un composé de formule (I) ou sel d'addition d'acide de ce composé, tel que défini dans la revendication 17.